# EUROPEAN PATENT SPECIFICATION

(11) **EP 2 966 146 B1**
(45) Date of publication and mention of the grant of the patent: **11.09.2019**
(21) Application number: 15176020.4
(22) Date of filing: 09.07.2015
(51) Int. Cl.: C09K 11/06, H01L 51/50, C07D 401/14, C07D 405/14, C07D 409/14, C07D 209/88

(54) **CONDENSED CYCLIC COMPOUND AND ORGANIC LIGHT-EMITTING DEVICE INCLUDING THE SAME**
KONDENSIERTE CYCLISCHE VERBINDUNG UND ORGANISCHE LICHTEMITTIERENDE VORRICHTUNG DAMIT
COMPOSÉ CYCLIQUE CONDENSÉ ET DISPOSITIF ÉLECTROLUMINESCENT ORGANIQUE COMPRENANT CELUI-CI

(30) Priority: 09.07.2014 KR 20140086329; 08.07.2015 KR 20150097108
(43) Date of publication of application: 13.01.2016
(73) Proprietor: Samsung Electronics Co., Ltd., Gyeonggi-do 443-742 (KR); Samsung SDI Co., Ltd., Yongin-si, Gyeonggi-do (KR)
(72) Inventor: Huh, Dalho, Gyeonggi-do 443-803 (KR); Chae, Miyoung, Gyeonggi-do 443-803 (KR); Kim, Hyunjung, Gyeonggi-do 443-803 (KR); Jeon, Soonok, Gyeonggi-do 443-803 (KR); Chung, Yeonsook, Gyeonggi-do 443-803 (KR); Jung, Yongsik, Gyeonggi-do 443-803 (KR); Kim, Wook, Gyeonggi-do 443-803 (KR); Son, Jhunmo, Gyeonggi-do 443-803 (KR); Lee, Namheon, Gyeonggi-do 443-803 (KR); Kim, Sangmo, Gyeonggi-do 443-803 (KR)
(74) Representative: Elkington and Fife LLP

(56) References cited:
- WO-A1-2015/140073
- US-A1- 2014 183 467
- MING-SHIANG LIN ET AL: "Incorporation of a CN group into mCP: a new bipolar host material for highly efficient blue and white electrophosphorescent devices", JOURNAL OF MATERIALS CHEMISTRY, vol. 22, no. 31, 13 June 2012 (2012-06-13) , page 16114, XP055224872, GB ISSN: 0959-9428, DOI: 10.1039/c2jm32717a

## Description

### FIELD OF THE INVENTION

The present disclosure relates to a condensed cyclic compound and an organic light-emitting device including the same.

### BACKGROUND OF THE INVENTION

Organic light-emitting devices are self-emission devices that have wide viewing angles, high contrast ratios, short response times, and excellent brightness, driving voltage, and response speed characteristics, and produce full-color images, compared to electronic devices of the related art.

In an example, an organic light-emitting device includes an anode, a cathode, and an organic layer that is disposed between the anode and the cathode and includes an emission layer. A hole transport region may be disposed between the anode and the emission layer, and an electron transport region may be disposed between the emission layer and the cathode. Holes provided from the anode may move toward the emission layer through the hole transport region, and electrons provided from the cathode may move toward the emission layer through the electron transport region. Carriers, such as holes and electrons, are recombined in the emission layer to produce excitons. These excitons change from an excited state to a ground state, thereby generating light.

Lin et al. (J. Mater. Chem., 2012, 22, pp. 16114-16129) provide examples of condensed cyclic compounds for use in electro phosphorescent devices.

### SUMMARY OF THE INVENTION

Provided are novel condensed cyclic compounds and organic light-emitting devices including the same.

Additional aspects will be set forth in part in the description which follows and, in part, will be apparent from the description, or may be learned by practice of the presented exemplary embodiments.

According to an aspect of an exemplary embodiment, a condensed cyclic compound is represented by one of Formulae 1B to 1D: 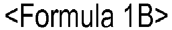 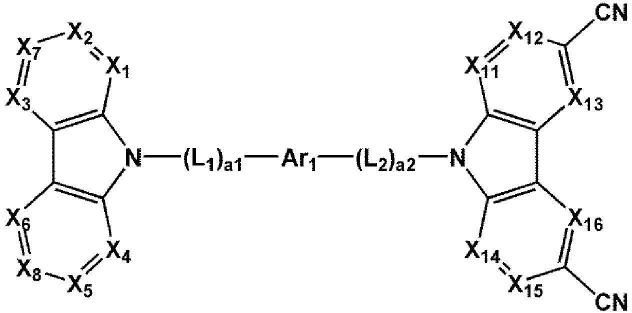 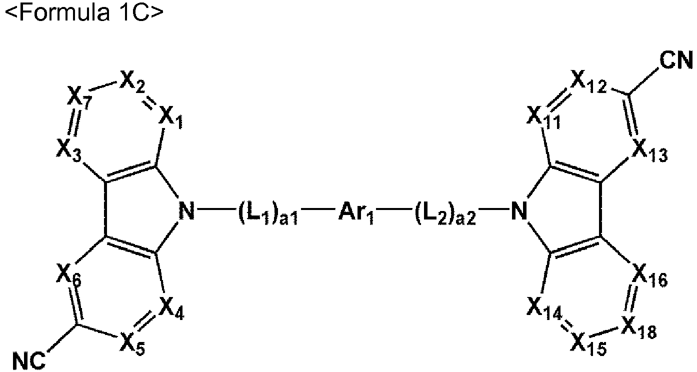 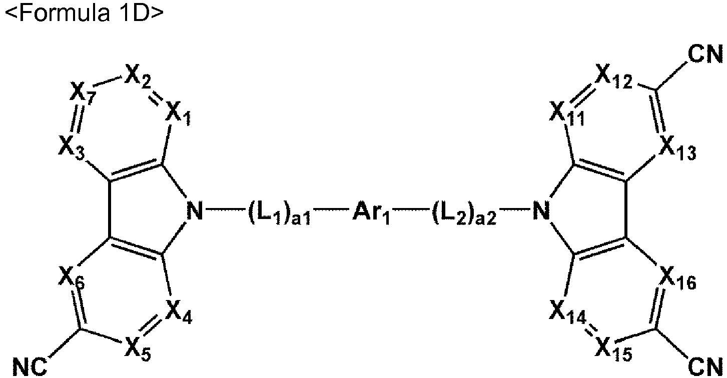 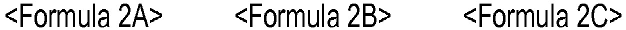 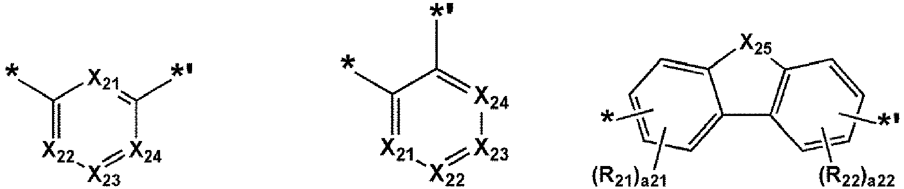 wherein in formulae 1B to 1D and 2A to 2C,
X₁ may be N or C(R₁), X₂ may be N or C(R₂), X₃ may be N or C(R₃), X₄ may be N or C(R₄), X₅ may be N or C(R₅), X₆ may be N or C(R₆), X₇ may be N or C(R₇), X₈ may be N or C(R₈), X₁₁ may be N or C(R₁₁), X₁₂ may be N or C(R₁₂), X₁₃ may be N or C(R₁₃), X₁₄ may be N or C(R₁₄), X₁₅ may be N or C(R₁₅), X₁₆ may be N or C(R₁₆), X₁₇ may be N or C(R₁₇), X₁₈ may be N or C(R₁₈),
R₁ to R₈ and R₁₁ to R₁₈ may be each independently selected from a hydrogen, a deuterium, -F, -Cl, -Br, -I, a hydroxyl group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, provided that from among the substituted or unsubstituted monovalent non-aromatic condensed heteropolycyclic groups, a substituted or unsubstituted carbazolyl group is excluded, and -Si(Q₁)(Q₂)(Q₃),
Ar₁ is represented by one of Formulae 2A to 2C,
X₂₁ may be N or C(R₂₁), X₂₂ may be N or C(R₂₂), X₂₃ may be N or C(R₂₃), X₂₄ may be N or C(R₂₄), X₂₅ may be O, S, P(=O)(R₂₅), Se, or Si(R₂₅)(R₂₆),
R₂₁ to R₂₆ may be each independently selected from a hydrogen, a deuterium, C₁-C₄ alkyl group, a phenyl group, a pyridinyl group, a pyrimidinyl group, a pyrazinyl group, a pyridazinyl group, a triazinyl group, and -Si(Q₁₁)(Q₁₂)(Q₁₃),
a21 and a22 may be each independently an integer selected from 0 to 3, a21 is 2 or more, 2 or more R₂₁(s) may be identical to or different from each other, a22 is 2 or more, 2 or more R₂₂(s) may be identical to or different from each other;
L₁ and L₂ may be each independently selected from
a phenylene group, a pyridinylene group, a pyrimidinylene group, a pyrazinylene group, a pyridazinylene group, a triazinylene group, a dibenzofuranylene group, and a dibenzothiophenylene group; and
a phenylene group, a pyridinylene group, a pyrimidinylene group, a pyrazinylene group, a pyridazinylene group, a triazinylene group, a dibenzofuranylene group, and a dibenzothiophenylene group, each substituted with at least one selected from a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group, a C₁-C₁₀ alkoxy group, a phenyl group, a naphthyl group, a pyridinyl group, a pyrimidinyl group, a pyrazinyl group, a pyridazinyl group, a triazinyl group and - Si(Q₂₁)(Q₂₂)(Q₂₃),
a1 and a2 may be each independently an integer selected from 0 to 5, and when a1 is 2 or more, 2 or more L₁(s) may be identical to or different from each other, and when a2 is 2 or more, 2 or more L₂(s) may be identical to or different from each other;
each of * and *' indicates a binding site to a neighboring atom; and
at least one of the substituted C₁-C₆₀ alkyl group, substituted C₂-C₆₀ alkenyl group, substituted C₂-C₆₀ alkynyl group, substituted C₁-C₆₀ alkoxy group, substituted C₃-C₁₀ cycloalkyl group, substituted C₁-C₁₀ heterocycloalkyl group, substituted C₃-C₁₀ cycloalkenyl group, substituted C₁-C₁₀ heterocycloalkenyl group, substituted C₆-C₆₀ aryl group, substituted C₆-C₆₀ aryloxy group, substituted C₆-C₆₀ arylthio group, substituted C₁-C₆₀ heteroaryl group, substituted monovalent non-aromatic condensed polycyclic group, and substituted monovalent non-aromatic condensed heteropolycyclic group may be selected from a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, provided that the monovalent non-aromatic condensed heteropolycyclic group is not a carbazolyl group, and -Si(Q₃₁)(Q₃₂)(Q₃₃),
wherein Q₁ to Q₃, Q₁₁ to Q₁₃, Q₂₁ to Q₂₃, and Q₃₁ to Q₃₃ may be each independently selected from a hydrogen, a C₁-C₆₀ alkyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₁-C₆₀ heteroaryl group, a monovalent non-aromatic condensed polycyclic group, and a monovalent non-aromatic condensed heteropolycyclic group, provided that the monovalent non-aromatic condensed heteropolycyclic group is not a carbazolyl group,
provided that the condensed cyclic compound is not one of the below compounds: 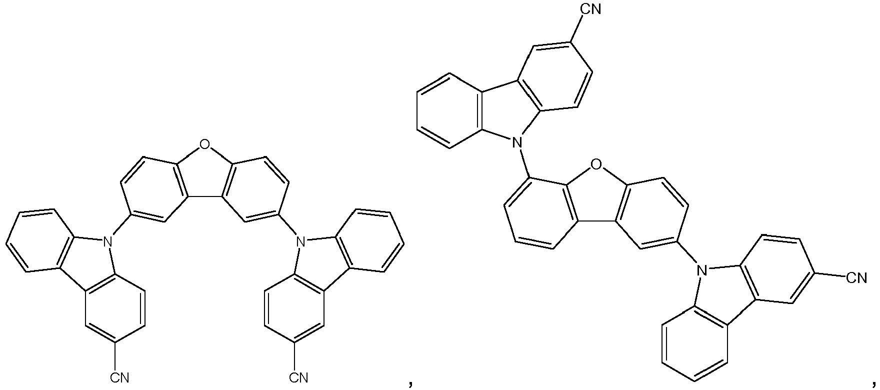 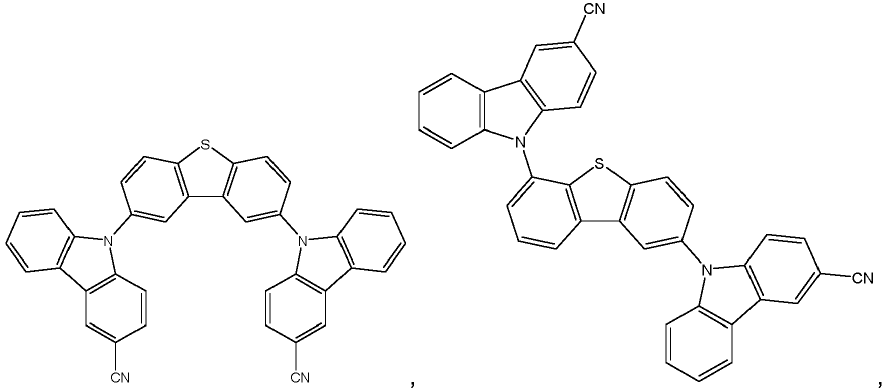 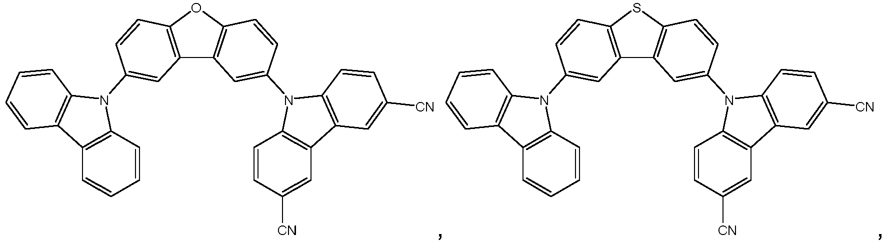 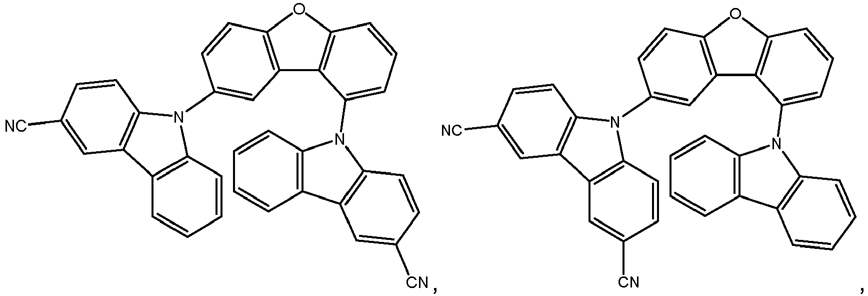 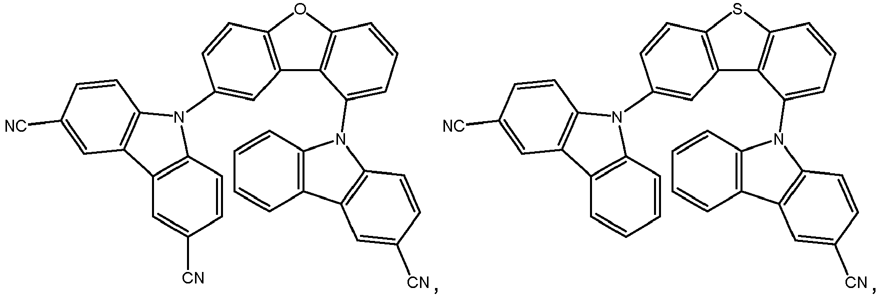 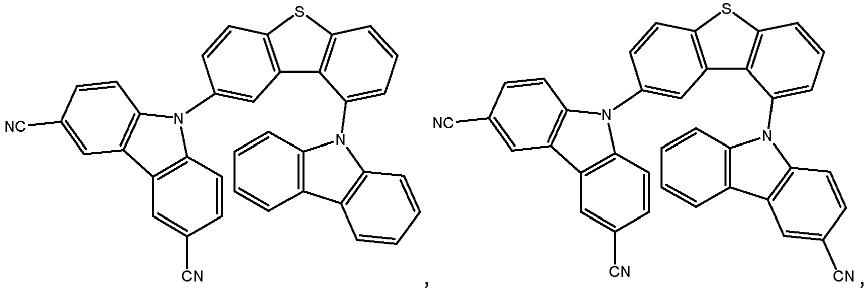 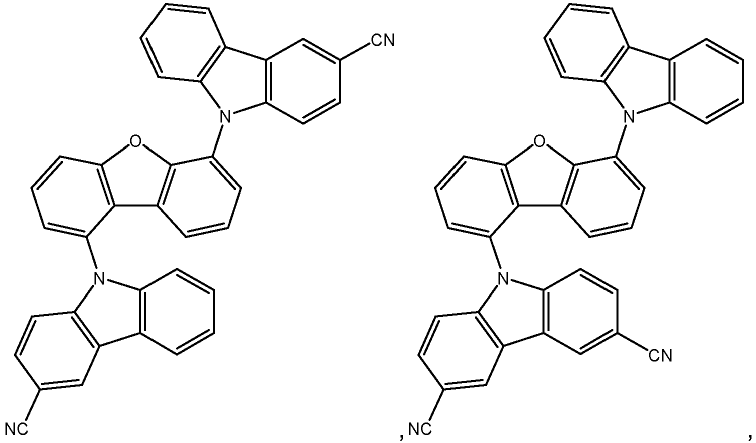 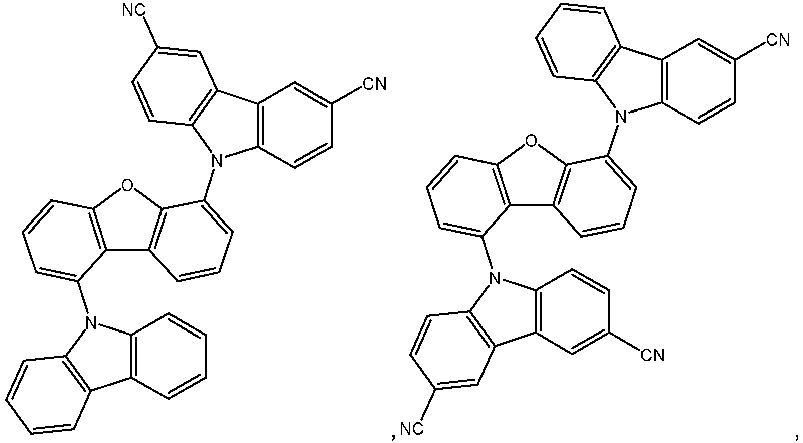 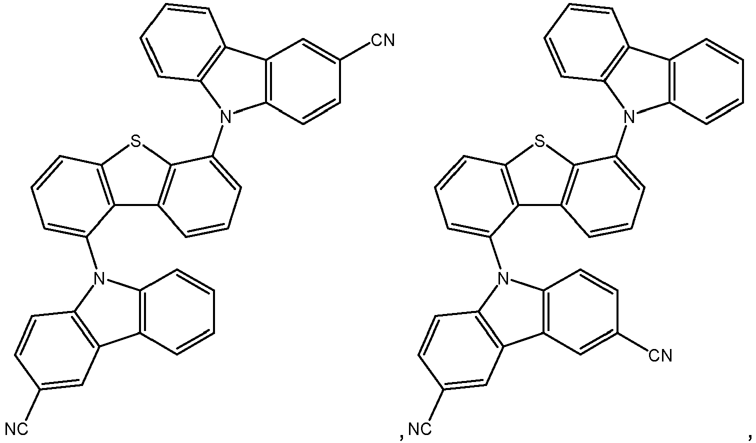 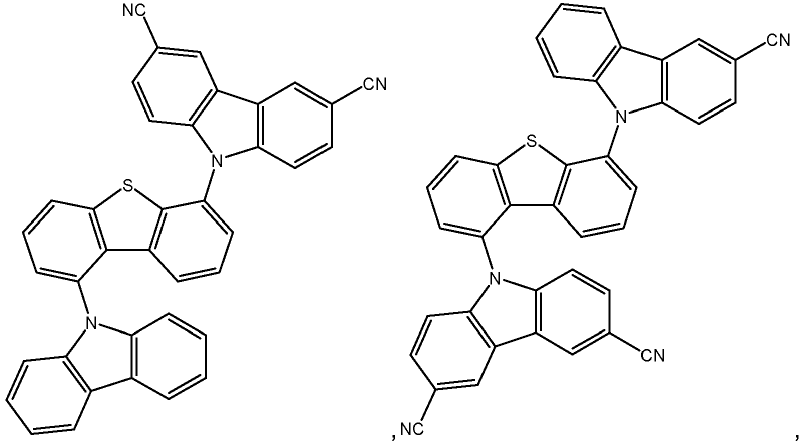 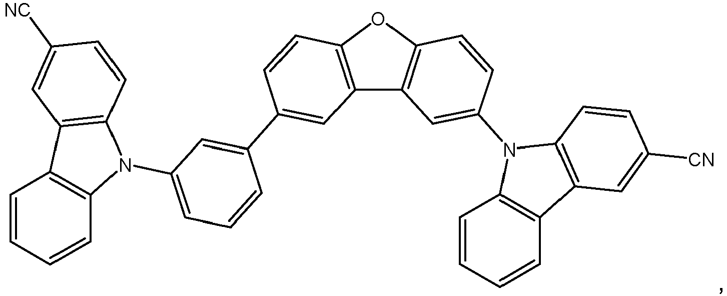 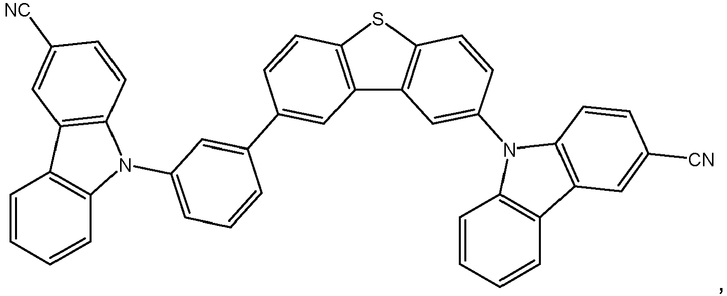 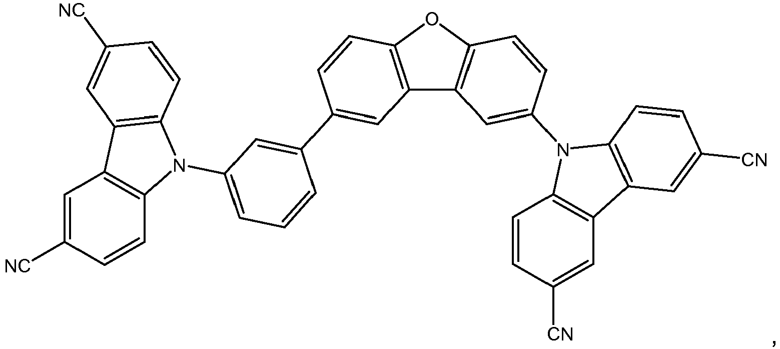 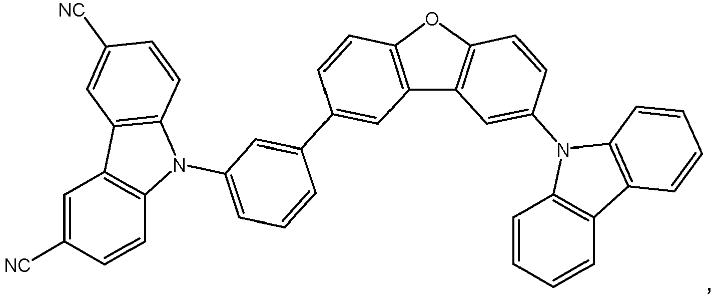 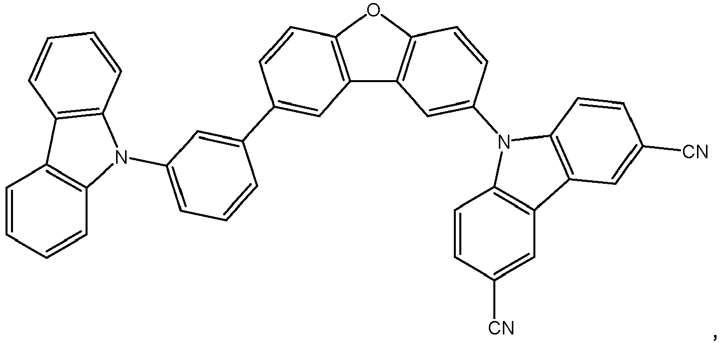 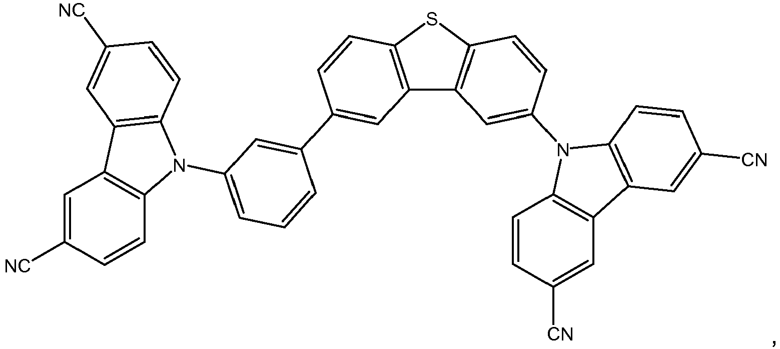 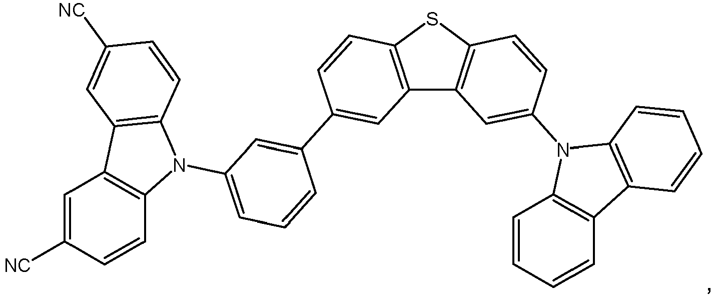 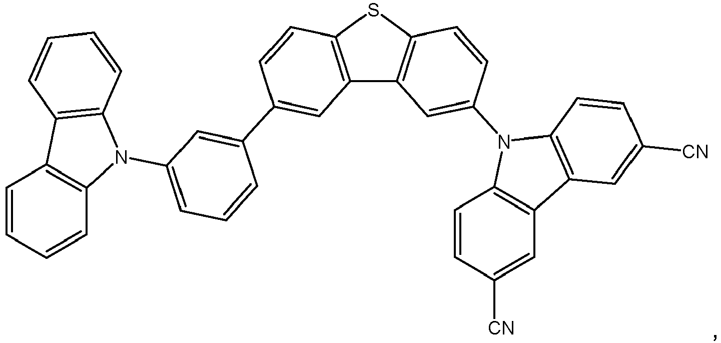 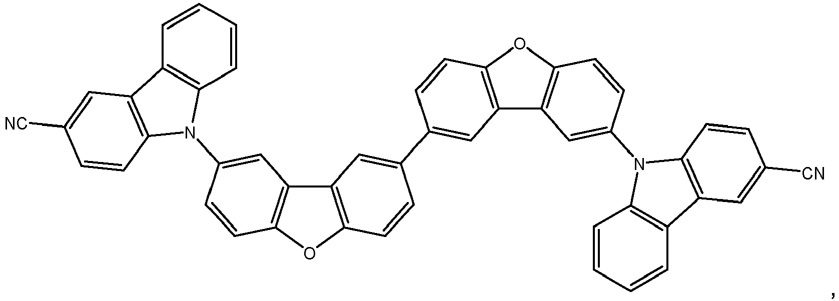 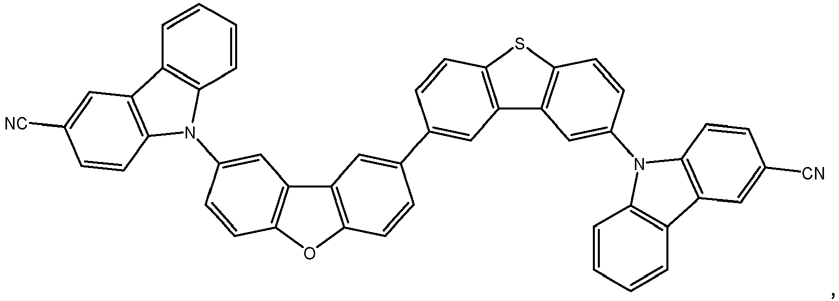 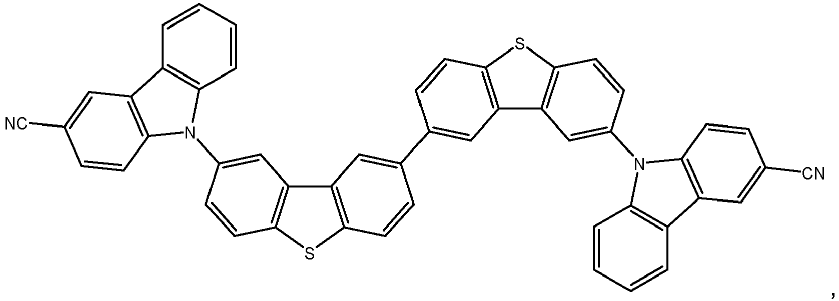 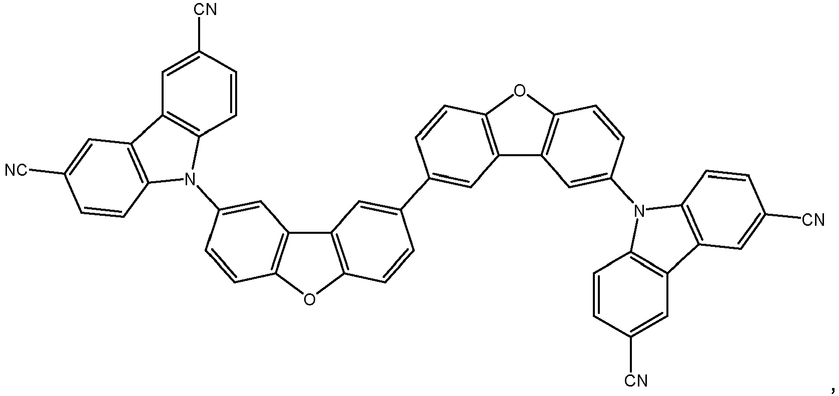 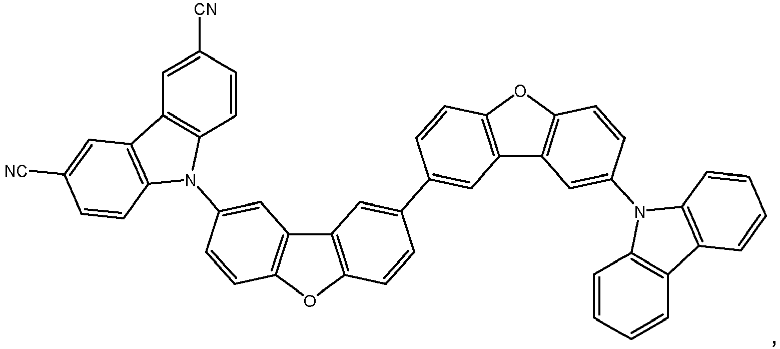 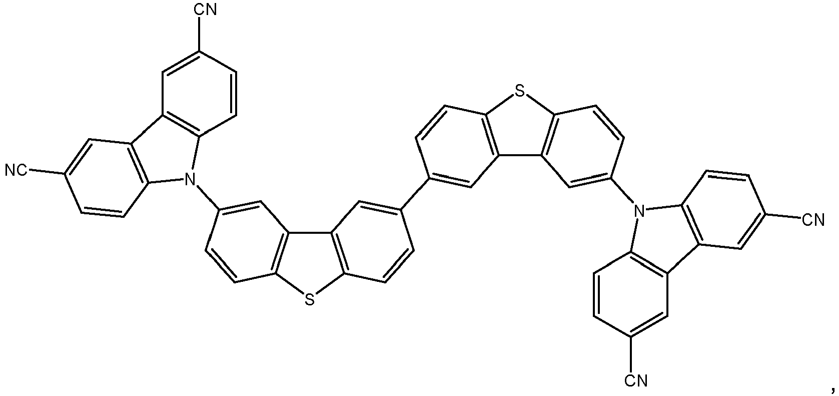 or

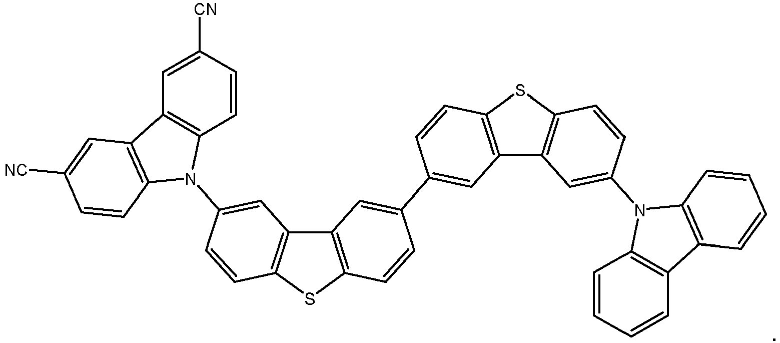

According to another aspect of an exemplary embodiment, an organic light-emitting device includes: a first electrode, a second electrode, and an organic layer between the first electrode and the second electrode, the organic layer including an emission layer and at least one of the condensed cyclic compounds represented by Formulae 1B to 1D described above.

### BRIEF DESCRIPTION OF THE DRAWINGS

These and/or other aspects will become apparent and more readily appreciated from the following description of the exemplary embodiments, taken in conjunction with the accompanying drawings in which:
FIG. 1 shows a schematic cross-sectional view of an organic light-emitting device according to an exemplary embodiment;
FIG. 2 shows heat-resistance evaluation results of a mCP-thin film, a Compound A-thin film, and a Compound 4-thin film;
FIG. 3 shows a luminance-efficiency graph of organic light-emitting devices manufactured according to Examples 1 to 3 and Comparative Examples 1 and 2;
FIG. 4 shows a voltage-current density graph of organic light-emitting devices manufactured according to Examples 1 to 3 and Comparative Examples 1 and 2; and
FIG. 5 shows a time-luminance graph of organic light-emitting devices manufactured according to Examples 1 to 3 and Comparative Examples 1 and 2.

### DETAILED DESCRIPTION OF THE EMBODIMENTS

Reference will now be made in detail to exemplary embodiments, examples of which are illustrated in the accompanying drawings, wherein like reference numerals refer to like elements throughout. In this regard, the present exemplary embodiments may have different forms and should not be construed as being limited to the descriptions set forth herein. Accordingly, the exemplary embodiments are merely described below, by referring to the figures, to explain aspects. As used herein, the term "and/or" includes any and all combinations of one or more of the associated listed items. Expressions such as "at least one of," when preceding a list of elements, modify the entire list of elements and do not modify the individual elements of the list.

A condensed cyclic compound according to an embodiment is represented by one of Formulae 1B to 1D:

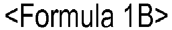

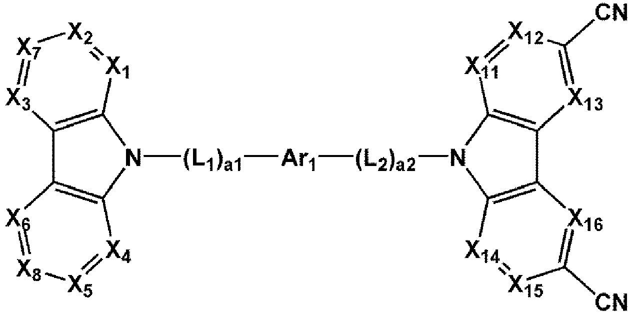

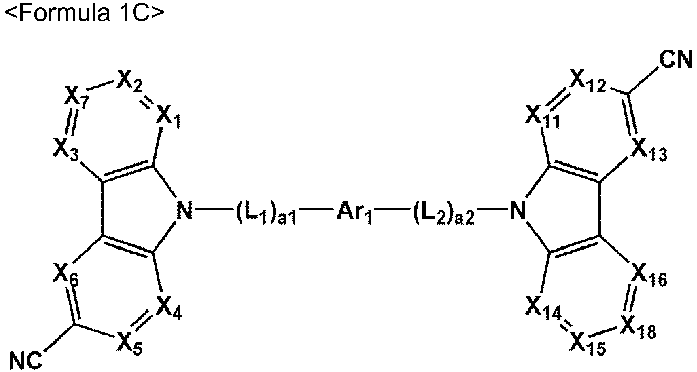

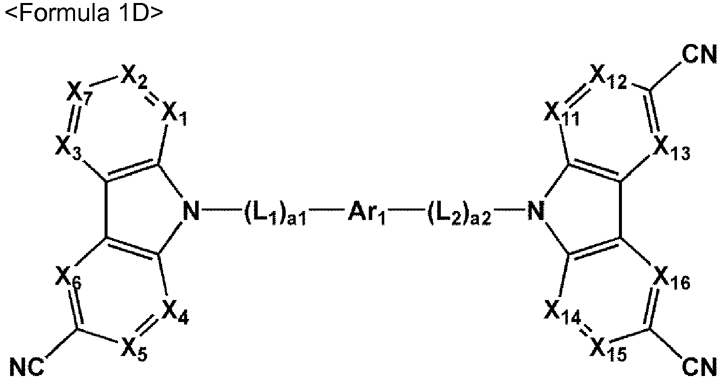

X₁ in Formulae 1B to 1D may be N or C(R₁), X₂ may be N or C(R₂), X₃ may be N or C(R₃), X₄ may be N or C(R₄), X₅ may be N or C(R₅), X₆ may be N or C(R₆), X₇ may be N or C(R₇), X₈ may be N or C(R₈), X₁₁ may be N or C(R₁₁), X₁₂ may be N or C(R₁₂), X₁₃ may be N or C(R₁₃), X₁₄ may be N or C(R₁₄), X₁₅ may be N or C(R₁₅), X₁₆ may be N or C(R₁₆), X₁₇ may be N or C(R₁₇), and X₁₈ may be N or C(R₁₈).

In some embodiments, each of X₁ to X₈ and X₁₁ to X₁₈ in Formulae 1B to 1D may not be N.

In some embodiments, one selected from X₁ to X₈ and X₁₁ to X₁₈ in Formulae 1B to 1D may be N, and the others may not be N.

In some embodiments, X₁ may be N, and each of X₂ to X₈ and X₁₁ to X₁₈ may not be N, but they are not limited thereto.

In some embodiments, X₇ may be N, and each of X₁ to X₆, X₈ and X₁₁ to X₁₈ may not be N, but they are not limited thereto.

R₁ to R₈ and R₁₁ to R₁₈ may be each independently selected from a hydrogen, a deuterium, -F, -Cl, -Br, -I, a hydroxyl group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group,provided that from among the substituted or unsubstituted monovalent non-aromatic condensed heteropolycyclic groups, a substituted or unsubstituted carbazolyl group is excluded, and -Si(Q₁)(Q₂)(Q₃).

For example, R₁ to R₈ and R₁₁ to R₁₈ may be each independently selected from
a hydrogen, a deuterium, -F, -CI, -Br, -I, a hydroxyl group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, and a C₁-C₂₀ alkoxy group;
a C₁-C₂₀ alkyl group and a C₁-C₂₀ alkoxy group, each substituted with at least one selected from a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a phenyl group, a naphthyl group, a pyridinyl group, a pyrimidinyl group, a pyrazinyl group, a pyridazinyl group, and a triazinyl group;
a cyclopentyl group, a cyclohexyl group, a cyclopentenyl group, a cyclohexenyl group, a cycloheptenyl group, a phenyl group, a pentalenyl group, an indenyl group, a naphthyl group, an azulenyl group, a heptalenyl group, an indacenyl group, an acenaphthyl group, a fluorenyl group, a spiro-fluorenyl group, a phenalenyl group, a phenanthrenyl group, an anthracenyl group, a fluoranthrenyl group, a triphenylenyl group, a pyrenyl group, a chrysenyl group, a naphthacenyl group, a picenyl group, a perylenyl group, a pentaphenyl group, a hexacenyl group, a pyrrolyl group, an imidazolyl group, a pyrazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzoxazolyl group, a benzoimidazolyl group, a furanyl group, a benzofuranyl group, a thiophenyl group, a benzothiophenyl group, a thiazolyl group, an isothiazolyl group, a benzothiazolyl group, an isoxazolyl group, an oxazolyl group, a triazolyl group, a tetrazolyl group, an oxadiazolyl group, a triazinyl group, a dibenzofuranyl group, a dibenzothiophenyl group, an imidazopyridimidinyl group and an imidazopyridinyl group;
a cyclopentyl group, a cyclohexyl group, a cyclopentenyl group, a cyclohexenyl group, a cycloheptenyl group, a phenyl group, a pentalenyl group, an indenyl group, a naphthyl group, an azulenyl group, a heptalenyl group, an indacenyl group, an acenaphthyl group, a fluorenyl group, a spiro-fluorenyl group, a phenalenyl group, a phenanthrenyl group, an anthracenyl group, a fluoranthrenyl group, a triphenylenyl group, a pyrenyl group, a chrysenyl group, a naphthacenyl group, a picenyl group, a perylenyl group, a pentaphenyl group, a hexacenyl group, a pyrrolyl group, an imidazolyl group, a pyrazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzoxazolyl group, a benzoimidazolyl group, a furanyl group, a benzofuranyl group, a thiophenyl group, a benzothiophenyl group, a thiazolyl group, an isothiazolyl group, a benzothiazolyl group, an isoxazolyl group, an oxazolyl group, a triazolyl group, a tetrazolyl group, an oxadiazolyl group, a triazinyl group, a dibenzofuranyl group, a dibenzothiophenyl group, an imidazopyridimidinyl group, and an imidazopyridinyl group, each substituted with at least one selected from a deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₂-C₂₀ alkenyl group, a C₂-C₂₀ alkynyl group, a C₁-C₂₀ alkoxy group, a phenyl group, a naphthyl group, an anthracenyl group, a pyrenyl group, a phenanthrenyl group, a fluorenyl group, a pyridinyl group, a pyrimidinyl group, a pyrazinyl group, a pyridazinyl group, a triazinyl group, a quinolinyl group, an isoquinolinyl group, a phthalazinyl group, a quinoxalinyl group, a cinnolinyl group, a quinazolinyl group, and -Si(Q₃₁)(Q₃₂)(Q₃₃); and
-Si(Q₁)(Q₂)(Q₃),
wherein Q₁ to Q₃ and Q₃₁ to Q₃₃ may be each independently selected from a hydrogen, a C₁-C₂₀ alkyl group, a C₁-C₂₀ alkoxy group, a phenyl group, a naphthyl group, an anthracenyl group, a pyrenyl group, a phenanthrenyl group, a fluorenyl group, a pyridinyl group, a pyrimidinyl group, a pyrazinyl group, a pyridazinyl group, a triazinyl group, a quinolinyl group, an isoquinolinyl group a phthalazinyl group, a quinoxalinyl group, a cinnolinyl group, and a quinazolinyl group.

In some embodiments, R₁ to R₆ and R₁₁ to R₁₆ may be each independently selected from
a hydrogen, a deuterium, -F, -Cl, -Br, -I, a hydroxyl group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group, and a C₁-C₁₀ alkoxy group;
a phenyl group, a naphthyl group, a pyridinyl group, a pyrimidinyl group, a pyrazinyl group, a pyridazinyl group, and a triazinyl group;
a phenyl group, a naphthyl group, a pyridinyl group, a pyrimidinyl group, a pyrazinyl group, a pyridazinyl group, and a triazinyl group, each substituted with at least one selected from a deuterium, -F, -Cl, -Br, -I, a hydroxyl group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group, a C₁-C₁₀ alkoxy group, a phenyl group, a naphthyl group, a pyridinyl group, a pyrimidinyl group, a pyrazinyl group, a pyridazinyl group, a triazinyl group, and - Si(Q₃₁)(Q₃₂)(Q₃₃); and
-Si(Q₁)(Q₂)(Q₃),
wherein R₇, R₈, R₁₇, and R₁₈ may be each independently selected from
a hydrogen, a deuterium, -F, -CI, -Br, -I, a hydroxyl group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group, and a C₁-C₁₀ alkoxy group;
a phenyl group, a naphthyl group, a pyridinyl group, a pyrimidinyl group, a pyrazinyl group, a pyridazinyl group, and a triazinyl group;
a phenyl group, a naphthyl group, a pyridinyl group, a pyrimidinyl group, a pyrazinyl group, a pyridazinyl group, and a triazinyl group, each substituted with at least one selected from a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group, a C₁-C₁₀ alkoxy group, a phenyl group, a naphthyl group, a pyridinyl group, a pyrimidinyl group, a pyrazinyl group, a pyridazinyl group, a triazinyl group, and -Si(Q₃₁)(Q₃₂)(Q₃₃); and
-Si(Q₁)(Q₂)(Q₃),
wherein Q₁ to Q₃ and Q₃₁ to Q₃₃ may be each independently selected from a hydrogen, a C₁-C₁₀ alkyl group, a C₁-C₁₀ alkoxy group, a phenyl group, a naphthyl group, a pyridinyl group, a pyrimidinyl group, a pyrazinyl group, a pyridazinyl group, and a triazinyl group, but are not limited thereto.

In some embodiments, R₁ to R₈ and R₁₁ to R₁₈ may be each independently selected from
a hydrogen, a deuterium, -F, -Cl, -Br, -I, a hydroxyl group, a nitro group, an amino group, an amidino group, a hydrazine group, a hydrazone group, a carboxylic acid group or a salt thereof, a sulfonic acid group or a salt thereof, a phosphoric acid group or a salt thereof, a methyl group, an ethyl group, an n-propyl group, an isopropyl group, an n-butyl group, an isobutyl group, a sec-butyl group, a tert-butyl group, an n-pentyl group, an isopentyl group, a sec-pentyl group, a tert-pentyl group, an n-hexyl group, an iso-hexyl group, a sec-hexyl group, a tert-hexyl group, an n-heptyl group, an iso-heptyl group, a sec-heptyl group, a tert-heptyl group, an n-octyl group, an iso-octyl group, a sec-octyl group, a tert-octyl group, an n-nonyl group, an iso-nonyl group, a sec-nonyl group, a tert-nonyl group, an n-decyl group, an isodecyl group, a sec-decyl group, a tert-decyl group, a methoxy group, an ethoxy group, a propoxy group, a butoxy group, a pentoxy group, a phenyl group, a naphthyl group, a pyridinyl group, a pyrimidinyl group, and a triazinyl group;
a methyl group, an ethyl group, an n-propyl group, an isopropyl group, an n-butyl group, an isobutyl group, a sec-butyl group, a tert-butyl group, an n-pentyl group, an isopentyl group, a sec-pentyl group, a tert-pentyl group, an n-hexyl group, an iso-hexyl group, a sec-hexyl group, a tert-hexyl group, an n-heptyl group, an iso-heptyl group, a sec-heptyl group, a tert-heptyl group, an n-octyl group, an iso-octyl group, a sec-octyl group, a tert-octyl group, an n-nonyl group, an iso-nonyl group, a sec-nonyl group, a tert-nonyl group, an n-decyl group, an isodecyl group, a sec-decyl group, a tert-decyl group, a methoxy group, an ethoxy group, a propoxy group, a butoxy group, a pentoxy group, a phenyl group, a naphthyl group, a pyridinyl group, a pyrimidinyl group, and a triazinyl group, each substituted with at least one selected from a deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof and a phosphoric acid group or a salt thereof; and
-Si(Q₁)(Q₂)(Q₃),
wherein Q₁ to Q₃ may be each independently selected from a hydrogen, a C₁-C₁₀ alkyl group, a C₁-C₁₀ alkoxy group and a phenyl group.

In some embodiments, R₁ to R₈ and R₁₁ to R₁₈ may be each independently selected from a hydrogen, a deuterium, a methyl group, an ethyl group, an n-propyl group, an isopropyl group, an n-butyl group, an isobutyl group, a sec-butyl group, a tert-butyl group, an n-pentyl group, an isopentyl group, a sec-pentyl group, a tert-pentyl group, an n-hexyl group, an iso-hexyl group, a sec-hexyl group, a tert-hexyl group, and -Si(Q₁)(Q₂)(Q₃),
wherein Q₁ to Q₃ may be each independently a hydrogen, a C₁-C₁₀ alkyl group, a C₁-C₁₀ alkoxy group, and a phenyl group.

In some embodiments, X₁ may be C(R₁), X₂ may be C(R₂), X₃ may be C(R₃), X₄ may be C(R₄), X₅ may be C(R₅), X₆ may be C(R₆), X₇ may be C(R₇), X₈ may be C(R₈), X₁₁ may be C(R₁₁), X₁₂ may be C(R₁₂), X₁₃ may be C(R₁₃), X₁₄ may be C(R₁₄), X₁₅ may be C(R₁₅), X₁₆ may be C(R₁₆), X₁₇ may be C(R₁₇), X₁₈ may be C(R₁₈). However, these are not limited thereto.

In some embodiments, X₁ may be N, X₂ may be C(R₂), X₃ may be C(R₃), X₄ may be C(R₄), X₅ may be C(R₅), X₆ may be C(R₆), X₇ may be C(R₇), X₈ may be C(R₈), X₁₁ may be C(R₁₁), X₁₂ may be C(R₁₂), X₁₃ may be C(R₁₃), X₁₄ may be C(R₁₄), X₁₅ may be C(R₁₅), X₁₆ may be C(R₁₆), X₁₇ may be C(R₁₇), X₁₈ may be C(R₁₈). However, these are not limited thereto.

In some embodiments, X₁ may be C(R₁), X₂ may be C(R₂), X₃ may be C(R₃), X₄ may be C(R₄), X₅ may be C(R₅), X₆ may be C(R₆), X₇ may be N, X₈ may be C(R₈), X₁₁ may be C(R₁₁), X₁₂ may be C(R₁₂), X₁₃ may be C(R₁₃), X₁₄ may be C(R₁₄), X₁₅ may be C(R₁₅), X₁₆ may be C(R₁₆), X₁₇ may be C(R₁₇), X₁₈ may be C(R₁₈). However, these are not limited thereto.

In an embodiment, R₁ to R₆ and R₁₁ to R₁₆ may each be not a cyano group.

Ar₁ in Formulae 1B to 1D may be represented by one of Formulae 2A to 2C:

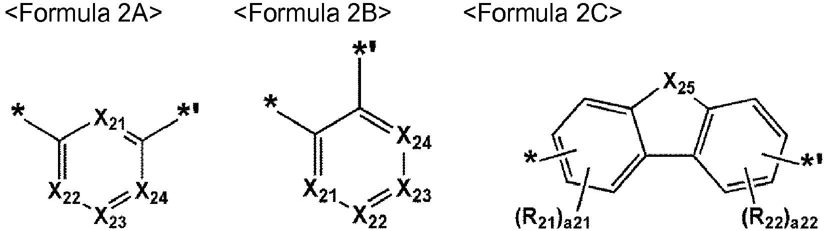

In Formulae 2A to 2C,
X₂₁ may be N or C(R₂₁), X₂₂ may be N or C(R₂₂), X₂₃ may be N or C(R₂₃), X₂₄ may be N or C(R₂₄), and X₂₅ may be O, S, P(=O)(R₂₅), Se, or Si(R₂₅)(R₂₆),
R₂₁ to R₂₆ may be each independently selected from a hydrogen, a deuterium, C₁-C₄ alkyl group, a phenyl group, a pyridinyl group, a pyrimidinyl group, a pyrazinyl group, a pyridazinyl group, a triazinyl group, and -Si(Q₁₁)(Q₁₂)(Q₁₃),
a21 and a22 may be each independently an integer selected from 0 to 3.

a21 indicates the number of R₂₁(s), and when a21 is 2 or more, 2 or more R₂₁(s) may be identical to or different from each other. a2 indicates the number of R₂₂(s), and when a22 is 2 or more, 2 or more R₂₂ may be identical to or different from each other.

In an embodiment, a1 and a2 may be each independently 0, 1, or 2.

In some embodiments, Ar₁ in Formulae 1B to 1D may be represented by one of Formulae 2B and 2C.

For example, Ar₁ in Formulae 1B to 1D may be represented by one of Formulae 2A-1 to 2A-10, 2B-1 to 2B-8, and 2C-1 to 2C-9:

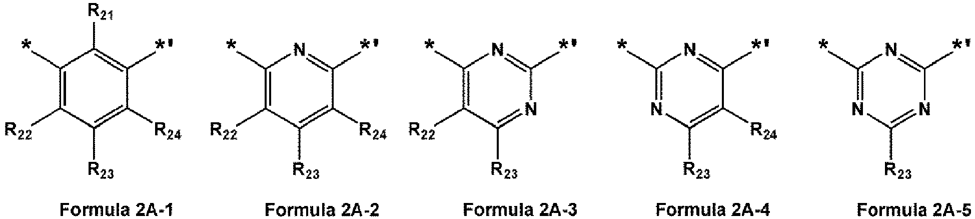

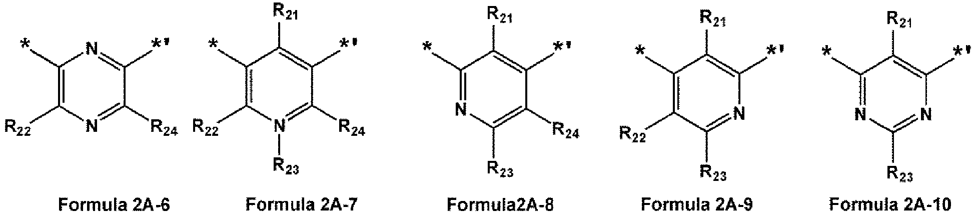

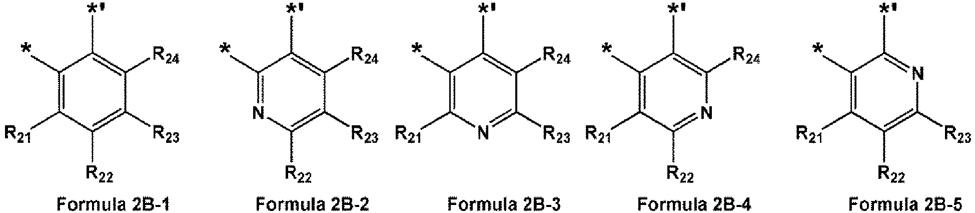

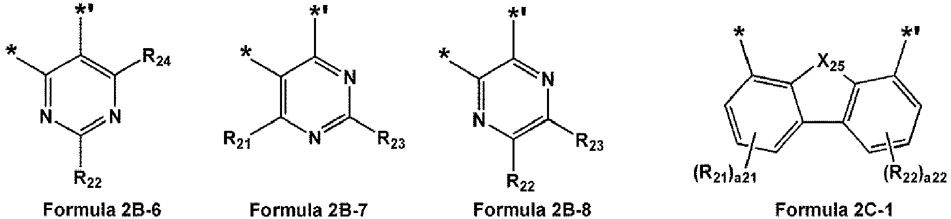

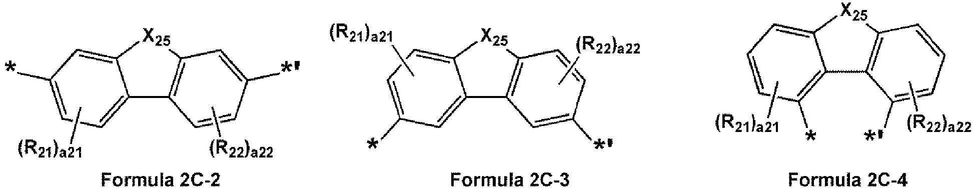

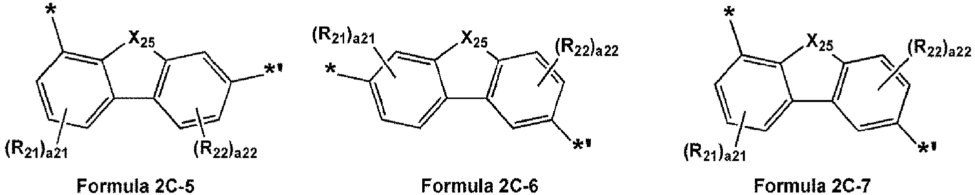

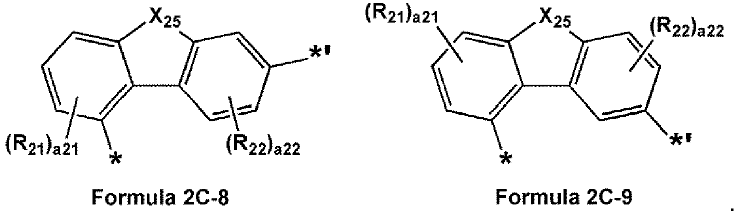

In Formulae 2A-1 to 2A-10, 2B-1 to 2B-8, and 2C-1 to 2C-9,
X₂₅ may be O, S, P(=O)(R₂₅), Se, or Si(R₂₅)(R₂₆),
R₂₁ to R₂₄ may be each independently selected from a hydrogen, a deuterium, a methyl group, an ethyl group, an n-propyl group, an isopropyl group, an n-butyl group, an isobutyl group, a sec-butyl group, a tert-butyl group, a phenyl group, a pyridinyl group, a pyrimidinyl group, a pyrazinyl group, a pyridazinyl group, a triazinyl group, and - Si(Q₁₁)(Q₁₂)(Q₁₃),
R₂₅ and R₂₆ may be each independently selected from a hydrogen, a deuterium, a methyl group, an ethyl group, an n-propyl group, an isopropyl group, an n-butyl group, an isobutyl group, a sec-butyl group, a tert-butyl group, a phenyl group, a pyridinyl group, a pyrimidinyl group, a pyrazinyl group, a pyridazinyl group, and a triazinyl group,
Q₁₁ to Q₁₃ may be each independently selected from a hydrogen, a C₁-C₁₀ alkyl group, a C₁-C₁₀ alkoxy group, and a phenyl group,
a21 and a22 may be each independently 0 or 1, and
each of * and *' indicates a binding site to a neighboring atom.

In an embodiment, when Ar₁ in Formulae 1B to 1D is represented by Formula 2A-1, the sum of a1 and a2 in Formulae 1B to 1D may not be 0.

L₁ and L₂ in Formulae 1B to 1D may be each independently selected from
a phenylene group, a pyridinylene group, a pyrimidinylene group, a pyrazinylene group, a pyridazinylene group, a triazinylene group, a dibenzofuranylene group, and a dibenzothiophenylene group; and
a phenylene group, a pyridinylene group, a pyrimidinylene group, a pyrazinylene group, a pyridazinylene group, a triazinylene group, a dibenzofuranylene group, and a dibenzothiophenylene group, each substituted with at least one selected from a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group, a C₁-C₁₀ alkoxy group, a phenyl group, a naphthyl group, a pyridinyl group, a pyrimidinyl group, a pyrazinyl group, a pyridazinyl group, a triazinyl group, and-Si(Q₂₁)(Q₂₂)(Q₂₃).

For example, L₁ and L₂ in Formulae 1B to 1D may be each independently selected from
a phenylene group, a pyridinylene group, a pyrimidinylene group, and a triazinylene group; and
a phenylene group, a pyridinylene group, a pyrimidinylene group, and a triazinylene group, each substituted with at least one selected from a deuterium, a methyl group, an ethyl group, an n-propyl group, an isopropyl group, an n-butyl group, an isobutyl group, a sec-butyl group, a tert-butyl group, a phenyl group, a pyridinyl group, a pyrimidinyl group, a pyrazinyl group, a pyridazinyl group, a triazinyl group, and -Si(Q₂₁)(Q₂₂)(Q₂₃), but are not limited thereto.

a1 and a2 in Formulae 1B to 1D may be each independently an integer selected from 0 to 5. a1 indicates the number of L₁(s), and when a1 is 0, *-(L₁)ₐ₁-*' indicates a single bond. When a1 is 2 or more, 2 or more L₁(s) may be identical to or different from each other. a2 indicates the number of L₂(s), when a2 is 0, *-(L₂)ₐ₂-*' indicates a single bond. When a2 is 2 or more, 2 or more L₂(s) may be identical to or different from each other.

In an embodiment, a1 and a2 may be each independently 0, 1, or 2.

In some embodiments, a1 and a2 may be each independently 0 or 1.

For example, L₁ and L₂ in Formulae 1B to 1D may be each independently selected from
a phenylene group; and
a phenylene group, substituted with at least one selected from a deuterium, a methyl group, an ethyl group, an n-propyl group, an isopropyl group, an n-butyl group, an isobutyl group, a sec-butyl group, a tert-butyl group, a phenyl group, a pyridinyl group, a pyrimidinyl group, a pyrazinyl group, a pyridazinyl group, a triazinyl group, and-Si(Q₂₁)(Q₂₂)(Q₂₃),
wherein Q₂₁ to Q₂₃ may be each independently selected from a hydrogen, a C₁-C₁₀ alkyl group, a C₁-C₁₀ alkoxy group, and a phenyl group,
a1 and a2 may be each independently 0 or 1, but are not limited thereto.

In some embodiments, in Formulae 1B to 1D, a1 and a2 may each be 0, and Ar₁ may be represented by one of Formulae 2A-2 to 2A-10, 2B-1 to 2B-8, and 2C-1 to 2C-9.

In some embodiments, in Formulae 1B to 1D, a1 and a2 may each be 0, and Ar₁ may be represented by one of Formulae 2A-1, 2A-2, 2A-10, 2B-1, 2B-8, and 2C-3.

In some embodiments, the sum of a1 and a2 in Formulae 1B to 1D is 1 or more, and *-(L₁)ₐ₁-Ar₁-(L₂)ₐ₂-*' in Formulae 1B to 1D may be represented by one of Formulae 3-1 to 3-57, but these are not limited thereto:

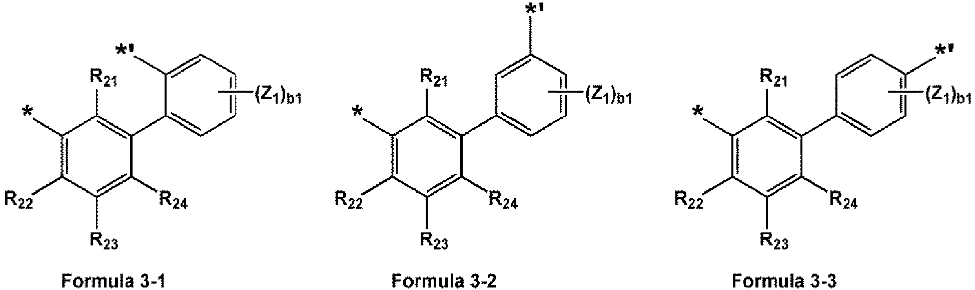

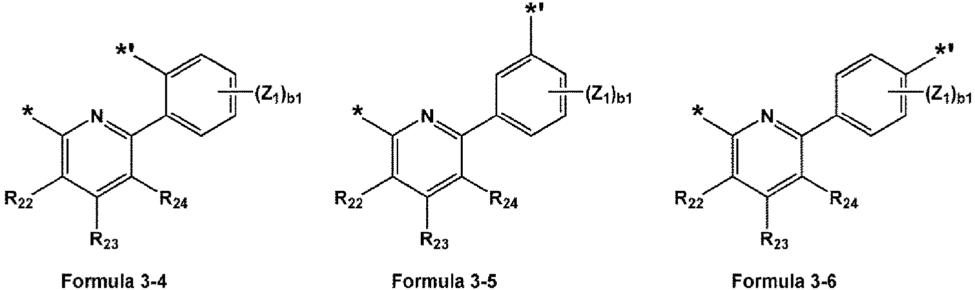

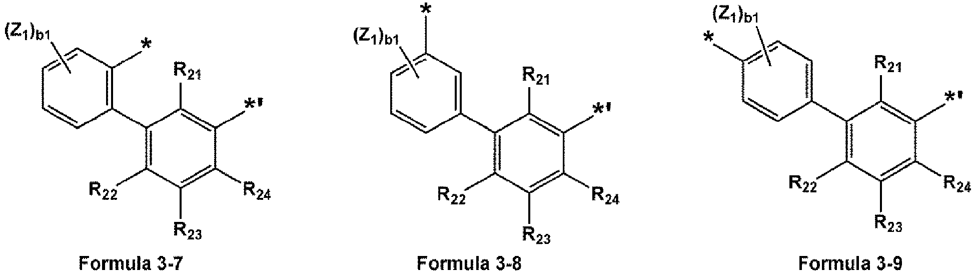

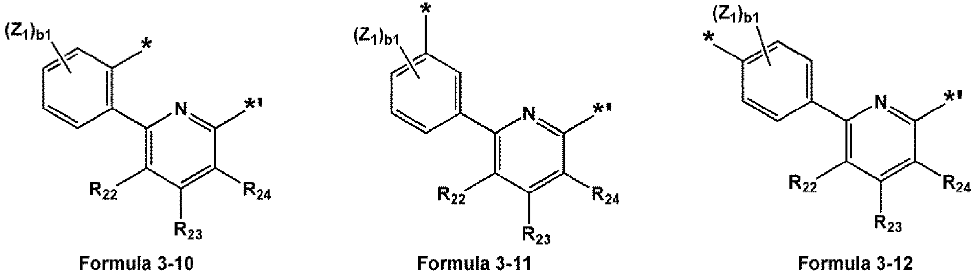

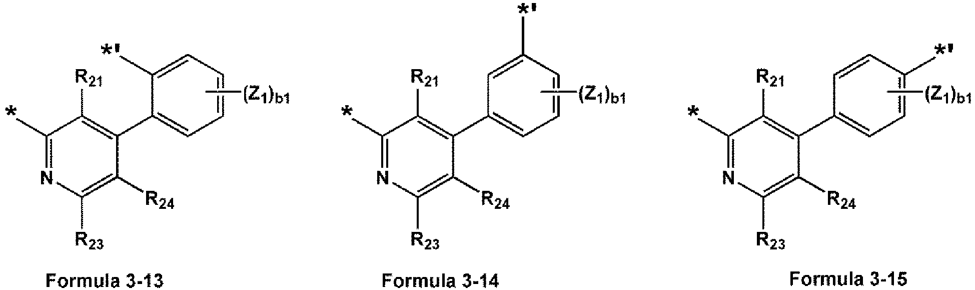

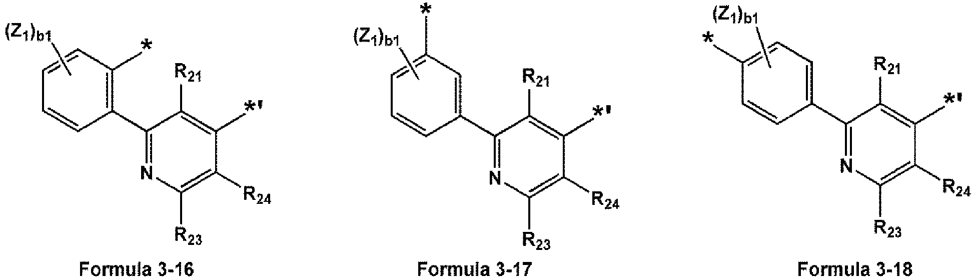

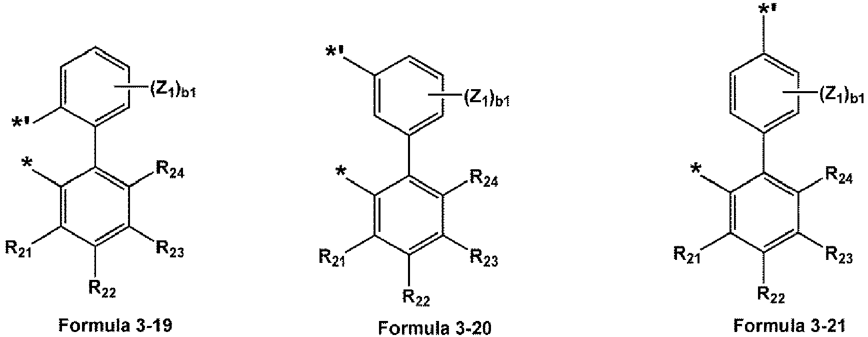

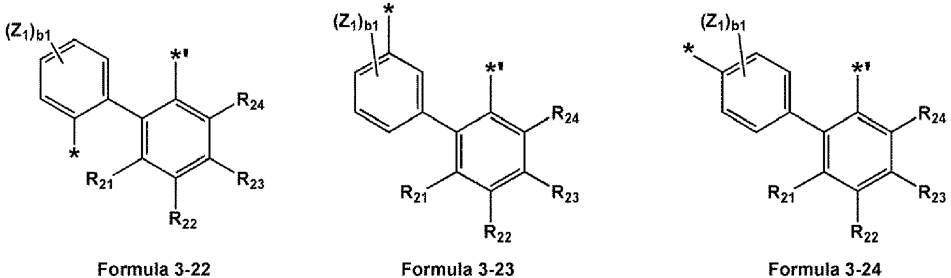

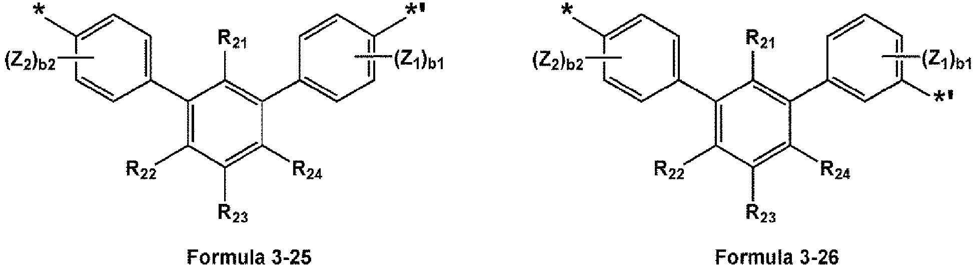

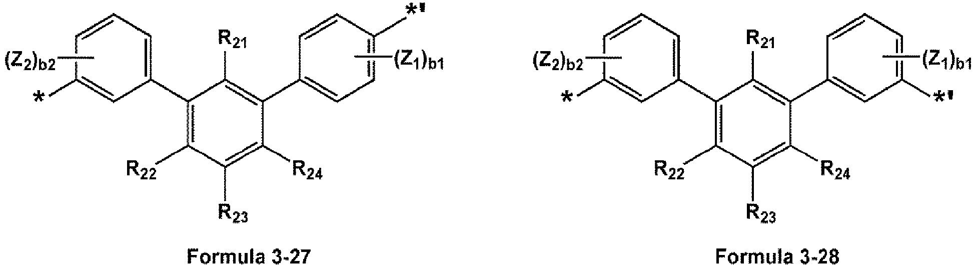

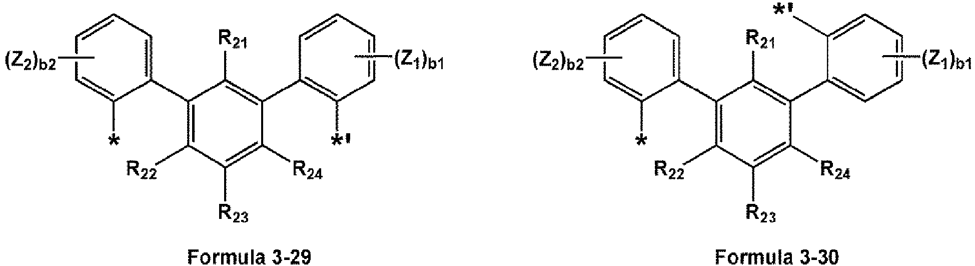

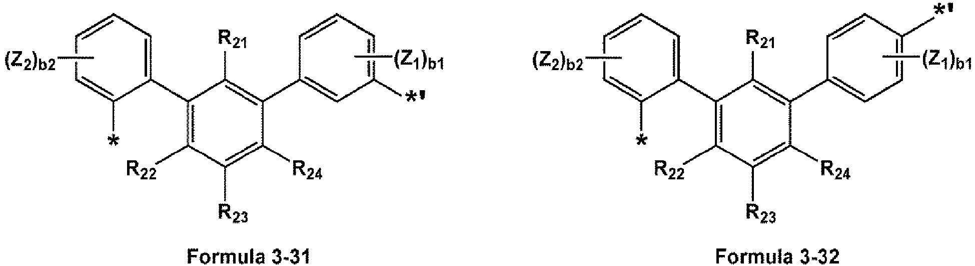

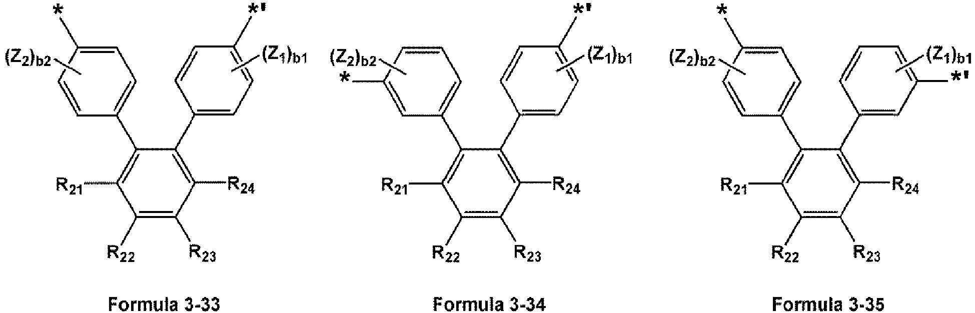

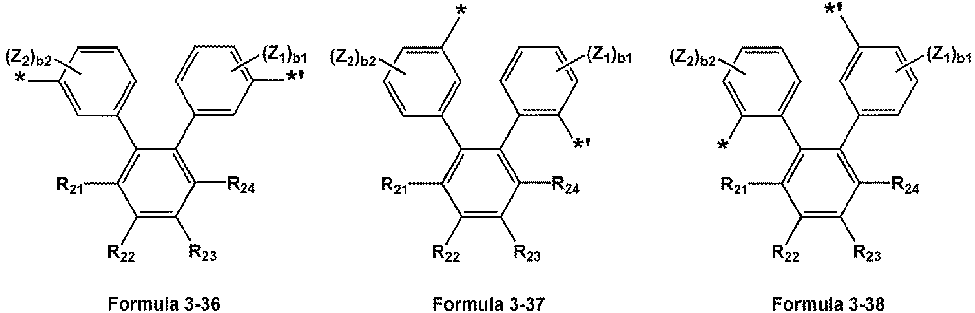

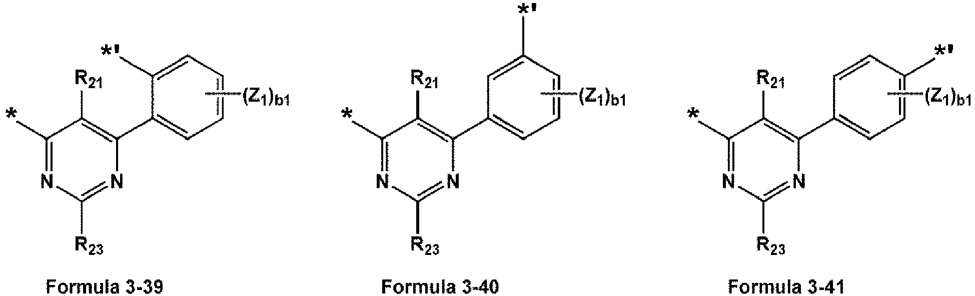

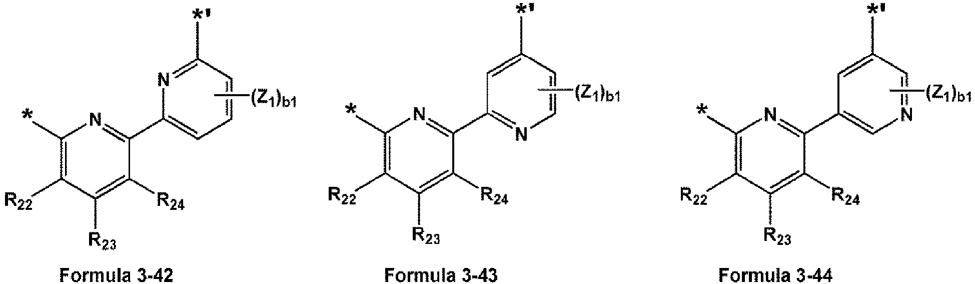

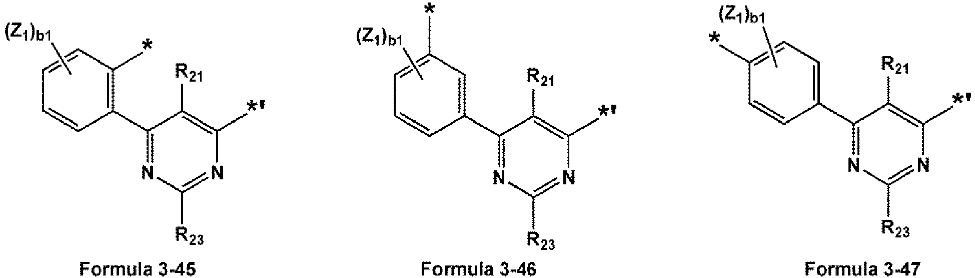

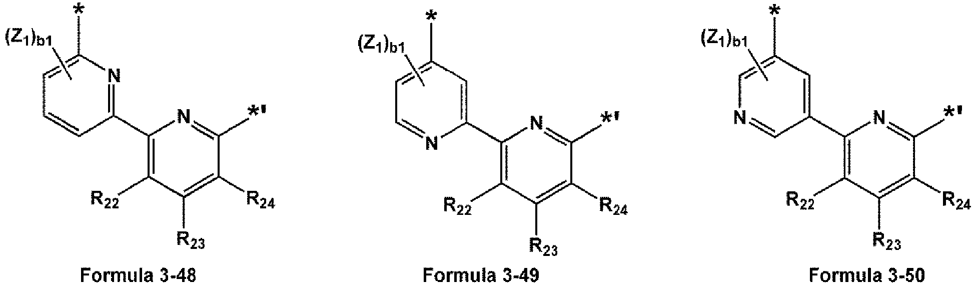

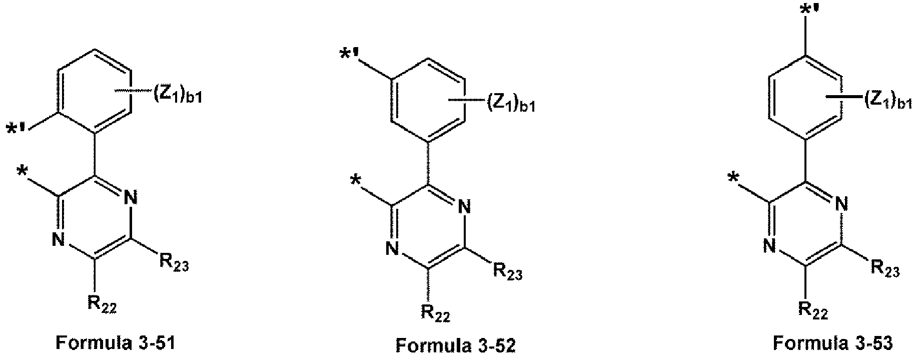

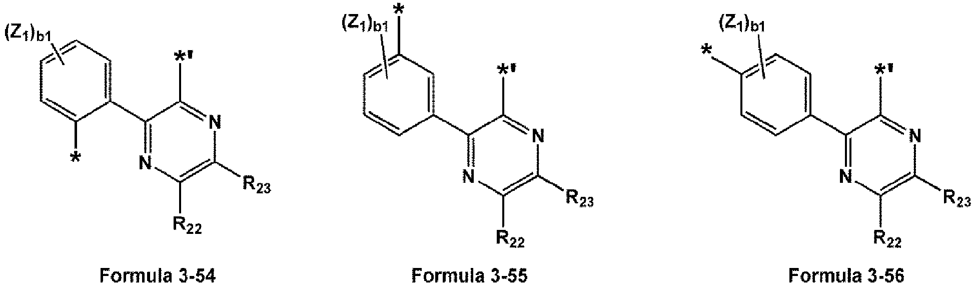

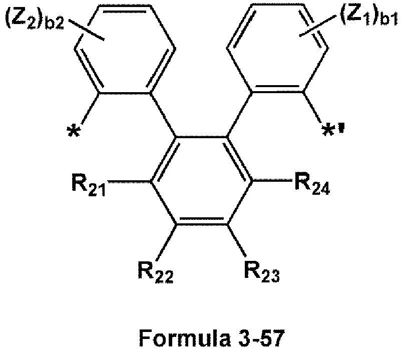

In Formulae 3-1 to 3-57,
R₂₁ to R₂₄, Z₁, and Z₂ may be each independently selected from a hydrogen, a deuterium, a methyl group, an ethyl group, an n-propyl group, an isopropyl group, an n-butyl group, an isobutyl group, a sec-butyl group, a tert-butyl group, a phenyl group, a pyridinyl group, a pyrimidinyl group, a pyrazinyl group, a pyridazinyl group, a triazinyl group, and -Si(Q₁₁)(Q₁₂)(Q₁₃),
Q₁₁ to Q₁₃ may be each independently selected from a hydrogen, a C₁-C₁₀ alkyl group, a C₁-C₁₀ alkoxy group, and a phenyl group,
b1 and b2 may be each independently 0, 1, or 2, and
each of *and*' indicates a binding site to a nitrogen atom.

In some embodiments, the condensed cyclic compound represented by Formulae 1B to 1D may be represented by one selected from Formulae 1B(1) to 1D(1), 1B(2) to 1D(2), and 1B(3) to 1D(3):

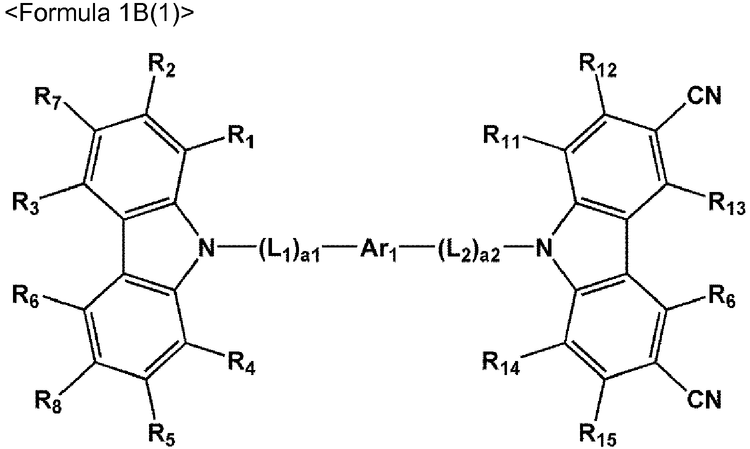

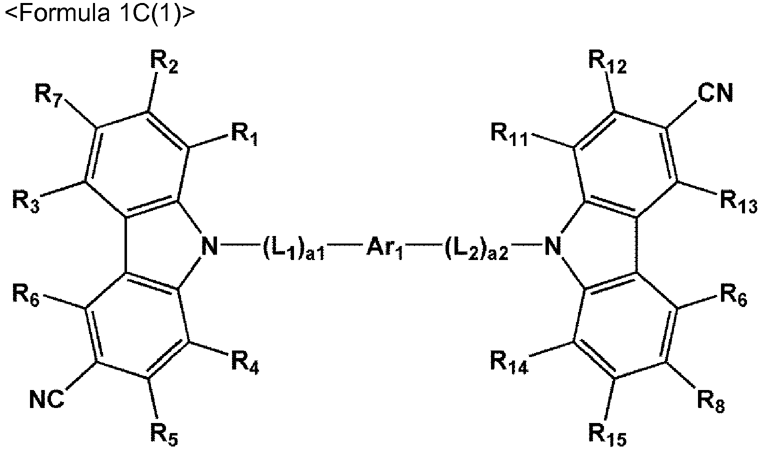

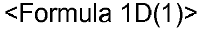

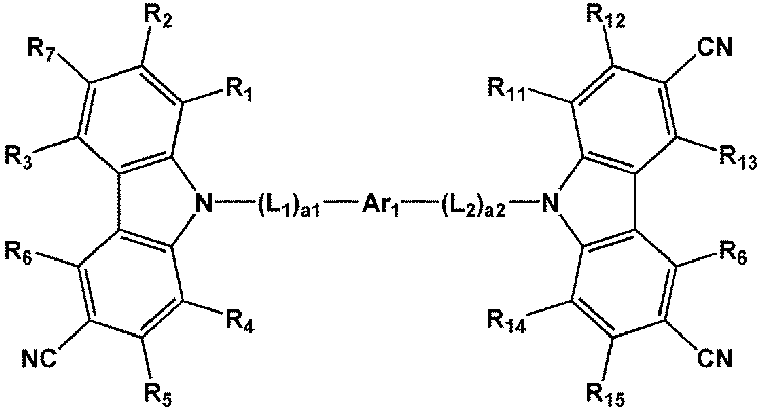

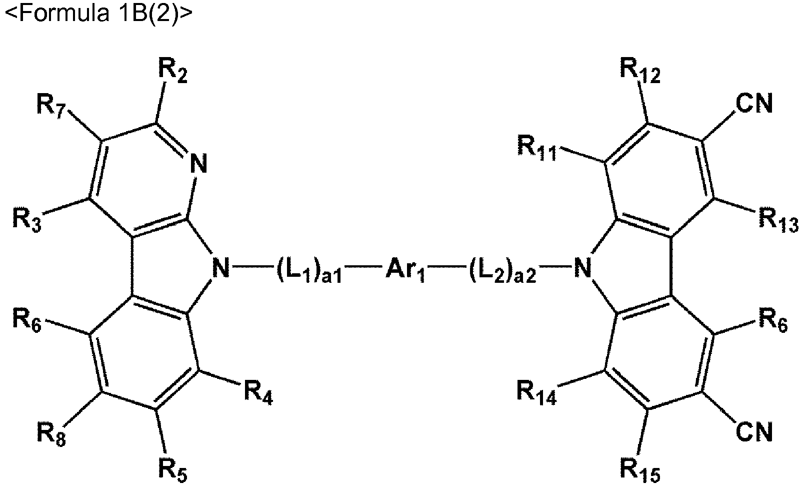

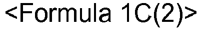

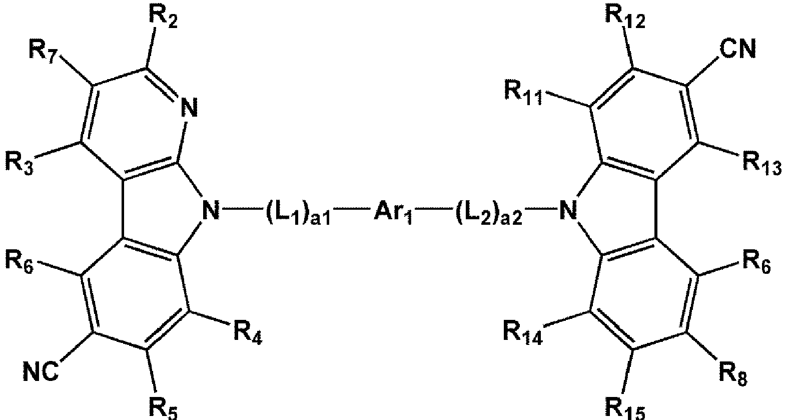

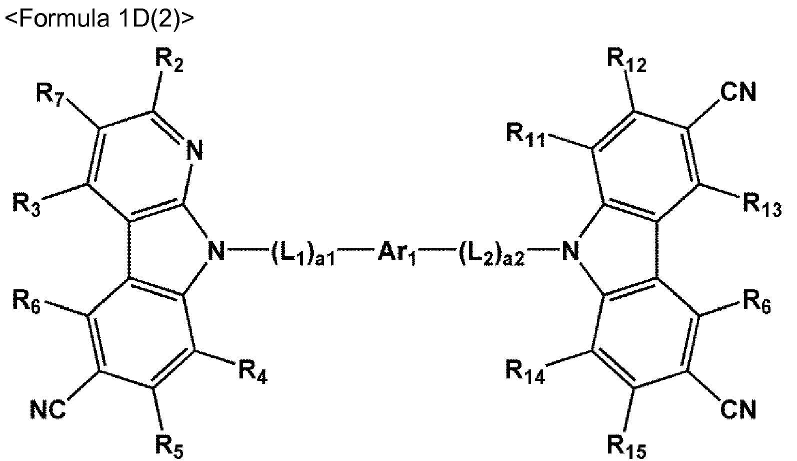

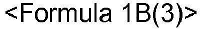

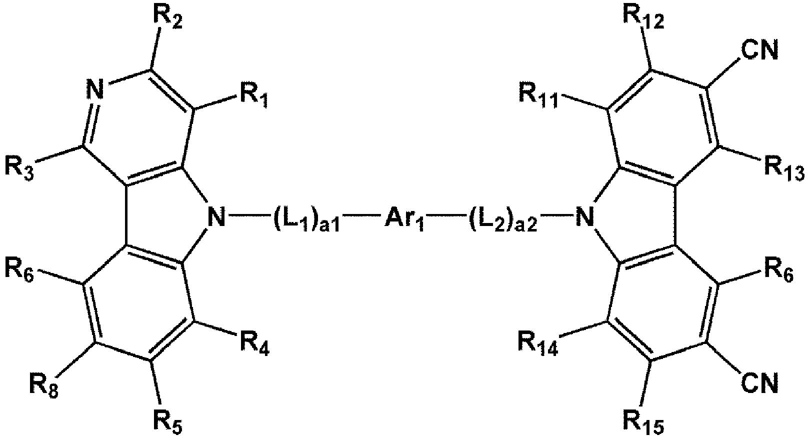

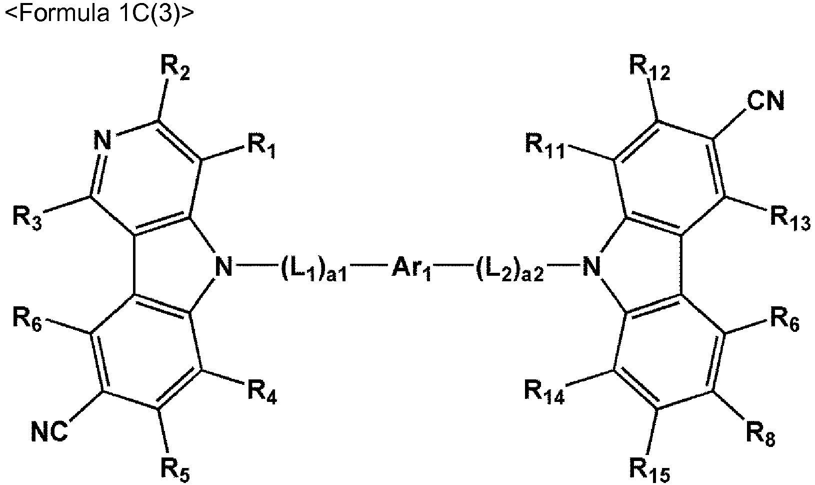

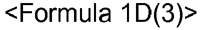

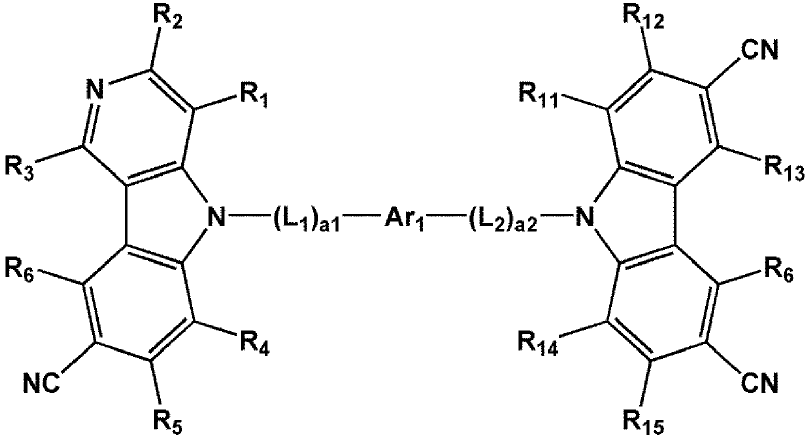

Descriptions of R₁ to R₈, R₁₁ to R₁₆, R₁₈, Ar₁, L₁, L₂, a1, a2 and *-(L₁)ₐ₁-Ar₁-(L₂)ₐ₂-*' in Formulae 1B(1) to 1D(1), 1B(2) to 1D(2), and 1B(3) to 1D(3) are the same as presented above.

For example, each of R₁ to R₈, R₁₁ to R₁₆, and R₁₈ in Formulae 1B(1) to 1D(1), 1B(2) to 1D(2), and 1B(3) to 1D(3) may not be a cyano group.

In some embodiments, R₁ to R₈, R₁₁ to R₁₆, and R₁₈ in Formulae 1B(1) to 1D(1), 1B(2) to 1D(2), and 1B(3) to 1D(3) may be each independently selected from a hydrogen, a deuterium, -F, -Cl, -Br, -I, a hydroxyl group, a nitro group, an amino group, an amidino group, a hydrazine group, a hydrazone group, a carboxylic acid group or a salt thereof, a sulfonic acid group or a salt thereof, a phosphoric acid group or a salt thereof, a methyl group, an ethyl group, an n-propyl group, an isopropyl group, an n-butyl group, an isobutyl group, a sec-butyl group, a tert-butyl group, an n-pentyl group, an isopentyl group, a sec-pentyl group, a tert-pentyl group, an n-hexyl group, an iso-hexyl group, a sec-hexyl group, a tert-hexyl group, an n-heptyl group, an iso-heptyl group, a sec-heptyl group, a tert-heptyl group, an n-octyl group, an iso-octyl group, a sec-octyl group, a tert-octyl group, an n-nonyl group, an iso-nonyl group, a sec-nonyl group, a tert-nonyl group, an n-decyl group, an isodecyl group, a sec-decyl group, a tert-decyl group, a methoxy group, an ethoxy group, a propoxy group, a butoxy group, a pentoxy group, a phenyl group, a naphthyl group, a pyridinyl group, a pyrimidinyl group, a triazinyl group, and -Si(Q₁)(Q₂)(Q₃),

Ar₁ is represented by one of Formulae 2A-1, 2A-2, 2A-10, 2B-1, 2B-8, or 2C-3:
L₁ and L₂ may be each independently selected from
a phenylene group, a pyridinylene group, a pyrimidinylene group, a pyrazinylene group, a pyridazinylene group, a triazinylene group, a dibenzofuranylene group, and a dibenzothiophenylene group; and
a phenylene group, a pyridinylene group, a pyrimidinylene group, a pyrazinylene group, a pyridazinylene group, a triazinylene group, a dibenzofuranylene group, and a dibenzothiophenylene group, each substituted with at least one selected from a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group, a C₁-C₁₀ alkoxy group, a phenyl group, a naphthyl group, a pyridinyl group, a pyrimidinyl group, a pyrazinyl group, a pyridazinyl group, a triazinyl group and-Si(Q₂₁)(Q₂₂)(Q₂₃);
a1 and a2 may be each independently 0 or 1,
wherein Q₁ to Q₃, Q₁₁ to Q₁₃ and Q₂₁ to Q₂₃ may be each independently selected from a hydrogen, a C₁-C₁₀ alkyl group, a C₁-C₁₀ alkoxy group, and a phenyl group, but are not limited thereto.

In an embodiment, the condensed cyclic compound represented by Formulae 1B to 1D may be one of Compounds 1 to 7, 13 to 18, 26 to 27, 31 to 34, 36, 39, 42 to 44, 46, 47, 49, 50, 52, 53, 55 and 56 but is not limited thereto:

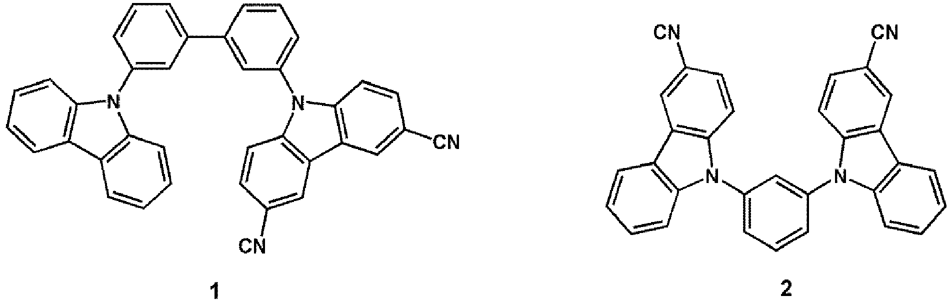

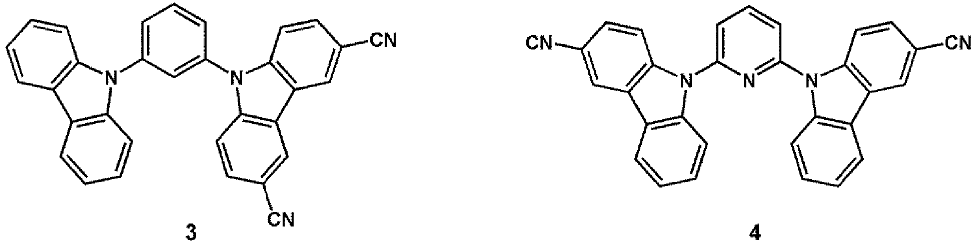

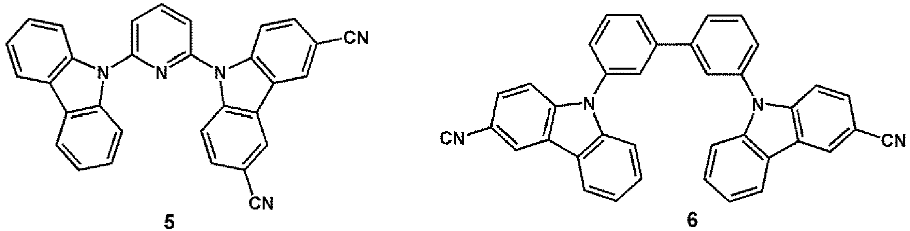

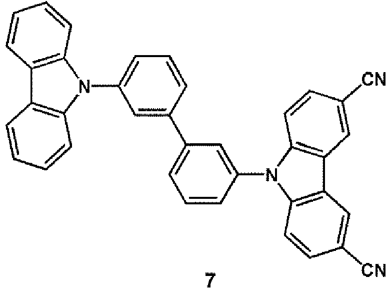

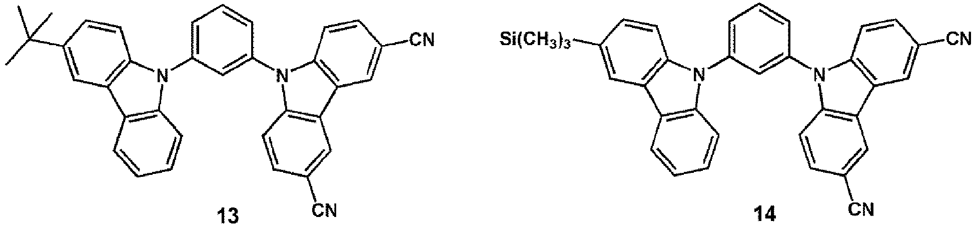

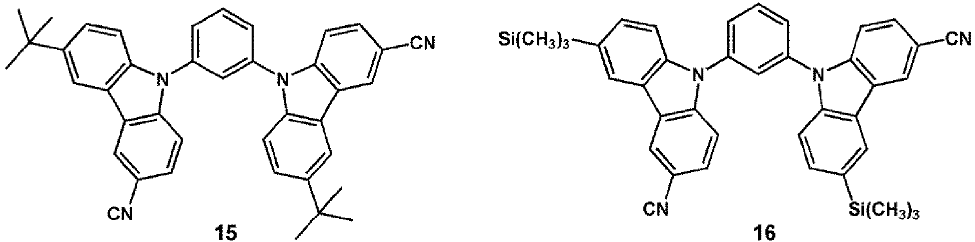

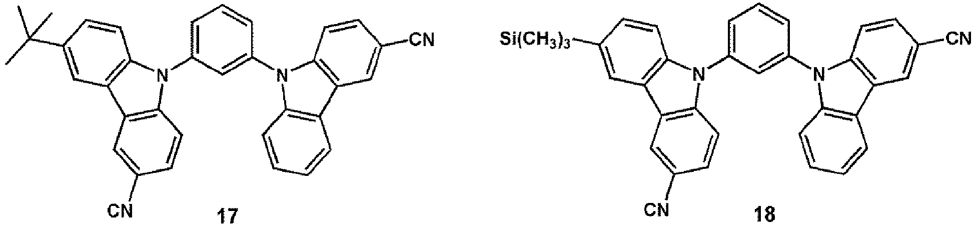

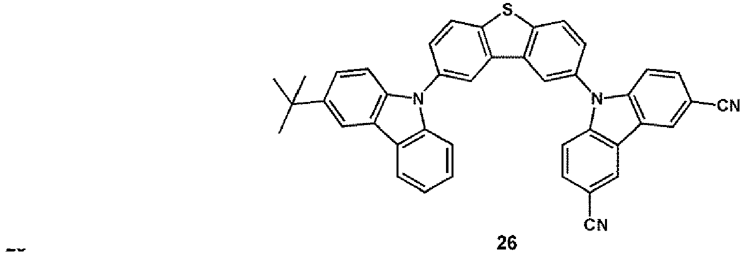

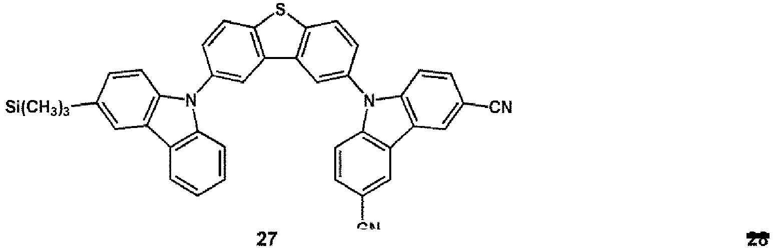

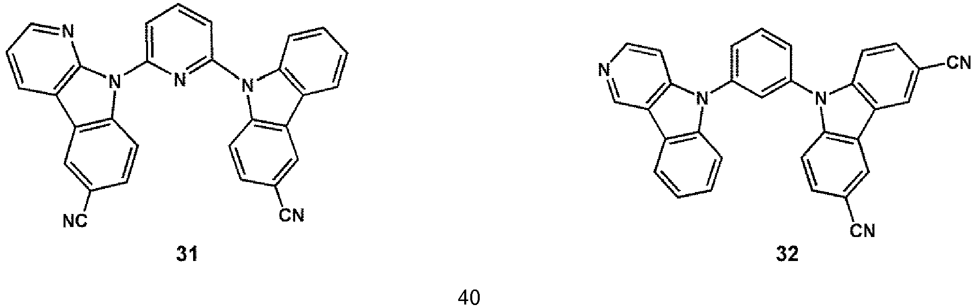

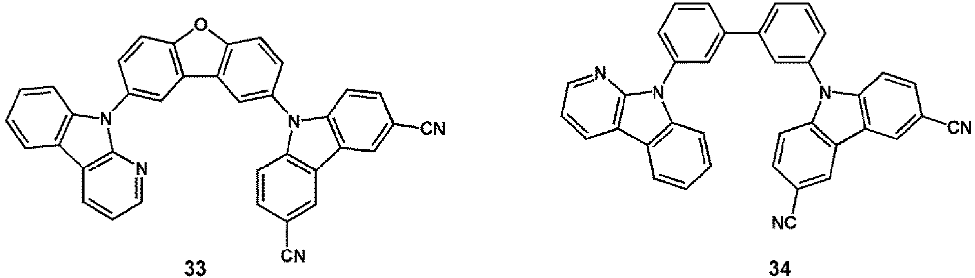

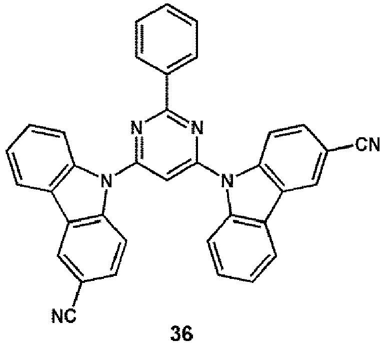

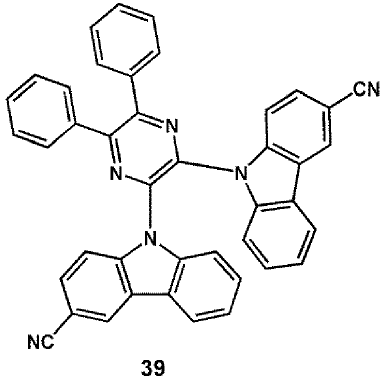

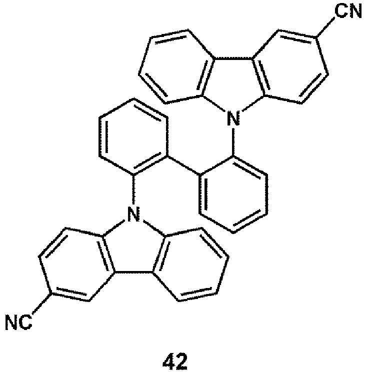

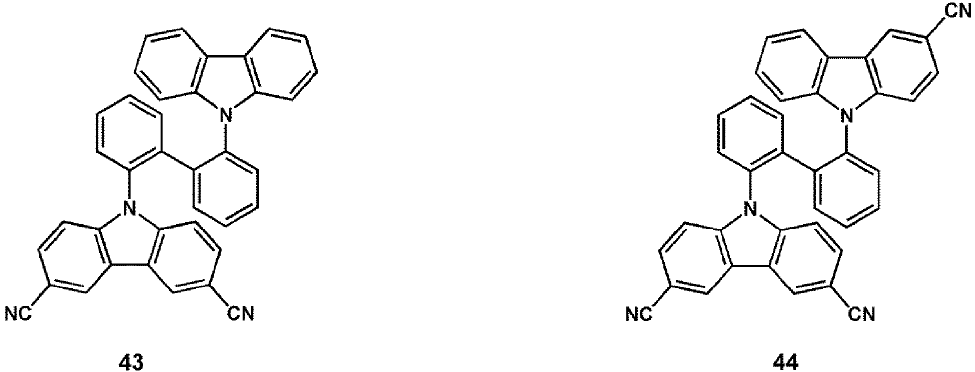

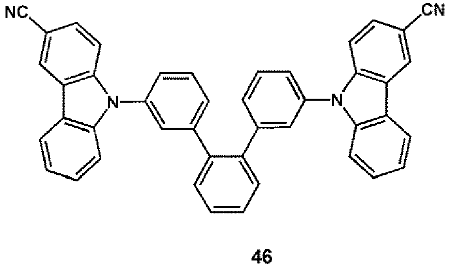

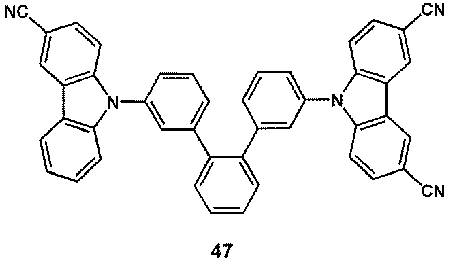

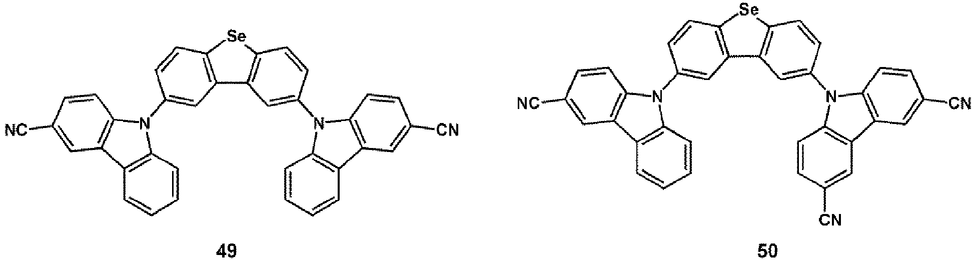

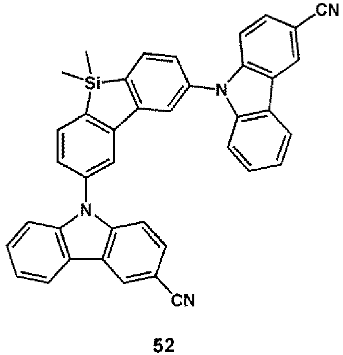

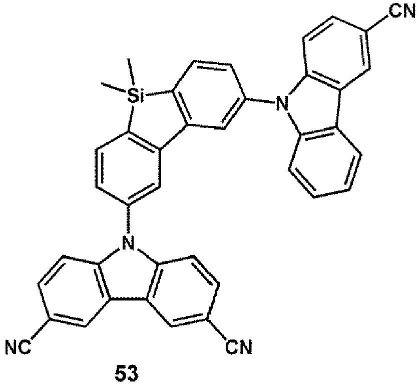

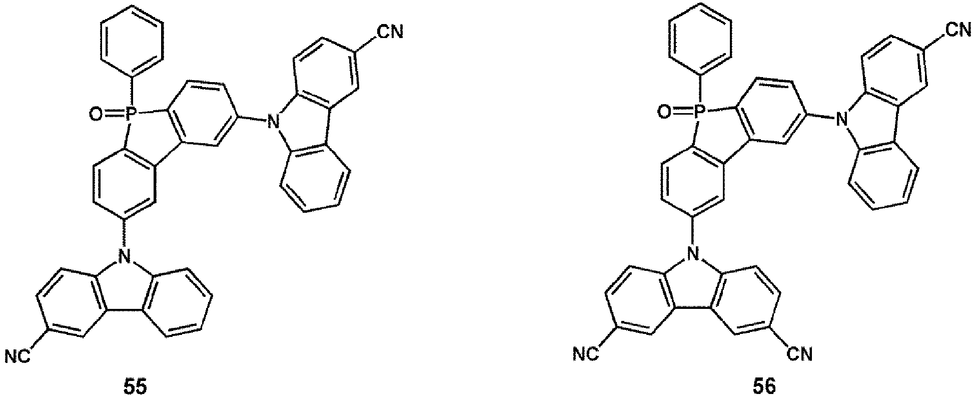

The condensed cyclic compound represented by Formulae 1B to 1D includes a linking group represented by "Ar₁", which is represented by one of Formulae 2A to 2C and the condensed cyclic compound represented by Formulae 1 B to 1 D is not one of the below compounds: 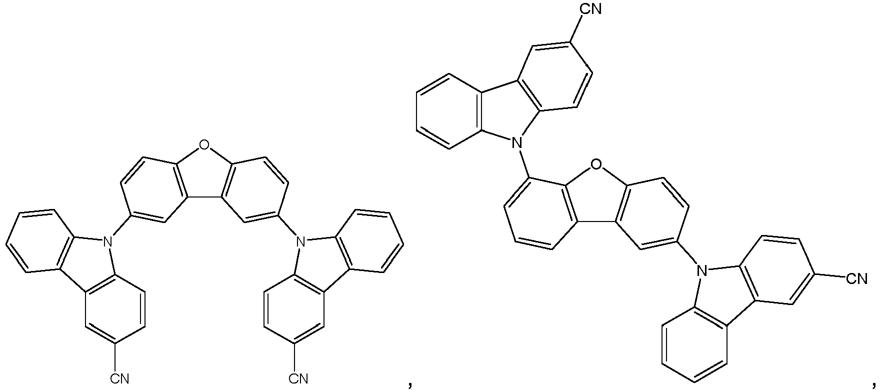 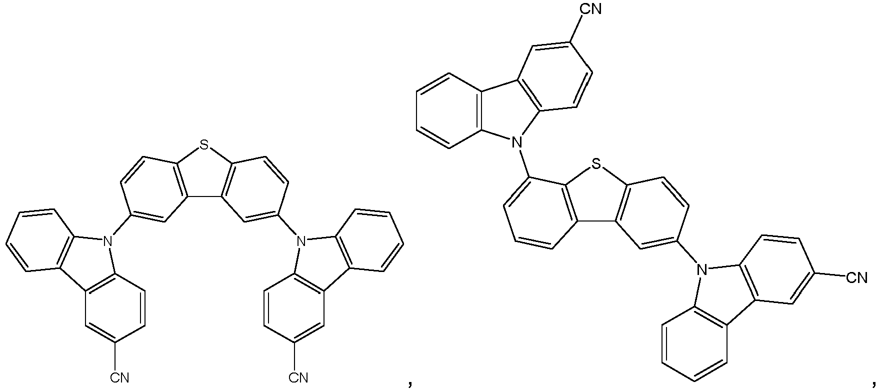 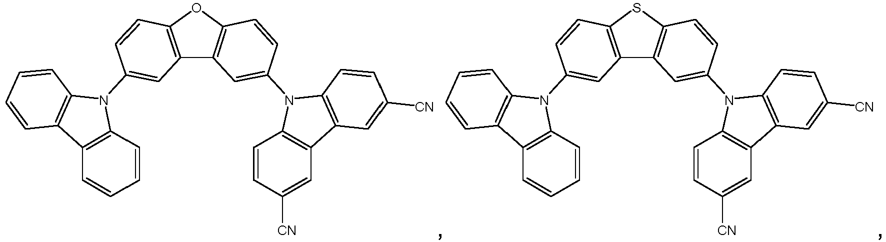 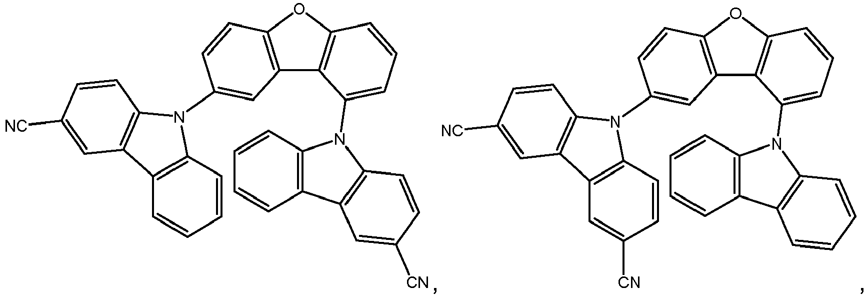 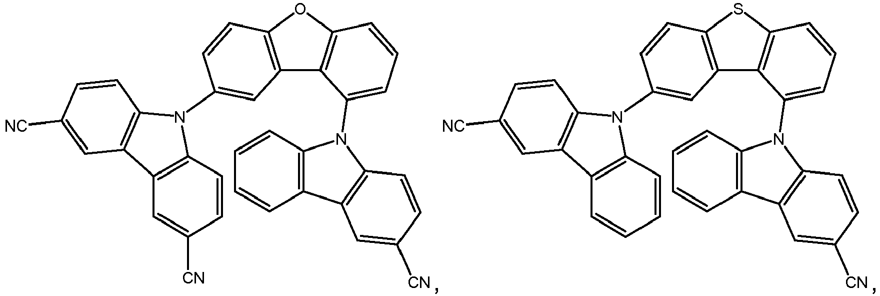 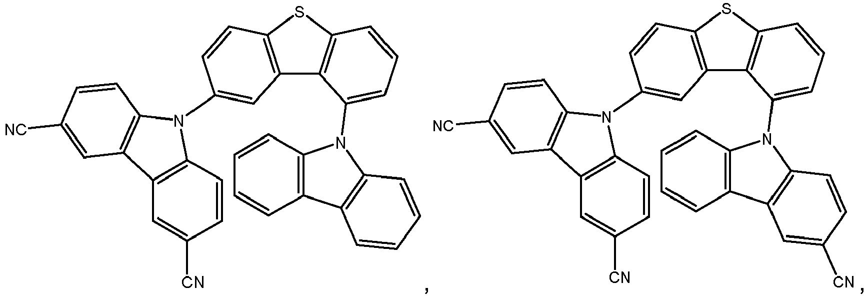 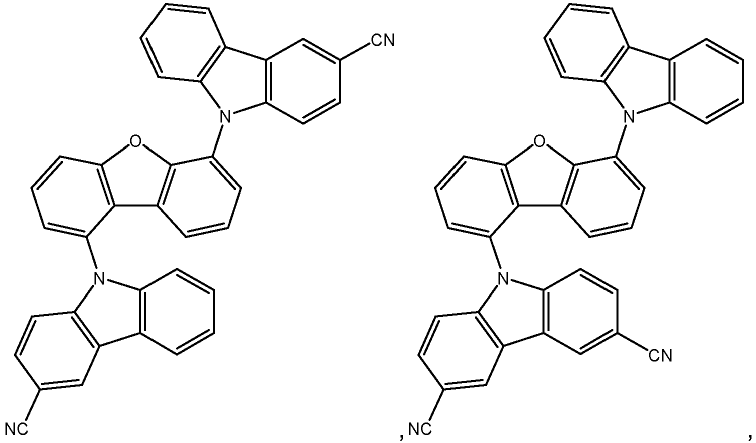 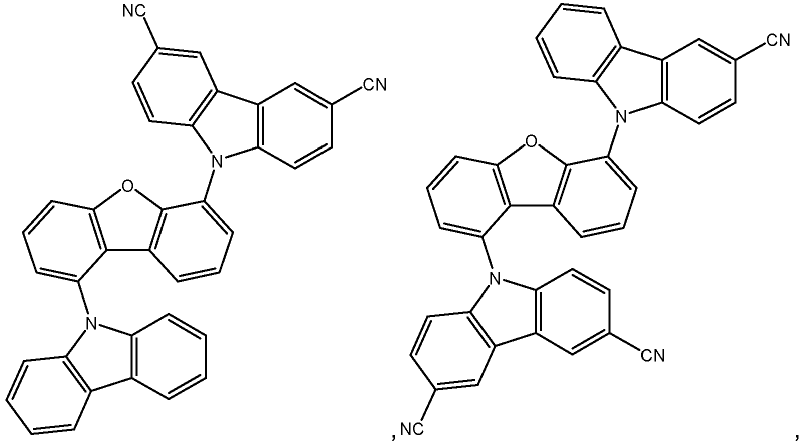 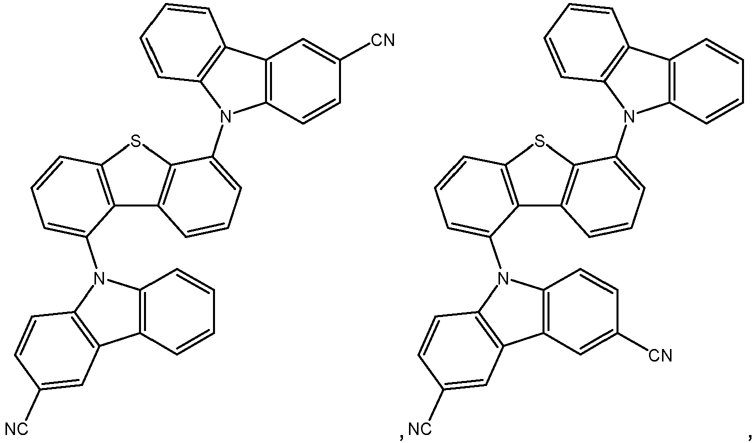 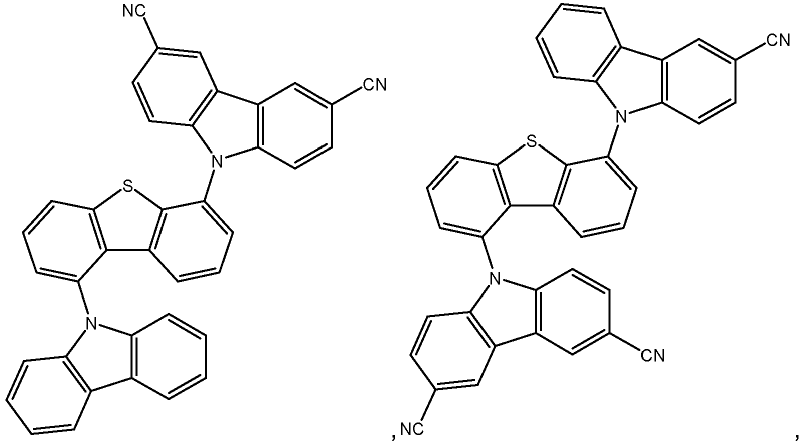 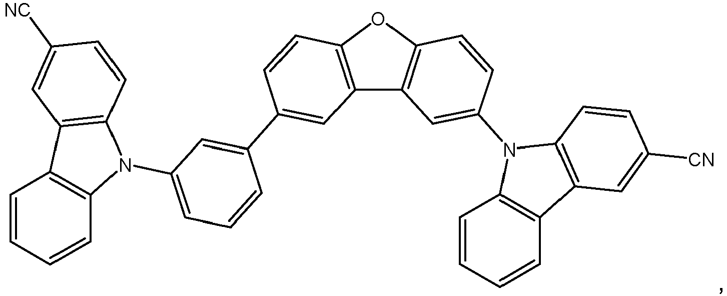 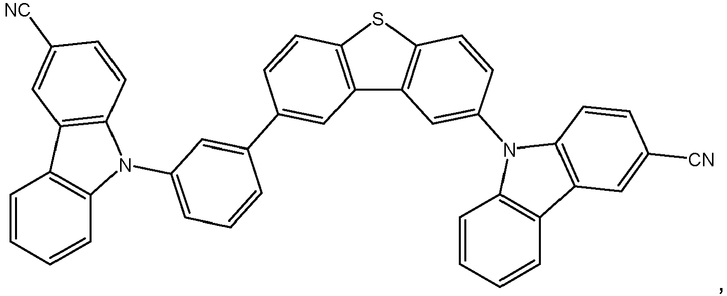 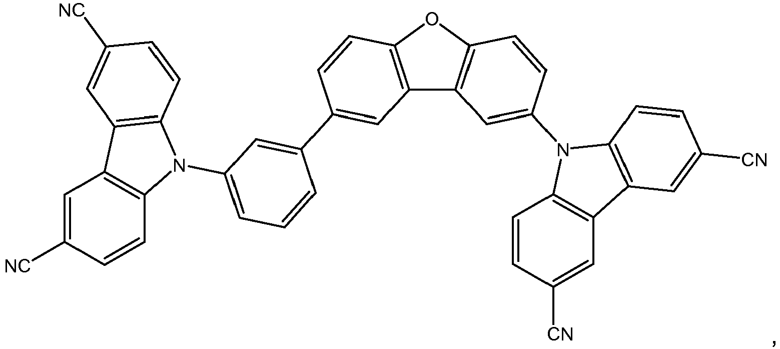 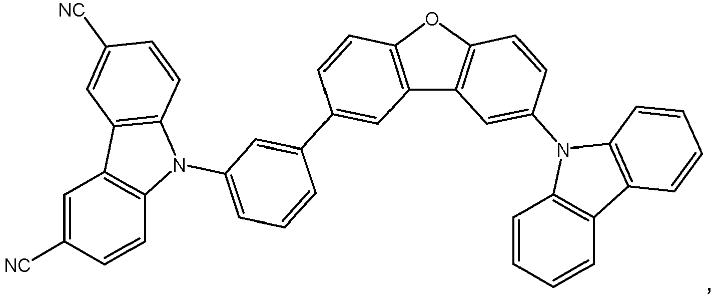 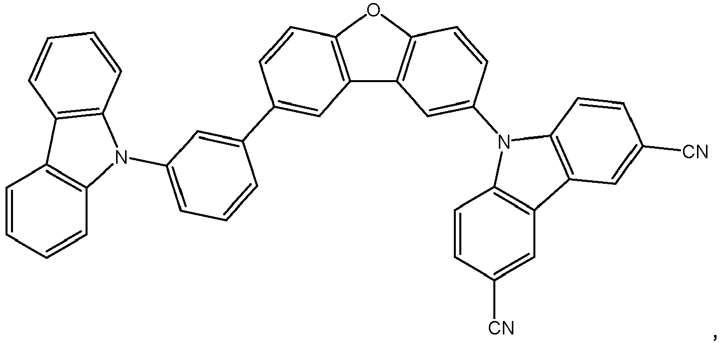 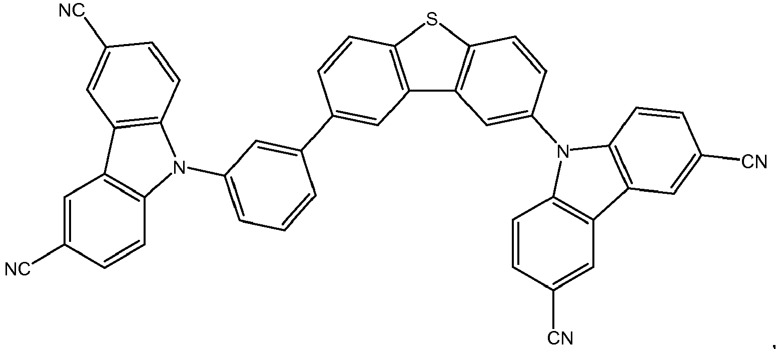 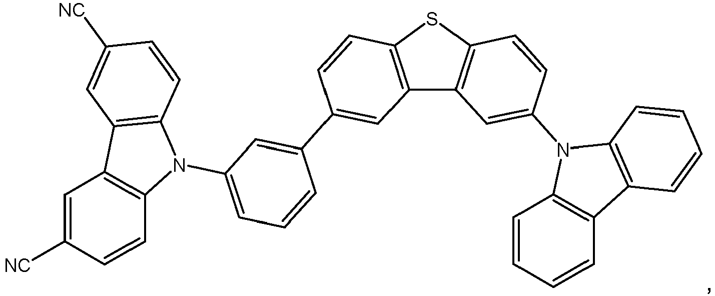 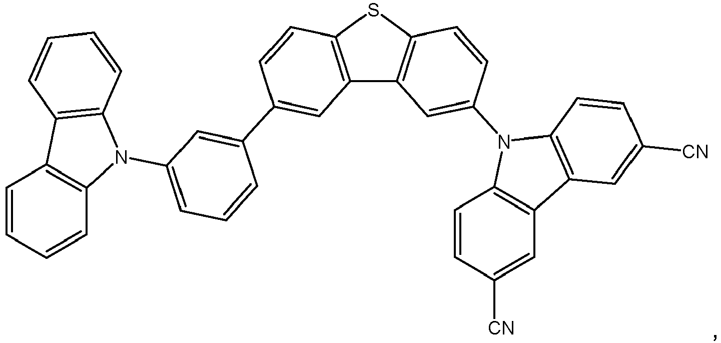 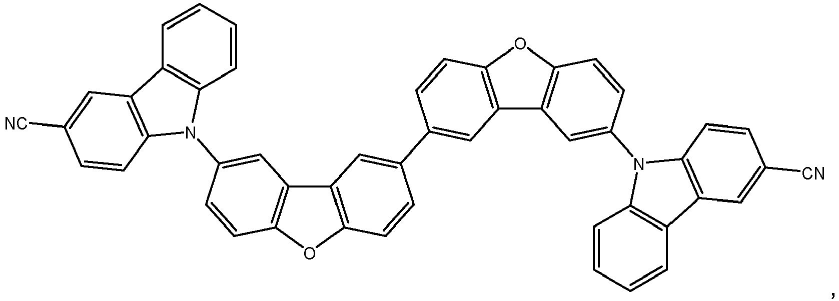 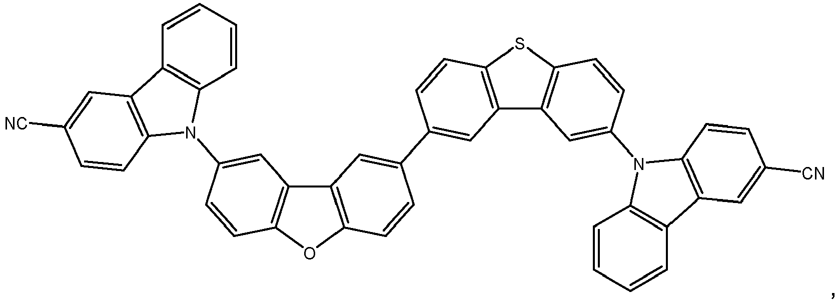 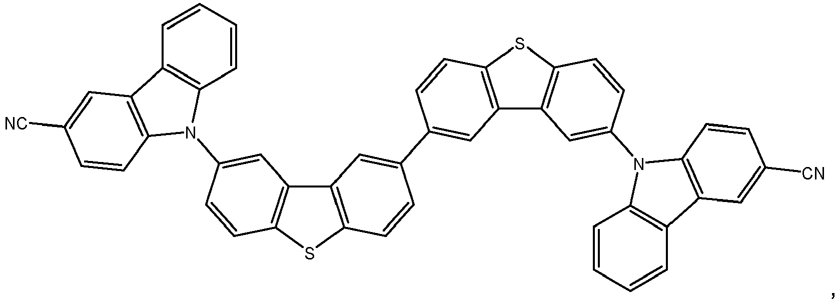 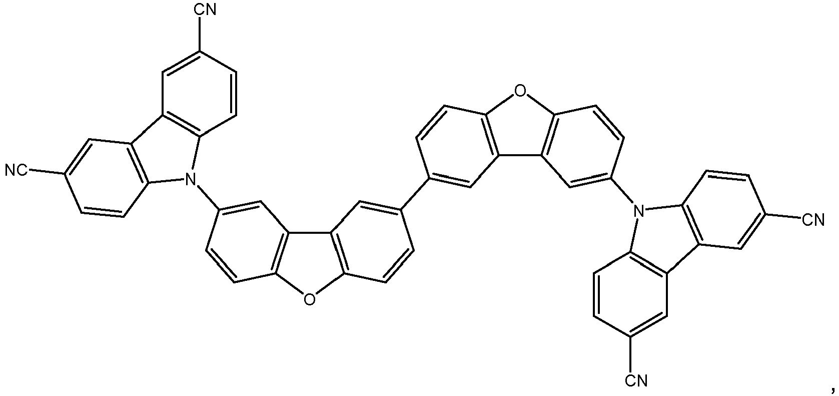 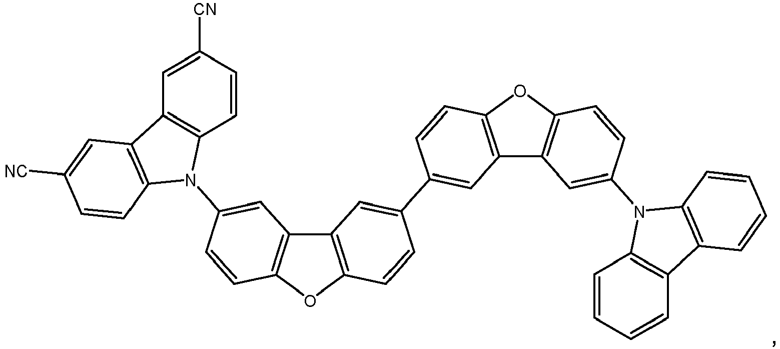 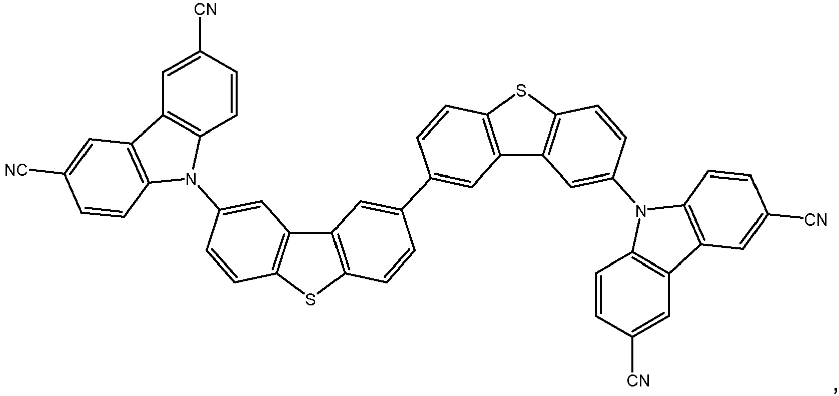 or 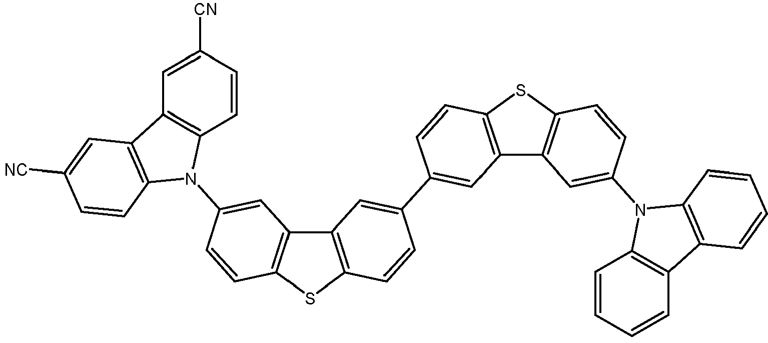 Accordingly the condensed cyclic compound represented by Formulae 1B to 1D may have excellent heat resistance and high triplet energy level.

In some embodiments, regarding the condensed cyclic compound represented by Formulae 1B to 1D, the list of R₁ to R₈ and R₁₁ to R₁₈ does not include "a substituted or unsubstituted carbazolyl group," the list of Ar₁, L₁ and L₂ does not include "a substituted or unsubstituted carbazolylene group," and the list of Ar₁, L₁ and L₂ does not include "a substituted or unsubstituted carbazolyl group." That is, the condensed cyclic compound represented by Formulae 1B to 1D has, as a carbazole-based ring, a carbazole-based ring 1 and a carbazole-based ring 2 (see Formula 1'). Accordingly, the condensed cyclic compound represented by Formulae 1B to 1D may have a triplet (T₁) energy level that is suitable for an electronic device, for example, for use as a material for an organic light-emitting device (for example, a host material in an emission layer) as exemplified below by Formula 1'.

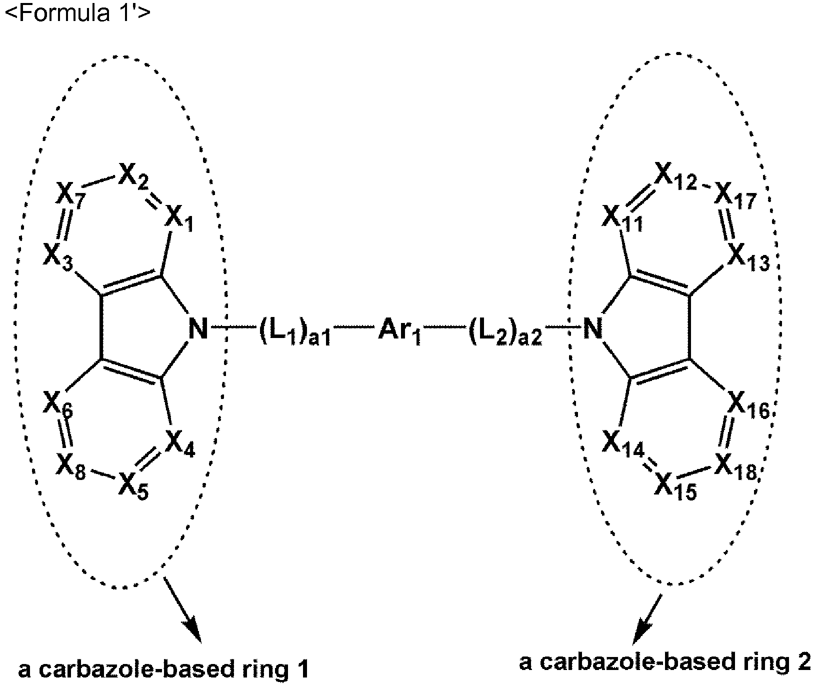

The condensed cyclic compound represented by Formulae 1B to 1D may have a relatively small difference between S₁ (singlet) energy and T₁ (triplet) energy. Accordingly, the condensed cyclic compound represented by Formulae 1B to 1D may be used as a thermally activated delayed fluorescence emitter (TADF emitter).

For example, the highest occupied molecular orbital (HOMO), lowest unoccupied molecular orbital (LUMO), T₁ and S₁ energy levels of Compounds 1 to 30 and A were simulated by using Gaussian program DFT method (the structure is optimized at B3LYP, 6-31 G(d,p) level), and simulation evaluation results are shown in Table 1 below (in the below table compounds 8 to 12, 19 to 23 and 30 are outside the scope of claim 1 and are provided for reference purposes only):

**[Table 1]**

| | HOMO (eV) | LUMO (eV) | T₁ (eV) | S₁ (eV) |
|---|---|---|---|---|
| Compound 1 | -5.594 | -1.654 | 3.080 | 3.435 |
| Compound 2 | -6.08 | -1.44 | 3.03 | 3.33 |
| Compound 3 | -6.08 | -1.44 | 3.03 | 3.33 |
| Compound 4 | -6.06 | -1.72 | 3.03 | 3.24 |
| Compound 5 | -5.929 | -1.700 | 3.085 | 3.606 |
| Compound 6 | -5.93 | -1.63 | 3.12 | 3.76 |
| Compound 7 | -5.594 | -1.654 | 3.080 | 3.435 |
| Compound 8 | -5.51 | -1.68 | 3.09 | 3.30 |
| Compound 9 | -5.42 | -1.66 | 3.04 | 3.23 |
| Compound 10 | -5.49 | -1.68 | 3.08 | 3.29 |
| Compound 11 | -5.54 | -1.24 | 3.12 | 3.85 |
| Compound 12 | -5.62 | -1.25 | 3.12 | 3.89 |
| Compound 13 | -5.71 | -1.69 | 3.08 | 3.63 |
| Compound 14 | -5.78 | -1.70 | 3.08 | 3.70 |
| Compound 15 | -5.90 | -1.37 | 3.10 | 3.94 |
| Compound 16 | -6.03 | -1.41 | 3.11 | 3.99 |
| Compound 17 | -5.95 | -1.41 | 3.10 | 3.95 |
| Compound 18 | -6.03 | -1.42 | 3.11 | 3.99 |
| Compound 19 | -5.60 | -1.38 | 3.11 | 3.61 |
| Compound 20 | -5.67 | -1.41 | 3.11 | 3.64 |
| Compound 21 | -5.50 | -1.66 | 3.06 | 3.30 |
| Compound 22 | -5.41 | -1.64 | 3.03 | 3.24 |
| Compound 23 | -5.49 | -1.66 | 3.06 | 3.30 |
| | | | | |
| Compound 26 | -5.52 | -1.85 | 2.97 | 3.15 |
| Compound 27 | -5.60 | -1.87 | 3.01 | 3.21 |
| Compound 30 | -5.733 | -1.438 | 3.117 | 3.553 |
| Compound A | -5.450 | -1.080 | 3.160 | 3.330 |

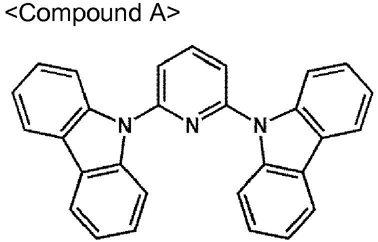

A synthesis method for the condensed cyclic compound represented by Formulae 1 B to 1 D may be understood by one of ordinary skill in the art by referring to the Synthesis Examples.

Accordingly, the condensed cyclic compound represented by Formulae 1B to 1D is suitable for an organic layer of an organic light-emitting device, for example, for use as a host or emitter (for example, a TADF emitter) of an emission layer in the organic layer. Thus, in another aspect, provided is an organic light-emitting device including a first electrode; a second electrode; and an organic layer between the first electrode and the second electrode, the organic layer including an emission layer and at least one of the condensed cyclic compounds represented by Formulae 1B to 1 D.

Due to the inclusion of the condensed cyclic compound represented by Formulae 1B to 1D, the organic light-emitting device may have a low driving voltage, high efficiency, high luminance, high quantum luminescent efficiency, and long lifespan.

The condensed cyclic compound represented by Formulae 1B to 1D may be used between a pair of electrodes that constitute an organic light-emitting device. For example, the condensed cyclic compound may be included in at least one selected from an emission layer, a hole transport region (for example, including at least one selected from a hole injection layer, a hole transport layer, and an electron blocking layer) between a first electrode and the emission layer, and an electron transport region (for example, including at least one selected from a hole blocking layer, an electron transport layer, and an electron injection layer) between the emission layer and a second electrode.

For example, the condensed cyclic compound represented by Formulae 1B to 1D may be included in the emission layer. The condensed cyclic compound included in the emission layer may act as a host, and the emission layer may further include a dopant (a fluorescent dopant or a phosphorescent dopant). The emission layer may be a green emission layer emitting green light or a blue emission layer emitting blue light. In an embodiment, the condensed cyclic compound represented by Formulae 1B to 1D may be included in the emission layer, the emission layer may further include a phosphorescent dopant, and the emission layer may emit blue light.

In some embodiments, the condensed cyclic compound represented by Formulae 1B to 1D may be included in the emission layer, and the condensed cyclic compound may be a TADF emitter. In some embodiments, the emission layer may include the condensed cyclic compound represented by Formulae 1B to 1D alone. In some embodiments, the emission layer may further include, in addition to the condensed cyclic compound represented by Formulae 1B to 1D, a host and/or a dopant.

The term "organic layer" used herein refers to a single layer and/or a plurality of layers between the first electrode and the second electrode of an organic light-emitting device. The "organic layer" may include, in addition to an organic compound, an organometallic complex including metal.

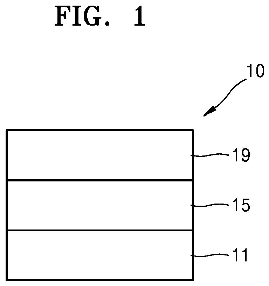

FIG. 1 is a schematic view of an organic light-emitting device 10 according to an embodiment. Hereinafter, the structure of an organic light-emitting device according to an embodiment and a method of manufacturing an organic light-emitting device according to an embodiment will be described in connection with FIG. 1. The organic light-emitting device 10 includes a first electrode 11, an organic layer 15, and a second electrode 19, which are sequentially stacked.

In FIG. 1, a substrate may be additionally disposed under the first electrode 11 or above the second electrode 19. For use as the substrate, any substrate that is used in general organic light-emitting devices may be used, and the substrate may be a glass substrate or transparent plastic substrate, each with excellent mechanical strength, thermal stability, transparency, surface smoothness, ease of handling, and water-resistance.

The first electrode 11 may be formed by depositing or sputtering a material for forming the first electrode on the substrate. The first electrode 11 may be an anode. The material for the first electrode 11 may be selected from materials with a high work function to allow holes be easily provided. The first electrode 11 may be a reflective electrode or a transmissive electrode. The material for the first electrode may be, for example, indium tin oxide (ITO), indium zinc oxide (IZO), tin oxide (SnO₂), and zinc oxide (ZnO). In some embodiments, magnesium (Mg), aluminum (Al), aluminum-lithium (Al-Li), calcium (Ca), magnesium-indium (Mg-ln), or magnesium-silver (Mg-Ag) may be used as the material for the first electrode.

The first electrode 11 may have a single-layer structure or a multi-layer structure including two or more layers. For example, the first electrode 11 may have a three-layered structure of ITO/Ag/ITO, but the structure of the first electrode 110 is not limited thereto.

An organic layer 15 is disposed on the first electrode 11.

The organic layer 15 may include a hole transport region, an emission layer, and an electron transport region.

The hole transport region may be disposed between the first electrode 11 and the emission layer.

The hole transport region may include at least one selected from a hole injection layer, a hole transport layer, an electron blocking layer, and a buffer layer.

The hole transport region may include only either a hole injection layer or a hole transport layer. In some embodiments, the hole transport region may have a structure of hole injection layer/hole transport layer or hole injection layer/hole transport layer/electron blocking layer, which are sequentially stacked in this stated order from the first electrode 11.

A hole injection layer hole injection layer may be formed on the first electrode 11 by using various methods, such as vacuum deposition, spin coating, casting, or Langmuir-Blodgett (LB).

When a hole injection layer is formed by vacuum deposition, the deposition conditions may vary according to a material that is used to form the hole injection layer, and the structure and thermal characteristics of the hole injection layer. For example, the deposition conditions may include a deposition temperature of about 100 to about 500°C, a vacuum pressure of about 10⁻⁸ to about 10⁻³ torr, and a deposition rate of about 1 to about 10,000 pm/sec (about 0.01 to about 100 Å/sec). However, the deposition conditions are not limited thereto.

When the hole injection layer is formed using spin coating, coating conditions may vary according to the material used to form the hole injection layer, and the structure and thermal properties of the hole injection layer. For example, a coating speed may be from about 2000 rpm to about 5000 rpm, and a temperature at which a heat treatment is performed to remove a solvent after coating may be from about 80°C to about 200°C. However, the coating conditions are not limited thereto.

Conditions for a hole transport layer and an electron blocking layer may be understood by referring to conditions for forming the hole injection layer.

The hole transport region may include at least one selected from m-MTDATA, TDATA, 2-TNATA, NPB, β-NPB, TPD, Spiro-TPD, Spiro-NPB, methylated-NPB, TAPC, HMTPD, 4,4',4"-tris(N-carbazolyl)triphenylamine (TCTA), polyaniline/dodecylbenzenesulfonic acid (Pani/DBSA), poly(3,4-ethylenedioxythiophene)/poly(4-styrenesulfonate) (PEDOT/PSS), polyaniline/camphor sulfonic acid (PANI/CSA), (polyaniline)/poly(4-styrenesulfonate) (Pani/PSS), a compound represented by Formula 201 below, and a compound represented by Formula 202 below:

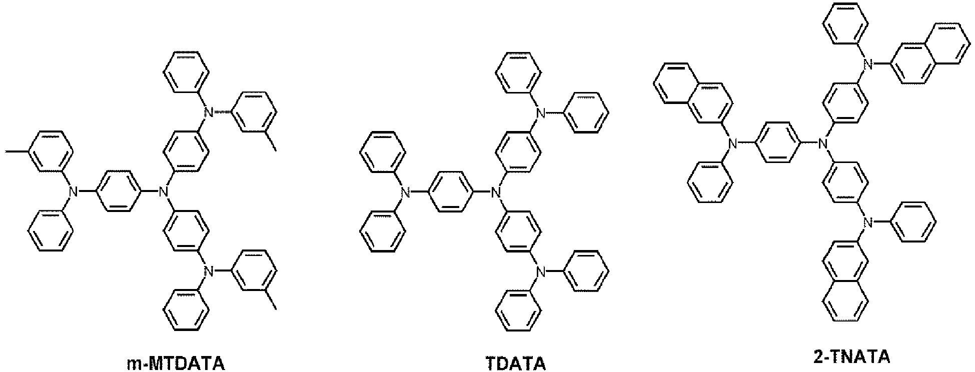

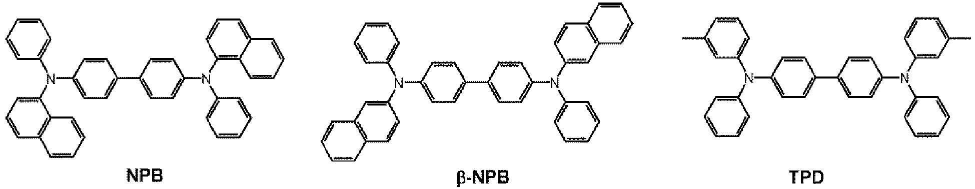

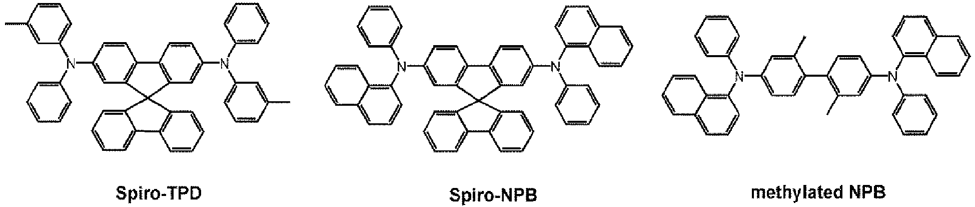

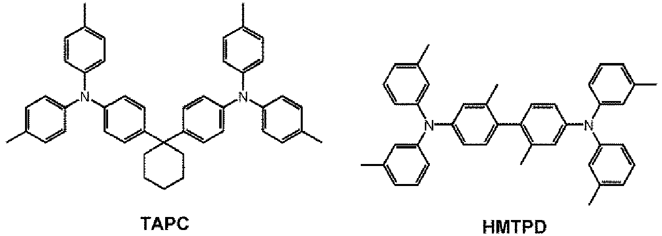

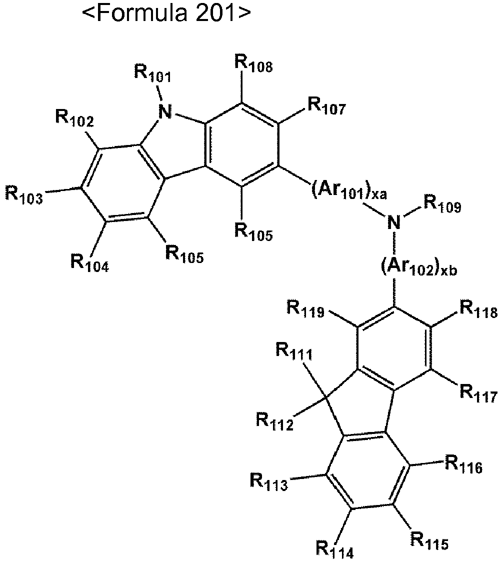

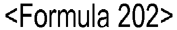

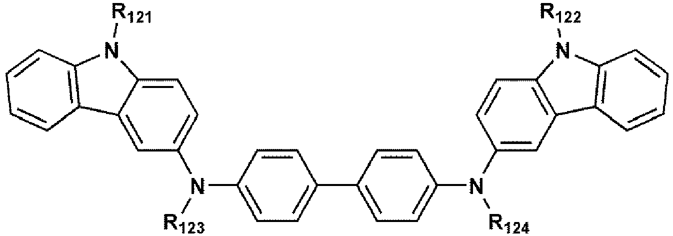

Ar₁₀₁ to Ar₁₀₂ in Formula 201 may be each independently selected from
a phenylene group, a pentalenylene group, an indenylene group, a naphthylene group, an azulenylene group, a heptalenylene group, an acenaphthylene group, a fluorenylene group, a phenalenylene group, a phenanthrenylene group, an anthracenylene group, a fluoranthenylene group, a triphenylenylene group, a pyrenylene group, a chrysenylenylene group, a naphthacenylene group, a picenylene group, a perylenylene group, and a pentacenylene group; and
a phenylene group, a pentalenylene group, an indenylene group, a naphthylene group, an azulenylene group, a heptalenylene group, an acenaphthylene group, a fluorenylene group, a phenalenylene group, a phenanthrenylene group, an anthracenylene group, a fluoranthenylene group, a triphenylenylene group, a pyrenylene group, a chrysenylenylene group, a naphthacenylene group, a picenylene group, a perylenylene group, and a pentacenylene group, each substituted with at least one selected from a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₃-C₁₀ cycloalkenyl group, a C₁-C₁₀ heterocycloalkyl group, a C₁-C₁₀ heterocycloalkenyl group, a C₆-C₆₀ aryl group, a C₆-C₆₀ aryloxy group, a C₆-C₆₀ arythio group, a C₁-C₆₀ heteroaryl group, a monovalent non-aromatic condensed polycyclic group, and a monovalent non-aromatic condensed heteropolycyclic group.

In Formula 201, xa and xb may be each independently an integer of 0 to 5, or 0, 1, or 2. For example, xa is 1 and xb is 0, but xa and xb are not limited thereto.

R₁₀₁ to R₁₀₈, R₁₁₁ to R₁₁₉ and R₁₂₁ to R₁₂₄ in Formulae 201 and 202 may be each independently selected from
a hydrogen, a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group (for example, a methyl group, an ethyl group, a propyl group, a butyl group, a pentyl group, a hexyl group, and so on), or a C₁-C₁₀ alkoxy group (for example, a methoxy group, an ethoxy group, a propoxy group, a butoxy group, a pentoxy group, and so on);
a C₁-C₁₀ alkyl group or a C₁-C₁₀ alkoxy group, each substituted with at least one selected from a deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, and a phosphoric acid group or a salt thereof;
a phenyl group, a naphthyl group, an anthracenyl group, a fluorenyl group, and a pyrenyl group; and
a phenyl group, a naphthyl group, an anthracenyl group, a fluorenyl group, and a pyrenyl group, each substituted with at least one selected from a deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group, and a C₁-C₁₀ alkoxy group,
but they are not limited thereto.

R₁₀₉ in Formula 201 may be selected from
a phenyl group, a naphthyl group, an anthracenyl group, and a pyridinyl group; and
a phenyl group, a naphthyl group, an anthracenyl group, and a pyridinyl group, each substituted with at least one selected from a deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₁-C₂₀ alkoxy group, a phenyl group, a naphthyl group, an anthracenyl group, and a pyridinyl group.

According to an embodiment, the compound represented by Formula 201 may be represented by Formula 201A, but is not limited thereto:

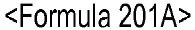

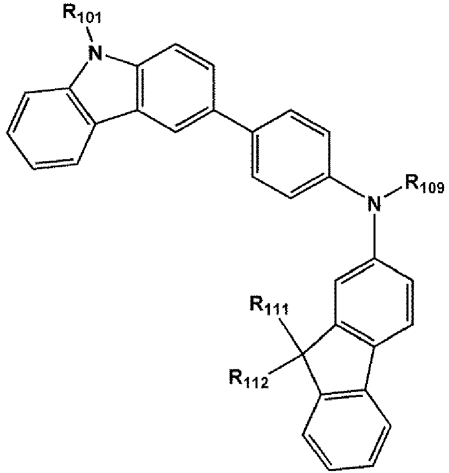

R₁₀₁, R₁₁₁, R₁₁₂, and R₁₀₉ in Formula 201A may be understood by referring to the description provided herein.

For example, the compound represented by Formula 201, and the compound represented by Formula 202 may include Compounds HT1 to HT20 illustrated below, but are not limited thereto.

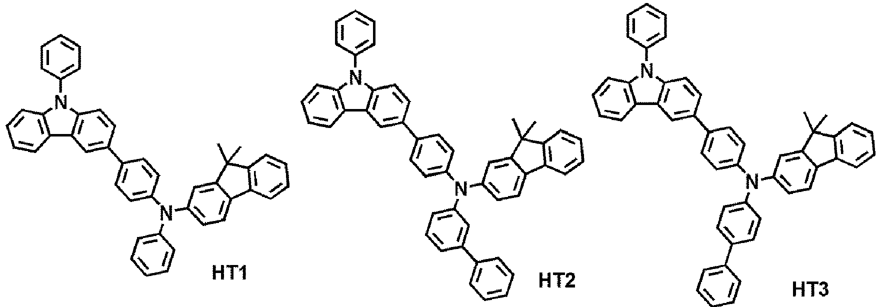

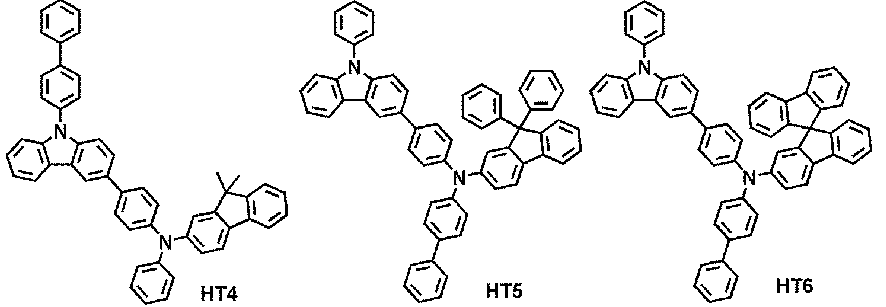

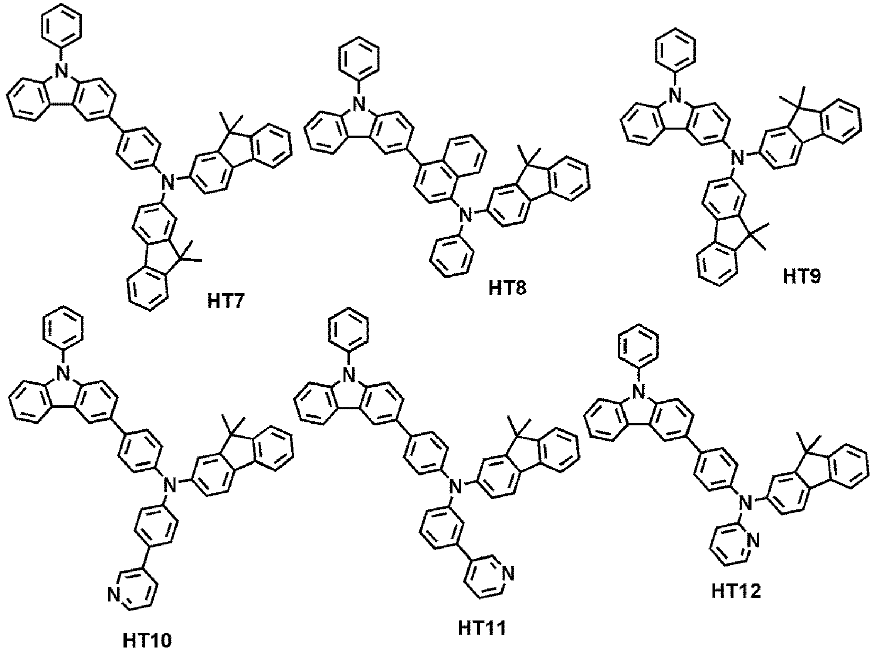

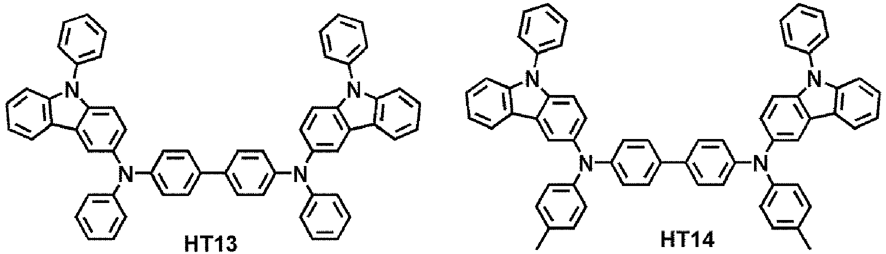

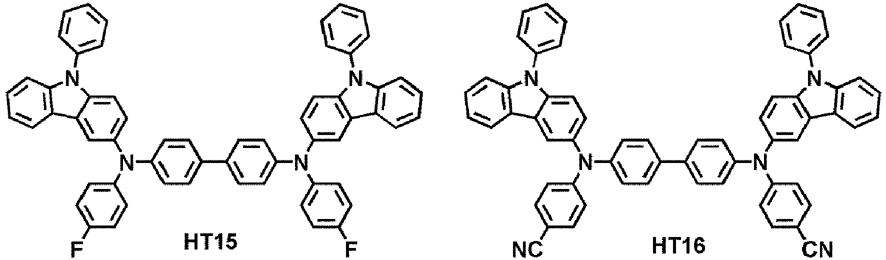

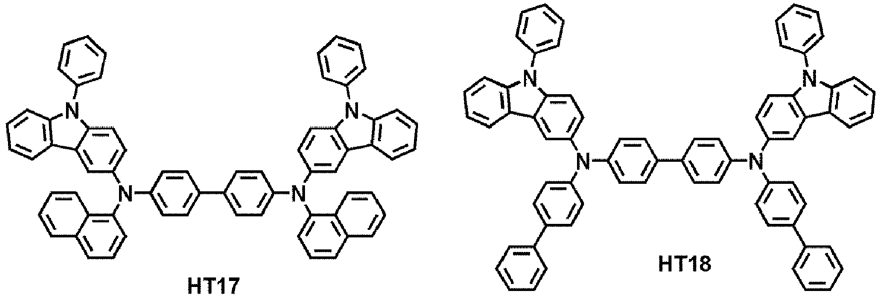

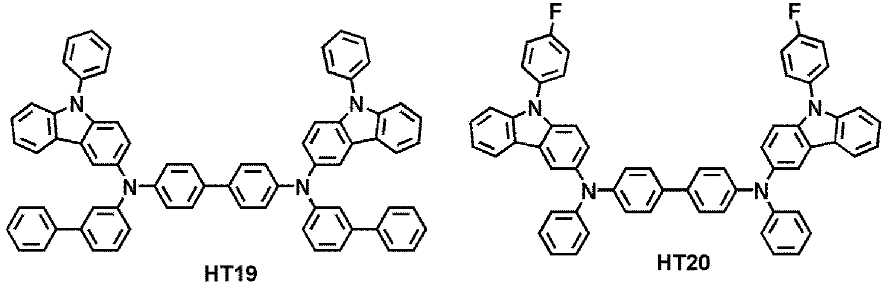

A thickness of the hole transport region may be in a range of about 10 nm to about 1,000 nm (about 100 Ǻ to about 10000 Ǻ), for example, about 10 nm to about 100 nm (about 100 Ǻ to about 1000 Ǻ). When the hole transport region includes a hole injection layer and a hole transport layer, the thickness of the hole injection layer may be in a range of about 10 nm to about 1,000 nm (about 100 Ǻ to about 10000 Ǻ), and for example, about 10 nm to about 100 nm (about 100 Ǻ to about 1000 Ǻ), and the thickness of the hole transport layer may be in a range of about 5 nm to about 200 nm (about 50 Ǻ to about 2000 Ǻ), and for example, about 10 nm to about 150 nm (about 100 Ǻ to about 1500 Ǻ). When the thicknesses of the hole transport region, the hole injection layer, and the hole transport layer are within these ranges, satisfactory hole transporting characteristics may be obtained without a substantial increase in driving voltage.

The hole transport region may further include, in addition to these materials, a charge-generation material for the improvement of conductive properties. The charge-generation material may be homogeneously or unhomogeneously dispersed in the hole transport region.

The charge-generation material may be, for example, a p-dopant. The p-dopant may be one selected from a quinone derivative, a metal oxide, and a cyano group-containing compound, but embodiments are not limited thereto. Non-limiting examples of the p-dopant are a quinone derivative, such as tetracyanoquinonedimethane (TCNQ) or 2,3,5,6-tetrafluoro-tetracyano-1,4-benzoquinonedimethane (F4-TCNQ); a metal oxide, such as a tungsten oxide or a molybdenum oxide; and a cyano group-containing compound, such as Compound HT-D1 or HP-1, but are not limited thereto.

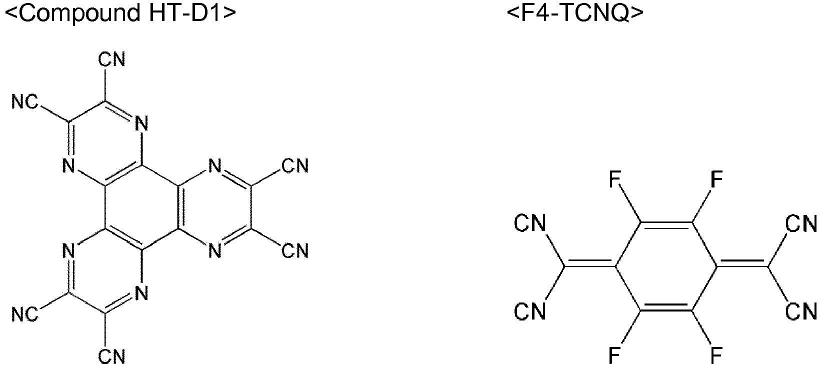

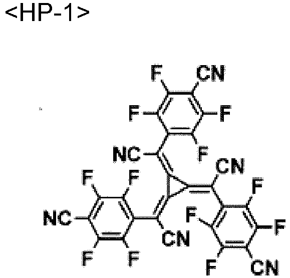

The hole transport region may include a buffer layer.

Also, the buffer layer may compensate for an optical resonance distance according to a wavelength of light emitted from the emission layer, and thus, efficiency of a formed organic light-emitting device may be improved.

Then, an emission layer (EML) may be formed on the hole transport region by vacuum deposition, spin coating, casting, LB deposition, or the like. When the emission layer is formed by vacuum deposition or spin coating, the deposition or coating conditions may be similar to those applied to form the hole injection layer although the deposition or coating conditions may vary according to the material that is used to form the emission layer.

The electron transport region may further include an electron blocking layer. The electron blocking layer may include, for example, mCP, but a material therefor is not limited thereto.

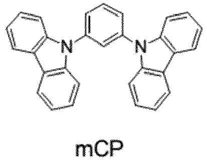

When the organic light-emitting device is a full color organic light-emitting device, the emission layer may be patterned into a red emission layer, a green emission layer, and a blue emission layer. In some embodiments, due to a stack structure including a red emission layer, a green emission layer, and/or a blue emission layer, the emission layer may emit white light.

The emission layer may include the condensed cyclic compound represented by Formulae 1B to 1 D. The emission layer may include a dopant. The dopant may be at least one selected from a phosphorescent dopant and a fluorescent dopant.

In some embodiments, the emission layer may include the condensed cyclic compound represented by Formulae 1B to 1D alone, and the condensed cyclic compound may be a TADF emitter.

In some embodiments, the emission layer may include the condensed cyclic compound represented by Formulae 1B to 1D, the condensed cyclic compound may be a TADF emitter, and the emission layer may further include a host.

For example, a host in the emission layer may include the condensed cyclic compound represented by Formulae 1B to 1 D.

A dopant in the emission layer may be a fluorescent dopant that emits light according to a fluorescent emission mechanism or a phosphorescent dopant that emits light according to a phosphorescent emission mechanism.

According to an embodiment, the dopant in the emission layer may be a phosphorescent dopant, and the phosphorescent dopant may include an organometallic compound represented by Formula 81 below: 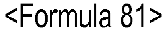 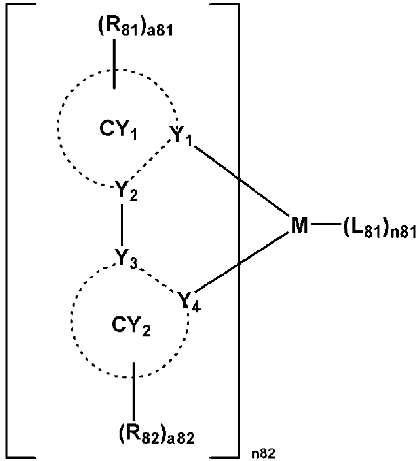 wherein in Formula 81,
M may be selected from iridium (Ir), platinum (Pt), osmium (Os), titanium (Ti), zirconium (Zr), hafnium (Hf), europium (Eu), terbium (Tb), and thulium (Tm);
Y₁ to Y₄ are each independently carbon (C) or nitrogen (N);
Y₁ and Y₂ are linked via a single bond or a double bond, and Y₃ and Y₄ are linked via a single bond or a double bond;
CY₁ and CY₂ are each independently selected from a benzene, a naphthalene, a fluorene, a spiro-fluorene, an indene, a pyrrole, a thiophene, a furan, an imidazole, a pyrazole, a thiazole, an isothiazole, an oxazole, an isoxazole, a pyridine, a pyrazine, a pyrimidine, a pyridazine, a quinoline, an isoquinoline, a benzoquinoline, a quinoxaline, a quinazolin, a carbazole, a benzoimidazole, a benzofuran, a benzothiophene, an isobenzothiophene, a benzoxazole, an isobenzoxazole, a triazole, a tetrazole, an oxadiazole, a triazine, a dibenzofuran, and a dibenzothiophene, and CY₁ and CY₂ are optionally linked to each other through a single bond or an organic linking group;
R₈₁ and R₈₂ are each independently selected from a hydrogen, a deuterium, -F,-Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, -SF₅, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic heterocondensed polycyclic group, -N(Q₁)(Q₂), -Si(Q₃)(Q₄)(Q₅), and -B(Q₆)(Q₇);
a81 and a82 are each independently an integer of 1 to 5;
n81 is an integer of 0 to 4;
n82 is 1, 2, or 3; and
L₈₁ is a monovalent organic ligand, a divalent organic ligand, or a trivalent organic ligand.
R₈₁ and R₈₂ may be understood by referring to the description provided herein in connection with R₁₁.

The phosphorescent dopant may include at least one selected from Compounds PD1 to PD78 and Flr₆, but embodiments are not limited thereto:

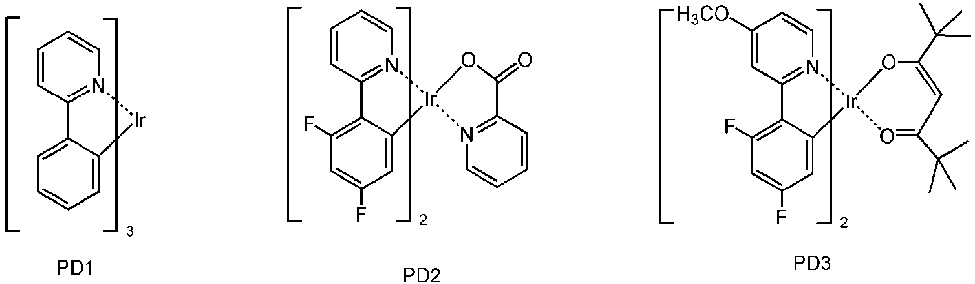

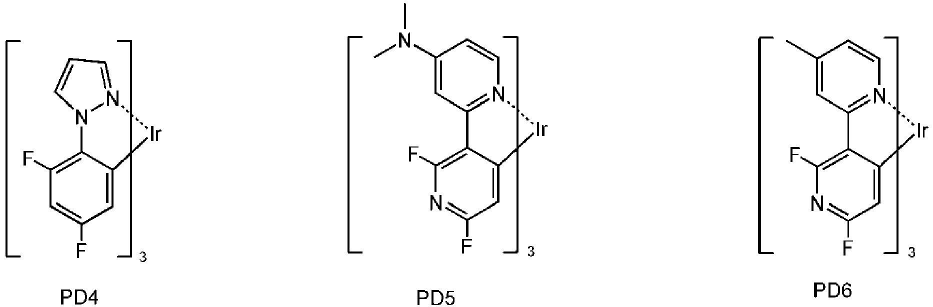

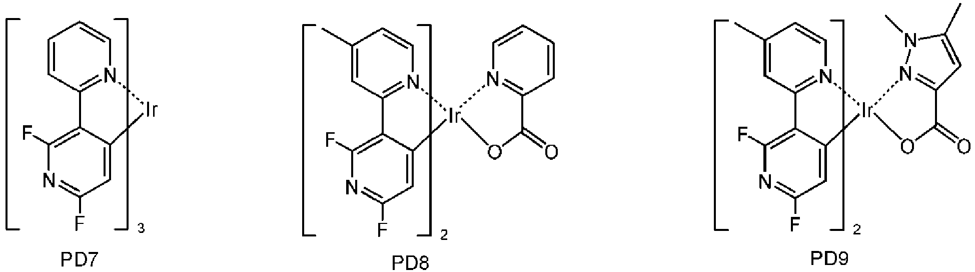

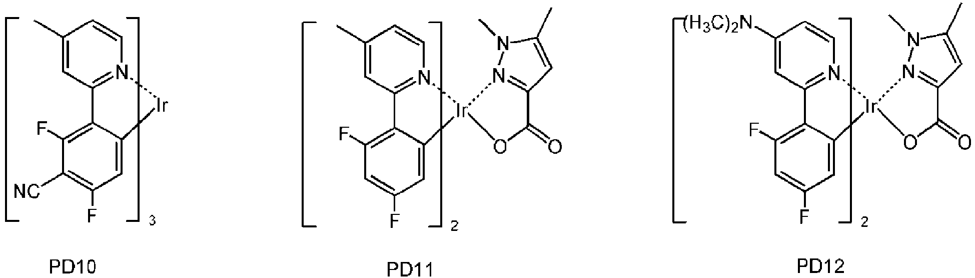

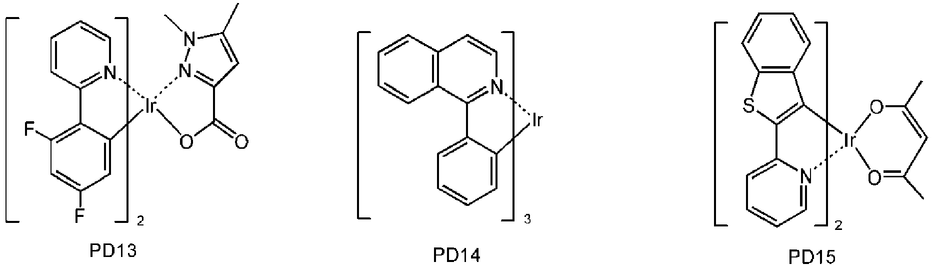

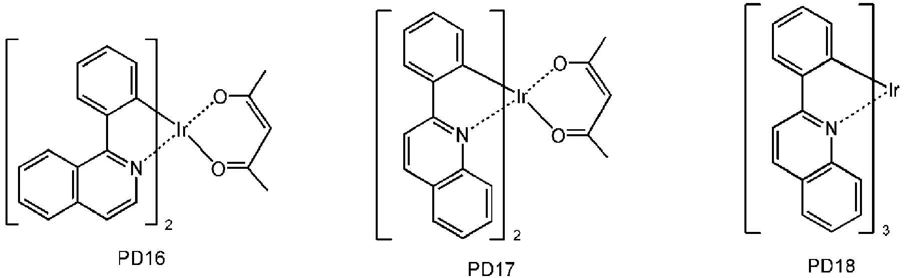

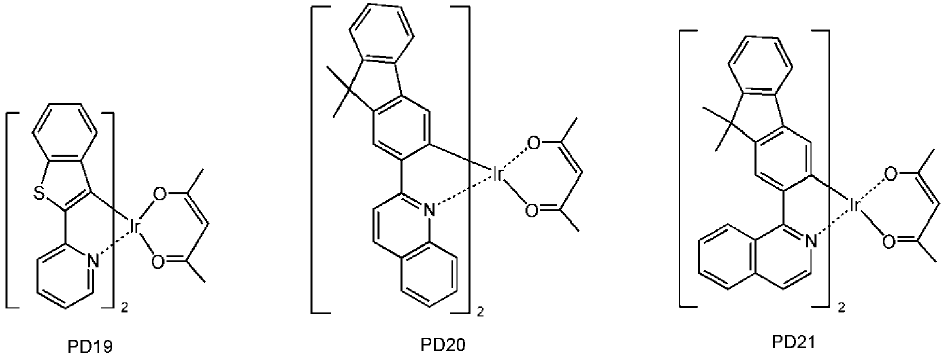

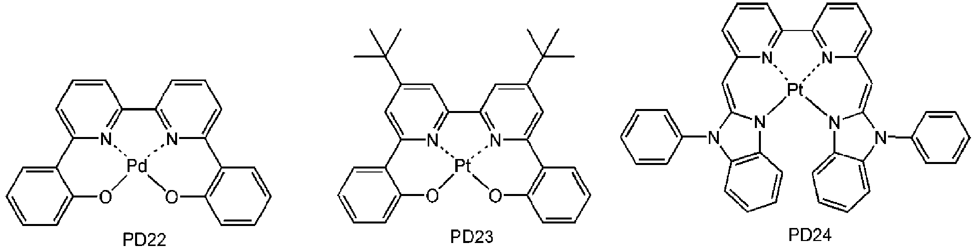

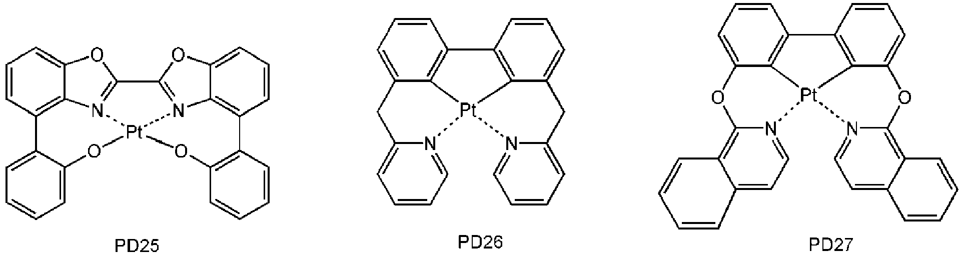

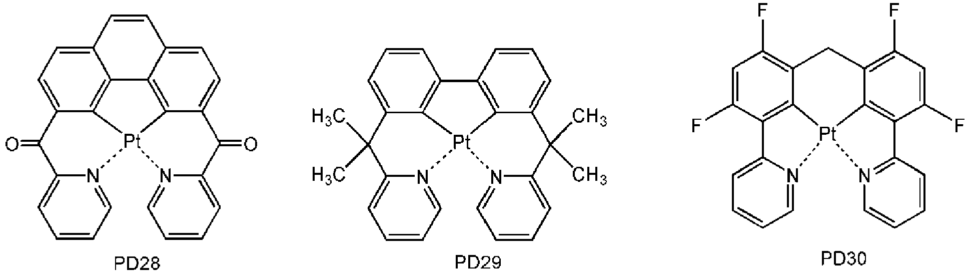

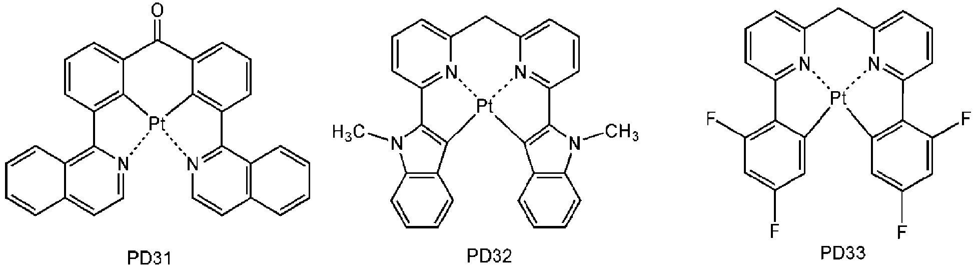

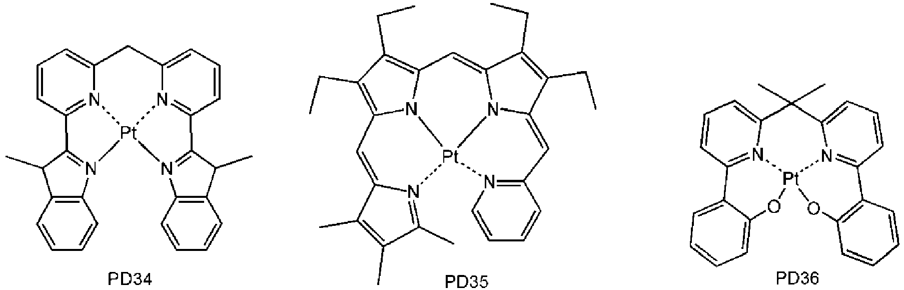

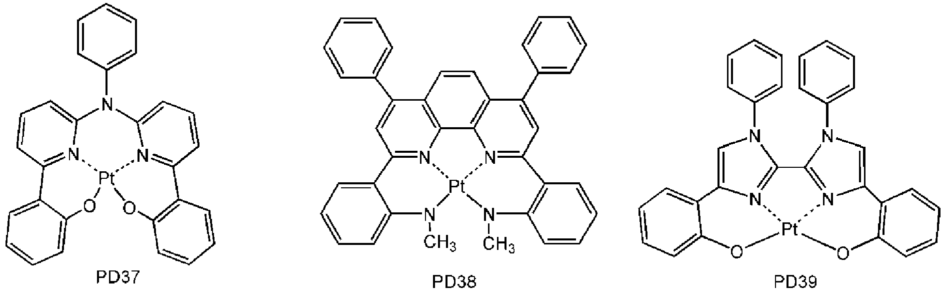

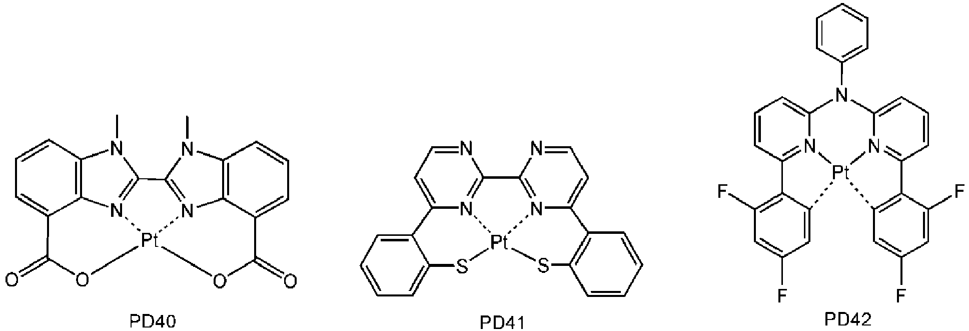

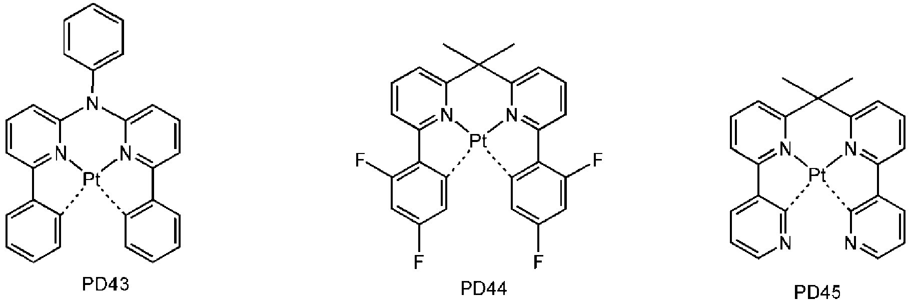

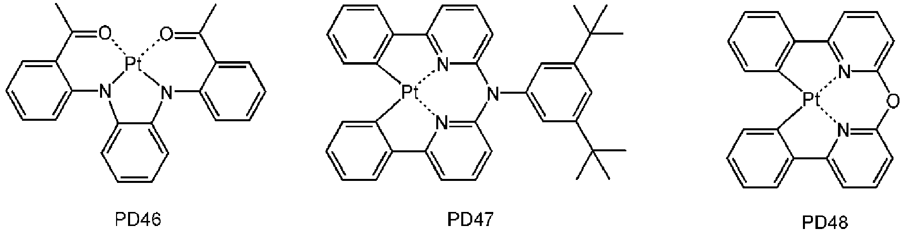

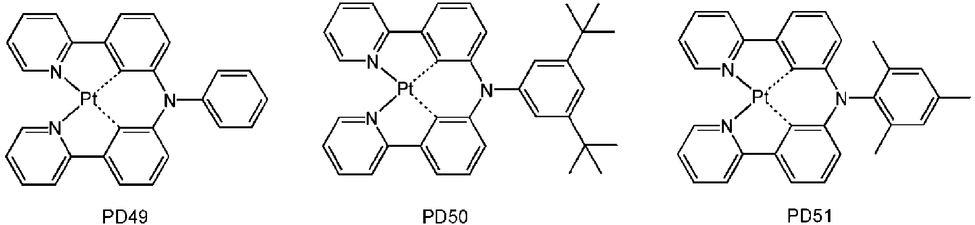

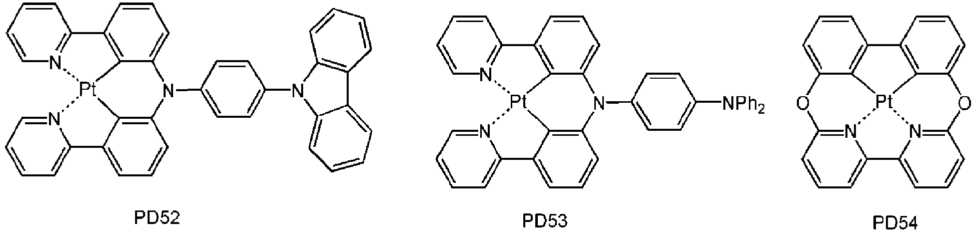

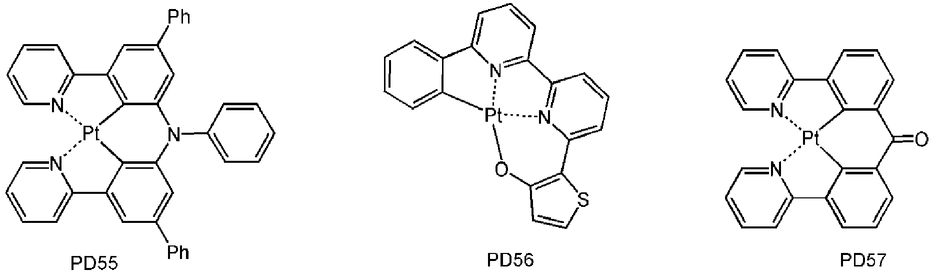

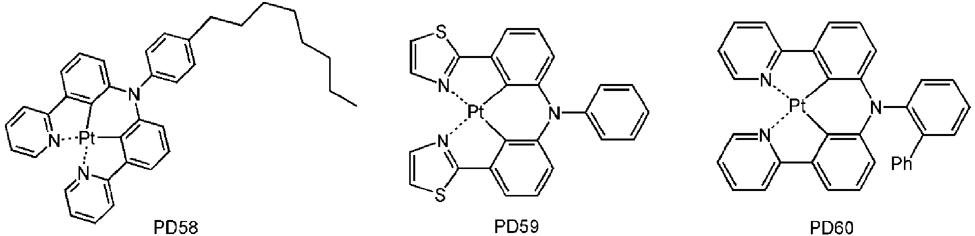

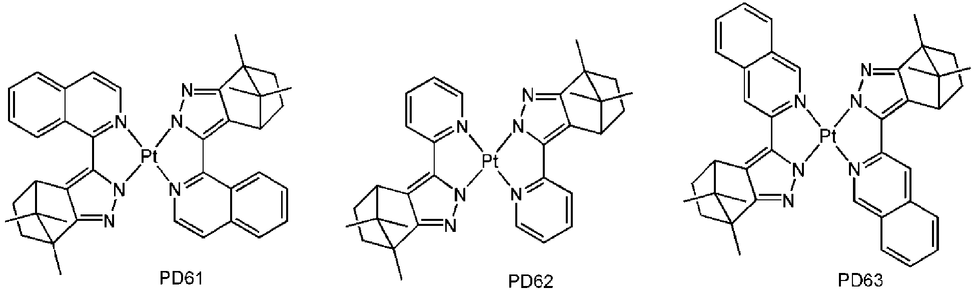

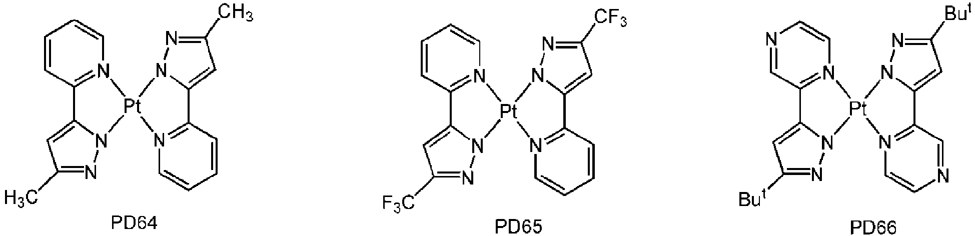

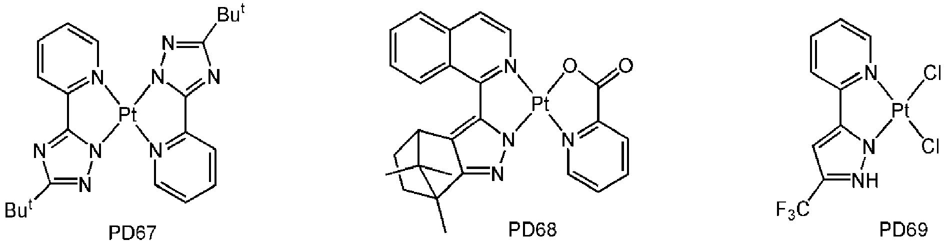

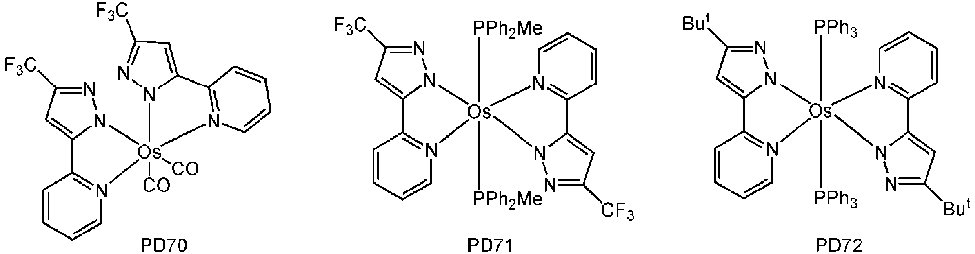

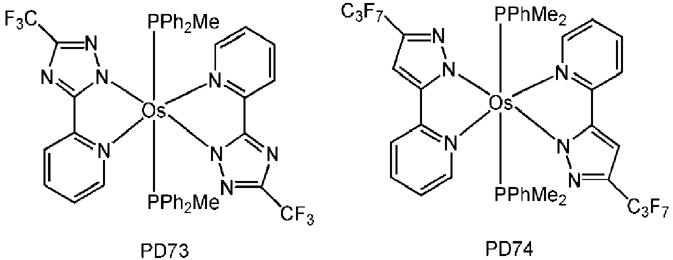

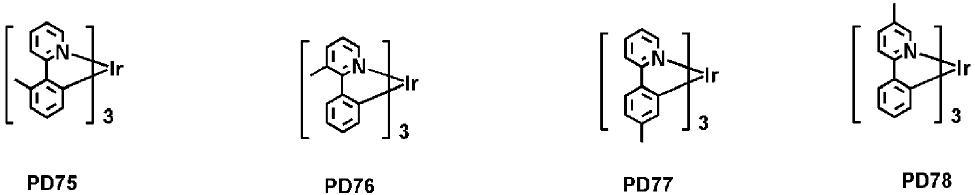

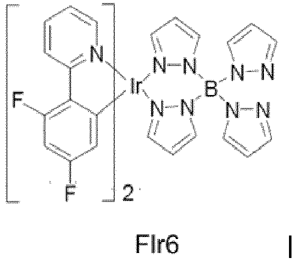

In some embodiments, the phosphorescent dopant may include PtOEP:

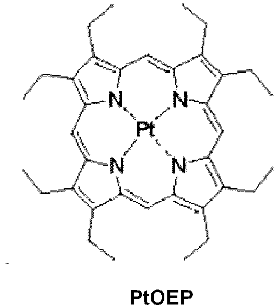

When the emission layer includes a host and a dopant, an amount of the dopant may be in a range of about 0.01 to about 20 parts by weight based on 100 parts by weight of the host, but is not limited thereto.

A thickness of the emission layer may be in a range of about 10 nm to about 100 nm (about 100 Ǻ to about 1000 Ǻ), for example, about 20 nm to about 60 nm (about 200 Ǻ to about 600 Ǻ). When the thickness of the emission layer is within this range, excellent light-emission characteristics may be obtained without a substantial increase in driving voltage.

Then, an electron transport region may be disposed on the emission layer.

The electron transport region may include at least one selected from a hole blocking layer, an electron transport layer, and an electron injection layer.

For example, the electron transport region may have a structure of hole blocking layer/electron transport layer/electron injection layer or a structure of electron transport layer/electron injection layer, but the structure of the electron transport region is not limited thereto. The electron transport layer may have a single-layered structure or a multi-layer structure including two or more different materials.

Conditions for forming the hole blocking layer, the electron transport layer, and the electron injection layer which constitute the electron transport region may be understood by referring to the conditions for forming the hole injection layer.

When the electron transport layer includes a hole blocking layer, the hole blocking layer may include, for example, at least one selected from BCP and Bphen, but embodiments are not limited thereto.

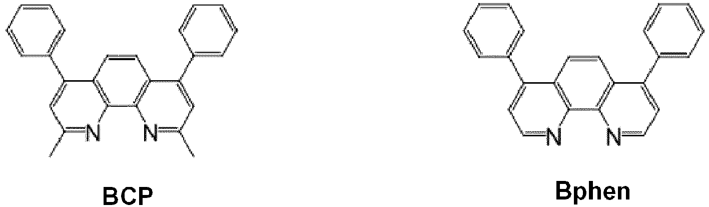

A thickness of the hole blocking layer may be in a range of about 2 nm to about 100 nm (about 20 Ǻ to about 1000 Ǻ), for example, about 3 nm to about 30 nm (about 30 Ǻ to about 300 Ǻ). When the thickness of the hole blocking layer is within these ranges, the hole blocking layer may have improved hole blocking ability without a substantial increase in driving voltage.

The electron transport layer may further include at least one selected from BCP, Bphen, Alq₃, Balq, TAZ, and NTAZ.

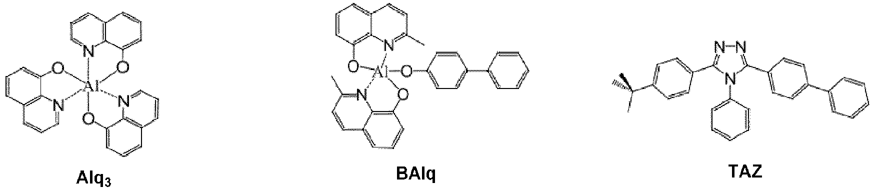

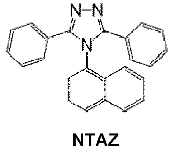

In some embodiments, the electron transport layer may include at least one selected from Compounds ET1, ET2, and ET3, but embodiments are not limited thereto:

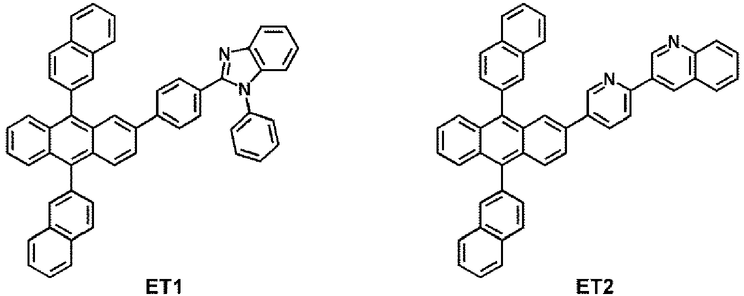

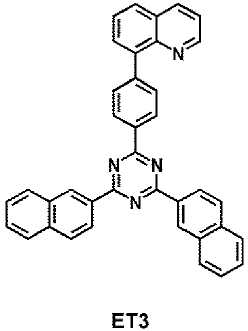

A thickness of the electron transport layer may be in a range of about 10 nm to about 100 nm (about 100 Ǻ to about 1000 Ǻ), for example, about 15 nm to about 50 nm (about 150 Ǻ to about 500 Ǻ). When the thickness of the electron transport layer is within the range described above, the electron transport layer may have satisfactory electron transport characteristics without a substantial increase in driving voltage.

Also, the electron transport layer may further include, in addition to the materials described above, a metal-containing material.

The metal-containing material may include a Li complex. The Li complex may include, for example, Compound ET-D1 (lithium quinolate, LiQ) or ET-D2.

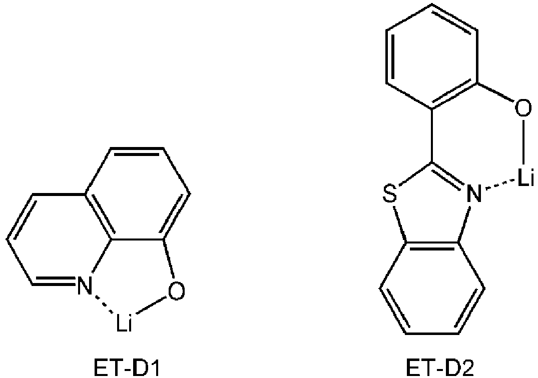

The electron transport layer may include an electron injection layer (EIL) that promotes flow of electrons from the second electrode 19 thereinto.

The electron injection layer may include at least one selected from, LiF, NaCl, CsF, Li₂O, BaO, and LiQ.

A thickness of the electron injection layer may be in a range of about 0.1 nm to about 10 nm (about 1 Ǻ to about 100 Ǻ), such as about 0.3 nm to about 9 nm (about 3 Ǻ to about 90 Ǻ). When the thickness of the electron injection layer is within the range described above, the electron injection layer may have satisfactory electron injection characteristics without a substantial increase in driving voltage.

The second electrode 19 is disposed on the organic layer 15. The second electrode 19 may be a cathode. A material for forming the second electrode 19 may be selected from metal, an alloy, an electrically conductive compound, and a combination thereof, which have a relatively low work function. For example, lithium (Li), magnesium (Mg), aluminum (Al), aluminum-lithium (Al-Li), calcium (Ca), magnesium-indium (Mg-In), or magnesium-silver (Mg-Ag) may be formed as a material for forming the second electrode 19. In some embodiments, to manufacture a top emission type light-emitting device, a transmissive electrode formed using ITO or IZO may be used as the second electrode 19.

Hereinbefore, the organic light-emitting device has been described with reference to FIG. 1, but is not limited thereto.

A C₁-C₆₀ alkyl group used herein refers to a linear or branched aliphatic hydrocarbon monovalent group having 1 to 60 carbon atoms, and detailed examples thereof are a methyl group, an ethyl group, a propyl group, an isobutyl group, a sec-butyl group, a ter-butyl group, a pentyl group, an iso-amyl group, and a hexyl group. A C₁-C₆₀ alkylene group used herein refers to a divalent group having the same structure as the C₁-C₆₀ alkyl group.

A C₁-C₆₀ alkoxy group used herein refers to a monovalent group represented by-OA₁₀₁ (wherein A₁₀₁ is the C₁-C₆₀ alkyl group), and detailed examples thereof are a methoxy group, an ethoxy group, and an isopropyloxy group.

A C₂-C₆₀ alkenyl group used herein refers to a hydrocarbon group formed by substituting at least one carbon double bond in the middle or at the terminal of the C₂-C₆₀ alkyl group, and detailed examples thereof are an ethenyl group, a prophenyl group, and a butenyl group. A C₂-C₆₀ alkenylene group used herein refers to a divalent group having the same structure as the C₂-C₆₀ alkenyl group.

A C₂-C₆₀ alkynyl group used herein refers to a hydrocarbon group formed by substituting at least one carbon trip bond in the middle or at the terminal of the C₂-C₆₀ alkyl group, and detailed examples thereof are an ethynyl group, and a propynyl group. A C₂-C₆₀ alkynylene group used herein refers to a divalent group having the same structure as the C₂-C₆₀ alkynyl group.

A C₃-C₁₀ cycloalkyl group used herein refers to a monovalent hydrocarbon monocyclic group having 3 to 10 carbon atoms, and detailed examples thereof are a cyclopropyl group, a cyclobutyl group, a cyclopentyl group, a cyclohexyl group, and a cycloheptyl group. A C₃-C₁₀ cycloalkylene group used herein refers to a divalent group having the same structure as the C₃-C₁₀ cycloalkyl group.

A C₁-C₁₀ heterocycloalkyl group used herein refers to a monovalent monocyclic group having at least one hetero atom selected from N, O, P, and S as a ring-forming atom and 1 to 10 carbon atoms, and detailed examples thereof are a tetrahydrofuranyl group, and a tetrahydrothiophenyl group. A C₁-C₁₀ heterocycloalkylene group used herein refers to a divalent group having the same structure as the C₁-C₁₀ heterocycloalkyl group.

A C₃-C₁₀ cycloalkenyl group used herein refers to a monovalent monocyclic group that has 3 to 10 carbon atoms and at least one double bond in the ring thereof and does not have aromaticity, and detailed examples thereof are a cyclopentenyl group, a cyclohexenyl group, and a cycloheptenyl group. A C₃-C₁₀ cycloalkenylene group used herein refers to a divalent group having the same structure as the C₃-C₁₀ cycloalkenyl group.

A C₁-C₁₀ heterocycloalkenyl group used herein refers to a monovalent monocyclic group that has at least one hetero atom selected from N, O, P, and S as a ring-forming atom, 1 to 10 carbon atoms, and at least one double bond in its ring. Detailed examples of the C₂-C₁₀ heterocycloalkenyl group are a 2,3-hydrofuranyl group and a 2,3-hydrothiophenyl group. A C₁-C₁₀ heterocycloalkenylene group used herein refers to a divalent group having the same structure as the C₁-C₁₀ heterocycloalkenyl group.

A C₆-C₆₀ aryl group used herein refers to a monovalent group having a carbocyclic aromatic system having 6 to 60 carbon atoms, and a C₆-C₆₀ arylene group used herein refers to a divalent group having a carbocyclic aromatic system having 6 to 60 carbon atoms. Detailed examples of the C₆-C₆₀ aryl group are a phenyl group, a naphthyl group, an anthracenyl group, a phenanthrenyl group, a pyrenyl group, and a chrysenyl group. When the C₆-C₆₀ aryl group and the C₆-C₆₀ arylene group each include two or more rings, the rings may be fused to each other.

A C₁-C₆₀ heteroaryl group used herein refers to a monovalent group having a carbocyclic aromatic system that has at least one hetero atom selected from N, O, P, and S as a ring-forming atom, and 1 to 60 carbon atoms. A C₁-C₆₀ heteroarylene group used herein refers to a divalent group having a carbocyclic aromatic system that has at least one hetero atom selected from N, O, P, and S as a ring-forming atom, and 1 to 60 carbon atoms. Examples of the C₁-C₆₀ heteroaryl group are a pyridinyl group, a pyrimidinyl group, a pyrazinyl group, a pyridazinyl group, a triazinyl group, a quinolinyl group, and an isoquinolinyl group. When the C₁-C₆₀ heteroaryl group and the C₁-C₆₀ heteroarylene group each include two or more rings, the rings may be fused to each other.

A C₆-C₆₀ aryloxy group used herein indicates -OA₁₀₂ (wherein A₁₀₂ is the C₆-C₆₀ aryl group), and a C₆-C₆₀ arylthio group used herein indicates -SA₁₀₃ (wherein A₁₀₃ is the C₆-C₆₀ aryl group).

A monovalent non-aromatic condensed polycyclic group used herein refers to a monovalent group that has two or more rings condensed to each other, only carbon atoms (for example, the number of carbon atoms may be in a range of 8 to 60) as a ring forming atom, and non-aromacity in the entire molecular structure. An example of the monovalent non-aromatic condensed polycyclic group is a fluorenyl group. A divalent non-aromatic condensed polycyclic group used herein refers to a divalent group having the same structure as the monovalent non-aromatic condensed polycyclic group.

A monovalent non-aromatic condensed heteropolycyclic group used herein refers to a monovalent group that has two or more rings condensed to each other, has a heteroatom selected from N, O P, and S, other than carbon atoms (for example, the number of carbon atoms may be in a range of 1 to 60), as a ring forming atom, and has non-aromacity in the entire molecular structure. An example of the monovalent non-aromatic condensed heteropolycyclic group is a carbazolyl group. A divalent non-aromatic condensed heteropolycyclic group used herein refers to a divalent group having the same structure as the monovalent non-aromatic condensed heteropolycyclic group.

At least one of substituents of the substituted C₃-C₁₀ cycloalkylene group, substituted C₁-C₁₀ heterocycloalkylene group, substituted C₃-C₁₀ cycloalkenylene group, substituted C₁-C₁₀ heterocycloalkenylene group, substituted C₆-C₆₀ arylene group, substituted C₁-C₆₀ heteroarylene group, substituted a divalent non-aromatic condensed polycyclic group, substituted a divalent non-aromatic condensed heteropolycyclic group, substituted C₁-C₆₀ alkyl group, substituted C₂-C₆₀ alkenyl group, substituted C₂-C₆₀ alkynyl group, substituted C₁-C₆₀ alkoxy group, substituted C₃-C₁₀ cycloalkyl group, substituted C₁-C₁₀ heterocycloalkyl group, substituted C₃-C₁₀ cycloalkenyl group, substituted C₁-C₁₀ heterocycloalkenyl group, substituted C₆-C₆₀ aryl group, substituted C₆-C₆₀ aryloxy group, substituted C₆-C₆₀ arylthio group, substituted C₁-C₆₀ heteroaryl group, substituted monovalent non-aromatic condensed polycyclic group, and substituted monovalent non-aromatic condensed heteropolycyclic group used herein may be selected from a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, provided that the monovalent non-aromatic condensed heteropolycyclic group is not a carbazolyl group and -Si(Q₃₁)(Q₃₂)(Q₃₃),
wherein Q₁ to Q₃, Q₁₁ to Q₁₃, Q₂₁ to Q₂₃, and Q₃₁ to Q₃₃ may be each independently selected from a hydrogen, a C₁-C₆₀ alkyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₁-C₆₀ heteroaryl group, a monovalent non-aromatic condensed polycyclic group, and a monovalent non-aromatic condensed heteropolycyclic group, provided that the monovalent non-aromatic condensed heteropolycyclic group is not a carbazolyl group.

The "biphenyl group" used herein refers to "a phenyl group substituted with a phenyl group."

Hereinafter, a compound and an organic light-emitting device according to embodiments are described in detail with reference to Synthesis Example and Examples. However, the organic light-emitting device is not limited thereto. The wording "B was used instead of A" used in describing Synthesis Examples means that an amount of A used was identical to an amount of B used, in terms of a molar equivalent.

### [Example]

### Synthesis Example 1: Synthesis of Compound 28 (Reference example)

10 g (52 mmol) of 3-cyanocarbazole, 14.2 g of 2,8-diomodibenzo[b,d]furan, 8.8 g of Cul, 15.9 g of K₂CO₃, and 2.5 g of 1,10-phenanthroline were placed in a 250 mL 2-neck round-bottom flask, and then, 150 mL of DMF was added thereto. Thereafter, the mixture was stirred at a temperature of 150°C for 28 hours, and then, the reaction product was cooled and MeOH was added thereto to produce a solid, which was then filtered therefrom. A result obtained therefrom was mixed with 1 L of chloroform, and dissolved by heating, and then, filtered through a celite pad. Then, the residual solution obtained therefrom was concentrated under reduced pressure. The obtained result was recrystallized under a MC/Aceton condition to produce Compound 28 (12.3 g, yield of 43%). 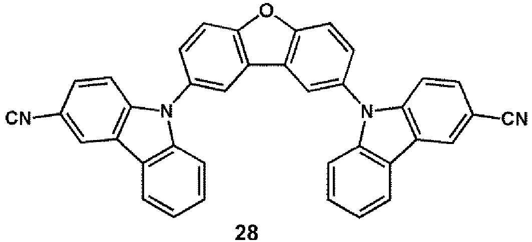 **Calc.: 548.16, found [M+H⁺] : 549.16**

### Synthesis Example 2: Synthesis of Compound 4

Compound 4 (10.3 g, yield of 61.6%) was prepared in the same manner as in Synthesis Example 1, except that 2,6-dichloropyridine was used instead of 2,8-dibromodibenzo[b,d]furan. 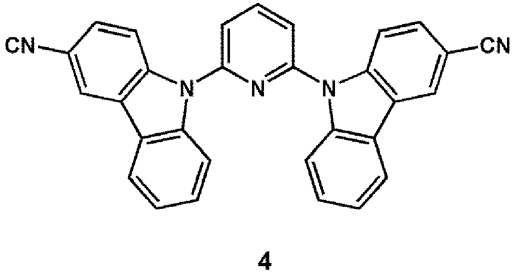 **Calc.: 459.15, found [M+H⁺]**: **460.15**

### Synthesis Example 3: Synthesis of Compound 3

Compound 3 (10.9 g, yield of 65.3%) was prepared in the same manner as in Synthesis Example 1, except that 3,6-dicyanocarbazole was used instead of 3-cyanocarbazole, and 9-(3-bromophenyl)-9H-carbazole was used instead of 2,8-dibromodibenzo[b,d]furan. 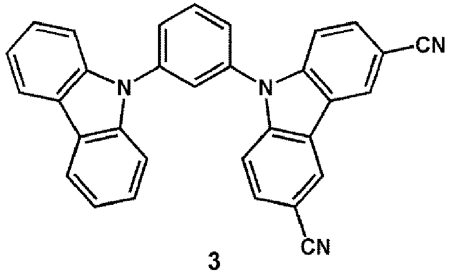 **Calc.: 458.15, found [M+H⁺]**: **459.16**

### Synthesis Example 4: Synthesis of Compound 6

Compound 6 (13.2 g, yield of 53.6%) was prepared in the same manner as in Synthesis Example 1, except that 3,3'-dibromo-1,1'-biphenyl was used instead of 2,8-dibromodibenzo[b,d]furan. 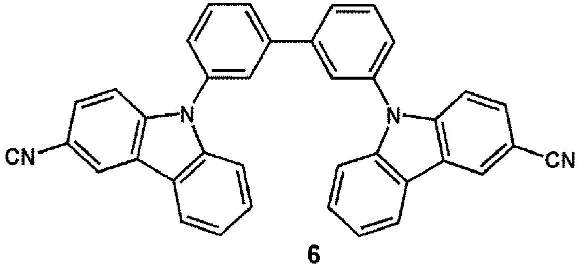 **Calc.: 534.18, found [M+H⁺]**: **535.18**

### Synthesis Example 5: Synthesis of Compound 29 (Reference example)

Compound 29 (11.7 g, yield of 64.4%) was prepared in the same manner as in Synthesis Example 1, except that 3,6-dicyanocarbazole was used instead of 3-cyanocarbazole, and 9-(8-bromodibenzo[b,d]furan-2-yl)-9H-carbazole) was used instead of 2,8-dibromodibenzo[b,d]furan. 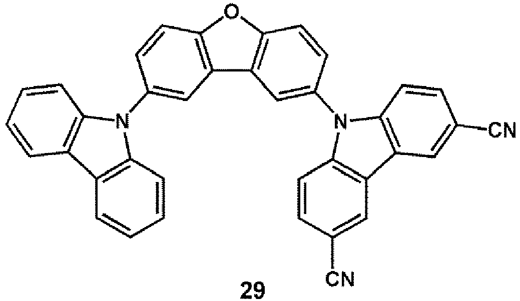 **Calc.: 548.16, found [M+H⁺]**: **549.16**

### Synthesis Example 6: Synthesis of Compound 1

Compound 1 (12.5 g, yield of 60.8%) was prepared in the same manner as in Synthesis Example 1, except that 3,6-dicyanocarbazole was used instead of 3-cyanocarbazole, and 9-(3'-bromo-[1,1'-biphenyl]-3-yl)-9H-carbazole was used instead of 2,8-dibromodibenzo[b,d]furan. 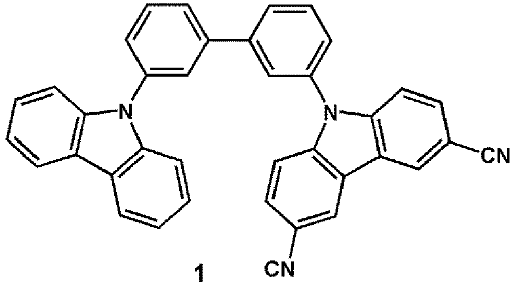 **Calc.: 534.18, found [M+H⁺]**: **535.17**

### Synthesis Example 7: Synthesis of Compound 5

Compound 5 (9.8 g, yield of 58.6%) was prepared in the same manner as in Synthesis Example 1, except that 3,6-dicyanocarbazole was used instead of 3-cyanocarbazole, and 9-(6-chloropyridin-2-yl)-9H-carbazole was used instead of 2,8-dibromodibenzo[b,d]furan. 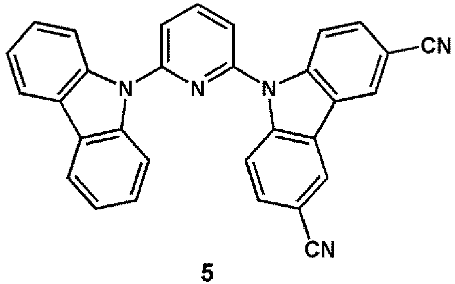 **Calc.: 459.15, found [M+H⁺]**: **460.16**

### Synthesis Example 8: Synthesis of Compound 2

Compound 2 (10.5 g, yield of 57.8%) was prepared in the same manner as in Synthesis Example 1, except that 1,3-dichloroa benzene was used instead of 2,8-dibromodibenzo[b,d]furan. 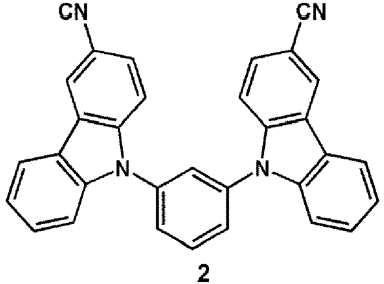 **Calc.: 458.15, found [M+H⁺]**: **459.16**

### Synthesis Example 9: Synthesis of Compound 24 (Reference example)

Compound 24 (10.3 g, yield of 73.4%) was prepared in the same manner as in Synthesis Example 1, except that 2,8-diomodibenzo[b,d]thiophene was used instead of 2,8-diomodibenzo[b,d]furan. 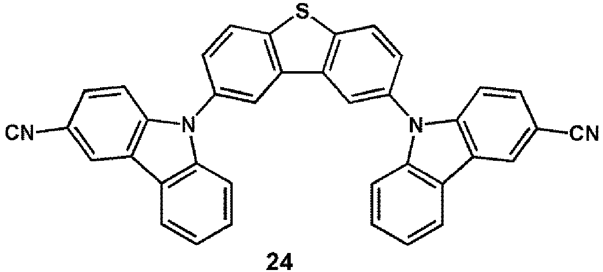 **Calc.: 564.14, found [M+H⁺****]**: **565.14**

### Evaluation Example 1: Evaluation of HOMO, LUMO and triplet(T1) energy level

HOMO, LUMO and T₁ energy levels of Compounds 4, 28, 6, 5, 3 and 2 were evaluated by using methods shown in Table 2. Results thereof are shown in Table 3.

**[Table 2]**

| | |
|---|---|
| HOMO energy level evaluation method | A potential (V)-current (A) graph of each compound was obtained by using cyclic voltammetry (CV) (electrolyte: 0.1 M Bu₄NClO₄ / solvent: CH₂Cl₂ / electrode: 3 electrode system (working electrode: GC, reference electrode: Ag/AgCl, auxiliary electrode: Pt)), and then, from reduction onset of the graph, a HOMO energy level of the compound was calculated. |
| LUMO energy level evaluation method | Each compound was diluted at a concentration of 1x10⁻⁵ M in CHCl₃, and an UV absorption spectrum thereof was measured at room temperature by using a Shimadzu UV-350 spectrometer, and a LUMO energy level thereof was calculated by using an optical band gap (Eg) from an edge of the absorption spectrum. |
| T1 energy level evaluation method | A mixture (each compound was dissolved in an amount of 1 mg in 3cc of toluene) of toluene and each compound was loaded into a quartz cell, and then, the resultant quartz cell was loaded into liquid nitrogen (77K) and a photoluminescence spectrum thereof was measured by using a device for measuring photoluminescence, and the obtained spectrum was compared with a photoluminescence spectrum measured at room temperature, and peaks observed only at low temperature were analyzed to calculate T₁ energy levels. |

**[Table 3]**

| Compound No. | HOMO(eV) (found) | LUMO(eV) (found) | T₁ energy level(eV) |
|---|---|---|---|
| Compound 4 | -6.00 | -2.43 | 3.04 |
| Compound 28 | -5.77 | -2.28 | 3.02 |
| Compound 6 | -5.89 | -2.36 | 2.82 |
| Compound 5 | -5.74 | -2.21 | 3.02 |
| Compound 3 | -5.71 | -2.15 | 3.02 |
| Compound 2 | -5.91 | -2.36 | 3.04 |

From Table 3, it may be seen that Compounds 4, 28, 6, 5, 3, and 2 are suitable for use as a material for an organic light-emitting device.

### Evaluation Example 2: Thermal characteristics evaluation

Each of Compounds 4, 28, 6, 5, 3, 2, and A was subjected to thermal analysis (N₂ atmosphere, temperature range: room temperature to 800°C (10°C /min)-TGA, room temperature to 400°C -DSC, Pan Type : Pt Pan in disposable Al Pan(TGA), disposable Al pan(DSC)) using thermo gravimetric analysis (TGA) and differential scanning calorimetry (DSC), and obtained results are shown in Table 4 below. Referring to Table 4, it may be seen that Compounds 4, 28, 6, 5, 3, and 2 may have higher thermal stability than Compound A.

**[Table 4]**

| Compound No. | Tg(°C) |
|---|---|
| 4 | 126 |
| 28 | 174 |
| 6 | 134 |
| 5 | 130 |
| 2 | 121 |
| 3 | 130 |
| Compound A | 72 |

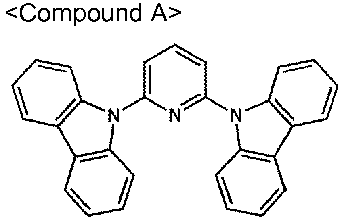

### Evaluation Example 3:Heat resistance of thin film

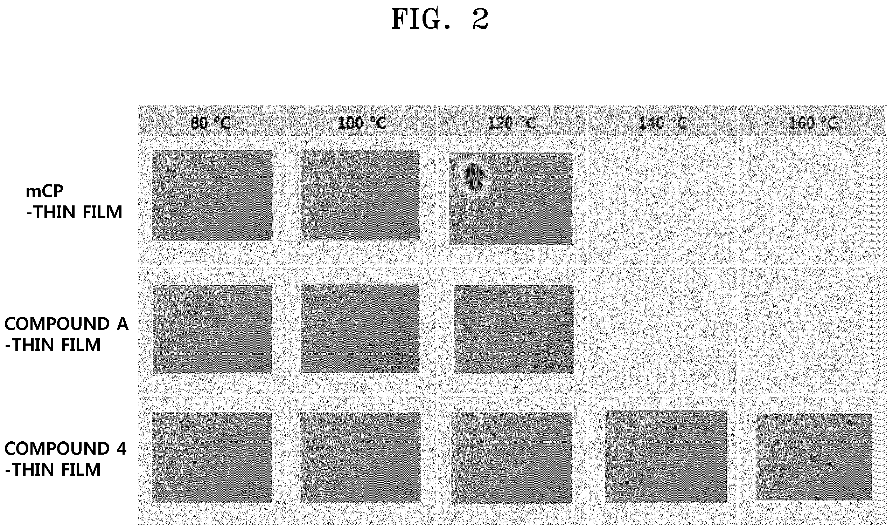

Each of mCP, Compound A, and Compound 4 was vacuum deposited on a glass substrate to form an mCP-thin film having a thickness of 50 nm (500Å), a Compound A-thin film having a thickness of 50 nm (500Å), and a Compound 4-thin film having a thickness of 50 nm (500Å). The mCP-thin film, the Compound A-thin film, and the Compound 4-thin film on the glass substrate were mounted on a hot-plate under atmospheric condition (relative humidity of 50%) at a temperature of 80°C, and then heated at incremental rates of 20°C every 10 minutes. In each step, images of surfaces of these thin films were captured, and FIG. 2 shows the captured images thereof.

Referring to FIG. 2, it may be seen that when the temperature of the hot-plate is 120°C, the surface of the mCP-thin film has black spots, and the surface of the Compound A-thin film is crystallized. However, in the case of the Compound 4-thin film, even at a temperature of 140°C, black spots and crystallization did not occur.

From these results, it may be seen that the Compound 4-thin film has excellent thin film heat resistance.

### Example 1

A glass substrate with a 150 nm (1500 A)-thick ITO (Indium tin oxide) electrode (first electrode, anode) formed thereon was washed with distilled water and ultrasonic waves. When the washing with distilled water was completed, sonification washing was performed using a solvent, such as isopropyl alcohol, acetone, or methanol. The result was dried and then transferred to a plasma washer, and the resultant substrate was washed with oxygen plasma for 5 minutes and then, transferred to a vacuum depositing device.

Compound HT3 and Compound HP-1 were co-deposited on the ITO electrode on the glass substrate to form a hole injection layer having a thickness of 10 nm (100Ǻ), and then, Compound HT3 was deposited on the hole injection layer to form a hole transport layer having a thickness of 130 nm (1300Ǻ), and mCP was deposited on the hole transport layer to form an electron blocking layer having a thickness of 15 nm (150Ǻ), thereby completing the manufacture of a hole transport region.

Compound 4 (host) and Flr6 (dopant, 10 wt%) were co-deposited on the hole transport region to form an emission layer having a thickness of 30 nm (300Ǻ).

BCP was vacuum deposited on the emission layer to form a hole blocking layer having a thickness of 10 nm (100Ǻ), Compound ET3 and Liq were vacuum deposited on the hole blocking layer to form an electron transport layer having a thickness of 25 nm (250Ǻ). Then, Liq was deposited on the electron transport layer to form an electron injection layer having a thickness of 0.5 nm (5Ǻ), and Al second electrode(cathode) having a thickness of 100 nm (1000Ǻ) was formed on the electron injection layer, thereby completing the manufacture of an organic light-emitting device.

### Examples 2 to 4 and Comparative Examples 1 to 5

Organic light-emitting devices were manufactured in the same manner as in Example 1, except that in forming an emission layer, as a host, the compounds shown in Table 5 were used instead of Compound 4.

### Evaluation Example 4: Evaluation on characteristics of organic light-emitting devices.

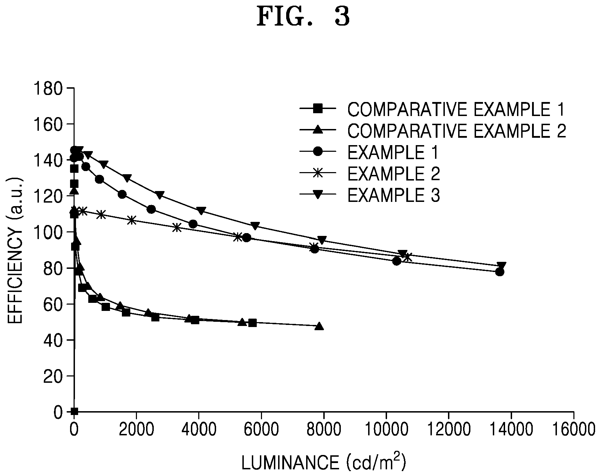

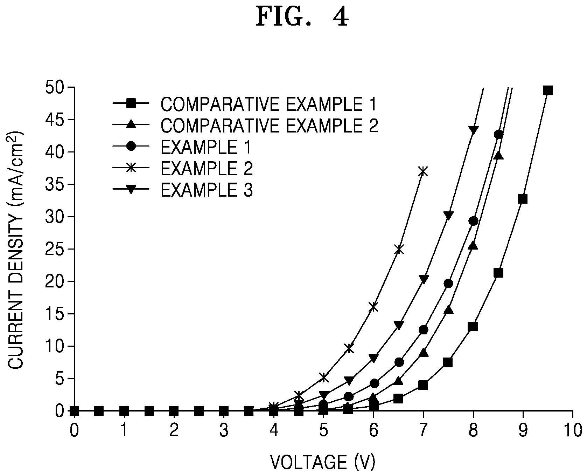

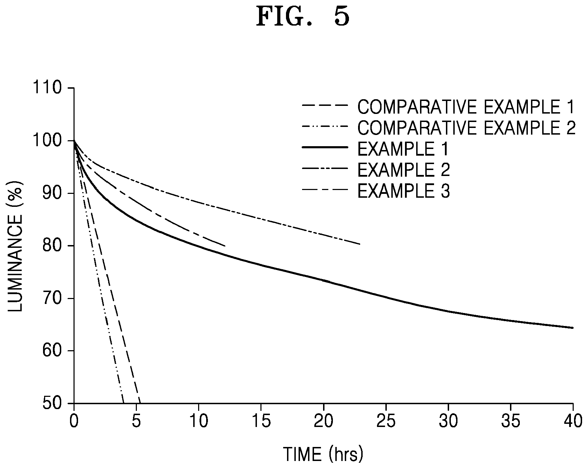

The driving voltage, current density, efficiency, power efficiency, quantum efficiency, and lifespan of the organic light-emitting devices of Examples 1 to 3 and Comparative Examples 1 to 5 were measured by using a current-voltage meter (Keithley 2400) and a luminance meter (Minolta Cs-1000A), and results thereof are shown in Table 5. FIGS. 3, 4, and 5 show a luminance-efficiency graph, a voltage-current density graph, and a time-luminance graph of the organic light-emitting devices of Examples 1 to 3 and Comparative Examples 1 and 2, respectively

T₉₅(at 500 cd/m²) in Table 5 indicates an amount of time that lapsed when 100% of the initial luminance was decreased to 95%.

**[Table 5]**

| | Host | Driving Voltage Voltage (V) | Current density (mA/cm²) | Efficiency (cd/A) | Power (Im/W) | Quantum efficiency (%) | T₉₅(hr) |
|---|---|---|---|---|---|---|---|
| Example 1 | Compound 4 | 5.12 | 1.29 | 39.25 | 24.16 | 19.5 | 0.7 |
| Example 2 | Compound 28 | 4.19 | 1.36 | 36.96 | 27.80 | 17.0 | 2.2 |
| Example 3 | Compound 6 | 4.55 | 1.27 | 39.43 | 27.26 | 20.3 | 1.3 |
| Example 4 | Compound 24 | 4.53 | 1.25 | 39.73 | 27.5 | 21.1 | 1.96 |
| Comparative Example 1 | Compound A | 6.85 | 3.32 | 15.27 | 7.03 | 8.9 | 0.5 |
| Comparative Example 2 | Compound B | 6.09 | 2.64 | 19.20 | 9.93 | 10.2 | 0.4 |
| Comparative Example 3 | Compound C | 5.22 | 1.89 | 13.83 | 8.32 | 7.4 | 0.2 |
| Comparative Example 4 | Compound D | 5.02 | 2.45 | 11.44 | 7.16 | 6.1 | 0.3 |
| Comparative Example 5 | Compound E | 5.92 | 1.29 | 22.51 | 11.90 | 12.0 | 0.2 |

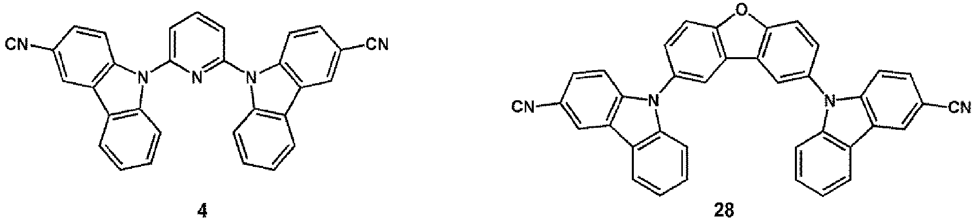

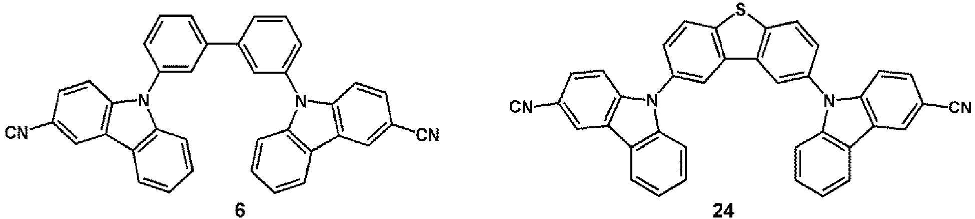

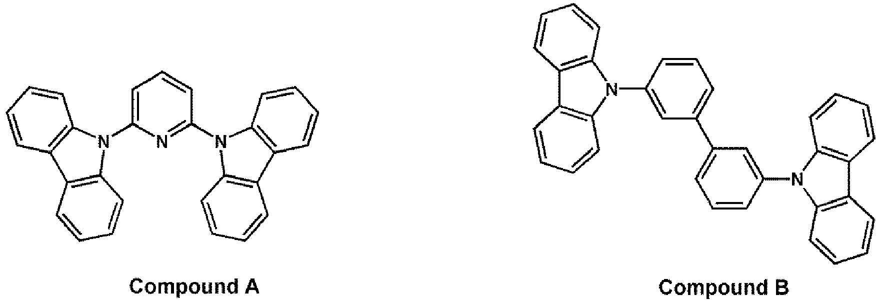

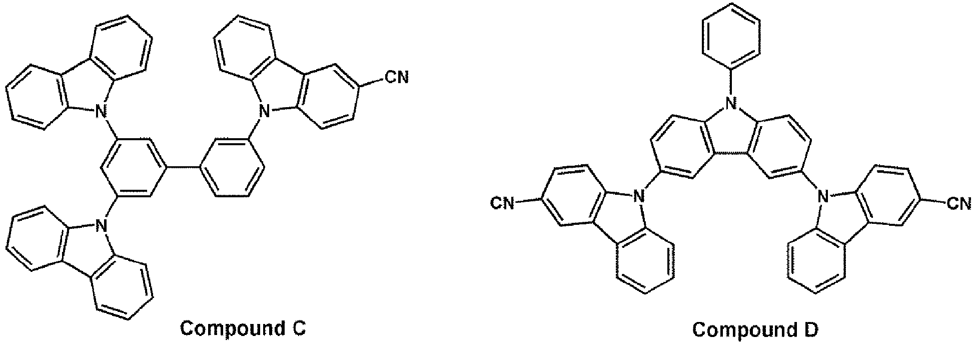

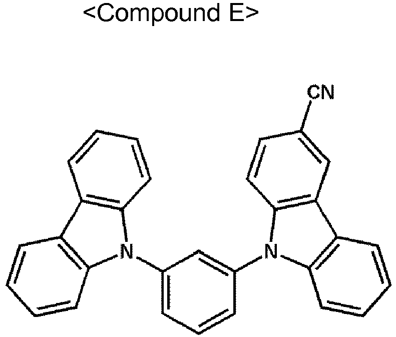

From Table 5 and FIGS. 3 to 5, it may be seen that the organic light-emitting devices of Examples 1 to 3 have a lower driving voltage, a higher current density, a higher efficiency, a higher power, a higher luminance, a high quantum luminescent efficiency, and a longer lifespan than the organic light-emitting device of Comparative Examples 1 to 5.

Although not limited to a particular theory, in the case of Comparative Examples 3 and 4 respectively including Compounds C and D, due to low T₁ energy levels of Compounds C and D, energy transition to a dopant in an emission layer is not smoothly carried out. Accordingly, it may be seen that the organic light-emitting devices of Comparative Examples 3 and 4 have lower quantum efficiency than the organic light-emitting devices of Examples 1 to 3.

The condensed cyclic compound according to embodiments has excellent electric characteristics and thermal stability. Accordingly, an organic light-emitting device using the condensed cyclic compound may have low driving voltage, high efficiency, high luminance, and a long lifespan.

It should be understood that exemplary embodiments described herein should be considered in a descriptive sense only and not for purposes of limitation. Descriptions of features or aspects within each exemplary embodiment should typically be considered as available for other similar features or aspects in other exemplary embodiments.

While one or more exemplary embodiments have been described with reference to the figures, it will be understood by those of ordinary skill in the art that various changes in form and details may be made therein without departing from the scope as defined by the following claims.

## Claims

1. A condensed cyclic compound represented by one of Formulae 1B to 1D: 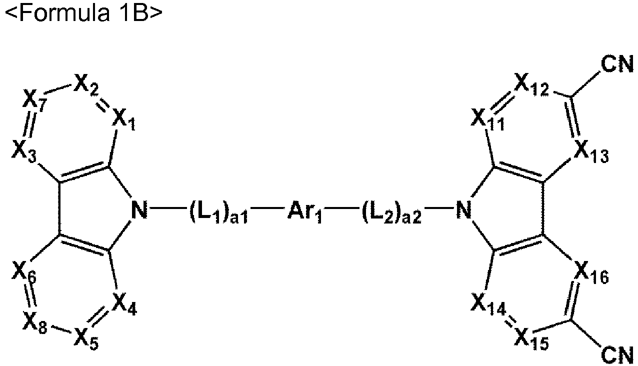 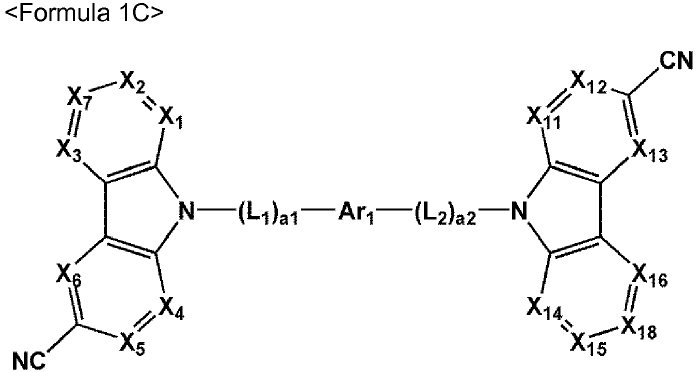 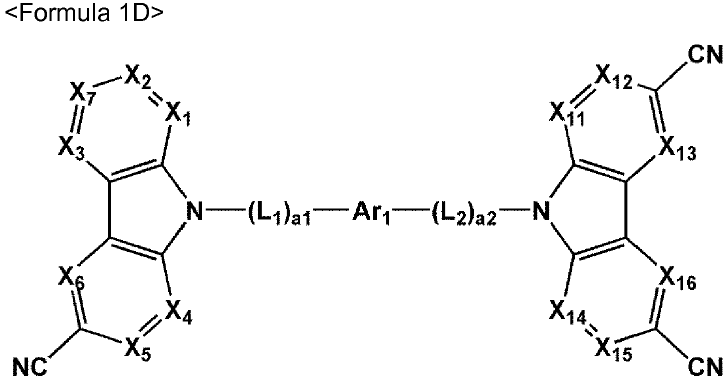 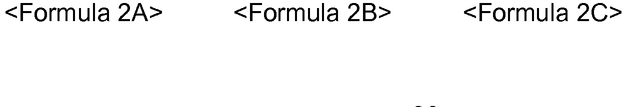 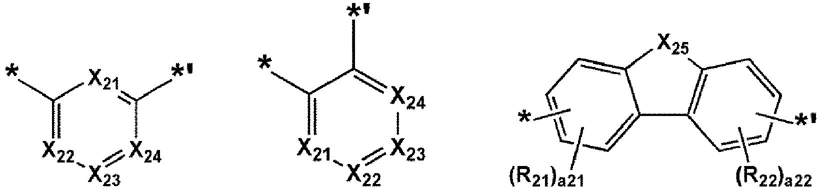 wherein in Formulae 1B to 1D and 2A to 2C,
X₁ is N or C(R₁), X₂ is N or C(R₂), X₃ is N or C(R₃), X₄ is N or C(R₄), X₅ is N or C(R₅), X₆ is N or C(R₆), X₇ is N or C(R₇), X₈ is N or C(R₈), X₁₁ is N or C(R₁₁), X₁₂ is N or C(R₁₂), X₁₃ is N or C(R₁₃), X₁₄ is N or C(R₁₄), X₁₅ is N or C(R₁₅), X₁₆ is N or C(R₁₆), X₁₇ is N or C(R₁₇), and X₁₈ is N or C(R₁₈),
R₁ to R₈ and R₁₁ to R₁₈ are each independently selected from a hydrogen, a deuterium, -F, -Cl, -Br, -I, a hydroxyl group, a nitro group, an amino group, an amidino group, a hydrazine group, a hydrazone group, a carboxylic acid group or a salt thereof, a sulfonic acid group or a salt thereof, a phosphoric acid group or a salt thereof, a substituted or unsubstituted C₁-C₆₀ alkyl group, a substituted or unsubstituted C₂-C₆₀ alkenyl group, a substituted or unsubstituted C₂-C₆₀ alkynyl group, a substituted or unsubstituted C₁-C₆₀ alkoxy group, a substituted or unsubstituted C₃-C₁₀ cycloalkyl group, a substituted or unsubstituted C₁-C₁₀ heterocycloalkyl group, a substituted or unsubstituted C₃-C₁₀ cycloalkenyl group, a substituted or unsubstituted C₁-C₁₀ heterocycloalkenyl group, a substituted or unsubstituted C₆-C₆₀ aryl group, a substituted or unsubstituted C₆-C₆₀ aryloxy group, a substituted or unsubstituted C₆-C₆₀ arylthio group, a substituted or unsubstituted C₁-C₆₀ heteroaryl group, a substituted or unsubstituted monovalent non-aromatic condensed polycyclic group, a substituted or unsubstituted monovalent non-aromatic condensed heteropolycyclic group, provided that from among the substituted or unsubstituted monovalent non-aromatic condensed heteropolycyclic groups, a substituted or unsubstituted carbazolyl group is excluded, and -Si(Q₁)(Q₂)(Q₃),
Ar₁ is represented by one of Formulae 2A to 2C,
X₂₁ is N or C(R₂₁), X₂₂ is N or C(R₂₂), X₂₃ is N or C(R₂₃), X₂₄ is N or C(R₂₄), and X₂₅ is O, S, P(=O)(R₂₅), Se or Si(R₂₅)(R₂₆),
R₂₁ to R₂₆ are each independently selected from a hydrogen, a deuterium, C₁-C₄ alkyl group, a phenyl group, a pyridinyl group, a pyrimidinyl group, a pyrazinyl group, a pyridazinyl group, a triazinyl group, and -Si(Q₁₁)(Q₁₂)(Q₁₃),
a21 and a22 are each independently an integer selected from 0 to 3, and when a21 is 2 or more, 2 or more R₂₁(s) may be identical to or different from each other, and when a22 is 2 or more, 2 or more R₂₂(s) may be identical to or different from each other;
L₁ and L₂ are each independently selected from
a phenylene group, a pyridinylene group, a pyrimidinylene group, a pyrazinylene group, a pyridazinylene group, a triazinylene group, a dibenzofuranylene group, and a dibenzothiophenylene group; and
a phenylene group, a pyridinylene group, a pyrimidinylene group, a pyrazinylene group, a pyridazinylene group, a triazinylene group, a dibenzofuranylene group, and a dibenzothiophenylene group, each substituted with at least one selected from a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group, a C₁-C₁₀ alkoxy group, a phenyl group, a naphthyl group, a pyridinyl group, a pyrimidinyl group, a pyrazinyl group, a pyridazinyl group, a triazinyl group, and - Si(Q₂₁)(Q₂₂)(Q₂₃),
a1 and a2 are each independently an integer selected from 0 to 5, and when a1 is 2 or more, 2 or more L₁(s) may be identical to or different from each other, and when a2 is 2 or more, 2 or more L₂(s) may be identical to or different from each other;
each of * and *' indicates a binding site to a neighboring atom;
at least one of substituents of the substituted C₁-C₆₀ alkyl group, substituted C₂-C₆₀ alkenyl group, substituted C₂-C₆₀ alkynyl group, substituted C₁-C₆₀ alkoxy group, substituted C₃-C₁₀ cycloalkyl group, substituted C₁-C₁₀ heterocycloalkyl group, substituted C₃-C₁₀ cycloalkenyl group, substituted C₁-C₁₀ heterocycloalkenyl group, substituted C₆-C₆₀ aryl group, substituted C₆-C₆₀ aryloxy group, substituted C₆-C₆₀ arylthio group, substituted C₁-C₆₀ heteroaryl group, substituted monovalent non-aromatic condensed polycyclic group, and substituted monovalent non-aromatic condensed heteropolycyclic group is selected from a deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₆₀ alkyl group, a C₂-C₆₀ alkenyl group, a C₂-C₆₀ alkynyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₆-C₆₀ aryloxy group, a C₆-C₆₀ arylthio group, a C₁-C₆₀ heteroaryl group, a monovalent non-aromatic condensed polycyclic group, a monovalent non-aromatic condensed heteropolycyclic group, provided that the monovalent non-aromatic condensed heteropolycyclic group is not a carbazolyl group, and -Si(Q₃₁)(Q₃₂)(Q₃₃),
wherein Q₁ to Q₃, Q₁₁ to Q₁₃, Q₂₁ to Q₂₃, and Q₃₁ to Q₃₃ are each independently selected from a hydrogen, a C₁-C₆₀ alkyl group, a C₁-C₆₀ alkoxy group, a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₁-C₆₀ heteroaryl group, a monovalent non-aromatic condensed polycyclic group, and a monovalent non-aromatic condensed heteropolycyclic group, provided that the monovalent non-aromatic condensed heteropolycyclic group is not a carbazolyl group, with the proviso that the condensed cyclic compound is not one of the below compounds: 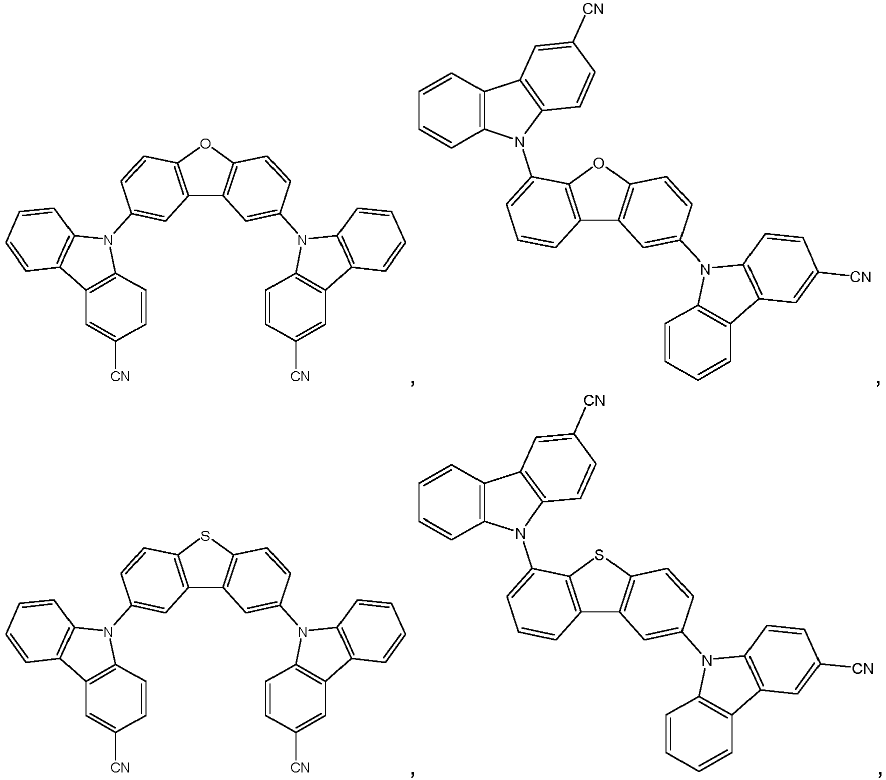 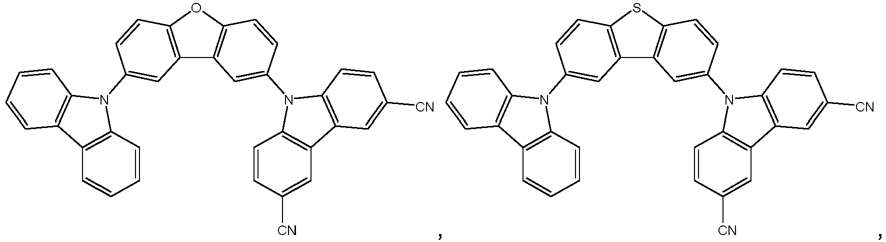 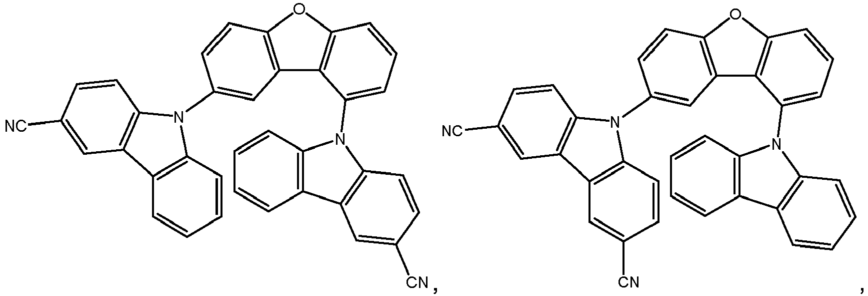 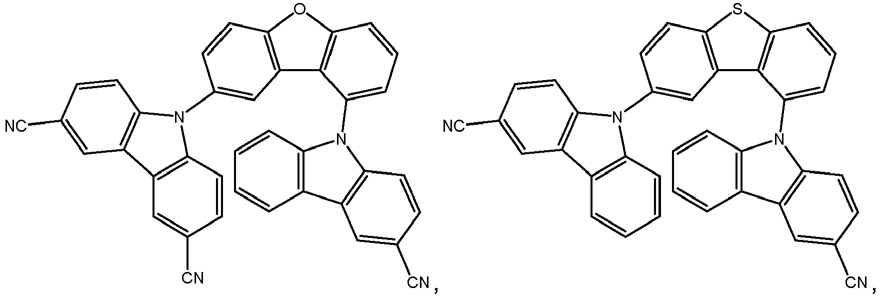 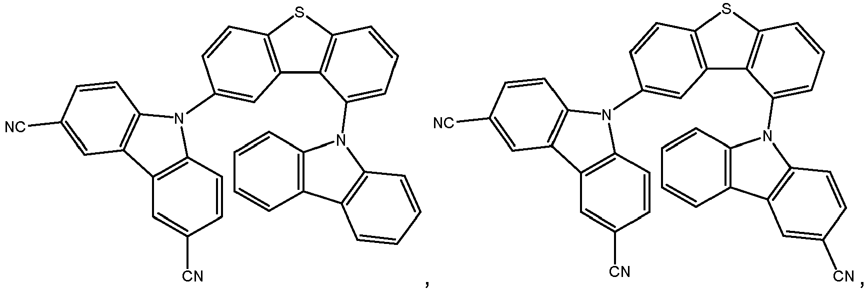 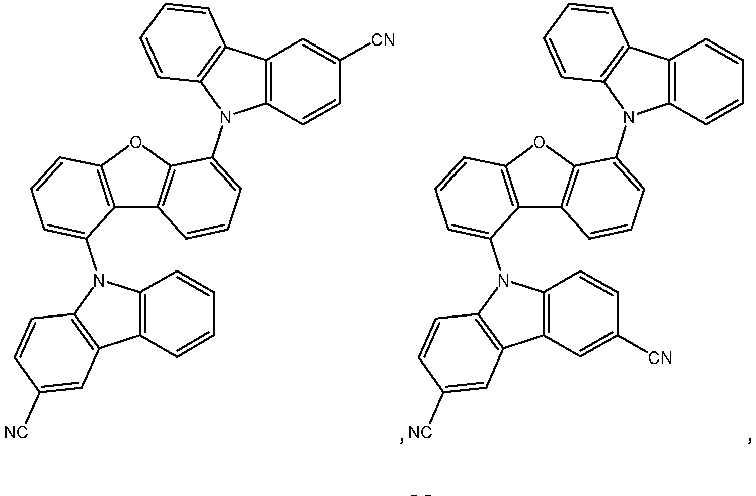 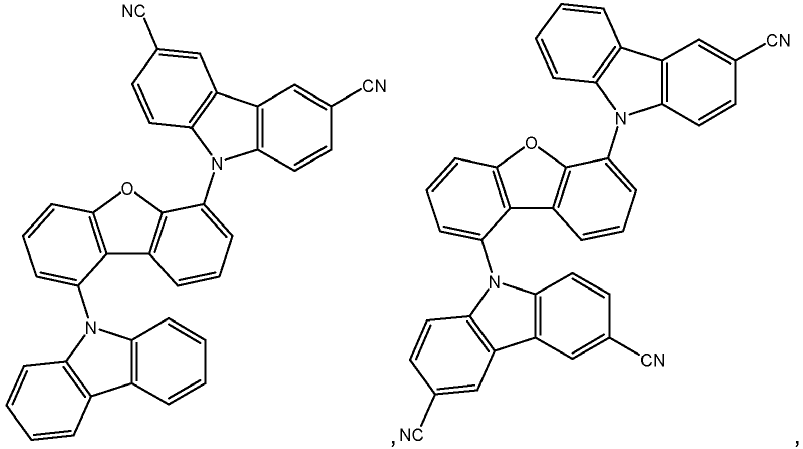 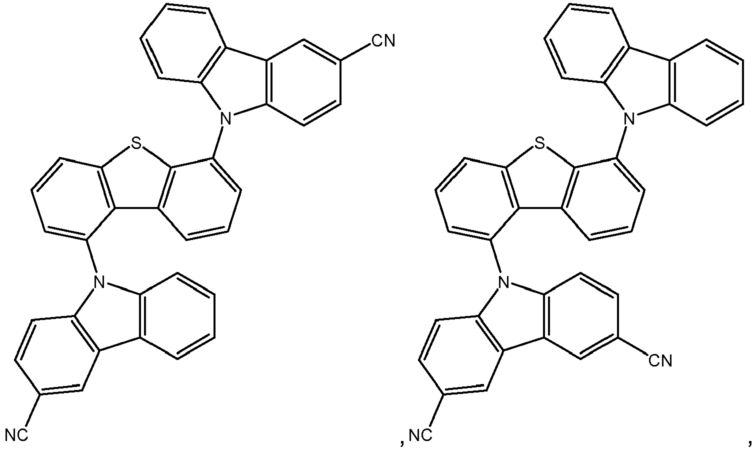 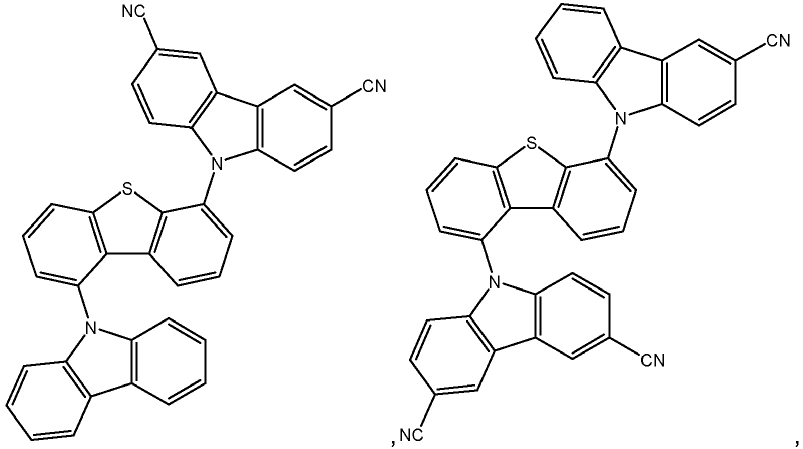 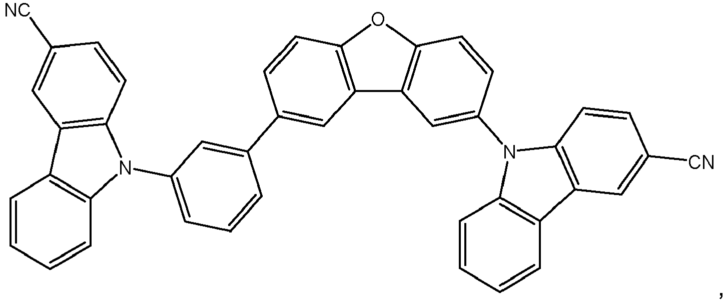 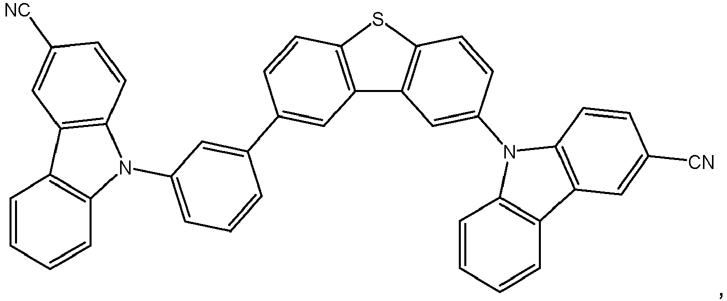 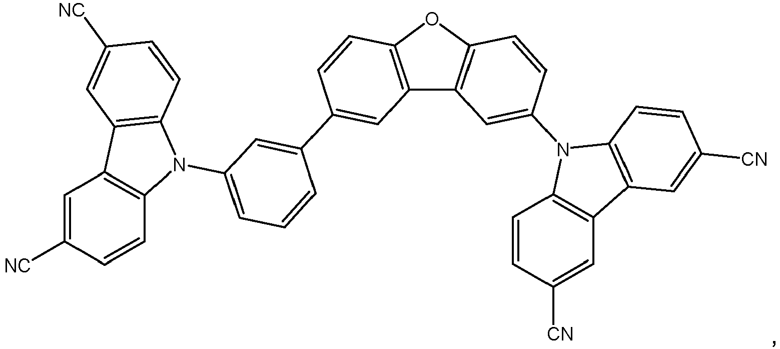 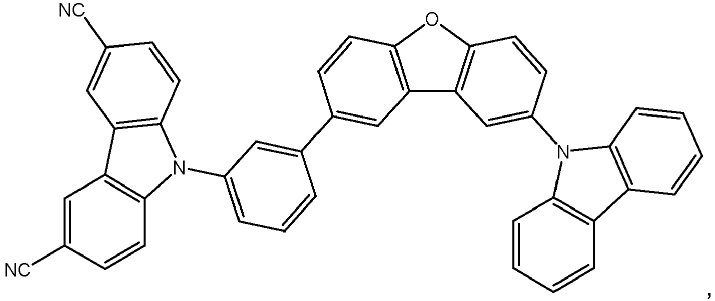 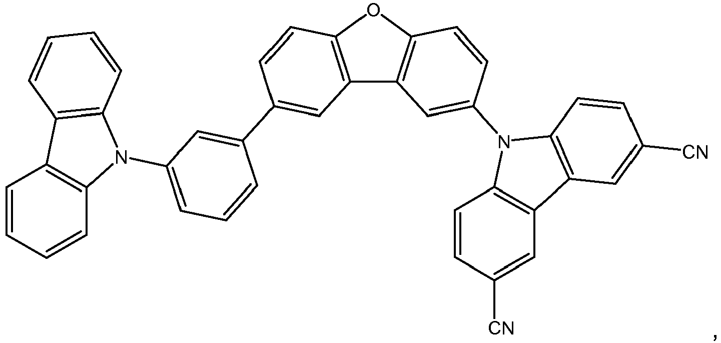 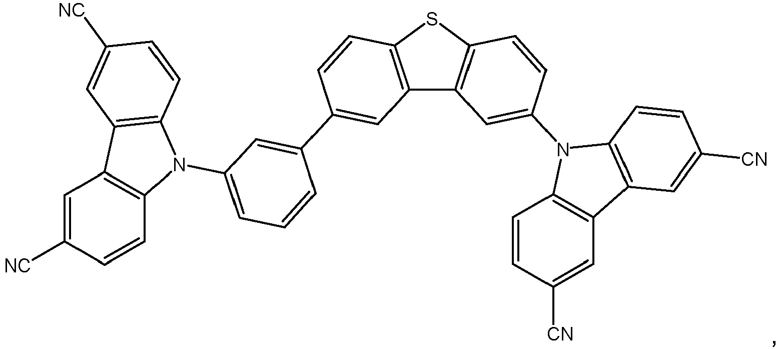 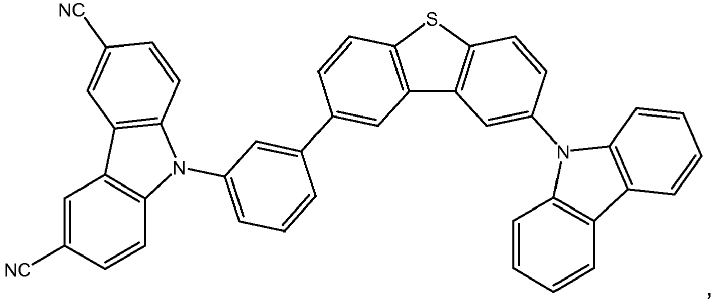 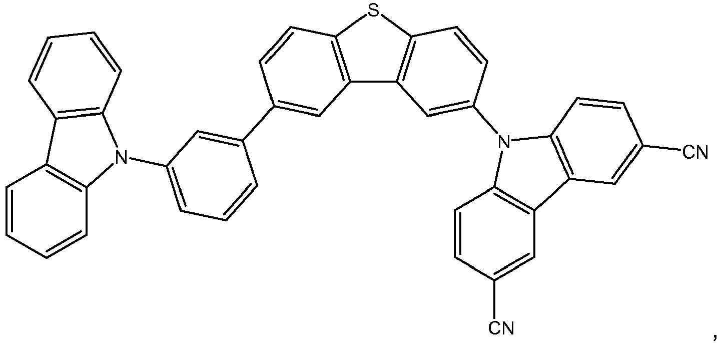 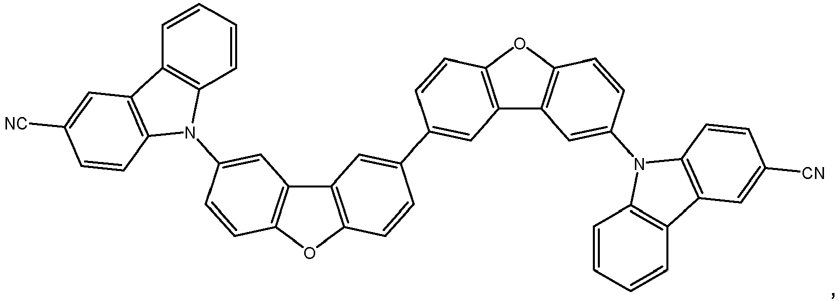 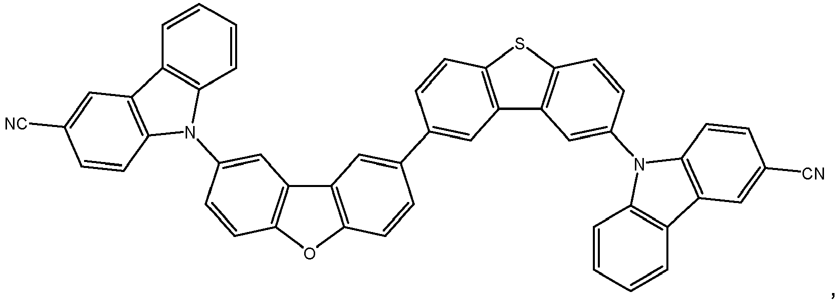 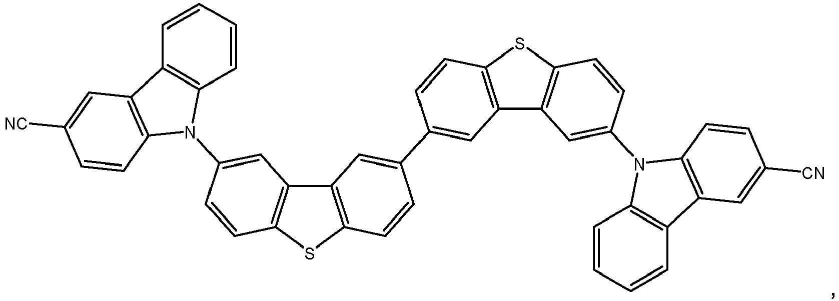 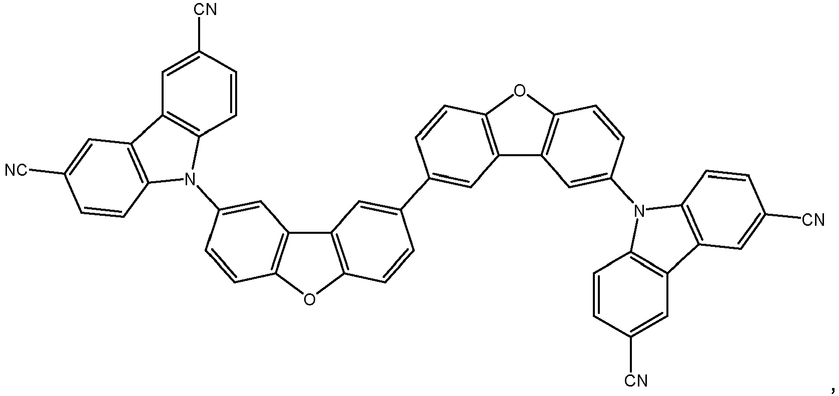 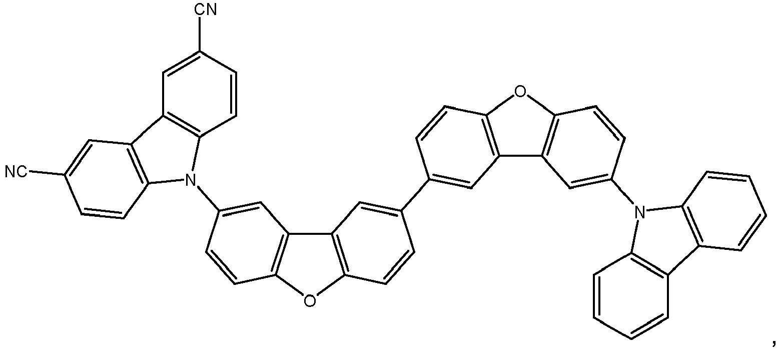 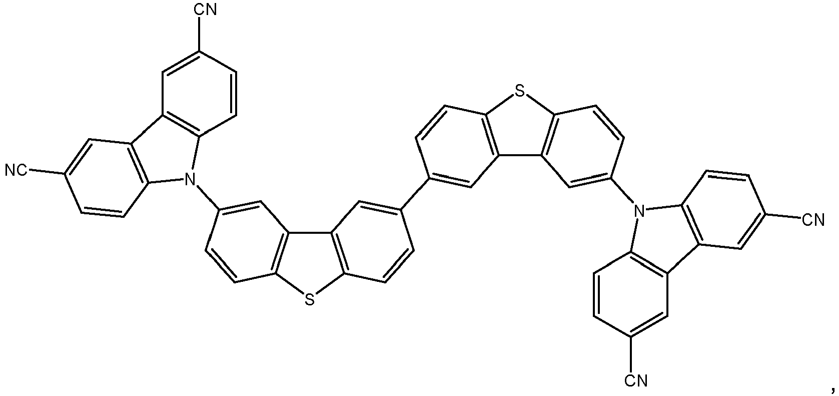
or

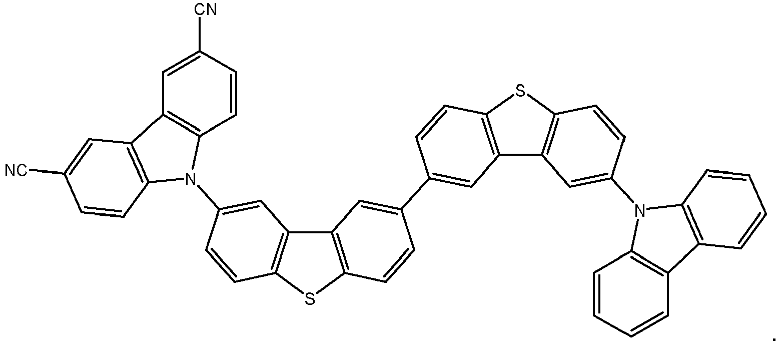

2. The condensed cyclic compound of claim 1, wherein
R₁ to R₈ and R₁₁ to R₁₈ are each independently selected from
a hydrogen, a deuterium, -F, -Cl, -Br, -I, a hydroxyl group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, and a C₁-C₂₀ alkoxy group;
a C₁-C₂₀ alkyl group and a C₁-C₂₀ alkoxy group, each substituted with at least one selected from a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a phenyl group, a naphthyl group, a pyridinyl group, a pyrimidinyl group, a pyrazinyl group, a pyridazinyl group, and a triazinyl group;
a cyclopentyl group, a cyclohexyl group, a cyclopentenyl group, a cyclohexenyl group, a cycloheptenyl group, a phenyl group, a pentalenyl group, an indenyl group, a naphthyl group, an azulenyl group, a heptalenyl group, an indacenyl group, an acenaphthyl group, a fluorenyl group, a spiro-fluorenyl group, a phenalenyl group, a phenanthrenyl group, an anthracenyl group, a fluoranthrenyl group, a triphenylenyl group, a pyrenyl group, a chrysenyl group, a naphthacenyl group, a picenyl group, a perylenyl group, a pentaphenyl group, a hexacenyl group, a pyrrolyl group, an imidazolyl group, a pyrazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzoxazolyl group, a benzoimidazolyl group, a furanyl group, a benzofuranyl group, a thiophenyl group, a benzothiophenyl group, a thiazolyl group, an isothiazolyl group, a benzothiazolyl group, an isoxazolyl group, an oxazolyl group, a triazolyl group, a tetrazolyl group, an oxadiazolyl group, a triazinyl group, a dibenzofuranyl group, a dibenzothiophenyl group, an imidazopyridimidinyl group, and an imidazopyridinyl group;
a cyclopentyl group, a cyclohexyl group, a cyclopentenyl group, a cyclohexenyl group, a cycloheptenyl group, a phenyl group, a pentalenyl group, an indenyl group, a naphthyl group, an azulenyl group, a heptalenyl group, an indacenyl group, an acenaphthyl group, a fluorenyl group, a spiro-fluorenyl group, a phenalenyl group, a phenanthrenyl group, an anthracenyl group, a fluoranthrenyl group, a triphenylenyl group, a pyrenyl group, a chrysenyl group, a naphthacenyl group, a picenyl group, a perylenyl group, a pentaphenyl group, a hexacenyl group, a pyrrolyl group, an imidazolyl group, a pyrazolyl group, a pyridinyl group, a pyrazinyl group, a pyrimidinyl group, a pyridazinyl group, an isoindolyl group, an indolyl group, an indazolyl group, a purinyl group, a quinolinyl group, an isoquinolinyl group, a benzoquinolinyl group, a phthalazinyl group, a naphthyridinyl group, a quinoxalinyl group, a quinazolinyl group, a cinnolinyl group, a phenanthridinyl group, an acridinyl group, a phenanthrolinyl group, a phenazinyl group, a benzoxazolyl group, a benzoimidazolyl group, a furanyl group, a benzofuranyl group, a thiophenyl group, a benzothiophenyl group, a thiazolyl group, an isothiazolyl group, a benzothiazolyl group, an isoxazolyl group, an oxazolyl group, a triazolyl group, a tetrazolyl group, an oxadiazolyl group, a triazinyl group, a dibenzofuranyl group, a dibenzothiophenyl group, an imidazopyridimidinyl group, and an imidazopyridinyl group, each substituted with at least one selected from a deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₂₀ alkyl group, a C₂-C₂₀ alkenyl group, a C₂-C₂₀ alkynyl group, a C₁-C₂₀ alkoxy group, a phenyl group, a naphthyl group, an anthracenyl group, a pyrenyl group, a phenanthrenyl group, a fluorenyl group, a pyridinyl group, a pyrimidinyl group, a pyrazinyl group, a pyridazinyl group, a triazinyl group, a quinolinyl group, an isoquinolinyl group, a phthalazinyl group, a quinoxalinyl group, a cinnolinyl group, a quinazolinyl group, and -Si(Q₃₁)(Q₃₂)(Q₃₃); and
-Si(Q₁)(Q₂)(Q₃),
wherein Q₁ to Q₃ and Q₃₁ to Q₃₃ are each independently selected from a hydrogen, a C₁-C₂₀ alkyl group, a C₁-C₂₀ alkoxy group, a phenyl group, a naphthyl group, an anthracenyl group, a pyrenyl group, a phenanthrenyl group, a fluorenyl group, a pyridinyl group, a pyrimidinyl group, a pyrazinyl group, a pyridazinyl group, a triazinyl group, a quinolinyl group, an isoquinolinyl group a phthalazinyl group, a quinoxalinyl group, a cinnolinyl group, and a quinazolinyl group, preferably wherein R₁ to R₈ and R₁₁ to R₁₈ are each independently selected from a hydrogen, a deuterium, a cyano group, a methyl group, an ethyl group, an n-propyl group, an isopropyl group, an n-butyl group, an isobutyl group, a sec-butyl group, a tert-butyl group, an n-pentyl group, an isopentyl group, a sec-pentyl group, a tert-pentyl group, an n-hexyl group, an iso-hexyl group, a sec-hexyl group, a tert-hexyl group, and -Si(Q₁)(Q₂)(Q₃),
wherein Q₁ to Q₃ are each independently selected from a hydrogen, a C₁-C₁₀ alkyl group, a C₁-C₁₀ alkoxy group, and a phenyl group.

3. The condensed cyclic compound of claim 1, wherein
R₁ to R₆ and R₁₁ to R₁₆ are each independently selected from
a hydrogen, a deuterium, -F, -Cl, -Br, -I, a hydroxyl group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group, and a C₁-C₁₀ alkoxy group;
a phenyl group, a naphthyl group, a pyridinyl group, a pyrimidinyl group, a pyrazinyl group, a pyridazinyl group, and a triazinyl group;
a phenyl group, a naphthyl group, a pyridinyl group, a pyrimidinyl group, a pyrazinyl group, a pyridazinyl group, and a triazinyl group, each substituted with at least one selected from a deuterium, -F, -Cl, -Br, -I, a hydroxyl group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group, a C₁-C₁₀ alkoxy group, a phenyl group, a naphthyl group, a pyridinyl group, a pyrimidinyl group, a pyrazinyl group, a pyridazinyl group, a triazinyl group, and - Si(Q₃₁)(Q₃₂)(Q₃₃); and
-Si(Q₁)(Q₂)(Q₃),
R₇, R₈, R₁₇, and R₁₈ are each independently selected from
a hydrogen, a deuterium, -F, -Cl, -Br, -I, a hydroxyl group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group, and a C₁-C₁₀ alkoxy group;
a phenyl group, a naphthyl group, a pyridinyl group, a pyrimidinyl group, a pyrazinyl group, a pyridazinyl group, and a triazinyl group;
a phenyl group, a naphthyl group, a pyridinyl group, a pyrimidinyl group, a pyrazinyl group, a pyridazinyl group, and a triazinyl group, each substituted with at least one selected from a deuterium, -F, -Cl, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group, a C₁-C₁₀ alkoxy group, a phenyl group, a naphthyl group, a pyridinyl group, a pyrimidinyl group, a pyrazinyl group, a pyridazinyl group, a triazinyl group, and -Si(Q₃₁)(Q₃₂)(Q₃₃); and
-Si(Q₁)(Q₂)(Q₃),
wherein Q₁ to Q₃ and Q₃₁ to Q₃₃ are each independently selected from a hydrogen, a C₁-C₁₀ alkyl group, a C₁-C₁₀ alkoxy group, a phenyl group, a naphthyl group, a pyridinyl group, a pyrimidinyl group, a pyrazinyl group, a pyridazinyl group, and a triazinyl group, and/or wherein each of R₁ to R₆ and R₁₁ to R₁₆ is not a cyano group.

4. The condensed cyclic compound of claim 1, wherein
Ar₁ is represented by one of Formulae 2A-1 to 2A-10, 2B-1 to 2B-8, and 2C-1 to 2C-9: 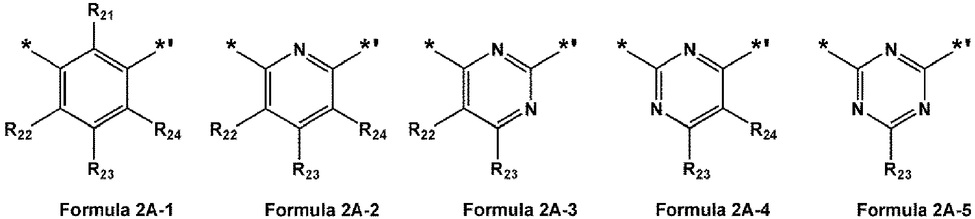 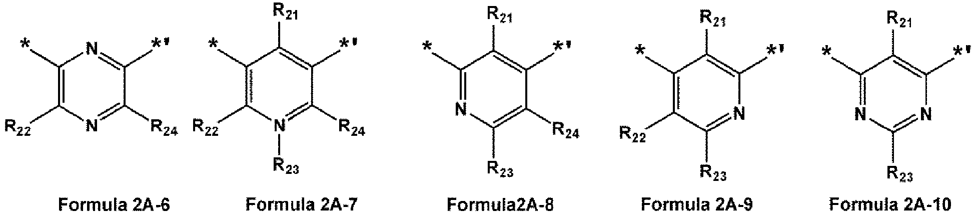 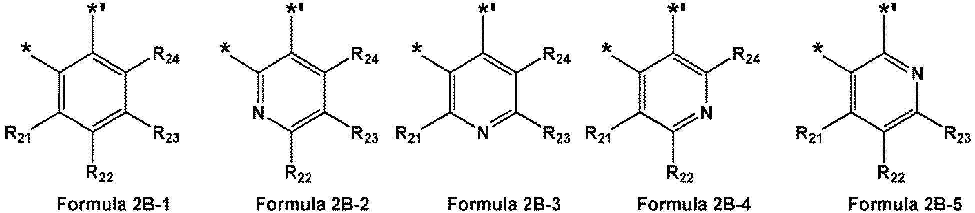 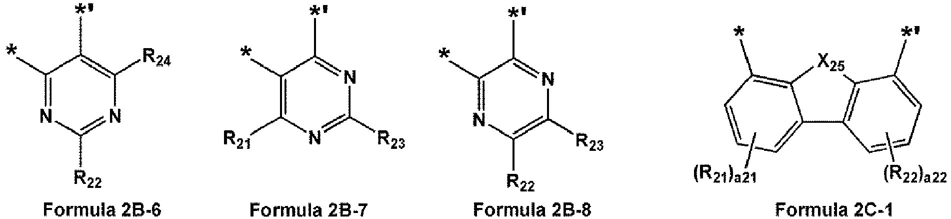 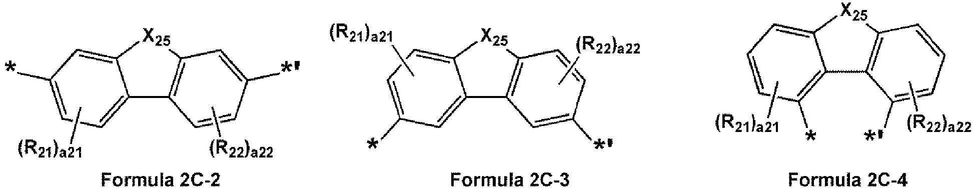 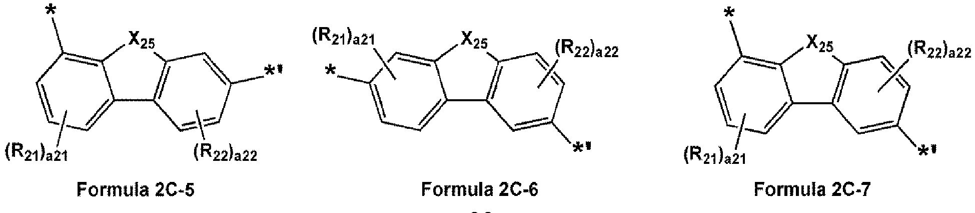 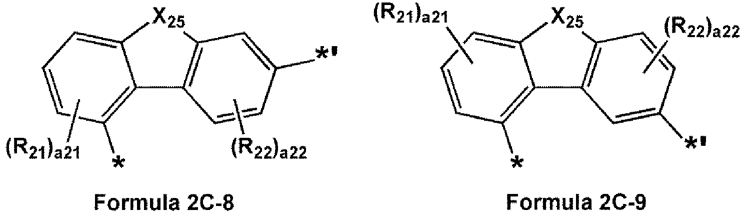 wherein in Formulae 2A-1 to 2A-10, 2B-1 to 2B-8, and 2C-1 to 2C-9,
X₂₅ is O, S, P(=O)(R₂₅), Se or Si(R₂₅)(R₂₆),
R₂₁ to R₂₄ are each independently selected from a hydrogen, a deuterium, a methyl group, an ethyl group, an n-propyl group, an isopropyl group, an n-butyl group, an isobutyl group, a sec-butyl group, a tert-butyl group, a phenyl group, a pyridinyl group, a pyrimidinyl group, a pyrazinyl group, a pyridazinyl group, a triazinyl group, and -Si(Q₁₁)(Q₁₂)(Q₁₃),
R₂₅ and R₂₆ are each independently selected from a hydrogen, a deuterium, a methyl group, an ethyl group, an n-propyl group, an isopropyl group, an n-butyl group, an isobutyl group, a sec-butyl group, a tert-butyl group, a phenyl group, a pyridinyl group, a pyrimidinyl group, a pyrazinyl group, a pyridazinyl group and a triazinyl group,
wherein Q₁₁ to Q₁₃ are each independently selected from a hydrogen, a C₁-C₁₀ alkyl group, a C₁-C₁₀ alkoxy group, and a phenyl group,
a21 and a22 are each independently 0 or 1, and
each of * and *' indicates a binding site to a neighboring atom.

5. The condensed cyclic compound of any of claims 1-3, wherein
L₁ and L₂ are each independently selected from
a phenylene group, a pyridinylene group, a pyrimidinylene group, and a triazinylene group; and
a phenylene group, a pyridinylene group, a pyrimidinylene group, and a triazinylene group, each substituted with at least one selected from a deuterium, a methyl group, an ethyl group, an n-propyl group, an isopropyl group, an n-butyl group, an isobutyl group, a sec-butyl group, a tert-butyl group, a phenyl group, a pyridinyl group, a pyrimidinyl group, a pyrazinyl group, a pyridazinyl group, a triazinyl group, and -Si(Q₂₁)(Q₂₂)(Q₂₃), preferably wherein
L₁ and L₂ are each independently selected from
a phenylene group; and
a phenylene group, substituted with at least one selected from a deuterium, a methyl group, an ethyl group, an n-propyl group, an isopropyl group, an n-butyl group, an isobutyl group, a sec-butyl group, a tert-butyl group, a phenyl group, a pyridinyl group, a pyrimidinyl group, a pyrazinyl group, a pyridazinyl group, a triazinyl group, and - Si(Q₂₁)(Q₂₂)(Q₂₃),
wherein Q₂₁ to Q₂₃ are each independently selected from a hydrogen, a C₁-C₁₀ alkyl group, a C₁-C₁₀ alkoxy group, and a phenyl group, and
a1 and a2 are each independently 0 or 1.

6. The condensed cyclic compound of claim 4, wherein
a1 and a2 in Formulae 1B to 1D is 0 and Ar₁ is represented by one of Formulae 2A-1, 2A-2, 2A-10, 2B-1, 2B-8 and 2C-3.

7. The condensed cyclic compound of claim 1, wherein
regarding Formulae 1B to 1D, the sum of a1 and a2 is 1 or more, and *-(L₁)ₐ₁-Ar₁-(L₂)ₐ₂-*' is represented by one of Formulae 3-1 to 3-57: 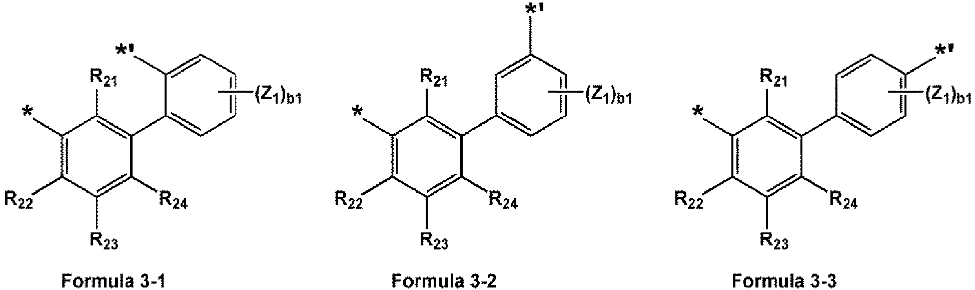 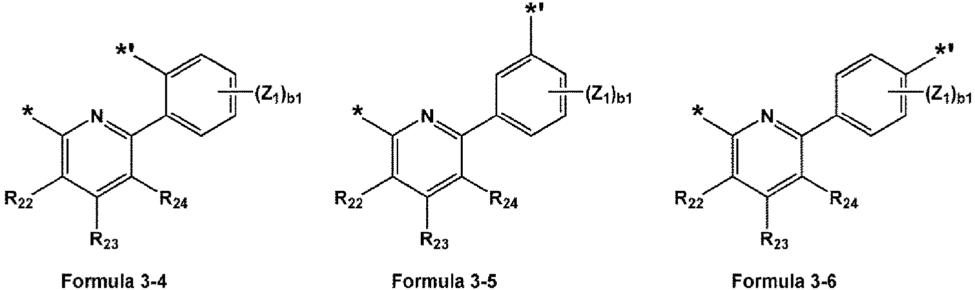 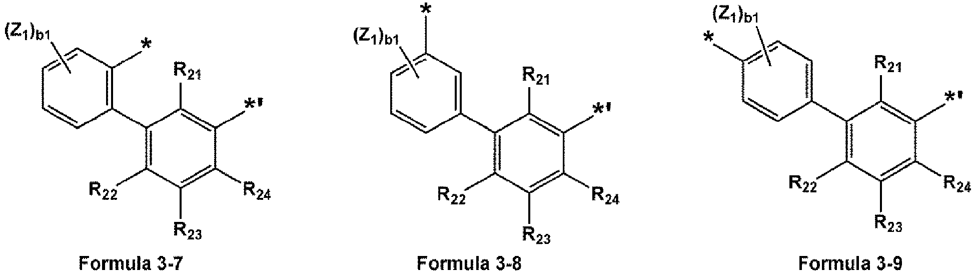 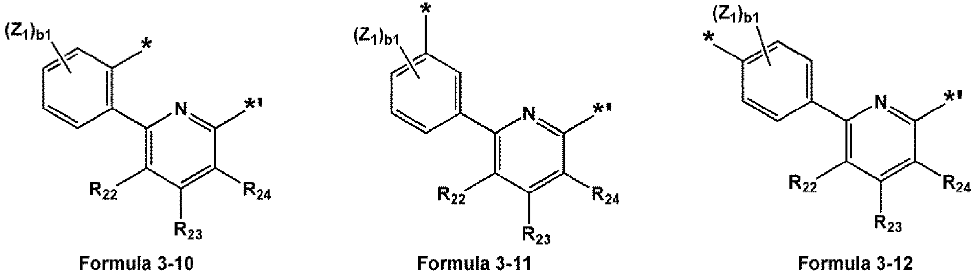 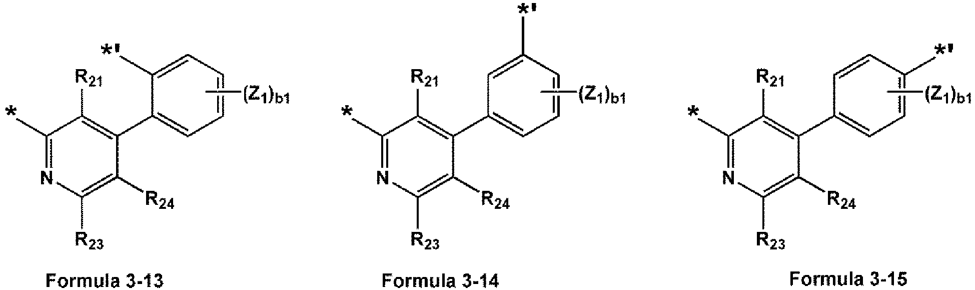 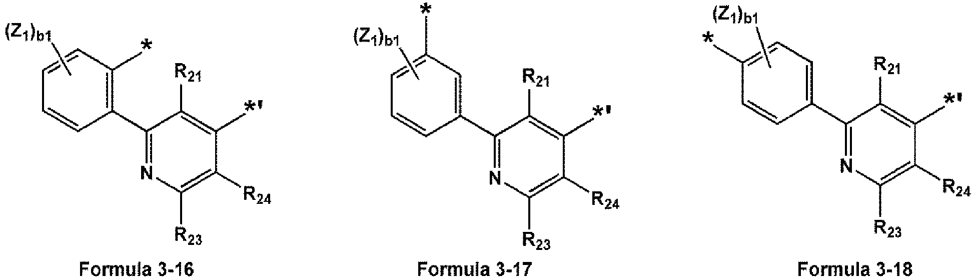 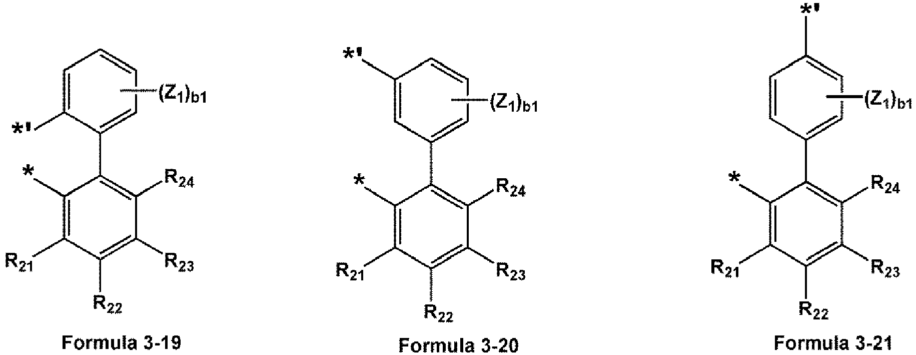 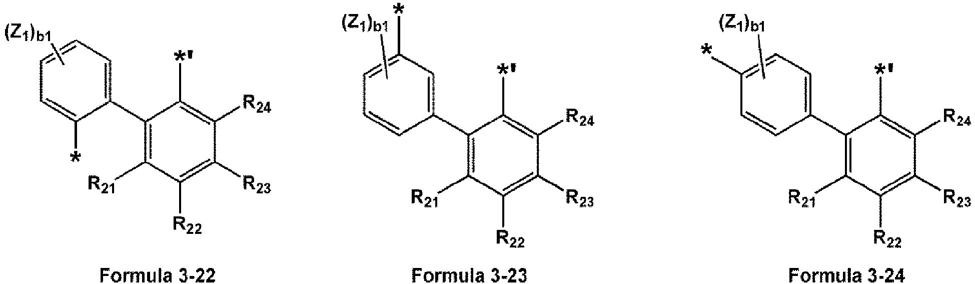 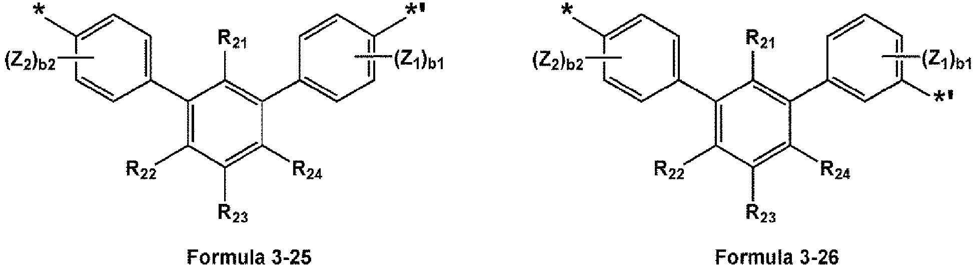 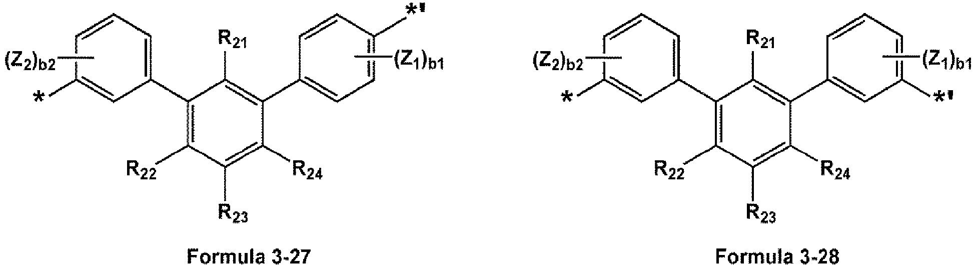 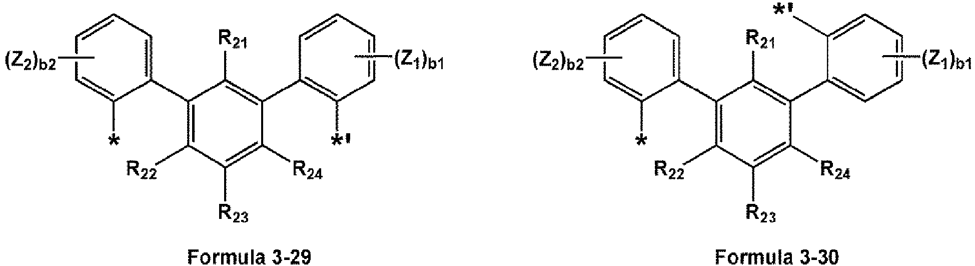 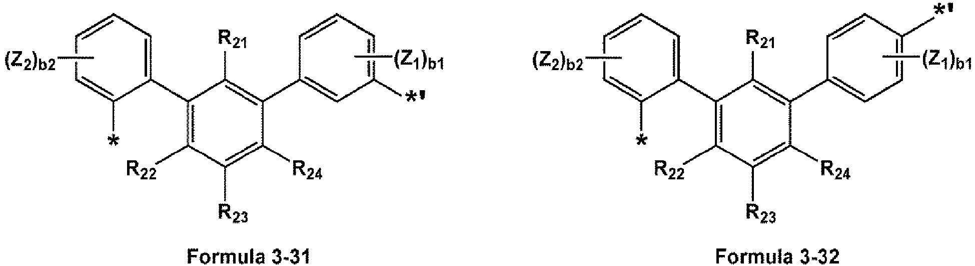 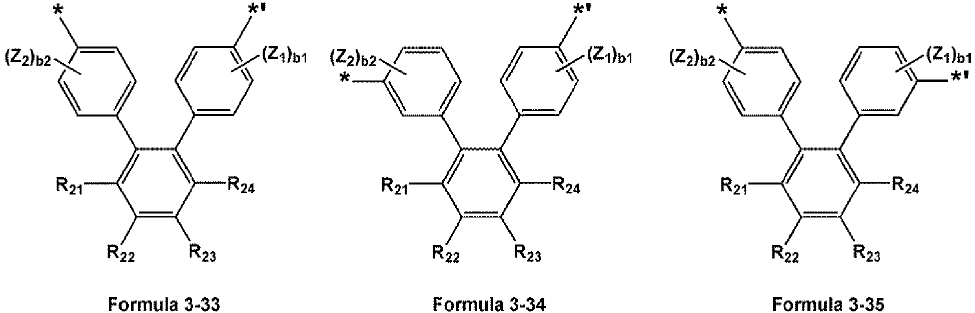 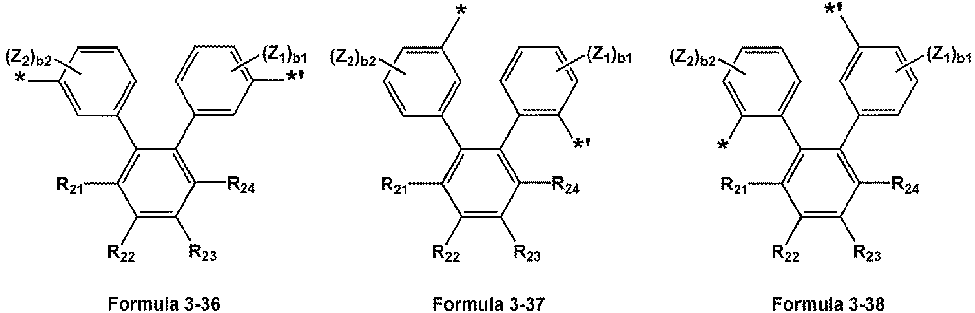 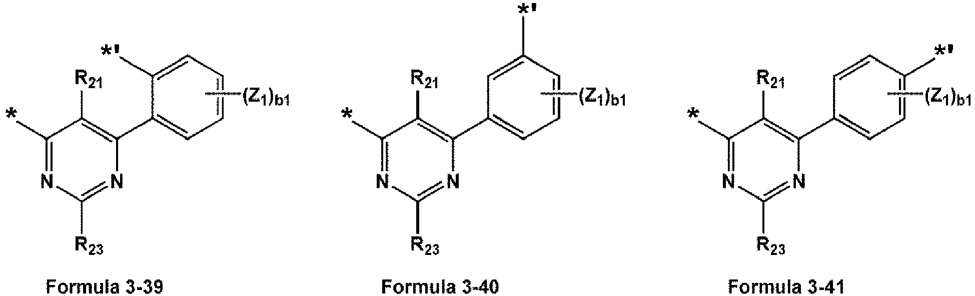 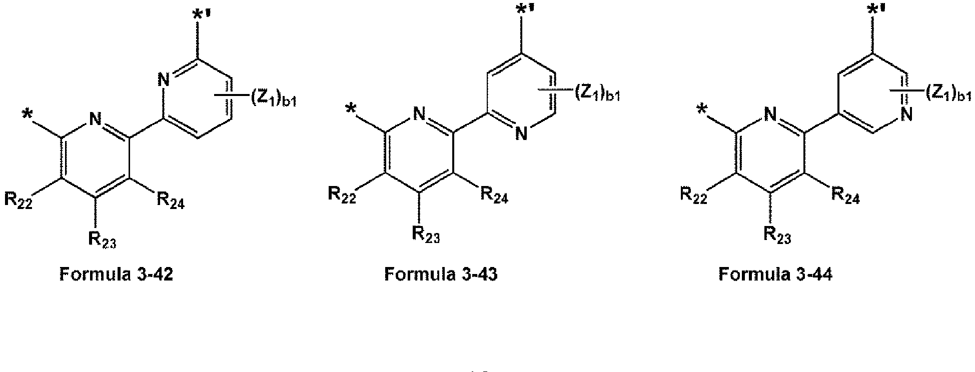 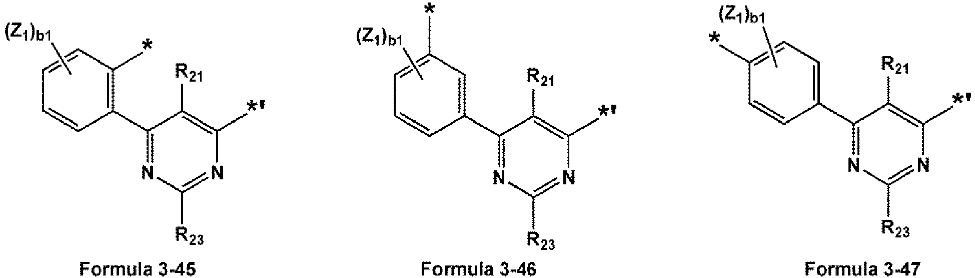 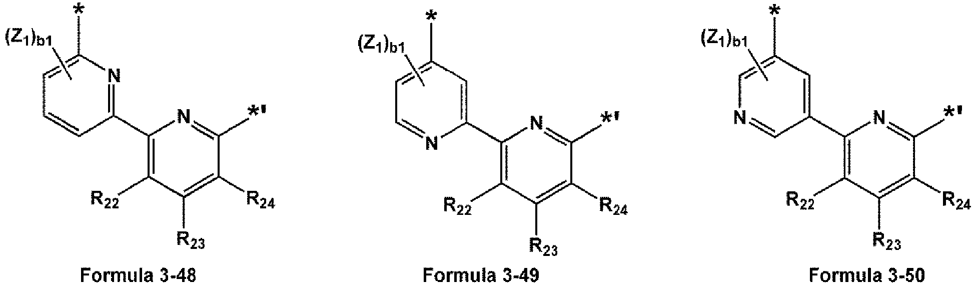 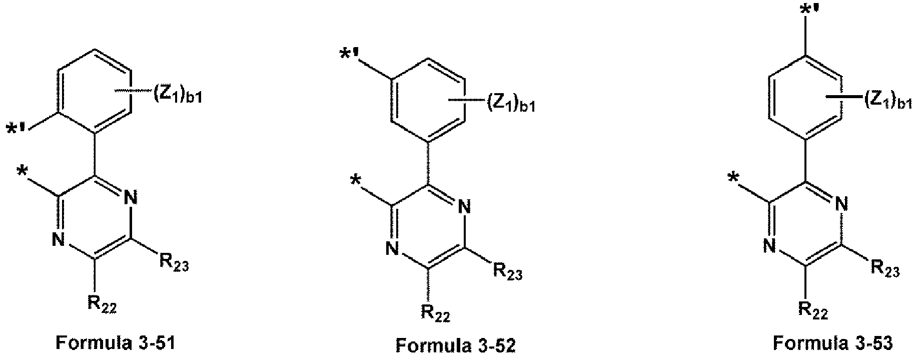 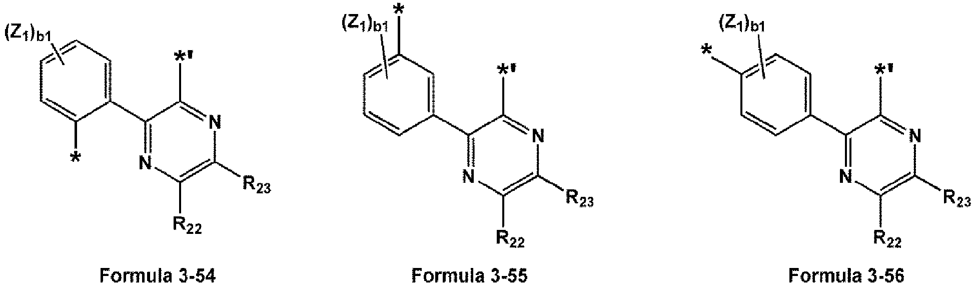 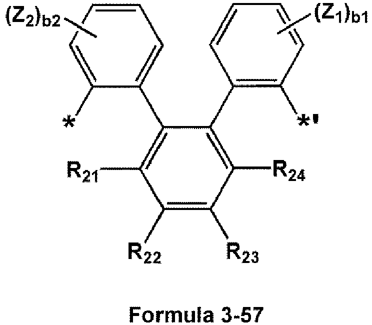 wherein in Formulae 3-1 to 3-57,
R₂₁ to R₂₄, Z₁, and Z₂ are each independently selected from a hydrogen, a deuterium, a methyl group, an ethyl group, an n-propyl group, an isopropyl group, an n-butyl group, an isobutyl group, a sec-butyl group, a tert-butyl group, a phenyl group, a pyridinyl group, a pyrimidinyl group, a pyrazinyl group, a pyridazinyl group, a triazinyl group, and -Si(Q₁₁)(Q₁₂)(Q₁₃),
wherein Q₁₁ to Q₁₃ are each independently selected from a hydrogen, a C₁-C₁₀ alkyl group, a C₁-C₁₀ alkoxy group, and a phenyl group,
b1 and b2 are each independently 0, 1, or 2, and
each of * and *' indicates a binding site to a neighboring nitrogen atom.

8. The condensed cyclic compound of claim 1, wherein
the condensed cyclic compound is represented by one of Formulae 1B(1) to 1D(1), 1B(2) to 1D(2) and 1B(3) to 1D(3): 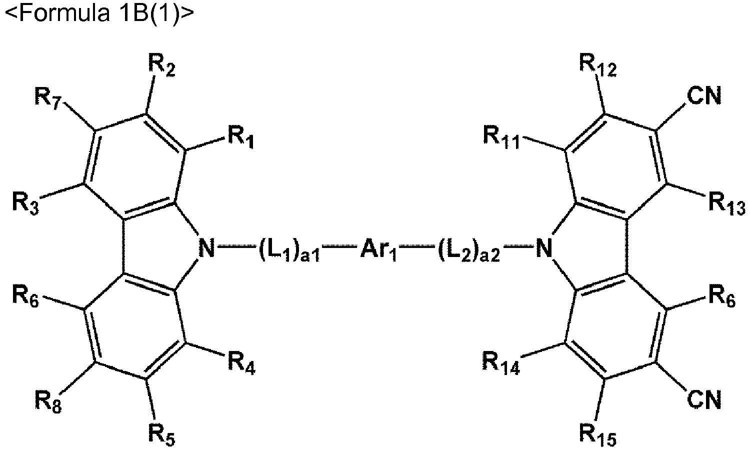 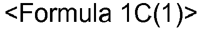 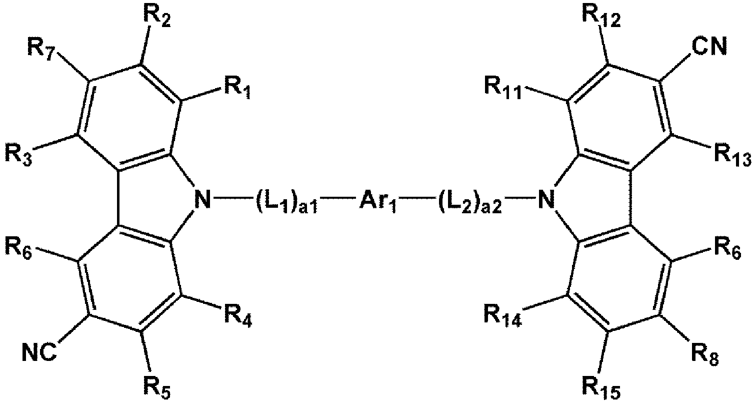 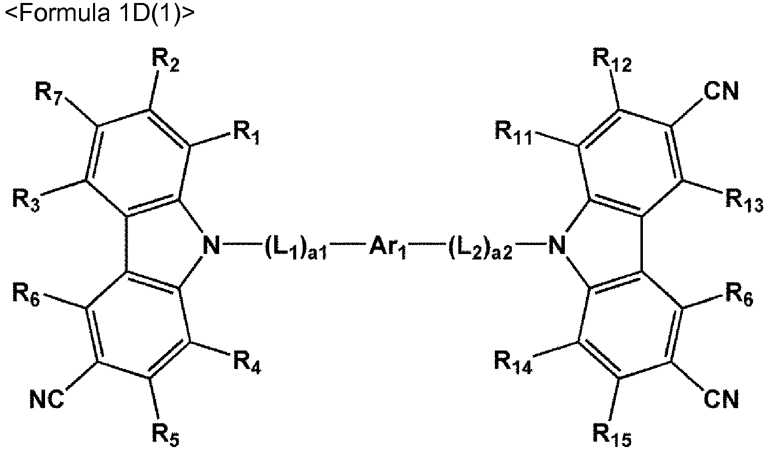 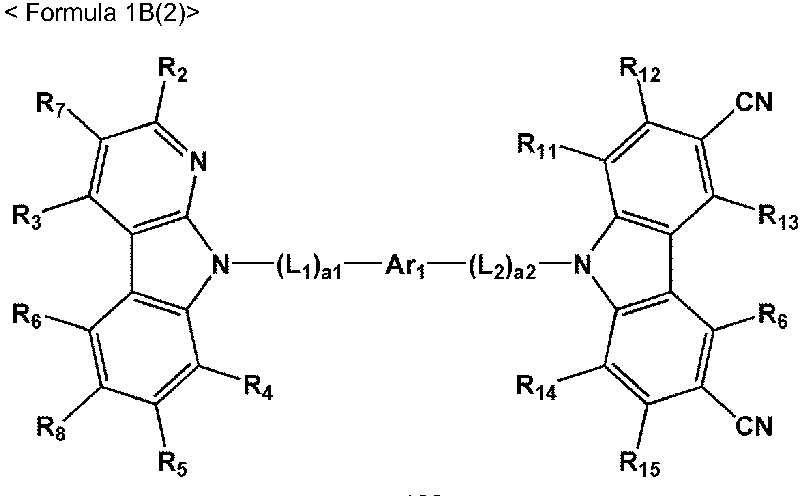 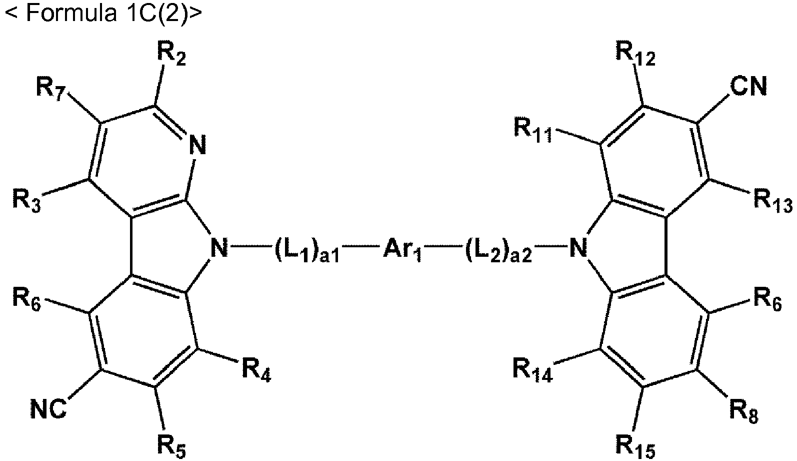 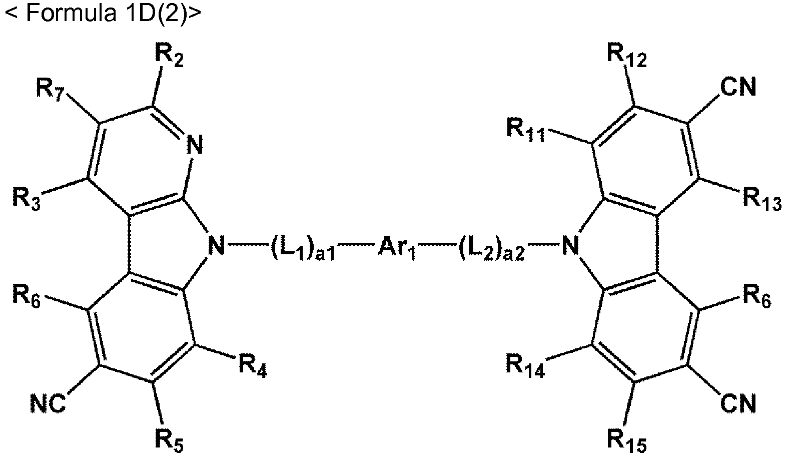 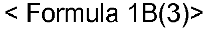 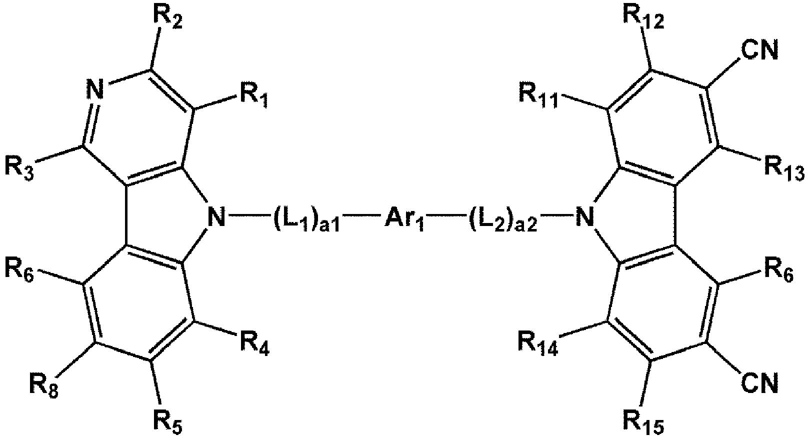 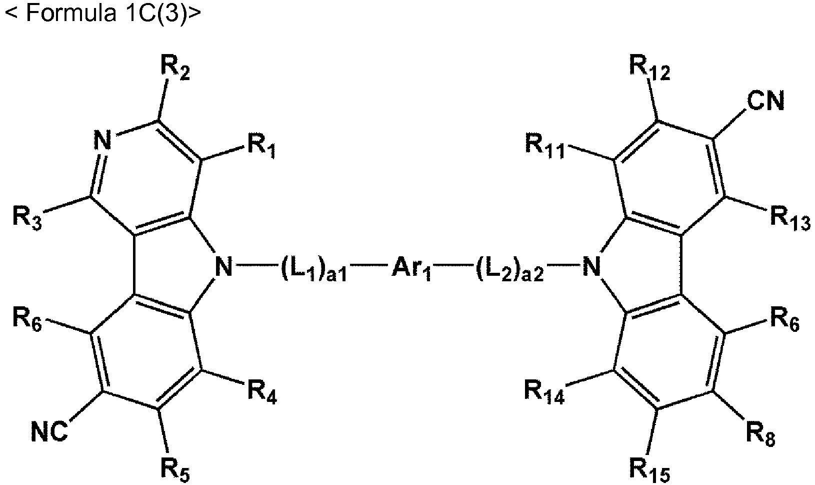 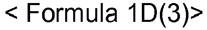 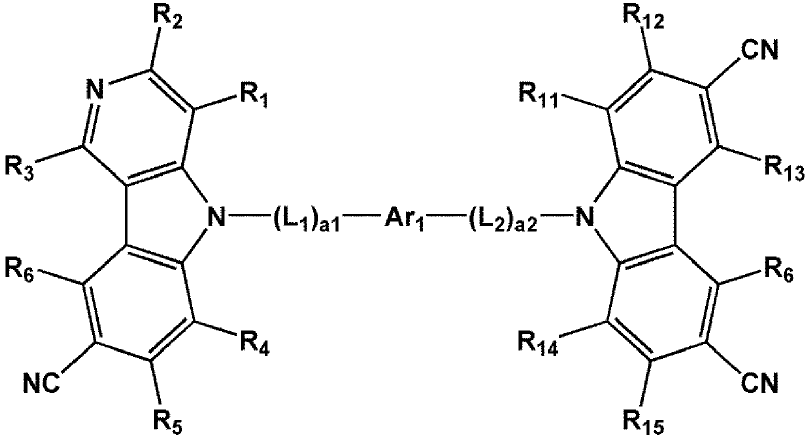 wherein in Formulae 1B(1) to 1D(1), 1B(2) to 1D(2) and 1B(3) to 1D(3),
R₁ to R₈, R₁₁ to R₁₆, and R₁₈ are each independently selected from a hydrogen, a deuterium, -F, -Cl, -Br, -I, a hydroxyl group, a nitro group, an amino group, an amidino group, a hydrazine group, a hydrazone group, a carboxylic acid group or a salt thereof, a sulfonic acid group or a salt thereof, a phosphoric acid group or a salt thereof, a methyl group, an ethyl group, an n-propyl group, an isopropyl group, an n-butyl group, an isobutyl group, a sec-butyl group, a tert-butyl group, an n-pentyl group, an isopentyl group, a sec-pentyl group, a tert-pentyl group, an n-hexyl group, an iso-hexyl group, a sec-hexyl group, a tert-hexyl group, an n-heptyl group, an iso-heptyl group, a sec-heptyl group, a tert-heptyl group, an n-octyl group, an iso-octyl group, a sec-octyl group, a tert-octyl group, an n-nonyl group, an iso-nonyl group, a sec-nonyl group, a tert-nonyl group, an n-decyl group, an isodecyl group, a sec-decyl group, a tert-decyl group, a methoxy group, an ethoxy group, a propoxy group, a butoxy group, a pentoxy group, a phenyl group, a naphthyl group, a pyridinyl group, a pyrimidinyl group, a triazinyl group, and -Si(Q₁)(Q₂)(Q₃),
Ar₁ is represented by Formulae 2A-1, 2A-2, 2A-10, 2B-1, 2B-8 or 2C-3:
L₁ and L₂ are each independently selected from:
a phenylene group, a pyridinylene group, a pyrimidinylene group, a pyrazinylene group, a pyridazinylene group, a triazinylene group, a dibenzofuranylene group, and a dibenzothiophenylene group; and
a phenylene group, a pyridinylene group, a pyrimidinylene group, a pyrazinylene group, a pyridazinylene group, a triazinylene group, a dibenzofuranylene group, and a dibenzothiophenylene group, each substituted with at least one selected from a deuterium, -F, -CI, -Br, -I, a hydroxyl group, a cyano group, a nitro group, an amino group, an amidino group, a hydrazine group, a hydrazone group, a carboxylic acid group or a salt thereof, a sulfonic acid group or a salt thereof, a phosphoric acid group or a salt thereof, a C₁-C₁₀ alkyl group, a C₁-C₁₀ alkoxy group, a phenyl group, a naphthyl group, a pyridinyl group, a pyrimidinyl group, a pyrazinyl group, a pyridazinyl group, a triazinyl group and - Si(Q₂₁)(Q₂₂)(Q₂₃),
a1 and a2 are each independently 0 or 1,
wherein Q₁ to Q₃, and Q₂₁ to Q₂₃ are each independently selected from a hydrogen, a C₁-C₁₀ alkyl group, a C₁-C₁₀ alkoxy group, and a phenyl group: 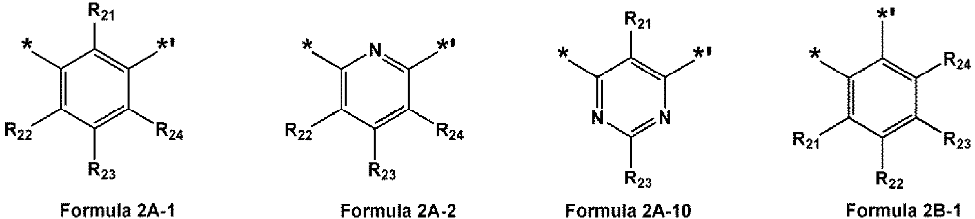 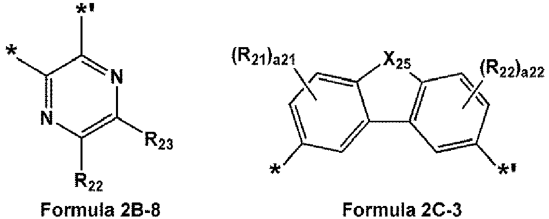 wherein in Formulae 2A-1, 2A-2, 2A-10, 2B-1, 2B-8 and 2C-3,
X₂₅ is O, S, P(=O)(R₂₅), Se or Si(R₂₅)(R₂₆),
R₂₁ to R₂₄ are each independently selected from a hydrogen, a deuterium, a methyl group, an ethyl group, an n-propyl group, an isopropyl group, an n-butyl group, an isobutyl group, a sec-butyl group, a tert-butyl group, a phenyl group, a pyridinyl group, a pyrimidinyl group, a pyrazinyl group, a pyridazinyl group, a triazinyl group, and -Si(Q₁₁)(Q₁₂)(Q₁₃),
R₂₅ and R₂₆ are each independently selected from a hydrogen, a deuterium, a methyl group, an ethyl group, an n-propyl group, an isopropyl group, an n-butyl group, an isobutyl group, a sec-butyl group, a tert-butyl group, a phenyl group, a pyridinyl group, a pyrimidinyl group, a pyrazinyl group, a pyridazinyl group and a triazinyl group,
a21 and a22 are each independently 0 or 1,
wherein Q₁₁ to Q₁₃ are each independently selected from a hydrogen, a C₁-C₁₀ alkyl group, a C₁-C₁₀ alkoxy group, and a phenyl group.

9. The condensed cyclic compound of claim 1, wherein the condensed cyclic compound is one of Compounds 1 to 7, 13 to 18, 26 to 27, 31 to 34, 36, 39, 42 to 44, 46, 47, 49, 50, 52, 53, 55 and 56:

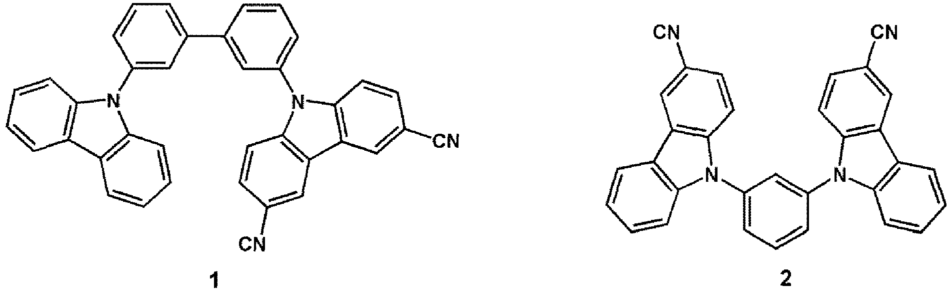

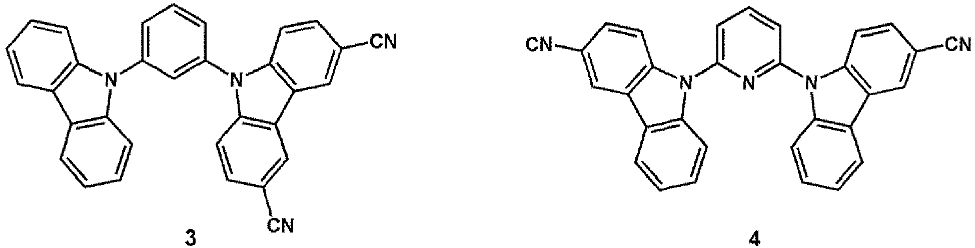

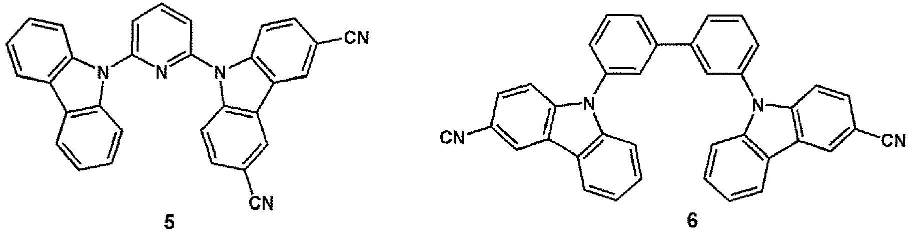

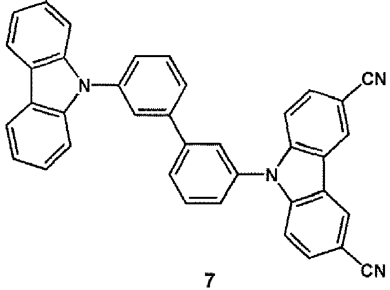

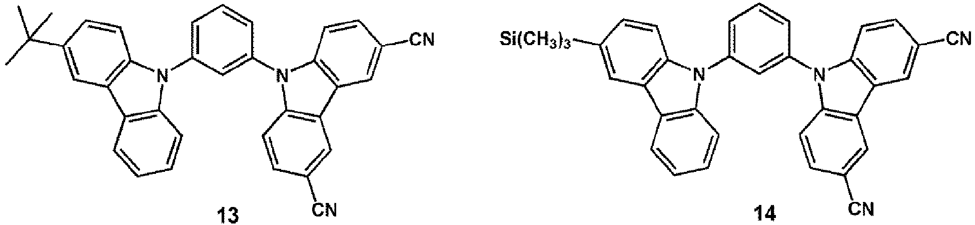

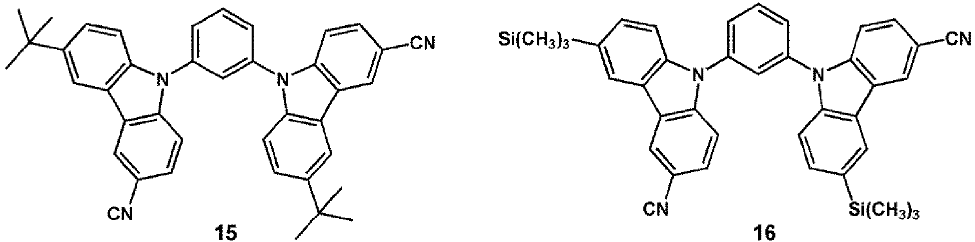

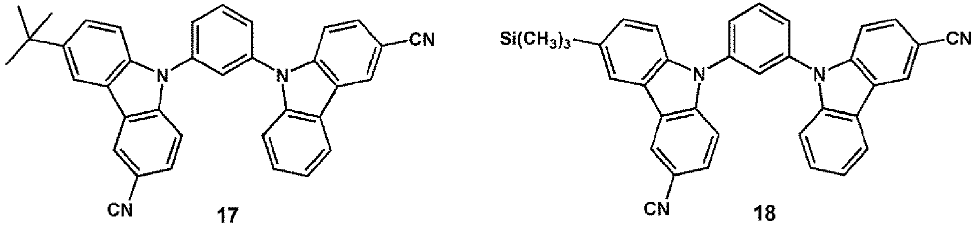

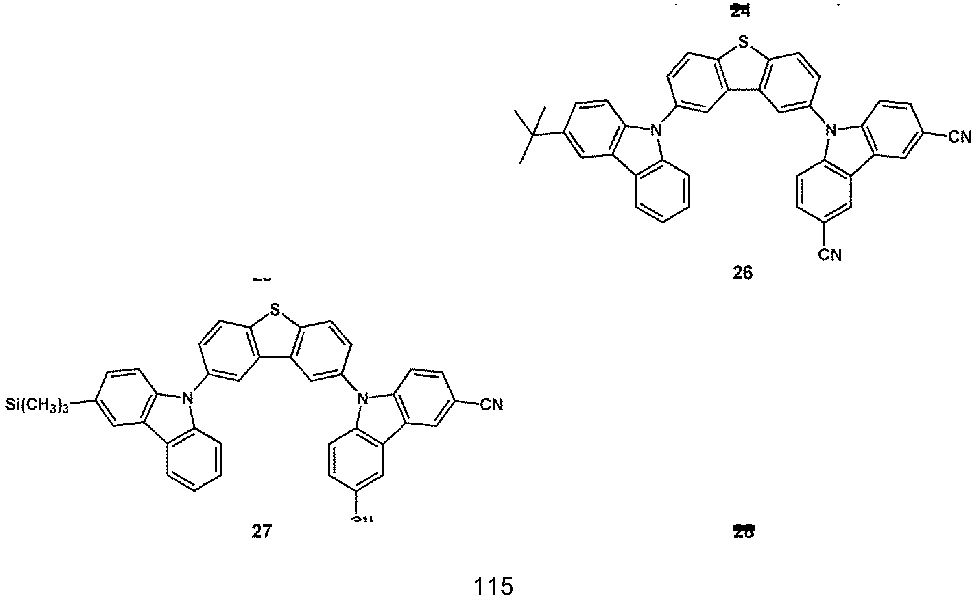

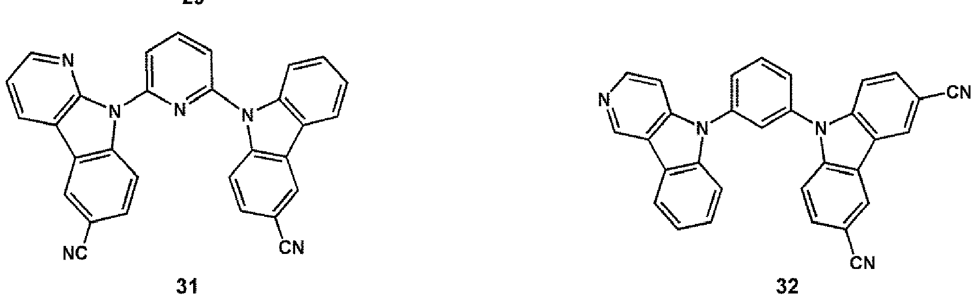

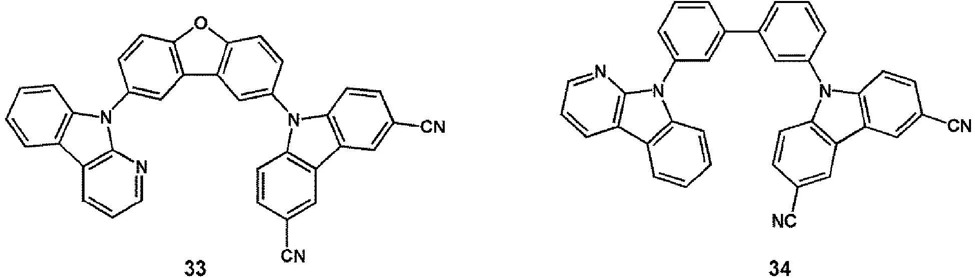

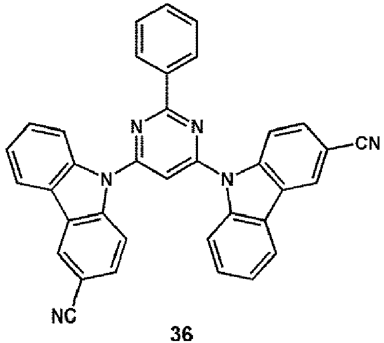

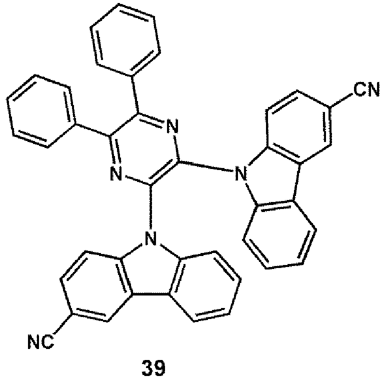

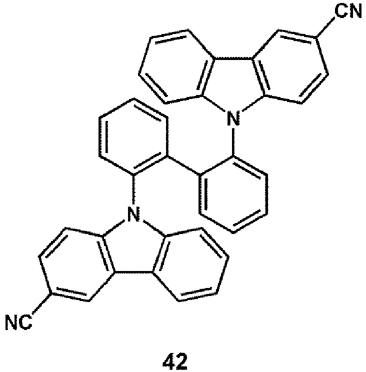

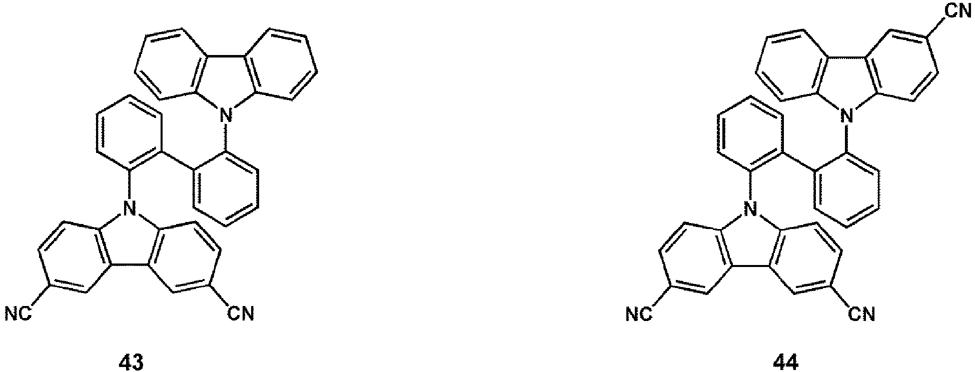

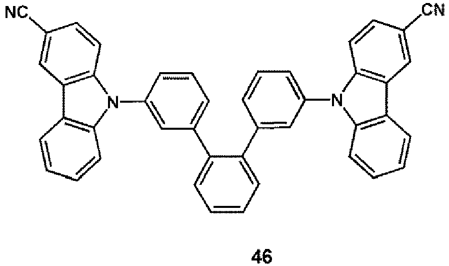

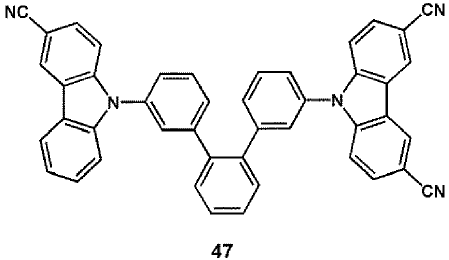

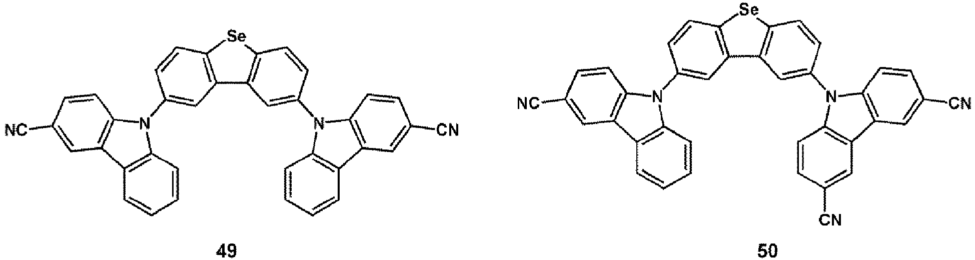

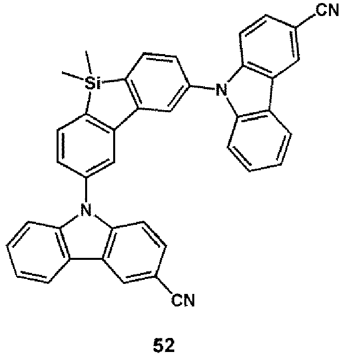

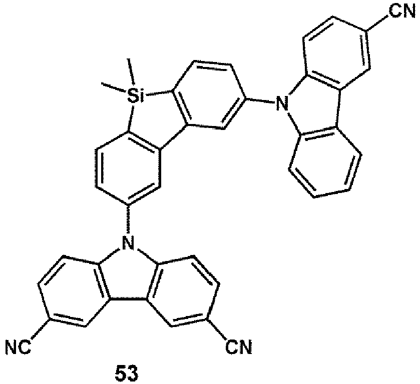

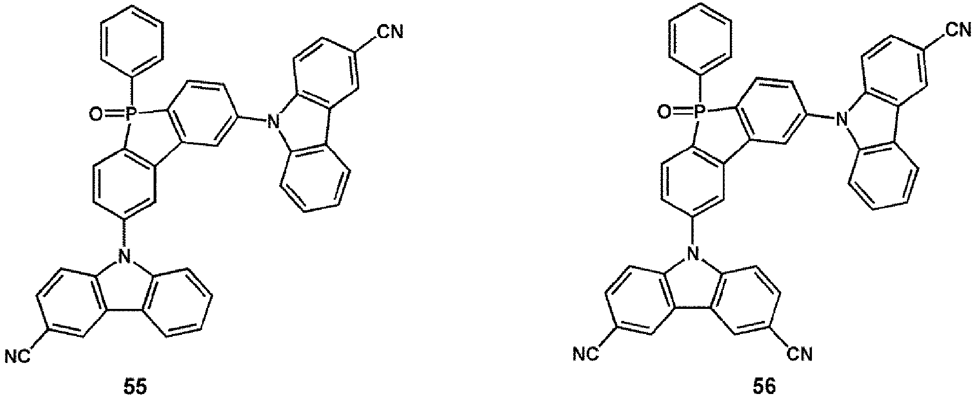

10. An organic light-emitting device comprising a first electrode; a second electrode; and an organic layer between the first electrode and the second electrode, the organic layer comprising an emission layer, wherein the organic layer comprises at least one of the condensed cyclic compounds represented by Formulae 1B to 1D of any of claims 1-9.

11. The organic light-emitting device of claim 10, wherein
the first electrode is an anode, the second electrode is a cathode, and
the organic layer comprises:
i) a hole transport region between the first electrode and the emission layer, the hole transport region comprising at least one selected from a hole injection layer, a hole transport layer, and an electron blocking layer; and
ii) an electron transport region between the emission layer and the second electrode, the electron transport region comprising at least one selected from a hole blocking layer, an electron transport layer, and an electron injection layer.

12. The organic light-emitting device of claim 10 or 11, wherein
The at least one condensed cyclic compound represented by Formulae 1B to 1D is included in the emission layer, the emission layer optionally emitting blue light and/or the condensed cyclic compound represented by Formulae 1B to 1D included in the emission layer optionally is a thermally activated delayed fluorescence emitter.

13. The organic light-emitting device of any of claims 10-12, wherein
The at least one condensed cyclic compound represented by Formulae 1B to 1D is included in the emission layer, the emission layer further comprises a phosphorescent dopant, and an amount of the condensed cyclic compound is greater than an amount of the phosphorescent dopant.

## Patentansprüche

1. Eine kondensierte Ringverbindung, dargestellt durch eine der Formeln 1B bis 1D: 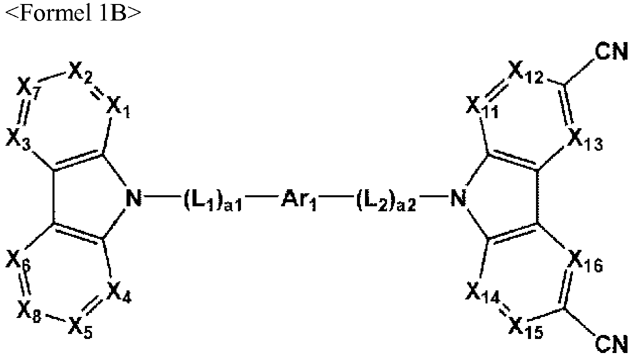 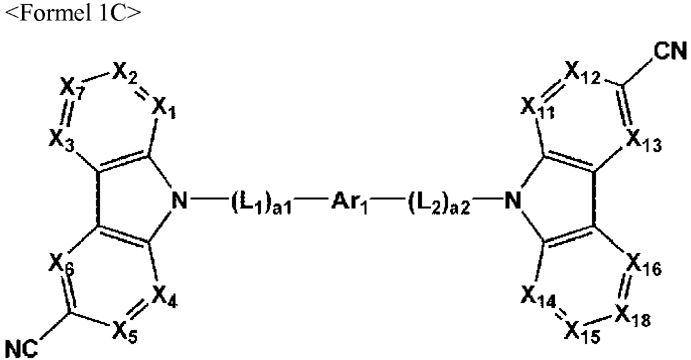 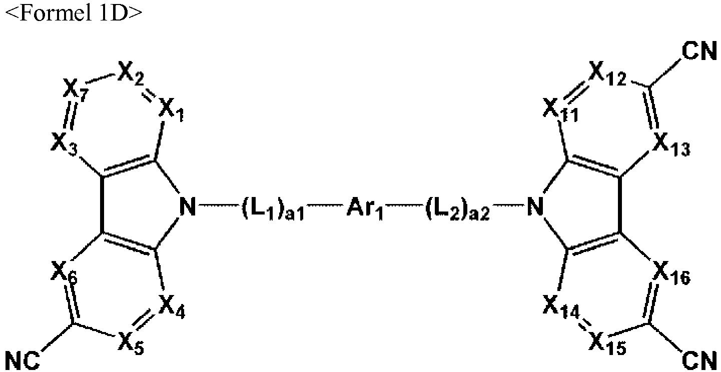 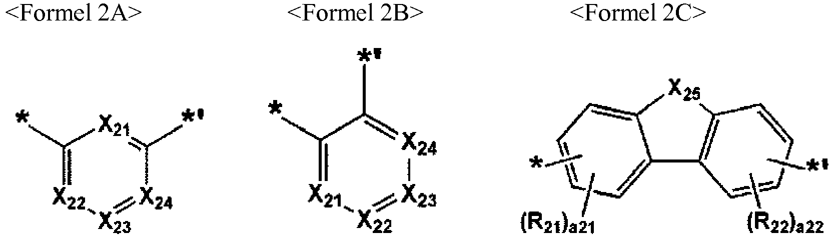 wobei in den Formeln 1B bis 1D und 2A bis 2C,
X₁ N oder C(R₁) ist, X₂ N oder C(R₂) ist, X₃ N oder C(R₃) ist, X₄ N oder C(R₄) ist, X₅ N oder C(R₅) ist, X₆ N oder C(R₆) ist, X₇ N oder C(R₇) ist, X₈ N oder C(R₈) ist, X₁₁ N oder C(R₁₁) ist, X₁₂ N oder C(R₁₂) ist, X₁₃ N oder C(R₁₃) ist, X₁₄ N oder C(R₁₄) ist, X₁₅ N oder C(R₁₅) ist, X₁₆ N oder C(R₁₆) ist, X₁₇ N oder C(R₁₇) ist und X₁₈ N oder C(R₁₈) ist,
R₁ bis R₈ und R₁₁ bis R₁₈ unabhängig ausgewählt sind aus einem Wasserstoff, einem Deuterium, -F, -Cl, -Br, -I, einer Hydroxylgruppe, einer Nitrogruppe, einer Aminogruppe, einer Amidinogruppe, einer Hydrazingruppe, einer Hydrazongruppe, einer Carbonsäuregruppe oder einem Salz davon, einer Schwefelsäuregruppe oder einem Salz davon, einer Phosphorsäuregruppe oder einem Salz davon, einer substituierten oder nicht substituierten C₁-C₆₀-Alkylgruppe, einer substituierten oder nicht substituierten C₂-C₆₀-Alkenylgruppe, einer substituierten oder nicht substituierten C₂-C₆₀-Alkynylgruppe, einer substituierten oder nicht substituierten C₁-C₆₀-Alkoxygruppe, einer substituierten oder nicht substituierten C₃-C₁₀-Cycloalkylgruppe, einer substituierten oder nicht substituierten C₁-C₁₀-Heterocycloalkylgruppe, einer substituierten oder nicht substituierten C₃-C₁₀-Cycloalkenylgruppe, einer substituierten oder nicht substituierten C₁-C₁₀-Heterocycloalkenylgruppe, einer substituierten oder nicht substituierten C₆-C₆₀-Arylgruppe, einer substituierten oder nicht substituierten C₆-C₆₀-Aryloxygruppe, einer substituierten oder nicht substituierten C₆-C₆₀-Arylthiogruppe, einer substituierten oder nicht substituierten C₁-C₆₀-Heteroarylgruppe, einer substituierten oder nicht substituierten, monovalenten, nicht aromatischen, kondensierten Polyringgruppe, einer substituierten oder nicht substituierten, monovalenten, nicht aromatischen, kondensierten Heteropolyringgruppe, vorausgesetzt, dass von den substituierten oder nicht substituierten, monovalenten, nicht aromatischen, kondensierten Heteropolyringgruppen eine substituierte oder nicht substituierte Carbazolylgruppe ausgeschlossen ist, und -Si(Q₁)(Q₂)(Q₃),
Ar₁ dargestellt ist durch eine der Formeln 2A bis 2C,
X₂₁ N oder C(R₂₁) ist, X₂₂ N oder C(R₂₂) ist, X₂₃ N oder C(R₂₃) ist, X₂₄ N oder C(R₂₄) ist und X₂₅ O, S, P(=O)(R₂₅), Se oder Si(R₂₅)(R₂₆) ist,
R₂₁ bis R₂₆ jeweils unabhängig ausgewählt sind aus einem Wasserstoff, einem Deuterium, einer C₁-C₄-Alkylgruppe, einer Phenylgruppe, einer Pyridinylgruppe, einer Pyrimidinylgruppe, einer Pyrazinylgruppe, einer Pyridazinylgruppe, einer Triazinylgruppe und -Si(Q₁₁)(Q₁₂)(Q₁₃),
a21 und a22 abhängig eine ganze Zahl sind, ausgewählt aus 0 bis 3, und wenn a21 2 oder mehr ist, 2 oder mehr R₂₁(s) identisch zueinander oder verschieden voneinander sein können, und wenn a22 2 oder mehr ist, 2 oder mehr R₂₂(s) identisch zueinander oder verschieden voneinander sein können;
L₁ und L₂ jeweils unabhängig ausgewählt sind aus
einer Phenylengruppe, einer Pyridinylengruppe, einer Pyrimidinylengruppe, einer Pyrazinylengruppe, einer Pyridazinylengruppe, einer Triazinylengruppe, einer Dibenzofuranylengruppe und einer Dibenzothiophenylengruppe; und
einer Phenylengruppe, einer Pyridinylengruppe, einer Pyrimidinylengruppe, einer Pyrazinylengruppe, einer Pyridazinylengruppe, einer Triazinylengruppe, einer Dibenzofuranylengruppe und einer Dibenzothiophenylengruppe, jeweils substituiert mit mindestens einem, ausgewählt aus einem Deuterium, -F, -Cl, -Br, -I, einer Hydroxylgruppe, einer Cyanogruppe, einer Nitrogruppe, einer Aminogruppe, einer Amidinogruppe, einer Hydrazingruppe, einer Hydrazongruppe, einer Carbonsäuregruppe oder einem Salz davon, einer Schwefelsäuregruppe oder einem Salz davon, einer Phosphorsäuregruppe oder einem Salz davon, einer C₁-C₁₀-Alkylgruppe, einer C₁-C₁₀-Alkoxygruppe, einer Phenylgruppe, einer Naphthylgruppe, einer Pyridinylgruppe, einer Pyrimidinylgruppe, einer Pyrazinylgruppe, einer Pyridazinylgruppe, einer Triazinylgruppe und -Si(Q₂₁)(Q₂₂)(Q₂₃),
a1 und a2 abhängig eine ganze Zahl sind, ausgewählt aus 0 bis 5, und wenn a1 2 oder mehr ist, 2 oder mehr L₁(s) identisch zueinander oder verschieden voneinander sein können, und wenn a2 2 oder mehr ist, 2 oder mehr L₂(s) identisch zueinander oder verschieden voneinander sein können;
jedes von * und *' eine Bindungsstelle an einem benachbarten Atom angibt;
mindestens einer der Substituenten der substituierten C₁-C₆₀-Alkylgruppe, substituierten C₂-C₆₀-Alkenylgruppe, substituierten C₂-C₆₀-Alkynylgruppe, substituierten C₁-C₆₀-Alkoxygruppe, substituierten C₃-C₁₀-cycloalkylgruppe, substituierten C₁-C₁₀-Heterocycloalkylgruppe, substituierten C₃-C₁₀-Cycloalkenylgruppe, substituierten C₁-C₁₀-Heterocycloalkenylgruppe, substituierten C₆-C₆₀-Arylgruppe, substituierten C₆-C₆₀-Aryloxygruppe, substituierten C₆-C₆₀-Arylthiogruppe, substituierten C₁-C₆₀-Heteroarylgruppe, substituierten, monovalenten, nicht aromatischen, kondensierten Polyringgruppe und substituierten, monovalenten, nicht aromatischen, kondensierten Heteropolyringgruppe ausgewählt ist aus einem Deuterium, -F, -Cl, -Br, -I, einer Hydroxylgruppe, einer Cyanogruppe, einer Nitrogruppe, einer Aminogruppe, einer Amidinogruppe, einer Hydrazingruppe, einer Hydrazongruppe, einer Carbonsäuregruppe oder einem Salz davon, einer Schwefelsäuregruppe oder einem Salz davon, einer Phosphorsäuregruppe oder einem Salz davon, einer C₁-C₆₀-Alkylgruppe, einer C₂-C₆₀-Alkenylgruppe, einer C₂-C₆₀Alkynylgruppe, einer C₁-C₆₀-Alkoxygruppe, einer C₃-C₁₀-Cycloalkylgruppe, einer C₁-C₁₀-Heterocycloalkylgruppe, einer C₃-C₁₀-Cycloalkenylgruppe, einer C₁-C₁₀-Heterocycloalkenylgruppe, einer C₆-C₆₀-Arylgruppe, einer C₆-C₆₀-Aryloxygruppe, einer C₆-C₆₀-Arylthiogruppe, einer C₁-C₆₀-Heteroarylgruppe, einer monovalenten, nicht aromatischen, kondensierten Polyringgruppe, einer monovalenten, nicht aromatischen, kondensierten Heteropolyringgruppe , vorausgesetzt, dass die monovalente, nicht aromatische, kondensierte Heteropolyringgruppe keine Carbazolylgruppe ist, und -Si(Q₃₁)(Q₃₂)(Q₃₃),
wobei Q₁ bis Q₃, Q₁₁ bis Q₁₃, Q₂₁ bis Q₂₃, und Q₃₁ bis Q₃₃ jeweils unabhängig ausgewählt sind aus einem Wasserstoff, einer C₁-C₆₀-Alkylgruppe, einer C₁-C₆₀-Alkoxygruppe, einer C₃-C₁₀-Cycloalkylgruppe, einer C₁-C₁₀-Heterocycloalkylgruppe, einer C₃-C₁₀-Cycloalkenylgruppe, einer C₁-C₁₀-Heterocycloalkenylgruppe, einer C₆-C₆₀-Arylgruppe, einer C₁-C₆₀-Heteroarylgruppe, einer monovalenten, nicht aromatischen, kondensierten Polyringgruppe und einer monovalenten, nicht aromatischen, kondensierten Heteropolyringgruppe, vorausgesetzt, dass die monovalente, nicht aromatische, kondensierte Heteropolyringgruppe keine Carbazolylgruppe ist, mit der Maßgabe, dass die Ringverbindung keine der unteren Verbindungen ist: 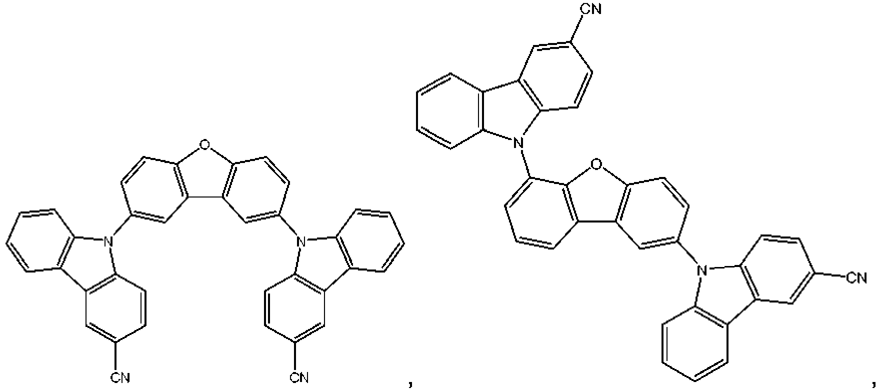 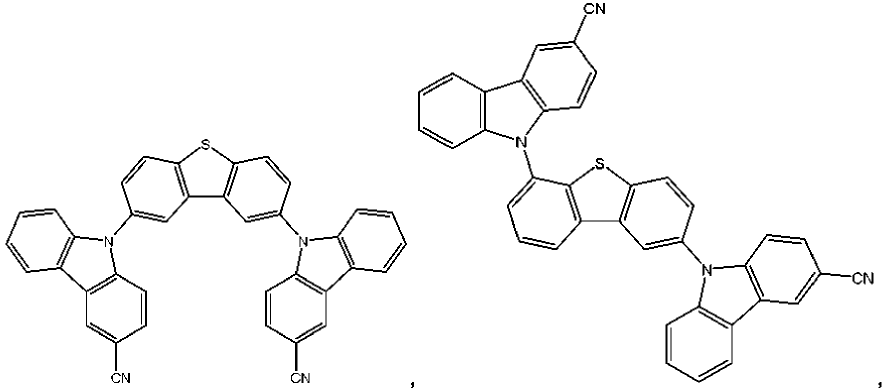 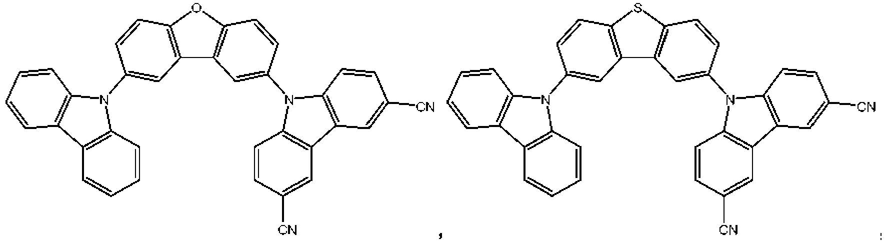 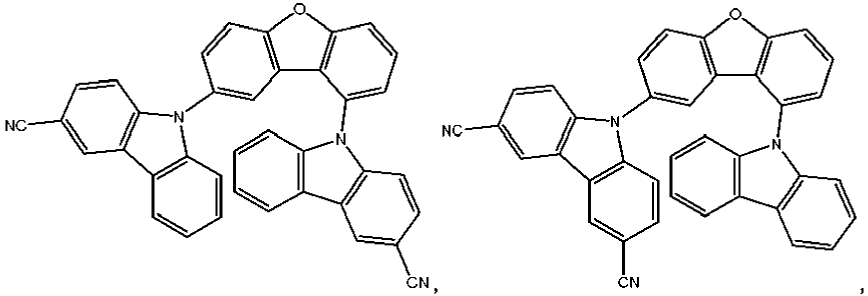 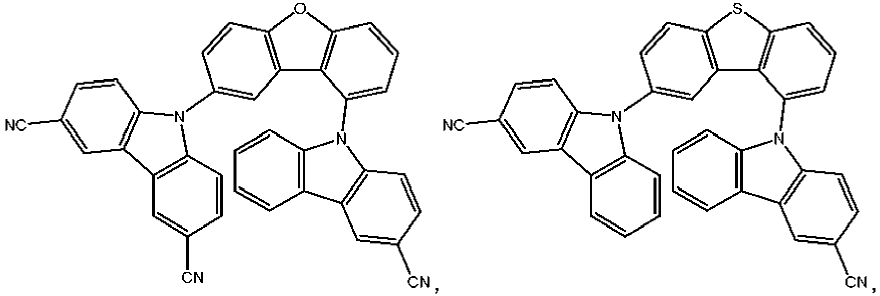 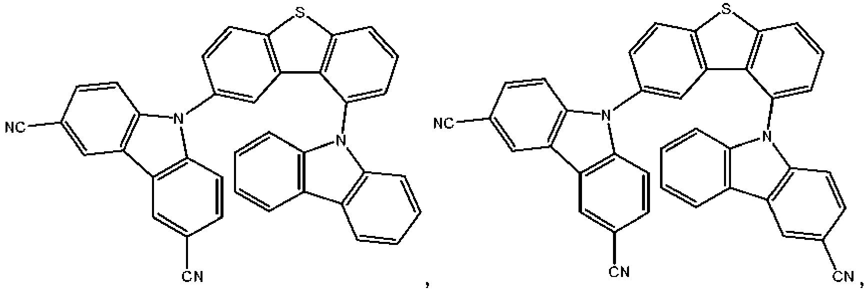 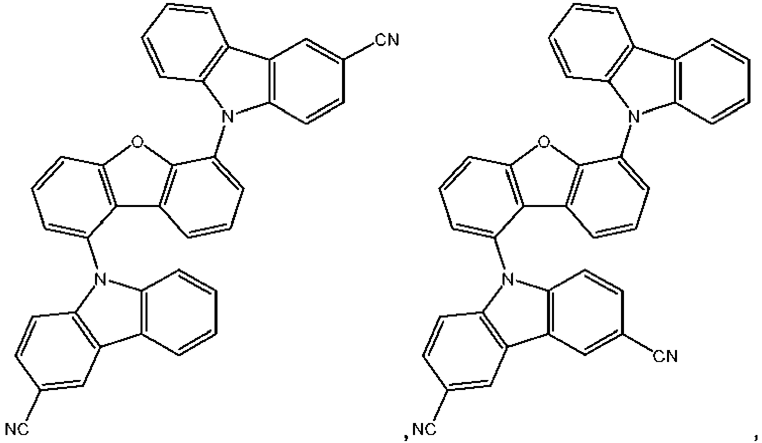 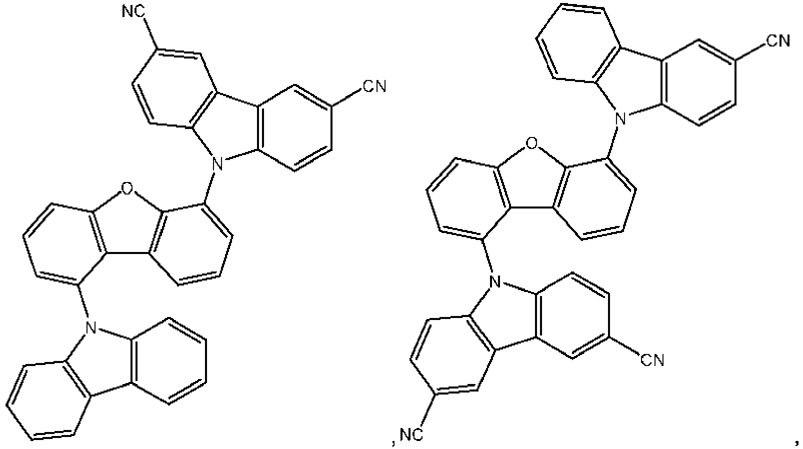 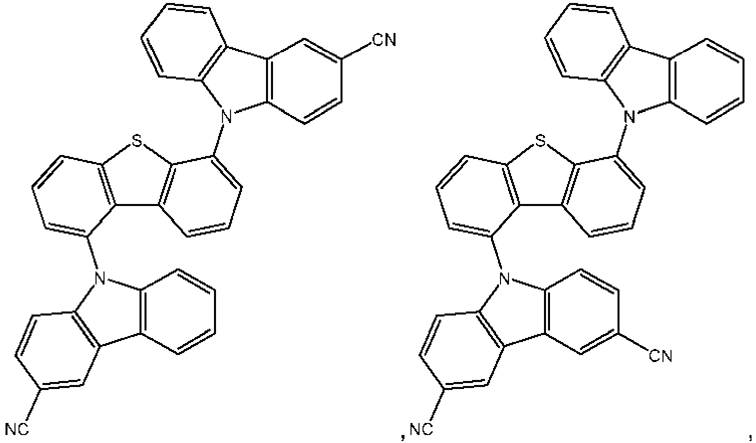 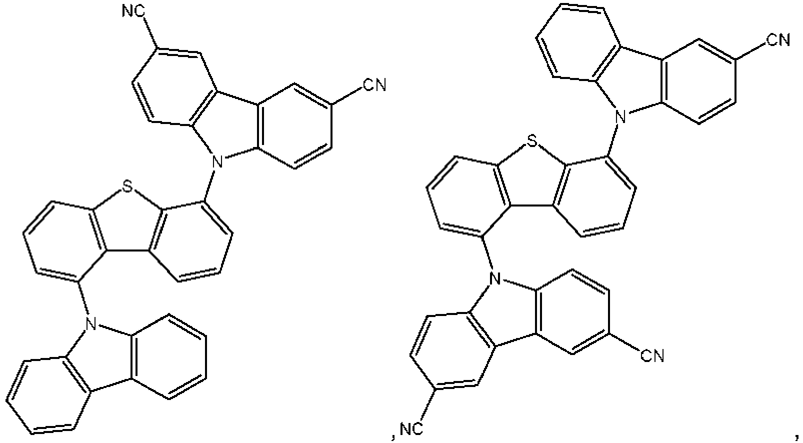 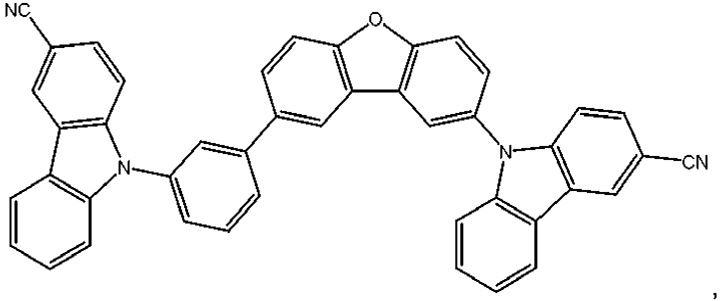 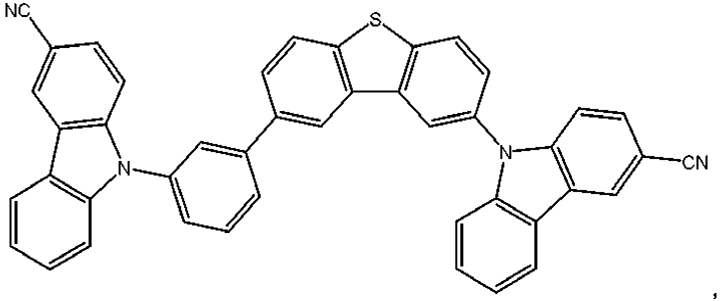 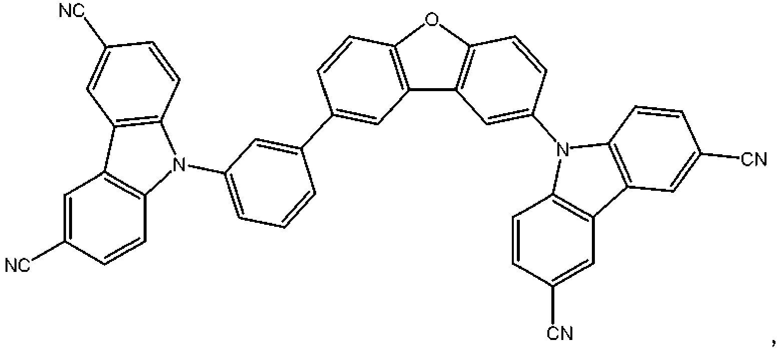 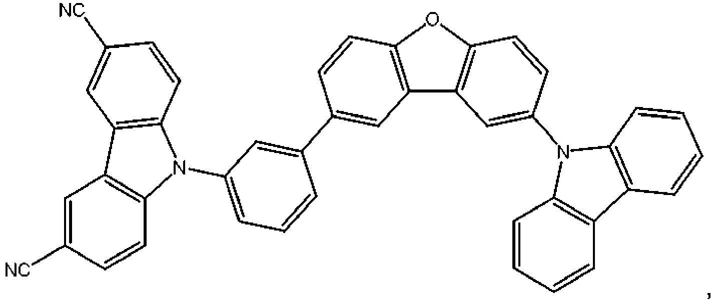 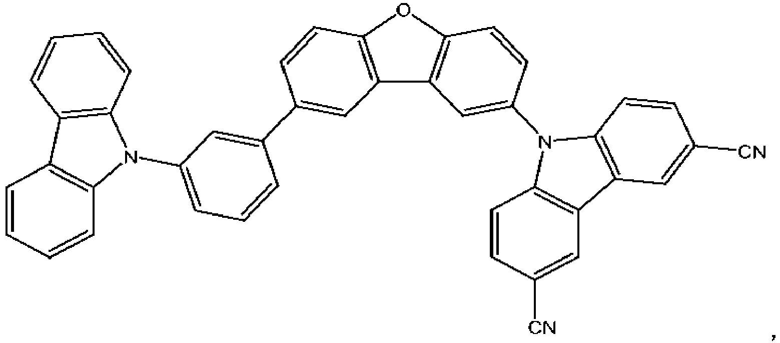 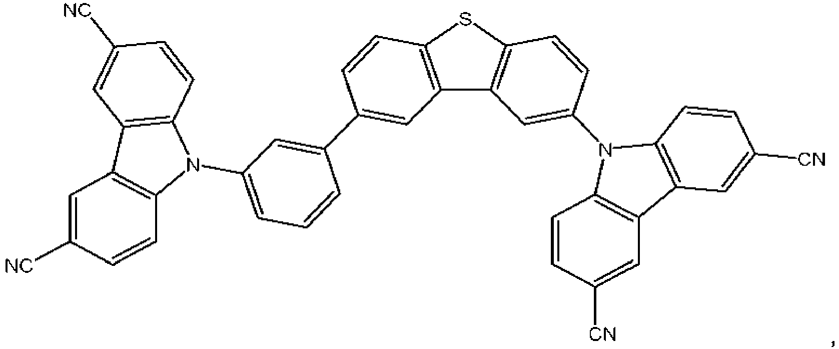 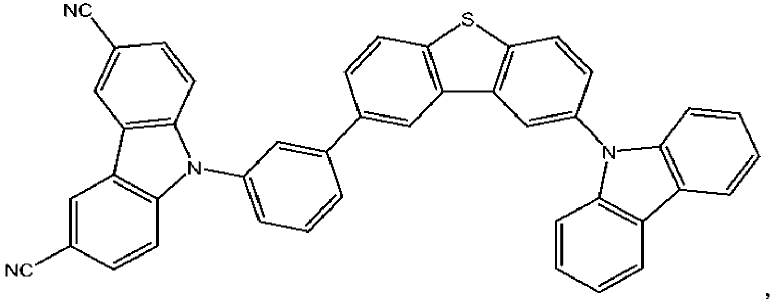 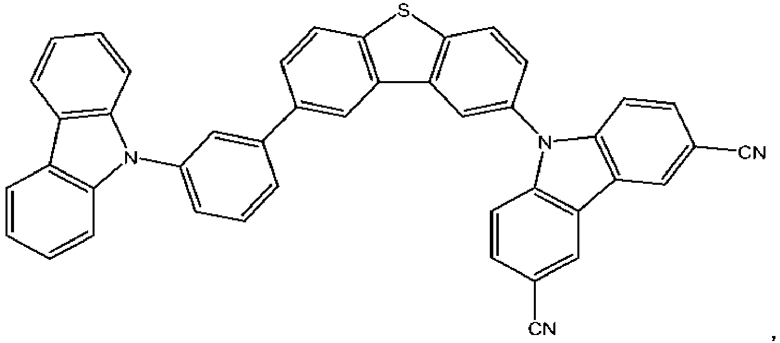 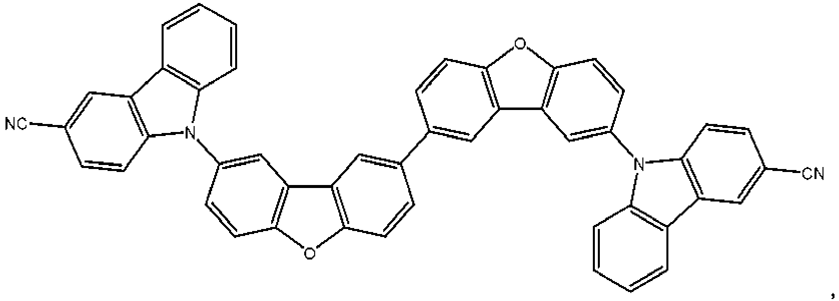 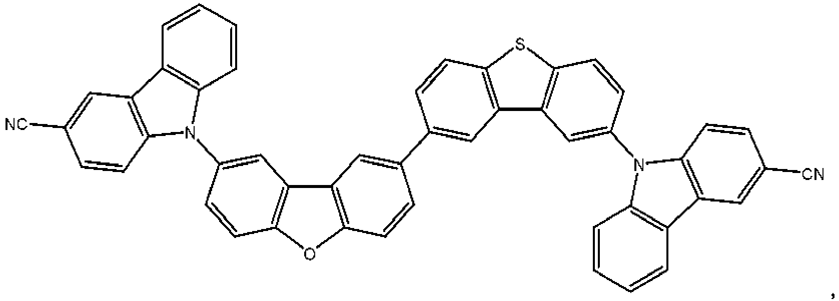 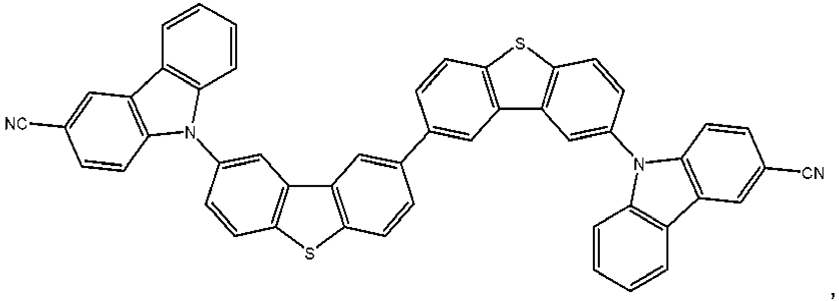 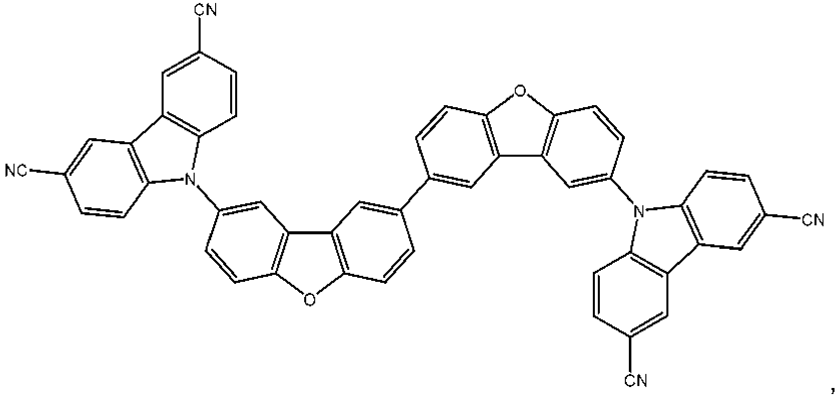 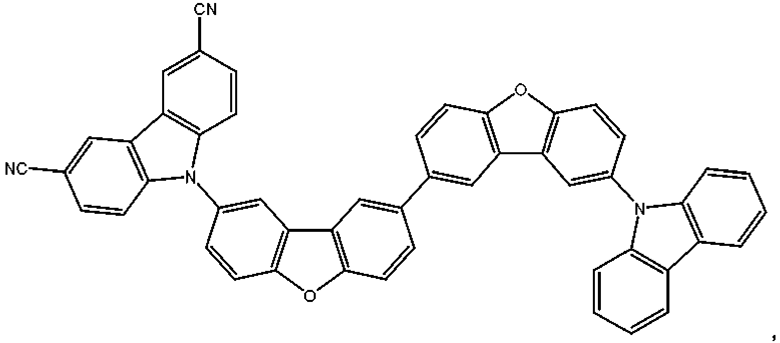 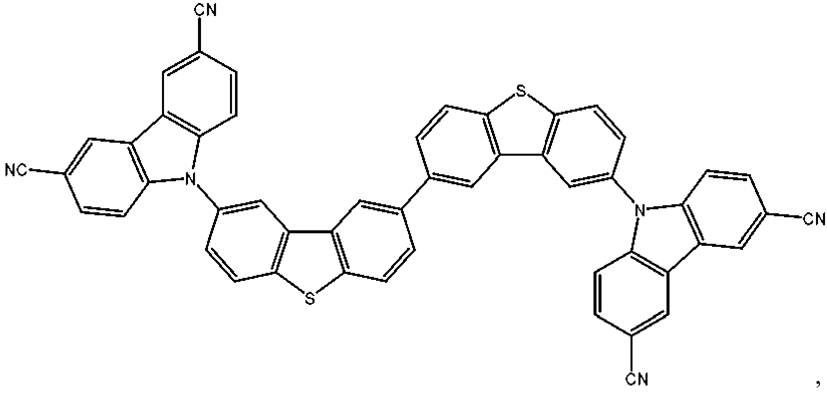 oder

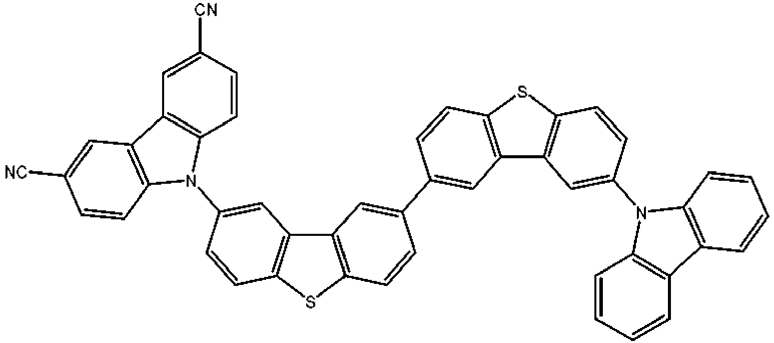

2. Die kondensierte Ringverbindung gemäß Anspruch 1, wobei R₁ bis R₈ und R₁₁ bis R₁₈ jeweils unabhängig ausgewählt sind aus einem Wasserstoff, einem Deuterium, -F, -Cl, -Br, -I, einer Hydroxylgruppe, einer Nitrogruppe, einer Aminogruppe, einer Amidinogruppe, einer Hydrazingruppe, einer Hydrazongruppe, einer Carbonsäuregruppe oder einem Salz davon, einer Schwefelsäuregruppe oder einem Salz davon, einer Phosphorsäuregruppe oder einem Salz davon, einer C₁-C₂₀-Alkylgruppe und einer C₁-C₂₀-Alkoxygruppe;
einer C₁-C₂₀-Alkylgruppe und einer C₁-C₂₀-Alkoxygruppe, jeweils substituiert mit mindestens einem, ausgewählt aus einem Deuterium, -F, -Cl, -Br, -I, einer Hydroxylgruppe, einer Cyanogruppe, einer Nitrogruppe, einer Aminogruppe, einer Amidinogruppe, einer Hydrazingruppe, einer Hydrazongruppe, einer Carbonsäuregruppe oder einem Salz davon, einer Schwefelsäuregruppe oder einem Salz davon, einer Phosphorsäuregruppe oder einem Salz davon, einer Phenylgruppe, einer Naphthylgruppe, einer Pyridinylgruppe, einer Pyrimidinylgruppe, einer Pyrazinylgruppe, einer Pyridazinylgruppe und einer Triazinylgruppe;
einer Cyclopentylgruppe, einer Cyclohexylgruppe, einer Cyclopentenylgruppe, einer Cyclohexenylgruppe, einer Cycloheptenylgruppe, einer Phenylgruppe, einer Pentalenylgruppe, einer Indenylgruppe, einer Naphthylgruppe, einer Azulenylgruppe, einer Heptalenylgruppe, einer Indacenylgruppe, einer Acenaphthylgruppe, einer Fluorenylgruppe, einer Spiro-Fluorenylgruppe, einer Phenalenylgruppe, einer Phenanthrenylgruppe, einer Anthracenylgruppe, einer Fluoranthrenylgruppe, einer Triphenylenylgruppe, einer Pyrenylgruppe, einer Chrysenylgruppe, einer Naphthacenylgruppe, einer Picenylgruppe, einer Perylenylgruppe, einer Pentaphenylgruppe, einer Hexacenylgruppe, einer Pyrrolylgruppe, einer Imidazolylgruppe, einer Pyrazolylgruppe, einer Pyridinylgruppe, einer Pyrazinylgruppe, einer Pyrimidinylgruppe, einer Pyridazinylgruppe, einer Isoindolylgruppe, einer Indolylgruppe, einer Indazolylgruppe, einer Purinylgruppe, einer Chinolinylgruppe, einer Isochinolinylgruppe, einer benzochinolinylgruppe, einer Phthalazinylgruppe, einer Naphthyridinylgruppe, einer chinoxalinylgruppe, einer chinazolinylgruppe, einer Cinnolinylgruppe, einer Phenanthridinylgruppe, einer Acridinylgruppe, einer Phenanthrolinylgruppe, einer Phenazinylgruppe, einer Benzoxazolylgruppe, einer Benzoimidazolylgruppe, einer Furanylgruppe, einer Benzofuranylgruppe, einer Thiophenylgruppe, einer Benzothiophenylgruppe, einer Thiazolylgruppe, einer Isothiazolylgruppe, einer Benzothiazolylgruppe, einer Isoxazolylgruppe, einer Oxazolylgruppe, einer Triazolylgruppe, einer Tetrazolylgruppe, einer Oxadiazolylgruppe, einer Triazinylgruppe, einer Dibenzofuranylgruppe, einer Dibenzothiophenylgruppe, einer Imidazopyridimidinylgruppe und einer Imidazopyridinylgruppe;
einer Cyclopentylgruppe, einer Cyclohexylgruppe, einer Cyclopentenylgruppe, einer Cyclohexenylgruppe, einer Cycloheptenylgruppe, einer Phenylgruppe, einer Pentalenylgruppe, einer Indenylgruppe, einer Naphthylgruppe, einer Azulenylgruppe, einer Heptalenylgruppe, einer Indacenylgruppe, einer Acenaphthylgruppe, einer Fluorenylgruppe, einer Spiro-Fluorenylgruppe, einer Phenalenylgruppe, einer Phenanthrenylgruppe, einer Anthracenylgruppe, einer Fluoranthrenylgruppe, einer Triphenylenylgruppe, einer Pyrenylgruppe, einer Chrysenylgruppe, einer Naphthacenylgruppe, einer Picenylgruppe, einer Perylenylgruppe, einer Pentaphenylgruppe, einer Hexacenylgruppe, einer Pyrrolylgruppe, einer Imidazolylgruppe, einer Pyrazolylgruppe, einer Pyridinylgruppe, einer Pyrazinylgruppe, einer Pyrimidinylgruppe, einer Pyridazinylgruppe, einer Isoindolylgruppe, einer Indolylgruppe, einer Indazolylgruppe, einer Purinylgruppe, einer Chinolinylgruppe, einer Isochinolinylgruppe, einer benzochinolinylgruppe, einer Phthalazinylgruppe, einer Naphthyridinylgruppe, einer Chinoxalinylgruppe, einer Chinazolinylgruppe, einer Cinnolinylgruppe, einer Phenanthridinylgruppe, einer Acridinylgruppe, einer Phenanthrolinylgruppe, einer Phenazinylgruppe, einer Benzoxazolylgruppe, einer Benzoimidazolylgruppe, einer Furanylgruppe, einer Benzofuranylgruppe, einer Thiophenylgruppe, einer Benzothiophenylgruppe, einer Thiazolylgruppe, einer Isothiazolylgruppe, einer Benzothiazolylgruppe, einer Isoxazolylgruppe, einer Oxazolylgruppe, einer Triazolylgruppe, einer Tetrazolylgruppe, einer Oxadiazolylgruppe, einer Triazinylgruppe, einer Dibenzofuranylgruppe, einer Dibenzothiophenylgruppe, einer Imidazopyridimidinylgruppe und einer Imidazopyridinylgruppe; jeweils substituiert mit mindestens einem, ausgewählt aus einem Deuterium, -F, -Cl, -Br, -I, einer Hydroxylgruppe, einer Cyanogruppe, einer Nitrogruppe, einer Aminogruppe, einer Amidinogruppe, einer Hydrazingruppe, einer Hydrazongruppe, einer Carbonsäuregruppe oder einem Salz davon, einer Schwefelsäuregruppe oder einem Salz davon, einer Phosphorsäuregruppe oder einem Salz davon, einer C₁-C₂₀-Alkylgruppe, einer C₂-C₂₀-Alkenylgruppe, einer C₂-C₂₀-Alkynylgruppe, einer C₁-C₂₀-Alkoxygruppe, einer Phenylgruppe, einer Naphthylgruppe, einer Anthracenylgruppe, einer Pyrenylgruppe, einer Phenanthrenylgruppe, einer Fluorenylgruppe, einer Pyridinylgruppe, einer Pyrimidinylgruppe, einer Pyrazinylgruppe, einer Pyridazinylgruppe, einer Triazinylgruppe, einer Chinolinylgruppe, einer Isochinolinylgruppe, einer Phthalazinylgruppe, einer Chinoxalinylgruppe, einer Cinnolinylgruppe, einer Chinazolinylgruppe und -Si(Q₃₁)(Q₃₂)(Q₃₃); und
-Si(Q₁)(Q₂)(Q₃),
wobei Q₁ bis Q₃ und Q₃₁ bis Q₃₃ jeweils unabhängig ausgewählt sind aus einem Wasserstoff, einer C₁-C₂₀-Alkylgruppe, einer C₁-C₂₀-Alkoxygruppe, einer Phenylgruppe, einer Naphthylgruppe, einer Anthracenylgruppe, einer Pyrenylgruppe, einer Phenanthrenylgruppe, einer Fluorenylgruppe, einer Pyridinylgruppe, einer Pyrimidinylgruppe, einer Pyrazinylgruppe, einer Pyridazinylgruppe, einer Triazinylgruppe, einer Chinolinylgruppe, einer Isochinolinylgruppe, einer Phthalazinylgruppe, einer Chinoxalinylgruppe, einer Cinnolinylgruppe und einer Chinazolinylgruppe, wobei bevorzugt R₁ bis R₈ und R₁₁ bis R₁₈ jeweils unabhängig ausgewählt sind aus einem Deuterium, einer Cyanogruppe, einer Methylgruppe, einer Ethylgruppe, einer n-Propylgruppe, einer Isopropylgruppe, einer n-Butylgruppe, einer Isobutylgruppe, einer sec-Butylgruppe, einer tert-Butylgruppe, einer n-Pentylgruppe, einer Isopentylgruppe, einer sec-Pentylgruppe, einer tert-Pentylgruppe, einer n-Hexylgruppe, einer iso-Hexylgruppe, einer sec-Hexylgruppe, einer tert-Hexylgruppe und -Si(Q₁)(Q₂)(Q₃),
wobei Q₁ bis Q₃ jeweils unabhängig ausgewählt sind aus einem Wasserstoff, einer C₁-C₁₀-Alkylgruppe, einer C₁-C₁₀-Alkoxygruppe und einer Phenylgruppe.

3. Die kondensierte Ringverbindung gemäß Anspruch 1, wobei
R₁ bis R₆ und R₁₁ bis R₁₆ unabhängig ausgewählt sind aus
einem Wasserstoff, einem Deuterium, -F, -Cl, -Br, -I, einer Hydroxylgruppe, einer Cyanogruppe, einer Nitrogruppe, einer Aminogruppe, einer Amidinogruppe, einer Hydrazingruppe, einer Hydrazongruppe, einer Carbonsäuregruppe oder einem Salz davon, einer Schwefelsäuregruppe oder einem Salz davon, einer Phosphorsäuregruppe oder einem Salz davon, einer C₁-C₁₀-Alkylgruppe und einer C₁-C₁₀-Alkoxygruppe;
einer Phenylgruppe, einer Naphthylgruppe, einer Pyridinylgruppe, einer Pyrimidinylgruppe, einer Pyrazinylgruppe und einer Triazinylgruppe;
einer Phenylgruppe, einer Naphthylgruppe, einer Pyridinylgruppe, einer Pyrimidinylgruppe, einer Pyrazinylgruppe, einer Pyridazinylgruppe, einer Triazinylgruppe, jeweils substituiert mit mindestens einem, ausgewählt aus einem Deuterium, -F, -Cl, -Br, -I, einer Hydroxylgruppe, einer Nitrogruppe, einer Aminogruppe, einer Amidinogruppe, einer Hydrazingruppe, einer Hydrazongruppe, einer Carbonsäuregruppe oder einem Salz davon, einer Schwefelsäuregruppe oder einem Salz davon, einer Phosphorsäuregruppe oder einem Salz davon, einer C₁-C₁₀-Alkylgruppe, einer C₁-C₁₀-Alkoxygruppe, einer Phenylgruppe, einer Naphthylgruppe, einer Pyridinylgruppe, einer Pyrimidinylgruppe, einer Pyrazinylgruppe, einer Pyridazinylgruppe, einer Triazinylgruppe und -Si(Q₃₁)(Q₃₂)(Q₃₃); und
-Si(Q₁)(Q₂)(Q₃),
R₇, R₈, R₁₇ und R₁₈ jeweils unabhängig ausgewählt sind aus
einem Wasserstoff, einem Deuterium, -F, -Cl, -Br, -I, einer Hydroxylgruppe, einer Nitrogruppe, einer Aminogruppe, einer Amidinogruppe, einer Hydrazingruppe, einer Hydrazongruppe, einer Carbonsäuregruppe oder einem Salz davon, einer Schwefelsäuregruppe oder einem Salz davon, einer Phosphorsäuregruppe oder einem Salz davon, einer C₁-C₁₀-Alkylgruppe und einer C₁-C₁₀-Alkoxygruppe;
einer Phenylgruppe, einer Naphthylgruppe, einer Pyridinylgruppe, einer Pyrimidinylgruppe, einer Pyrazinylgruppe und einer Triazinylgruppe;
einer Phenylgruppe, einer Naphthylgruppe, einer Pyridinylgruppe, einer Pyrimidinylgruppe, einer Pyrazinylgruppe, einer Pyridazinylgruppe, einer Triazinylgruppe, jeweils substituiert mit mindestens einem, ausgewählt aus einem Deuterium, -F, -Cl, -Br, -I, einer Hydroxylgruppe, einer Cyanogruppe, einer Nitrogruppe, einer Aminogruppe, einer Amidinogruppe, einer Hydrazingruppe, einer Hydrazongruppe, einer Carbonsäuregruppe oder einem Salz davon, einer Schwefelsäuregruppe oder einem Salz davon, einer Phosphorsäuregruppe oder einem Salz davon, einer C₁-C₁₀-Alkylgruppe, einer C₁-C₁₀-Alkoxygruppe, einer Phenylgruppe, einer Naphthylgruppe, einer Pyridinylgruppe, einer Pyrimidinylgruppe, einer Pyrazinylgruppe, einer Pyridazinylgruppe, einer Triazinylgruppe und -Si(Q₃₁)(Q₃₂)(Q₃₃); und
-Si(Q₁)(Q₂)(Q₃),
wobei Q₁ bis Q₃ und Q₃₁ bis Q₃₃ jeweils unabhängig ausgewählt sind aus einem Wasserstoff, einer C₁-C₁₀-Alkylgruppe, einer C₁-C₁₀-Alkoxygruppe, einer Phenylgruppe, einer Naphthylgruppe, einer Pyridinylgruppe, einer Pyrimidinylgruppe, einer Pyrazinylgruppe, einer Pyridazinylgruppe, und einer Triazinylgruppe.

4. Die kondensierte Ringverbindung gemäß Anspruch 1, wobei
Ar₁ dargestellt ist durch eine der Formeln 2A-1 bis 2A-10, 2B-1 bis 2B-8 und 2C-1 bis 2C-9: 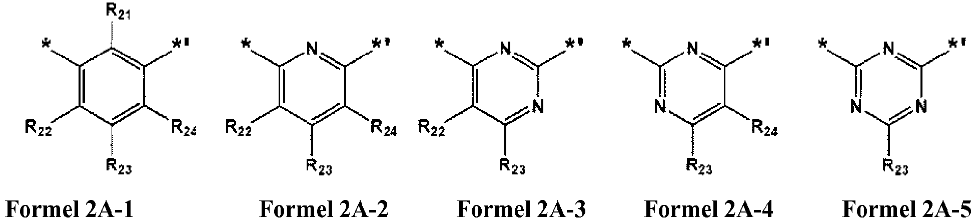 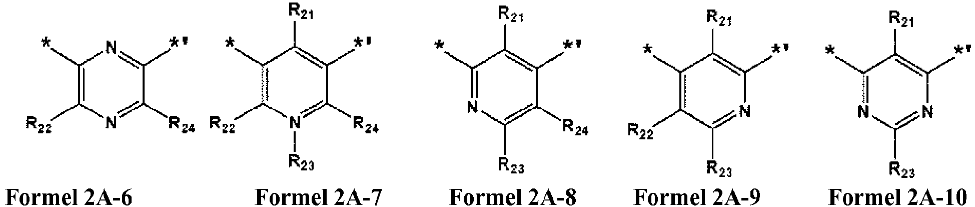 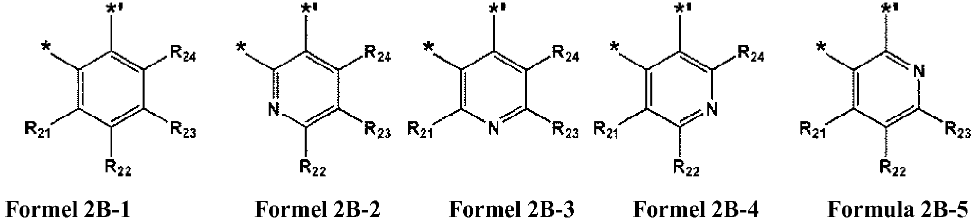 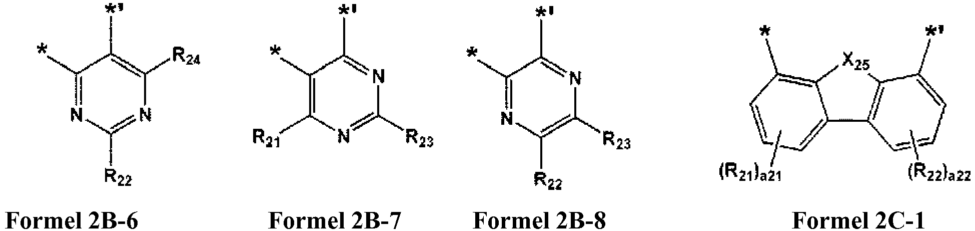 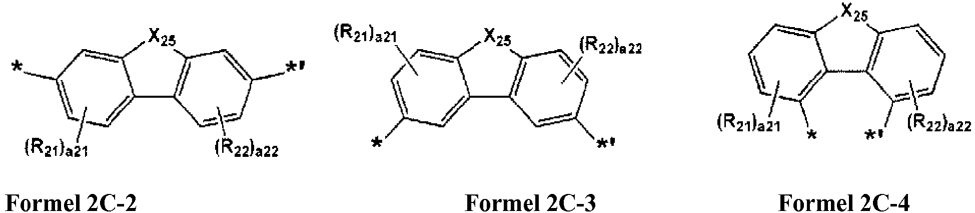 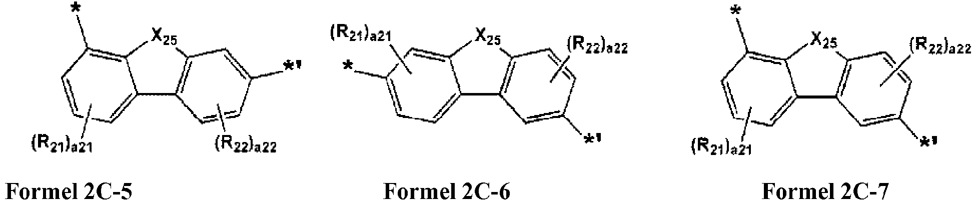 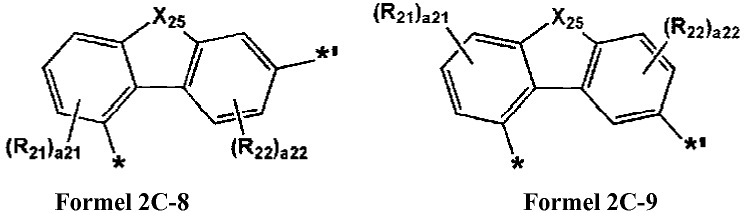 wobei in den Formeln 2A-1 bis 2A-10, 2B-1 bis 2B-8 und 2C-1 bis 2C-9,
X₂₅ O, S, P(=O)(R₂₅), Se oder Si(R₂₅)(R₂₆), ist,
R₂₁ to R₂₄ jeweils unabhängig ausgewählt sind aus einem Wasserstoff, einem Deuterium, einer Methylgruppe, einer Ethylgruppe, einer n-Propylgruppe, einer Isopropylgruppe, einer n-Butylgruppe, einer Isobutylgruppe, einer sec-Butylgruppe, einer tert-Butylgruppe, einer Phenylgruppe, einer Pyridinylgruppe, einer Pyrimidinylgruppe, einer Pyrazinylgruppe, einer Pyridazinylgruppe, einer Triazinylgruppe und -Si(Q₁₁)(Q₁₂)(Q₁₃),
R₂₅ und R₂₆ jeweils unabhängig ausgewählt sind aus einem Wasserstoff, einem Deuterium, einer Methylgruppe, einer Ethylgruppe, einer n-Propylgruppe, einer Isopropylgruppe, einer n-Butylgruppe, einer Isobutylgruppe, einer sec-Butylgruppe, einer tert-Butylgruppe, einer Phenylgruppe, einer Pyridinylgruppe, einer Pyrimidinylgruppe, einer Pyrazinylgruppe, einer Pyridazinylgruppe und einer Triazinylgruppe,
wobei Q₁₁ bis Q₁₃ jeweils unabhängig ausgewählt sind aus einem Wasserstoff, einer C₁-C₁₀-Alkylgruppe, einer C₁-C₁₀-Alkoxygruppe und einer Phenylgruppe,
a21 und a22 jeweils unabhängig 0 oder 1 sind, und
jedes von * und *' eine Bindungsstelle an einem benachbarten Atom angibt.

5. Die kondensierte Ringverbindung gemäß einem der Ansprüche 1-3, wobei
L₁ und L₂ jeweils unabhängig ausgewählt sind aus
einer Phenylengruppe, einer Pyridinylengruppe, einer Pyrimidinylengruppe, einer Triazinylengruppe; und
einer Phenylengruppe, einer Pyridinylengruppe, einer Pyrimidinylengruppe und einer Triazinylengruppe, jeweils substituiert mit mindestens einem, ausgewählt aus einem Deuterium, einer Methylgruppe, einer Ethylgruppe, einer n-Propylgruppe, einer Isopropylgruppe, einer n-Butylgruppe, einer Isobutylgruppe, einer sec-Butylgruppe, einer tert-Butylgruppe, einer Phenylgruppe, einer Pyridinylgruppe, einer Pyrimidinylgruppe, einer Pyrazinylgruppe, einer Pyridazinylgruppe, einer Triazinylgruppe und -Si(Q₂₁)(Q₂₂)(Q₂₃), wobei bevorzugt
L₁ und L₂ jeweils unabhängig ausgewählt sind aus
einer Phenylengruppe; und
einer Phenylengruppe, jeweils substituiert mit mindestens einem, ausgewählt aus einem Deuterium, einer Methylgruppe, einer Ethylgruppe, einer n-Propylgruppe, einer Isopropylgruppe, einer n-Butylgruppe, einer Isobutylgruppe, einer sec-Butylgruppe, einer tert-Butylgruppe, einer Phenylgruppe, einer Pyridinylgruppe, einer Pyrimidinylgruppe, einer Pyrazinylgruppe, einer Pyridazinylgruppe, einer Triazinylgruppe und
-Si(Q₂₁)(Q₂₂)(Q₂₃),
wobei Q₂₁ bis Q₂₃ jeweils unabhängig ausgewählt sind aus einem Wasserstoff, einer C₁-C₁₀-Alkylgruppe, einer C₁-C₁₀-Alkoxygruppe und einer Phenylgruppe und
a1 und a2 jeweils unabhängig 0 oder 1 sind.

6. Die kondensierte Ringverbindung gemäß Anspruch 4, wobei
a1 und a2 in den Formeln 1B bis 1D 0 ist und Ar₁ dargestellt ist durch eine der Formeln 2A-1, 2A-2, 2A-10, 2B-1, 2B-8 und 2C-3.

7. Die kondensierte Ringverbindung gemäß Anspruch 1, wobei
bezüglich den Formeln 1B bis 1D die Summe von a1 und a2 1 oder mehr ist, und *-(L₁)ₐ₁-Ar₁ (L₂)ₐ₂-*' dargestellt wird durch eine der Formen 3-1 bis 3-57: 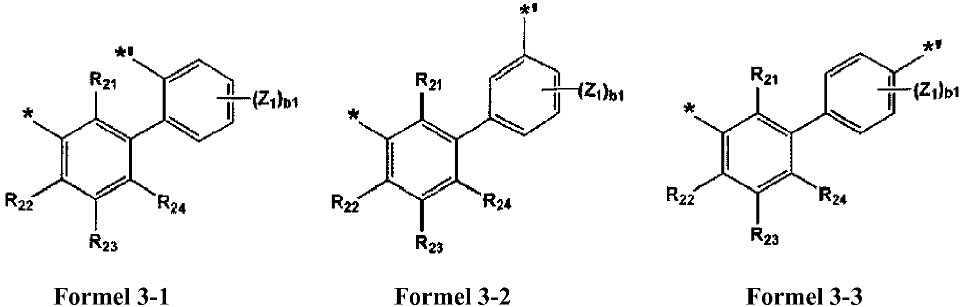 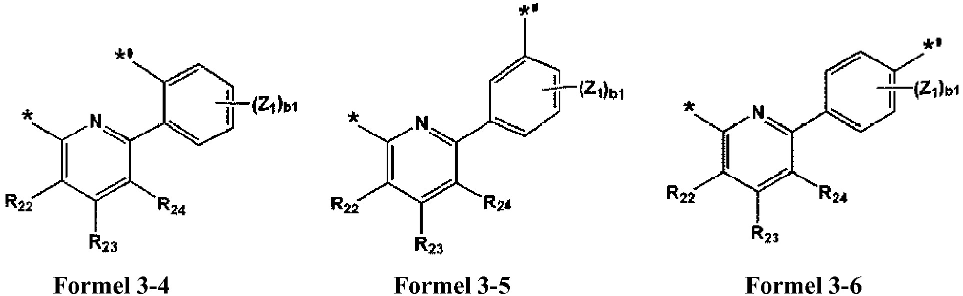 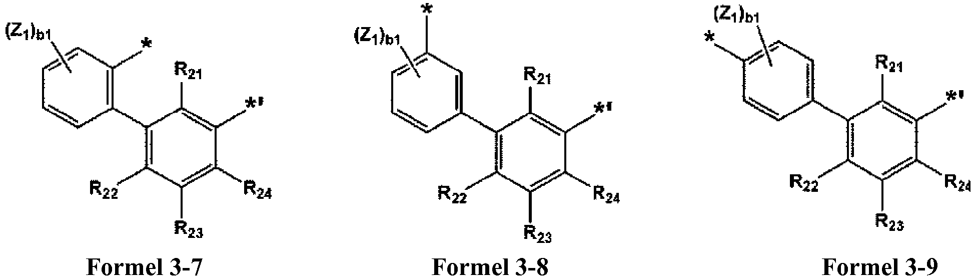 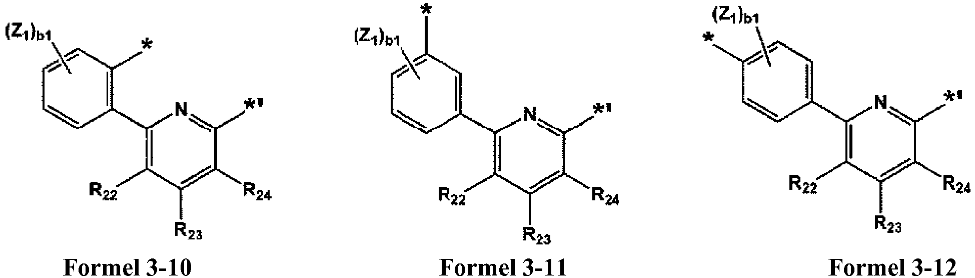 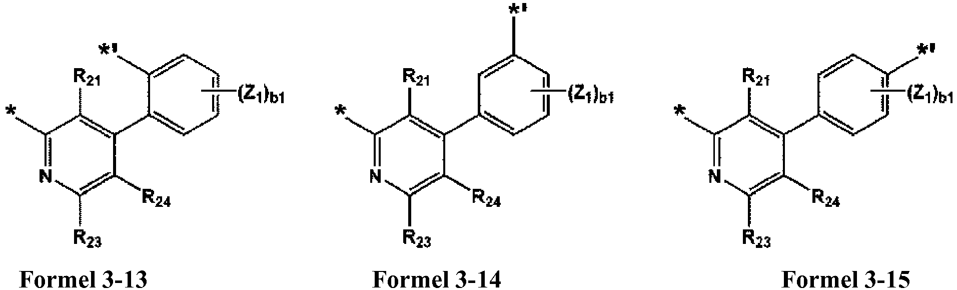 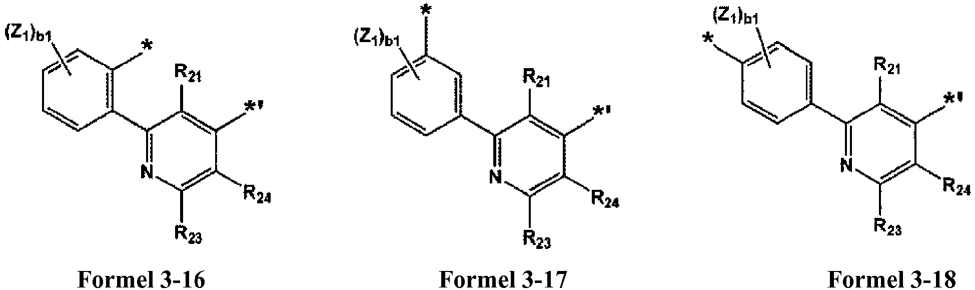 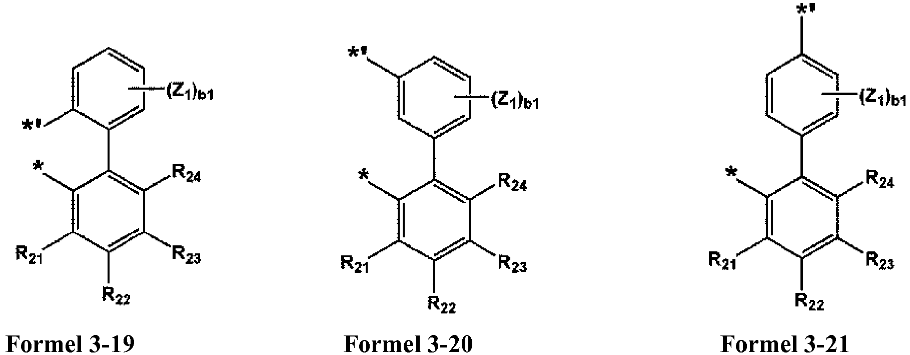 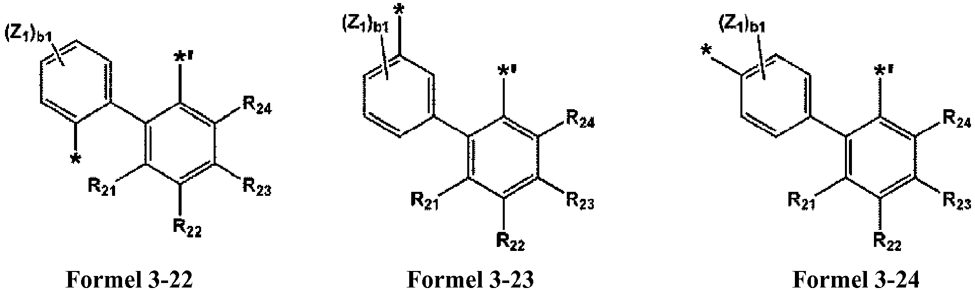 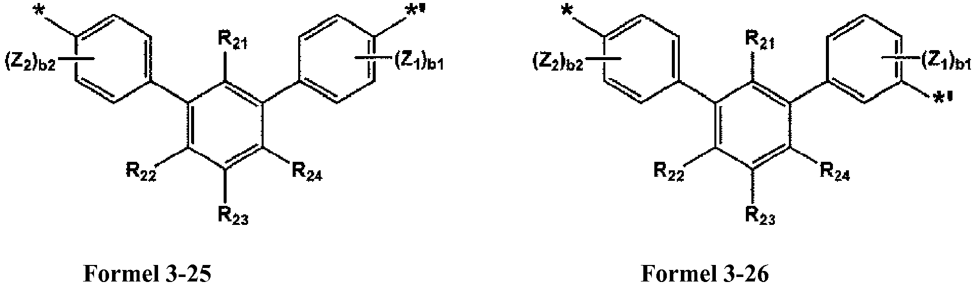 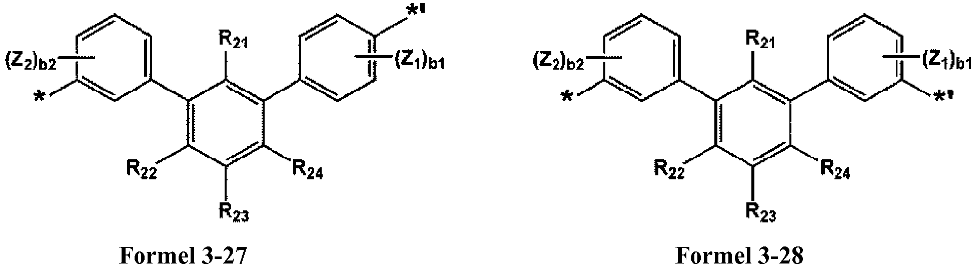 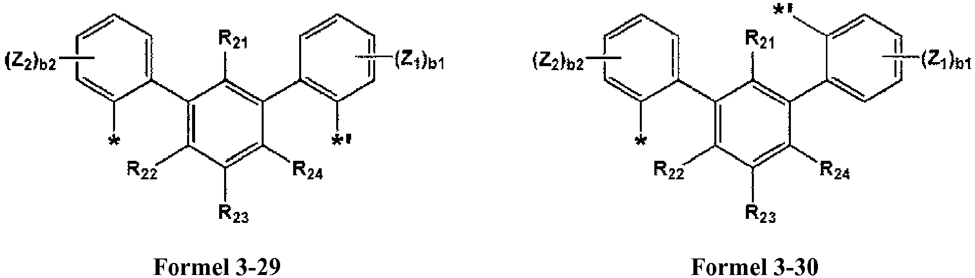 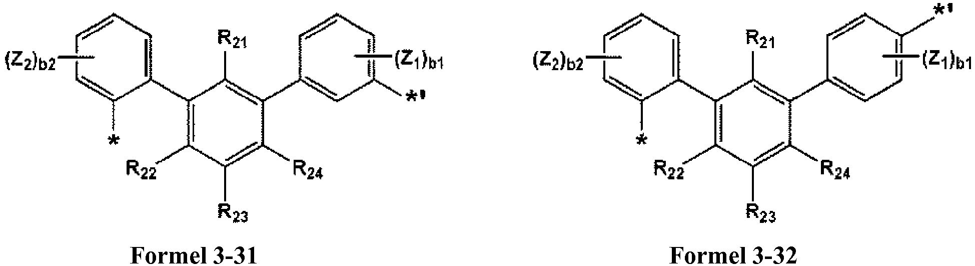 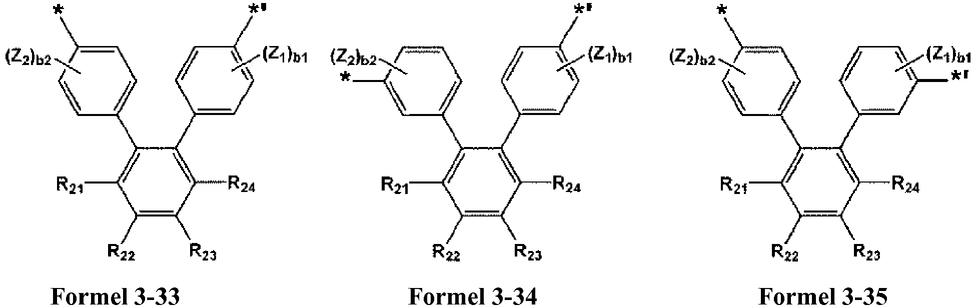 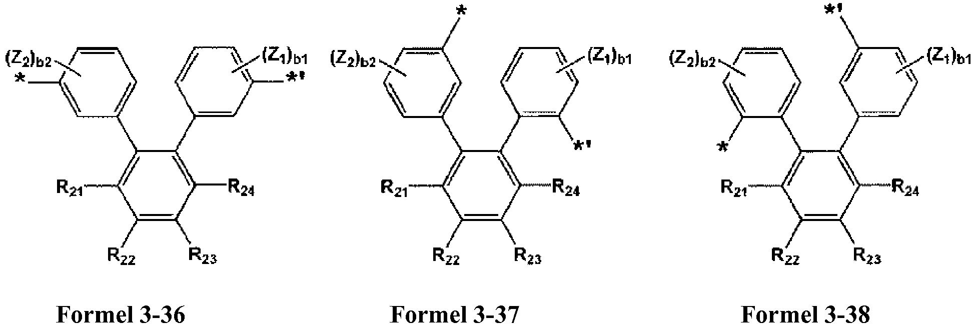 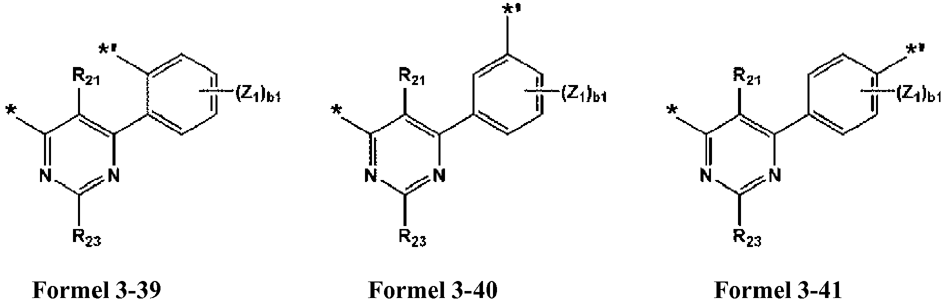 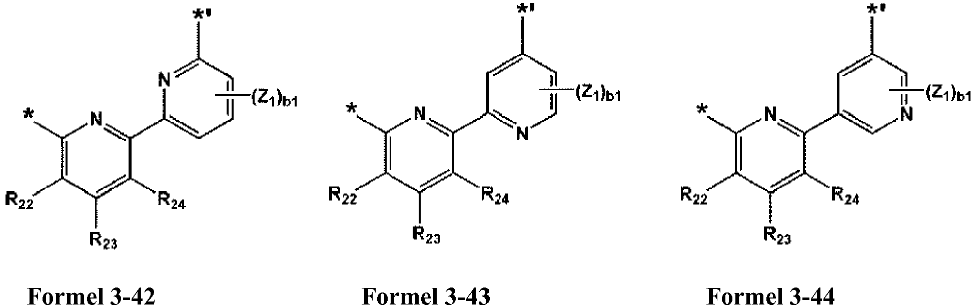 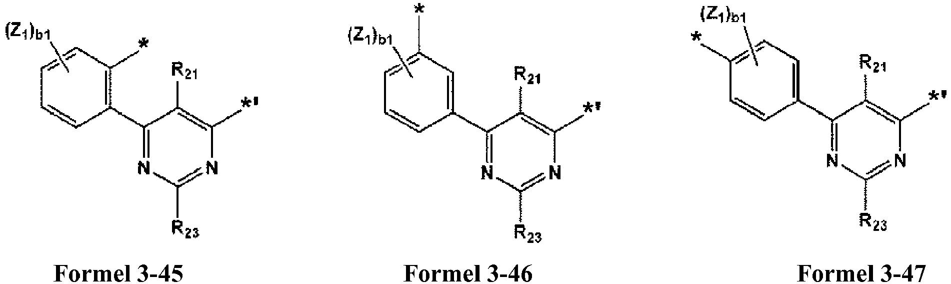 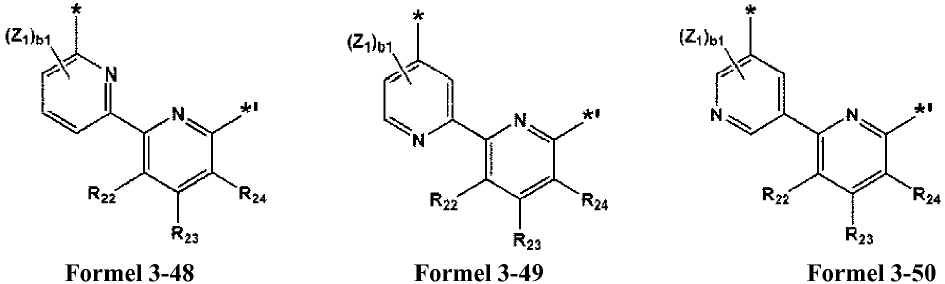 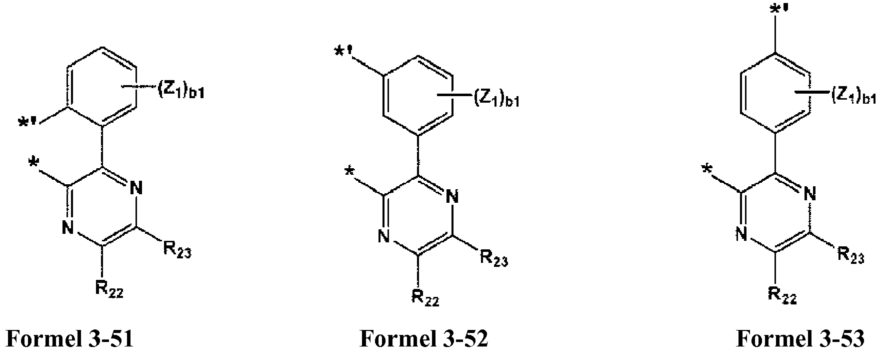 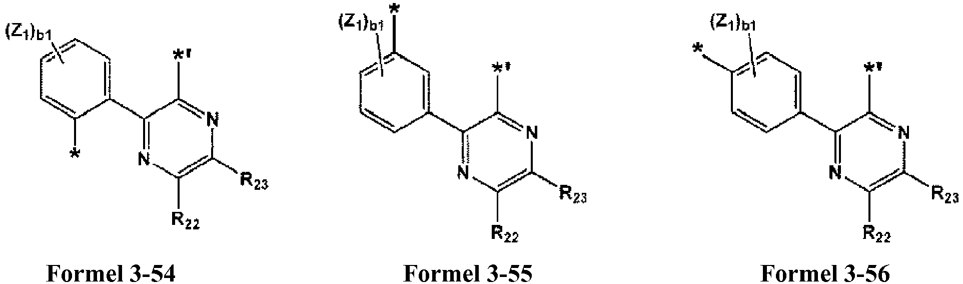 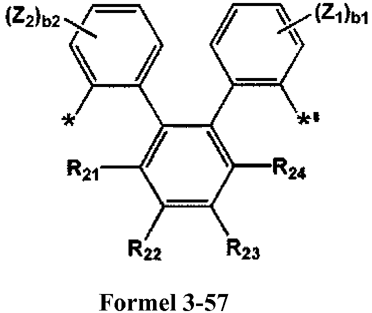 wobei in den Formeln 3-1 bis 3-57
R₂₁ bis R₂₄, Z₁ und Z₂ jeweils unabhängig ausgewählt sind aus einem Wasserstoff, einem Deuterium, einer Methylgruppe, einer Ethylgruppe, einer n-Propylgruppe, einer Isopropylgruppe, einer n-Butylgruppe, einer Isobutylgruppe, einer sec-Butylgruppe, einer tert-Butylgruppe, einer Phenylgruppe, einer Pyridinylgruppe, einer Pyrimidinylgruppe, einer Pyrazinylgruppe, einer Pyridazinylgruppe, einer Triazinylgruppe und -Si(Q₁₁)(Q₁₂)(Q₁₃),
wobei Q₁₁ bis Q₁₃ jeweils unabhängig ausgewählt sind aus einem Wasserstoff, einer C₁-C₁₀-Alkylgruppe, einer C₁-C₁₀-Alkoxygruppe und einer Phenylgruppe,
b1 und b2 jeweils unabhängig 0, 1 oder 2 sind, und
jedes von * und *' eine Bindungsstelle an einem benachbarten Stickstoffatom angibt.

8. Die kondensierte Ringverbindung gemäß Anspruch 1, wobei die kondensierte Ringverbindung dargestellt ist durch eine der Formeln 1B(1) bis 1D(1), 1B(2) bis 1D(2) und 1B(3) bis 1D(3): 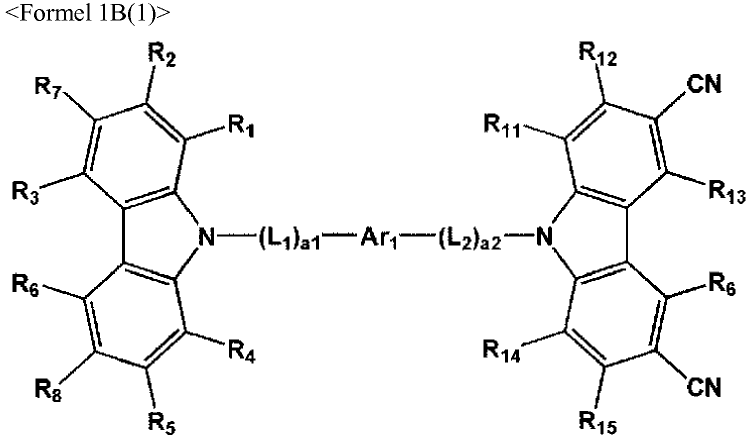 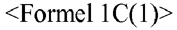 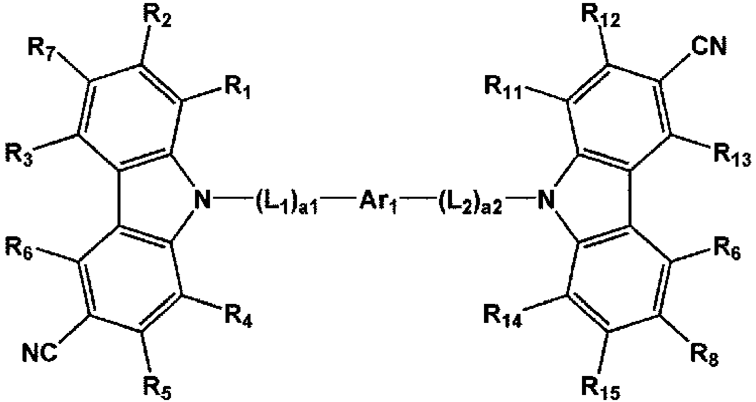 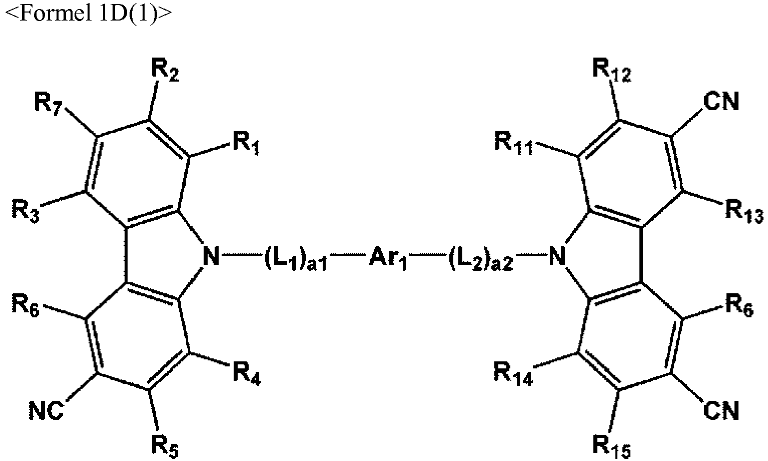 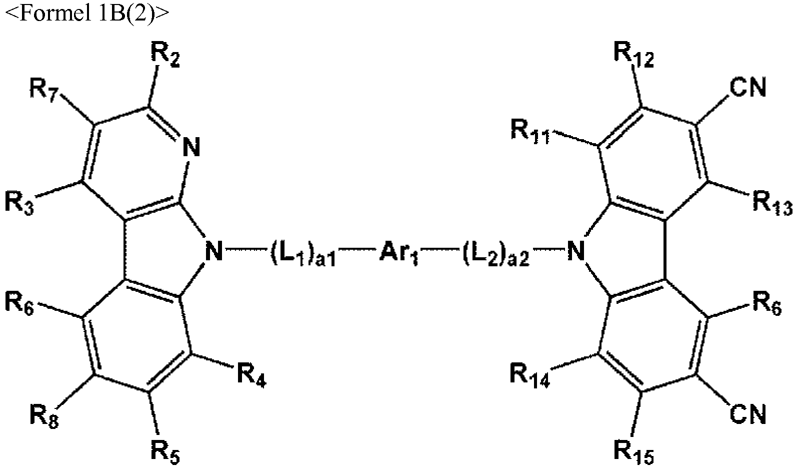 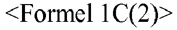 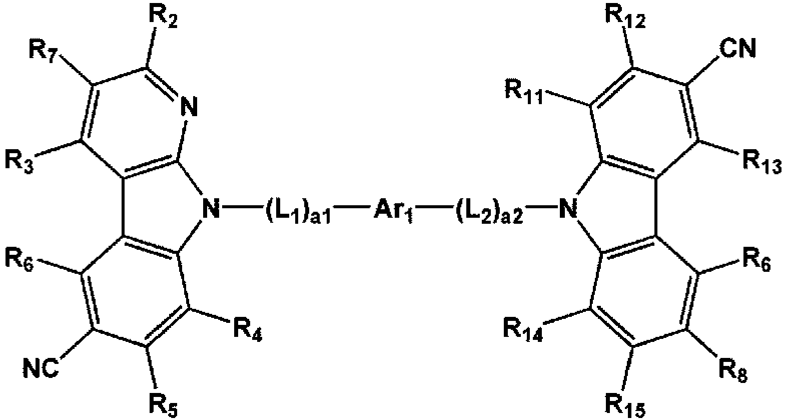 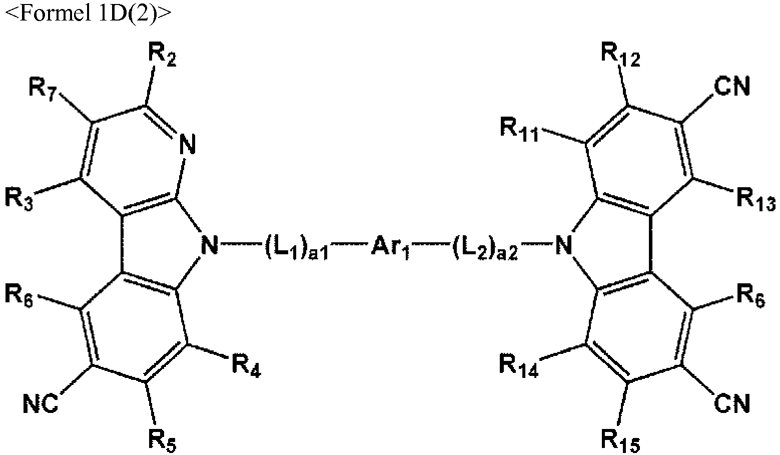 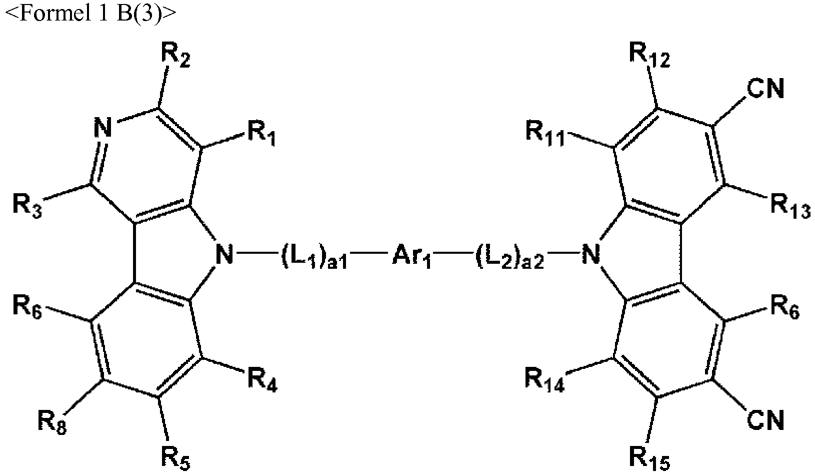 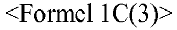 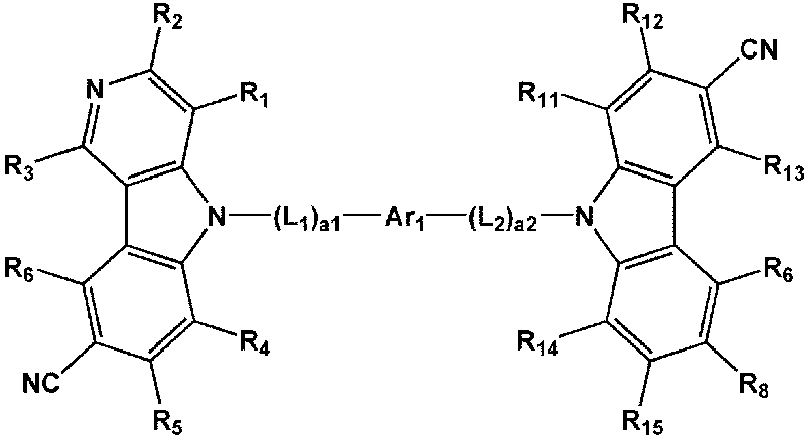 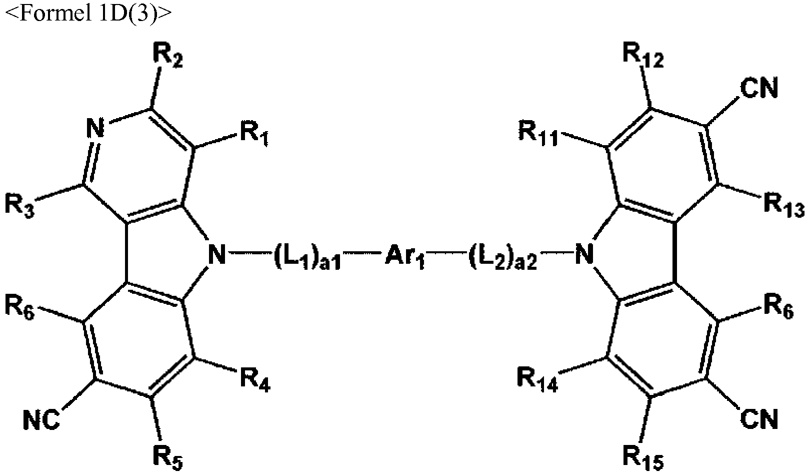 wobei in den Formeln 1 B(1) bis 1 D(1), 1 B(2) bis 1 D(2) und 1 B(3) bis 1 D(3),
R₁ bis R₈, R₁₁ bis R₁₆ und R₁₈ jeweils unabhängig ausgewählt sind aus einem Wasserstoff, einem Deuterium, -F, -Cl, -Br, -I, einer Hydroxylgruppe, einer Nitrogruppe, einer Aminogruppe, einer Amidinogruppe, einer Hydrazingruppe, einer Hydrazongruppe, einer Carbonsäuregruppe oder einem Salz davon, einer Schwefelsäuregruppe oder einem Salz davon, einer Phosphorsäuregruppe oder einem Salz davon, einer Methylgruppe, einer Ethylgruppe, einer n-Propylgruppe, einer Isopropylgruppe, einer n-Butylgruppe, einer Isobutylgruppe, einer sec-Butylgruppe, einer tert-Butylgruppe, einer n-Pentylgruppe, einer Isopentylgruppe, einer sec-Pentylgruppe, einer tert-Pentylgruppe, einer n-Hexylgruppe, einer iso-Hexylgruppe, einer sec-Hexylgruppe, einer tert-Hexylgruppe, einer n-Heptylgruppe, einer iso-Heptylgruppe, einer sec-Heptylgruppe, einer tert-Heptylgruppe, einer n-Octylgruppe, einer iso-Octylgruppe, einer sec-Octylgruppe, einer tert-Octylgruppe, einer n-Nonylgruppe, einer iso-Nonylgruppe, einer sec-Nonylgruppe, einer tert-nonylgruppe, einer n-Decylgruppe, einer Isodecylgruppe, einer sec-Decylgruppe, einer tert-Decylgruppe, einer Methoxygruppe, einer Ethoxygruppe, einer Propoxygruppe, einer Butoxygruppe, einer Pentoxygruppe, einer Phenylgruppe, einer Naphthylgruppe, einer Pyridinylgruppe, einer Pyrimidinylgruppe, einer Triazinylgruppe und -Si(Q₁)(Q₂)(Q₃),
Ar₁ dargestellt ist durch die Formeln 2A-1, 2A-2, 2A-10, 2B-1, 2B-8 oder 2C-3:
L₁ und L₂ jeweils unabhängig ausgewählt sind aus
einer Phenylengruppe, einer Pyridinylengruppe, einer Pyrimidinylengruppe, einer Pyrazinylengruppe, einer Pyridazinylengruppe, einer Triazinylengruppe, einer Dibenzofuranylengruppe und einer Dibenzothiophenylengruppe; und
einer Phenylengruppe, einer Pyridinylengruppe, einer Pyrimidinylengruppe, einer Pyrazinylengruppe, einer Pyridazinylengruppe, einer Triazinylengruppe, einer Dibenzofuranylengruppe und einer Dibenzothiophenylengruppe, jeweils substituiert mit mindestens einem, ausgewählt aus einem Deuterium, -F, -Cl, -Br, -I, einer Hydroxylgruppe, einer Cyanogruppe, einer Nitrogruppe, einer Aminogruppe, einer Amidinogruppe, einer Hydrazingruppe, einer Hydrazongruppe, einer Carbonsäuregruppe oder einem Salz davon, einer Schwefelsäuregruppe oder einem Salz davon, einer Phosphorsäuregruppe oder einem Salz davon, einer C₁-C₁₀-Alkylgruppe, einer C₁-C₁₀-Alkoxygruppe, einer Phenylgruppe, einer Naphthylgruppe, einer Pyridinylgruppe, einer Pyrimidinylgruppe, einer Pyrazinylgruppe, einer Pyridazinylgruppe, einer Triazinylgruppe und -Si(Q₂₁)(Q₂₂)(Q₂₃),
a1 und a2 jeweils unabhängig 0 oder 1 sind,
wobei Q₁ bis Q₃ und Q₂₁ bis Q₂₃ jeweils unabhängig ausgewählt sind aus einem Wasserstoff, einer C₁-C₁₀-Alkylgruppe, einer C₁-C₁₀-Alkoxygruppe und einer Phenylgruppe. 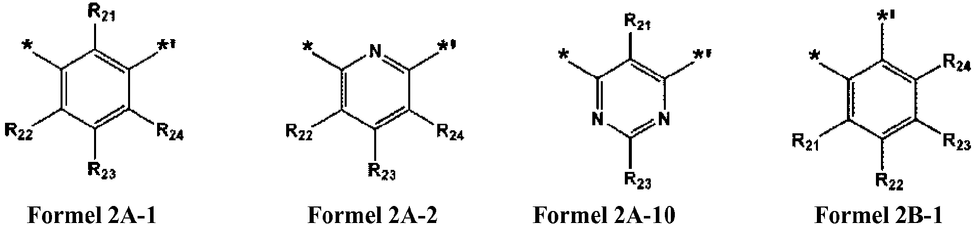 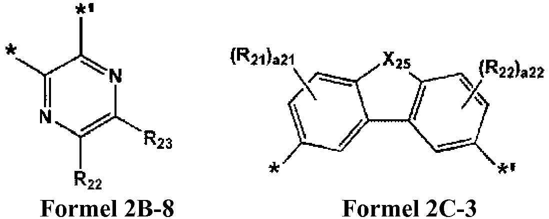 wobei in den Formeln 2A-1, 2A-2, 2A-10, 2B-1, 2B-8 und 2C-3,
X₂₅ O, S, P(=O)(R₂₅), Se oder Si(R₂₅)(R₂₆) ist,
R₂₁ to R₂₄ jeweils unabhängig ausgewählt sind aus einem Wasserstoff, einem Deuterium, einer Methylgruppe, einer Ethylgruppe, einer n-Propylgruppe, einer Isopropylgruppe, einer n-Butylgruppe, einer Isobutylgruppe, einer sec-Butylgruppe, einer tert-Butylgruppe, einer Phenylgruppe, einer Pyridinylgruppe, einer Pyrimidinylgruppe, einer Pyrazinylgruppe, einer Pyridazinylgruppe, einer Triazinylgruppe und -Si(Q₁₁)(Q₁₂)(Q₁₃),
R₂₅ und R₂₆ jeweils unabhängig ausgewählt sind aus einem Wasserstoff, einem Deuterium, einer Methylgruppe, einer Ethylgruppe, einer n-Propylgruppe, einer Isopropylgruppe, einer n-Butylgruppe, einer Isobutylgruppe, einer sec-Butylgruppe, einer tert-Butylgruppe, einer Phenylgruppe, einer Pyridinylgruppe, einer Pyrimidinylgruppe, einer Pyrazinylgruppe, einer Pyridazinylgruppe und einer Triazinylgruppe,
a21 und a22 jeweils unabhängig 0 oder 1 sind,
wobei Q₁₁ bis Q₁₃ jeweils unabhängig ausgewählt sind aus einem Wasserstoff, einer C₁-C₁₀-Alkylgruppe, einer C₁-C₁₀-Alkoxygruppe und einer Phenylgruppe.

9. Die kondensierte Ringverbindung gemäß Anspruch 1, wobei
die kondensierte Ringverbindung eine der Verbindungen 1 bis 7, 13 bis 18, 26 bis 27, 31 bis 34, 36, 39, 42 bis 44, 46, 47, 49, 50, 52, 53, 55 und 56 ist:

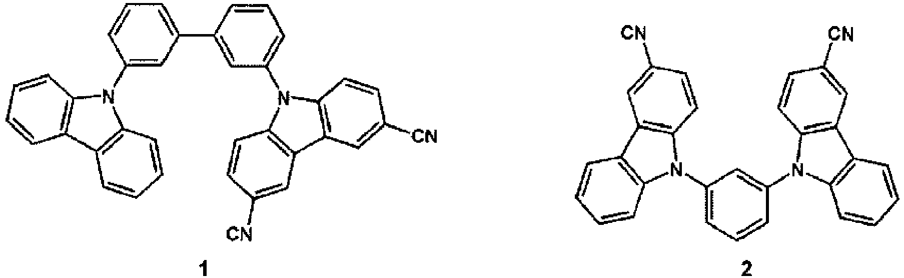

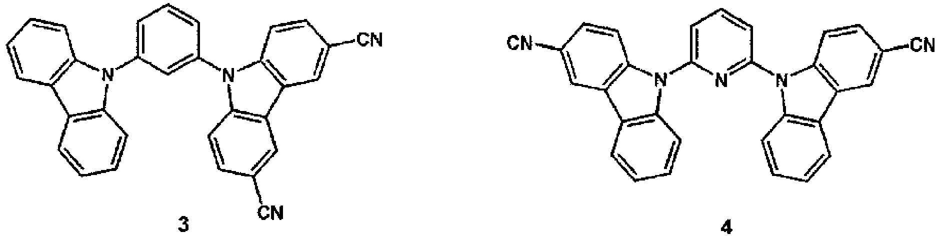

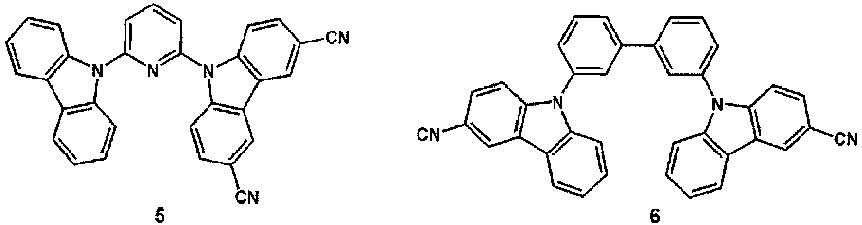

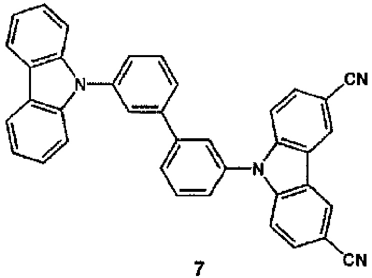

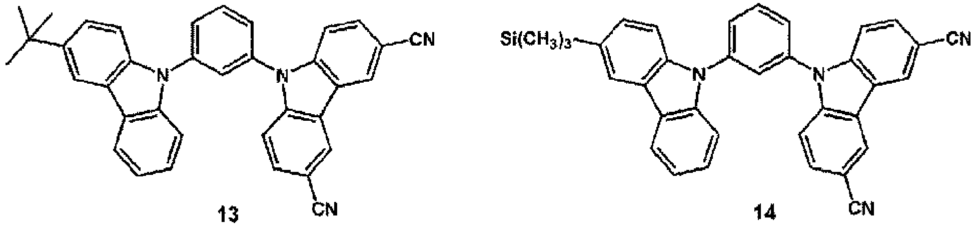

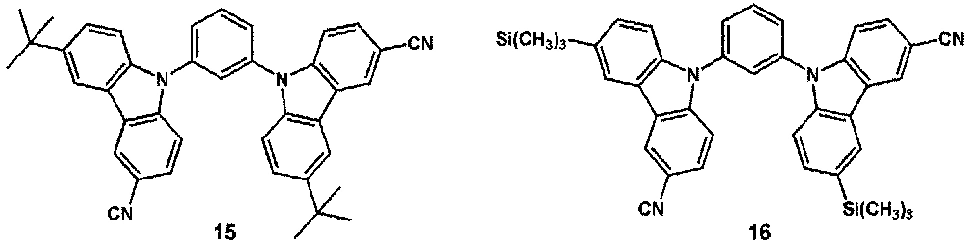

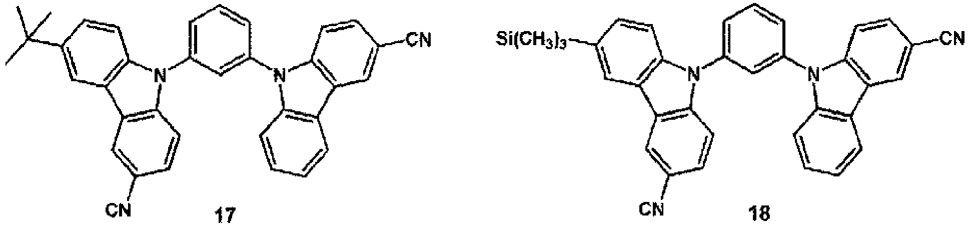

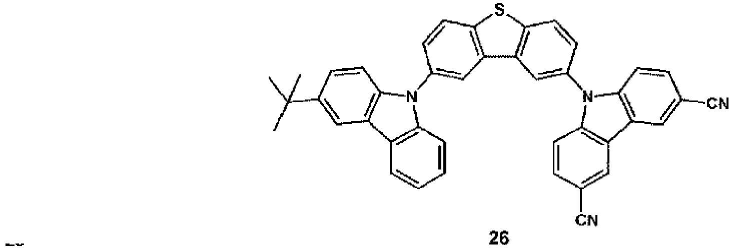

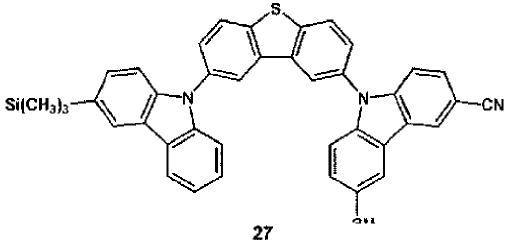

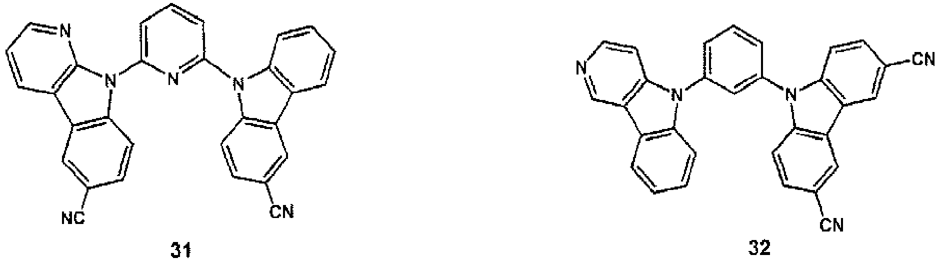

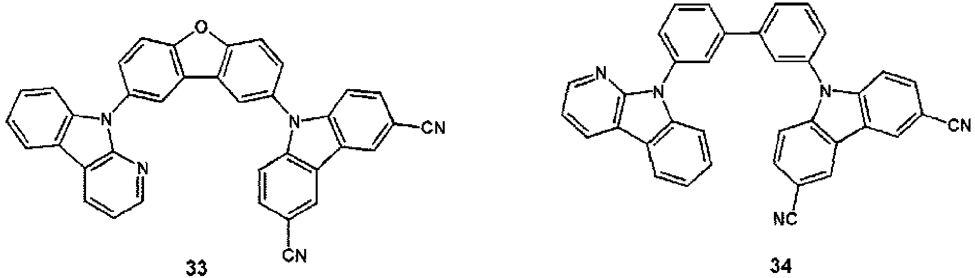

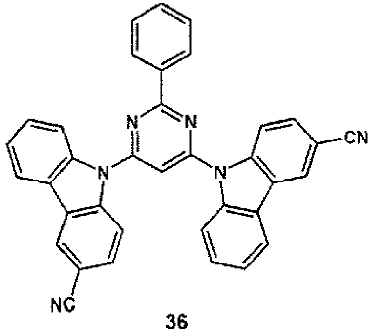

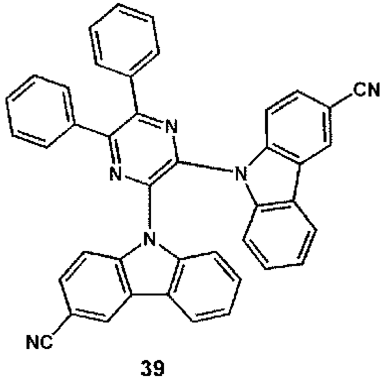

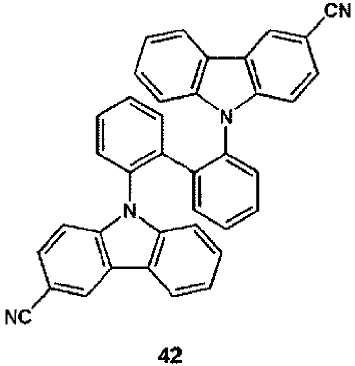

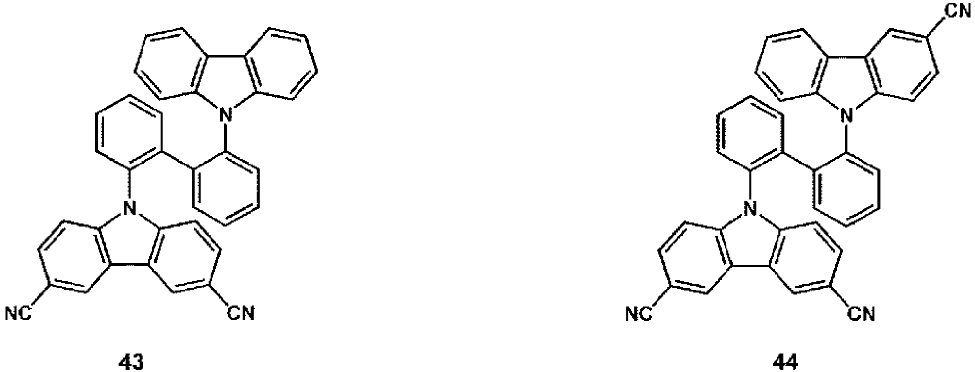

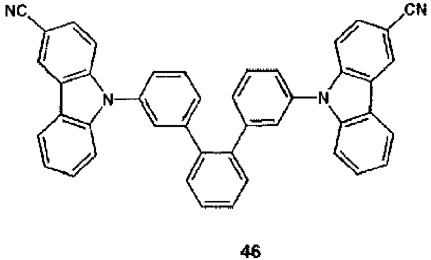

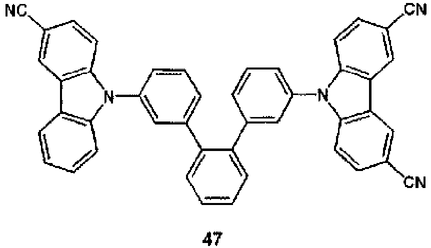

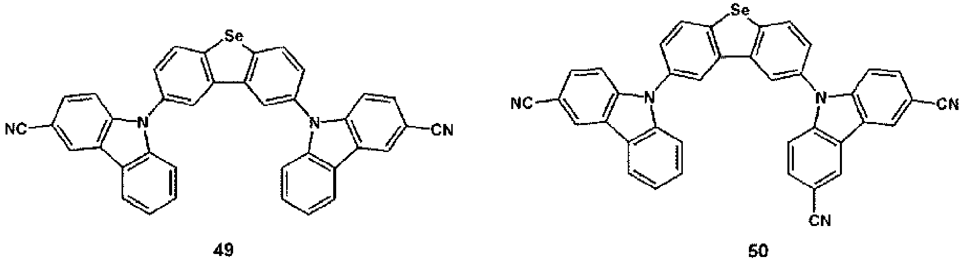

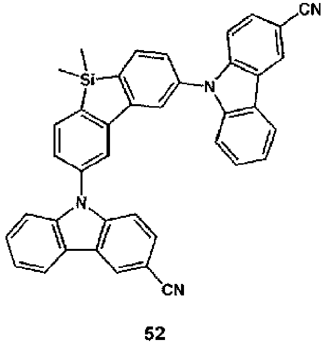

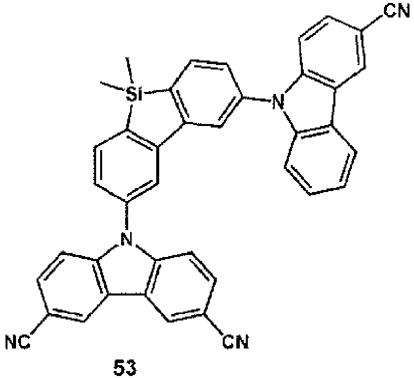

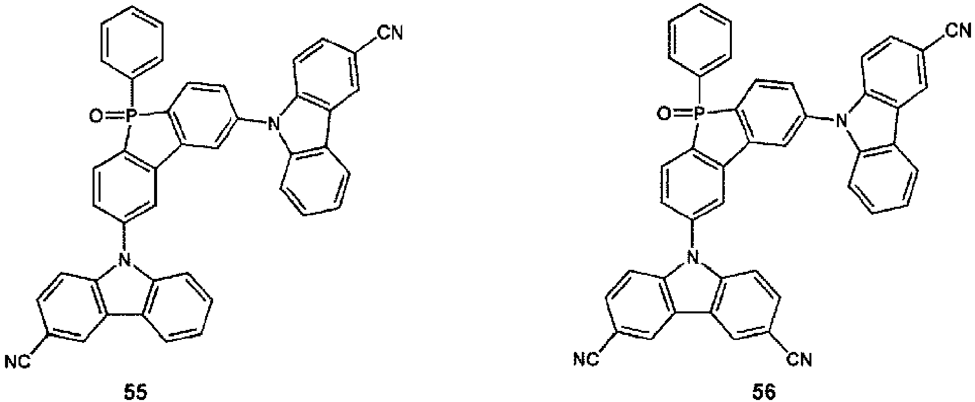

10. Eine organische lichtemittierende Vorrichtung, beinhaltend eine erste Elektrode; eine zweite Elektrode; und eine organische Schicht zwischen der ersten Elektrode und der zweiten Elektrode, wobei die organische Schicht eine Emissionsschicht beinhaltet, wobei die organische Schicht mindestens eine der kondensierten Ringverbindungen beinhaltet, die durch die Formeln 1B bis 1D gemäß einem der Ansprüche 1-9 dargestellt sind.

11. Die organische lichtemittierende Vorrichtung gemäß Anspruch 10, wobei
die erste Elektrode eine Anode ist, die zweite Elektrode eine Katode ist und die organische Schicht Folgendes beinhaltet:
i) eine Lochtransportregion zwischen der ersten Elektrode und der Emissionsschicht, wobei die Lochtransportregion mindestens eine ausgewählt von einer Lochinjektionsschicht, einer Lochtransportschicht und einer Elektronenblockierschicht beinhaltet; und
ii) eine Elektronentransportregion zwischen der Emissionsschicht und der zweiten Elektrode, wobei die Elektronentransportregion mindestens eine von einer Lochblockierschicht, einer Elektronentransportschicht und einer Elektroneninjektionsschicht beinhaltet.

12. Die organische lichtemittierende Vorrichtung gemäß Anspruch 10 oder 11, wobei die mindestens eine kondensierte Ringverbindung, dargestellt durch die Formeln 1B bis 1D, in der Emissionsschicht eingeschlossen ist, die Emissionsschicht optional blaues Licht emittiert und/oder die kondensierte Ringverbindung, dargestellt durch die Formeln 1B bis 1D, die in der Emissionsschicht eingeschlossen ist, optional ein thermisch aktivierter verzögerter Fluoreszenzemitter ist.

13. Die organische lichtemittierende Vorrichtung gemäß einem der Ansprüche 10-12, wobei
die mindestens eine kondensierte Ringverbindung, dargestellt durch die Formeln 1B bis 1D, in der Emissionsschicht eingeschlossen ist, die Emissionsschicht ferner einen phosphoreszierenden Dotierstoff beinhaltet und eine Menge der kondensierten Ringverbindung größer als eine Menge des phosphoreszierenden Dotierstoffs ist.

## Revendications

1. Composé cyclique condensé représenté par l'une des Formules 1B à 1D : 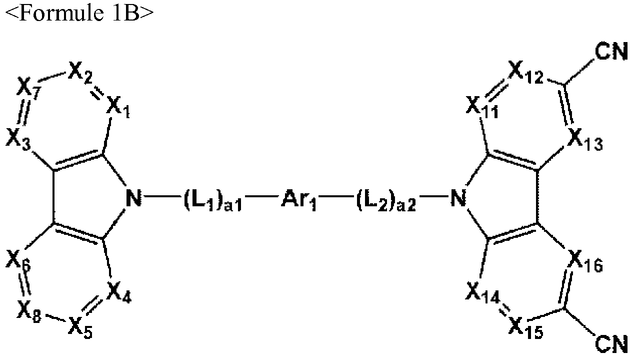 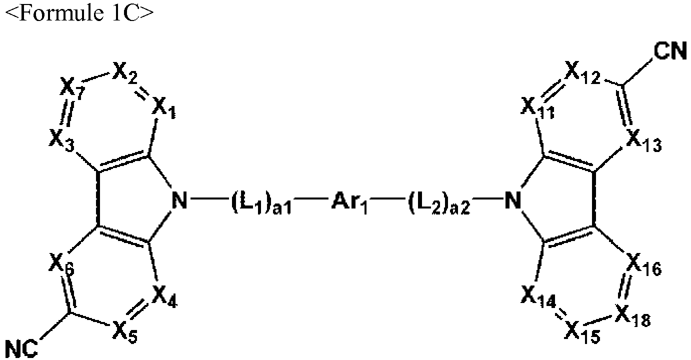 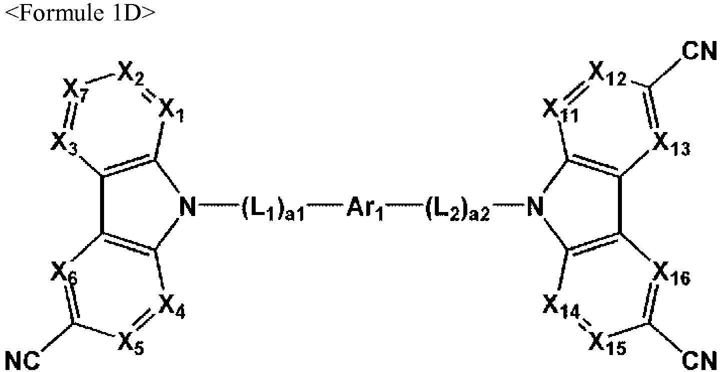 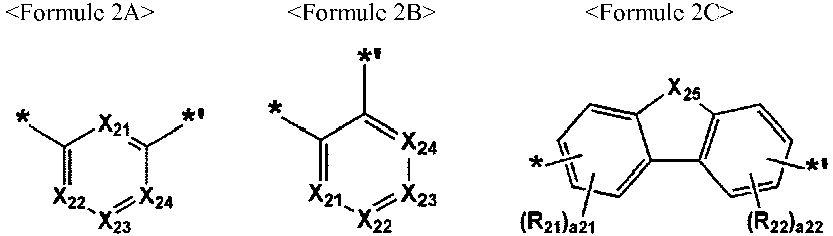 dans lequel dans les Formules 1B à 1D et 2A à 2C,
X₁ est N ou C(R₁), X₂ est N ou C(R₂), X₃ est N ou C(R₃), X₄ est N ou C(R₄), X₅ est N ou C(R₅), X₆ est N ou C(R₆), X₇ est N ou C(R₇), X₈ est N ou C(R₈), X₁₁ est N ou C(R₁₁), X₁₂ est N ou C(R₁₂), X₁₃ est N ou C(R₁₃), X₁₄ est N ou C(R₁₄), X₁₅ est N ou C(R₁₅), X₁₆ est N ou C(R₁₆), X₁₇ est N ou C(R₁₇), et X₁₈ est N ou C(R₁₈),
R₁ à R₈ et R₁₁ à R₁₈ sont chacun indépendamment sélectionnés parmi un hydrogène, un deutérium, -F, -Cl, -Br, -I, un groupe hydroxyle, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en C₁ à C₆₀ substitué ou non substitué, un groupe alcényle en C₂ à C₆₀ substitué ou non substitué, un groupe alcynyle en C₂ à C₆₀ substitué ou non substitué, un groupe alcoxy en C₁ à C₆₀ substitué ou non substitué, un groupe cycloalkyle en C₃ à C₁₀ substitué ou non substitué, un groupe hétérocycloalkyle en C₁ à C₁₀ substitué ou non substitué, un groupe cycloalcényle en C₃ à C₁₀ substitué ou non substitué, un groupe hétérocycloalcényle en C₁ à C₁₀ substitué ou non substitué, un groupe aryle en C₆ à C₆₀ substitué ou non substitué, un groupe aryloxy en C₆ à C₆₀ substitué ou non substitué, un groupe arylthio en C₆ à C₆₀ substitué ou non substitué, un groupe hétéroaryle en C₁ à C₆₀ substitué ou non substitué, un groupe polycyclique condensé non aromatique monovalent substitué ou non substitué, un groupe hétéropolycyclique condensé non aromatique monovalent substitué ou non substitué, à condition que parmi les groupes hétéropolycycliques condensés non aromatiques monovalents substitués ou non substitués, un groupe carbazolyle substitué ou non substitué soit exclu, et -Si(Q₁)(Q₂)(Q₃),
Ar₁ est représenté par l'une des Formules 2A à 2C,
X₂₁ est N ou C(R₂₁), X₂₂ est N ou C(R₂₂), X₂₃ est N ou C(R₂₃), X₂₄ est N ou C(R₂₄), et X₂₅ est O, S, P(=O)(R₂₅), Se ou Si(R₂₅)(R₂₆),
R₂₁ à R₂₆ sont chacun indépendamment sélectionnés parmi un hydrogène, un deutérium, un groupe alkyle en C₁ à C₄, un groupe phényle, un groupe pyridinyle, un groupe pyrimidinyle, un groupe pyrazinyle, un groupe pyridazinyle, un groupe triazinyle, et -Si(Q₁₁)(Q₁₂)(Q₁₃),
a21 et a22 sont chacun indépendamment un nombre entier sélectionné parmi 0 à 3, et quand a21 est 2 ou plus, 2 R₂₁ ou plus peuvent être identiques ou différents les uns des autres, et quand a22 est 2 ou plus, 2 R₂₂ ou plus peuvent être identiques ou différents les uns des autres ;
L₁ et L₂ sont chacun indépendamment sélectionnés parmi un groupe phénylène, un groupe pyridinylène, un groupe pyrimidinylène, un groupe pyrazinylène, un groupe pyridazinylène, un groupe triazinylène, un groupe dibenzofuranylène, et un groupe dibenzothiophénylène ;
et un groupe phénylène, un groupe pyridinylène, un groupe pyrimidinylène, un groupe pyrazinylène, un groupe pyridazinylène, un groupe triazinylène, un groupe dibenzofuranylène, et un groupe dibenzothiophénylène, chacun substitué par au moins un sélectionné parmi un deutérium, -F, -Cl, -Br, -I, un groupe hydroxyle, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en C₁ à C₁₀, un groupe alcoxy en C₁ à C₁₀, un groupe phényle, un groupe naphtyle, un groupe pyridinyle, un groupe pyrimidinyle, un groupe pyrazinyle, un groupe pyridazinyle, un groupe triazinyle, et - Si(Q₂₁)(Q₂₂)(Q₂₃),
a1 et a2 sont chacun indépendamment un nombre entier sélectionné parmi 0 à 5, et quand a1 est 2 ou plus, 2 L₁ ou plus peuvent être identiques ou différents les uns des autres, et quand a2 est 2 ou plus, 2 L₂ ou plus peuvent être identiques ou différents les uns des autres ;
chacun de * et *' indique un site de liaison à un atome voisin ;
au moins un des substituants du groupe alkyle en C₁ à C₆₀ substitué, du groupe alcényle en C₂ à C₆₀ substitué, du groupe alcynyle en C₂ à C₆₀ substitué, du groupe alcoxy en C₁ à C₆₀ substitué, du groupe cycloalkyle en C₃ à C₁₀ substitué, du groupe hétérocycloalkyle en C₁ à C₁₀ substitué, du groupe cycloalcényle en C₃ à C₁₀ substitué, du groupe hétérocycloalcényle en C₁ à C₁₀ substitué, du groupe aryle en C₆ à C₆₀ substitué, du groupe aryloxy en C₆ à C₆₀ substitué, du groupe arylthio en C₆ à C₆₀ substitué, du groupe hétéroaryle en C₁ à C₆₀ substitué, du groupe polycyclique condensé non aromatique monovalent substitué, et du groupe hétéropolycyclique condensé non aromatique monovalent substitué est sélectionné parmi un deutérium, -F, -Cl, -Br, -I, un groupe hydroxyle, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en C₁ à C₆₀, un groupe alcényle en C₂ à C₆₀, un groupe alcynyle en C₂ à C₆₀, un groupe alcoxy en C₁ à C₆₀, un groupe cycloalkyle en C₃ à C₁₀, un groupe hétérocycloalkyle en C₁ à C₁₀, un groupe cycloalcényle en C₃ à C₁₀, un groupe hétérocycloalcényle en C₁ à C₁₀, un groupe aryle en C₆ à C₆₀, un groupe aryloxy en C₆ à C₆₀, un groupe arylthio en C₆ à C₆₀, un groupe hétéroaryle en C₁ à C₆₀, un groupe polycyclique condensé non aromatique monovalent, et un groupe hétéropolycyclique condensé non aromatique monovalent, à condition que le groupe hétéropolycyclique condensé non aromatique monovalent ne soit par un groupe carbazolyle, et -Si(Q₃₁)(Q₃₂)(Q₃₃),
dans lequel Q₁ à Q₃, Q₁₁ à Q₁₃, Q₂₁ à Q₂₃, et Q₃₁ à Q₃₃ sont chacun indépendamment sélectionnés parmi un hydrogène, un groupe alkyle en C₁ à C₆₀, un groupe alcoxy en C₁ à C₆₀, un groupe cycloalkyle en C₃ à C₁₀, un groupe hétérocycloalkyle en C₁ à C₁₀, un groupe cycloalcényle en C₃ à C₁₀, un groupe hétérocycloalcényle en C₁ à C₁₀, un groupe aryle en C₆ à C₆₀, un groupe hétéroaryle en C₁ à C₆₀, un groupe polycyclique condensé non aromatique monovalent, et un groupe hétéropolycyclique condensé non aromatique monovalent, à condition que le groupe hétéropolycyclique condensé non aromatique monovalent ne soit pas un groupe carbazolyle, à condition que le composé cyclique condensé ne soit pas l'un des composés ci-dessous :

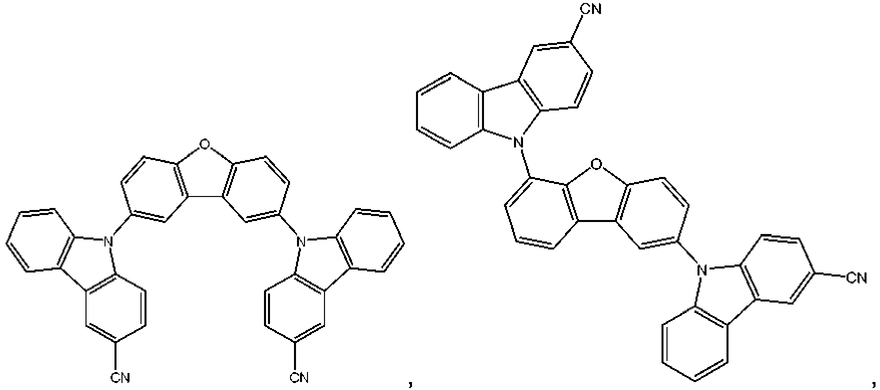

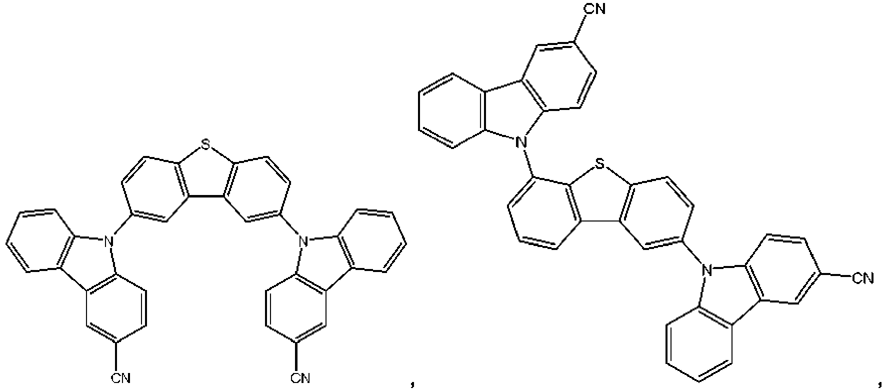

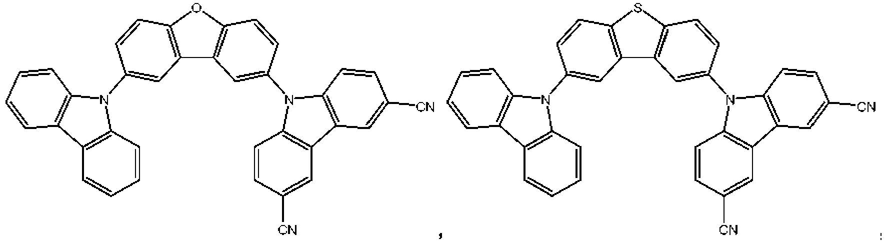

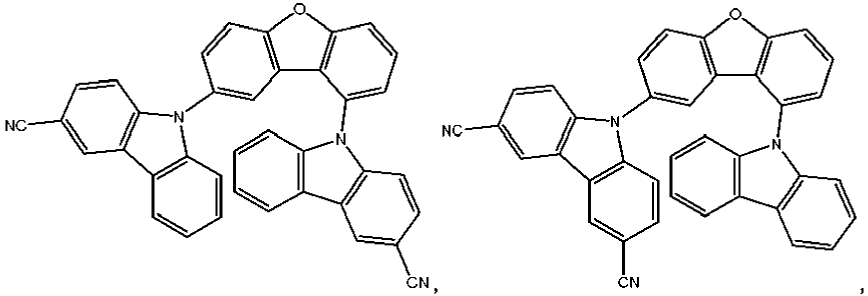

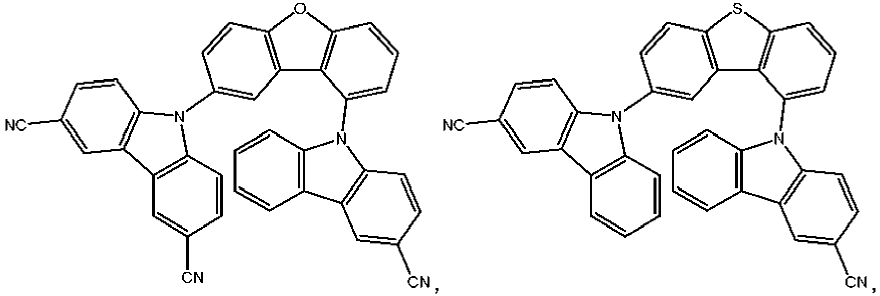

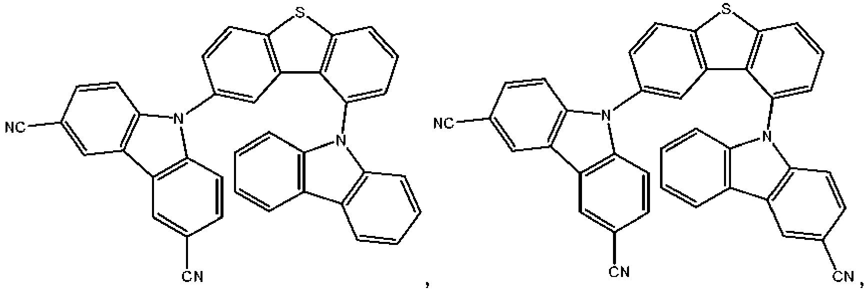

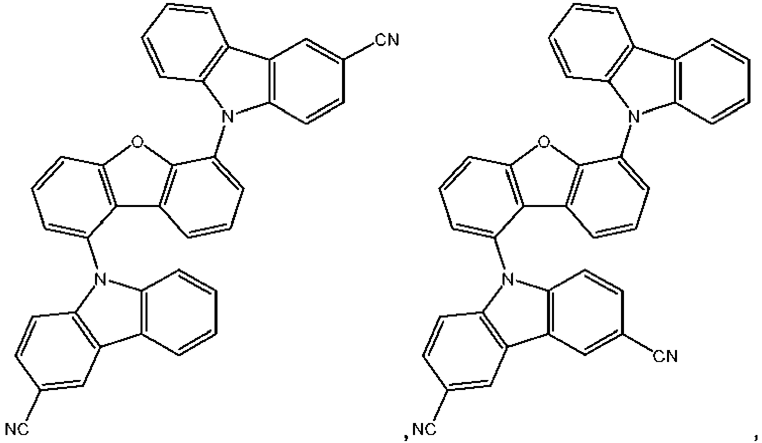

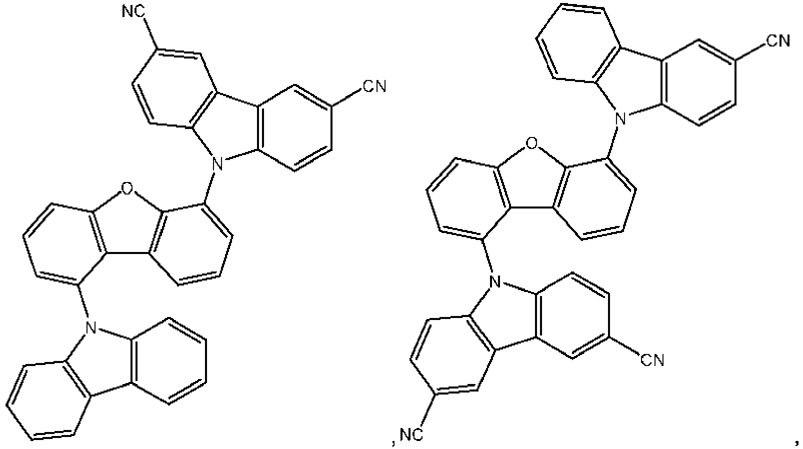

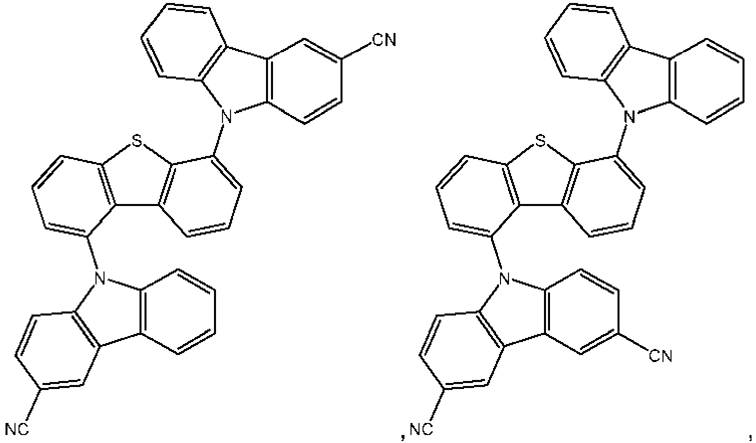

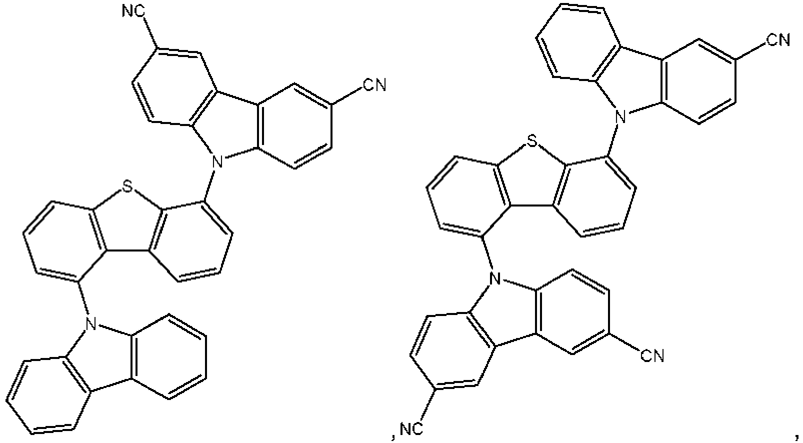

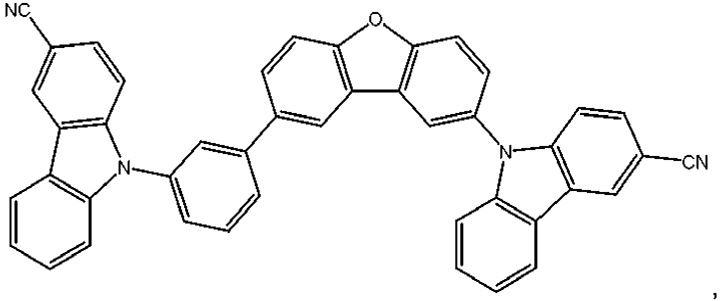

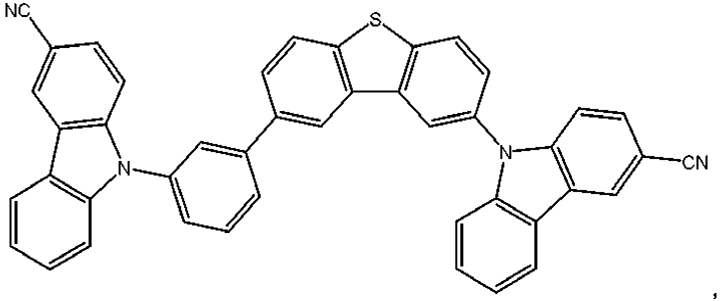

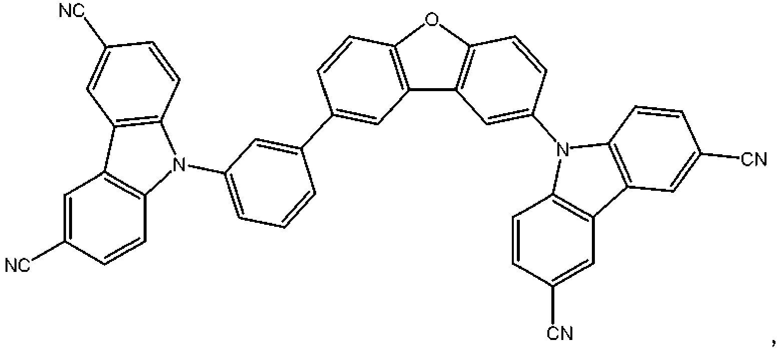

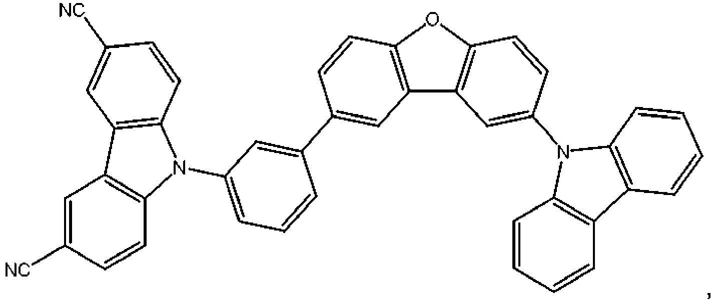

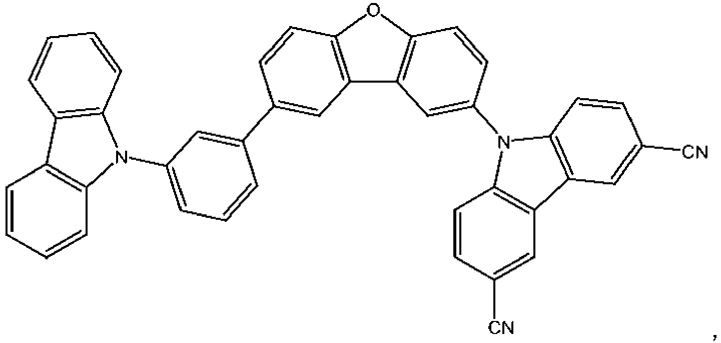

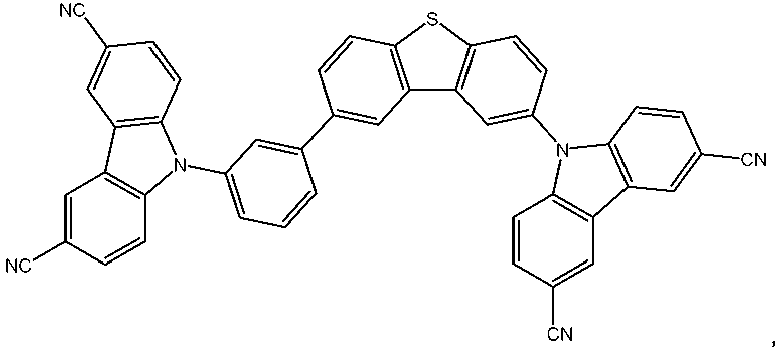

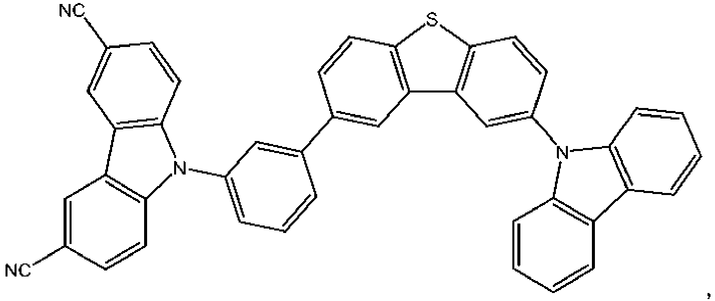

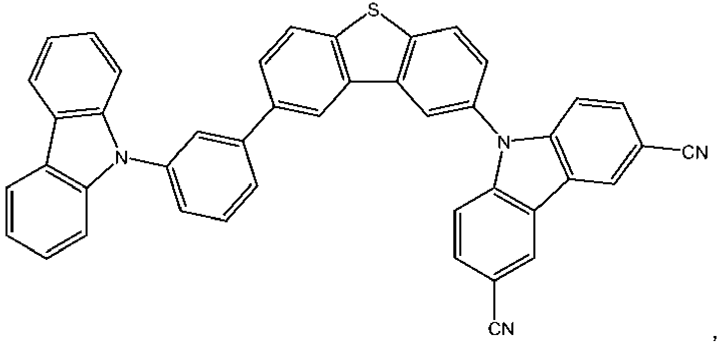

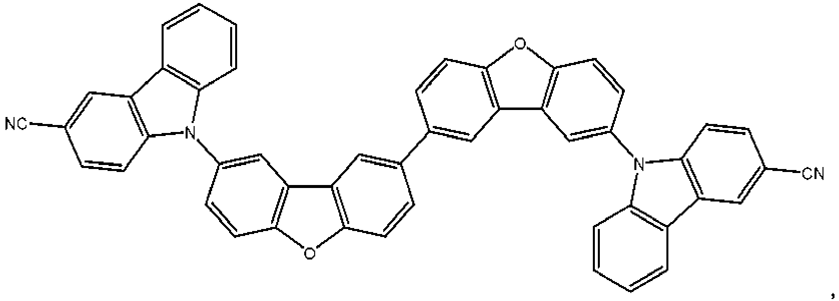

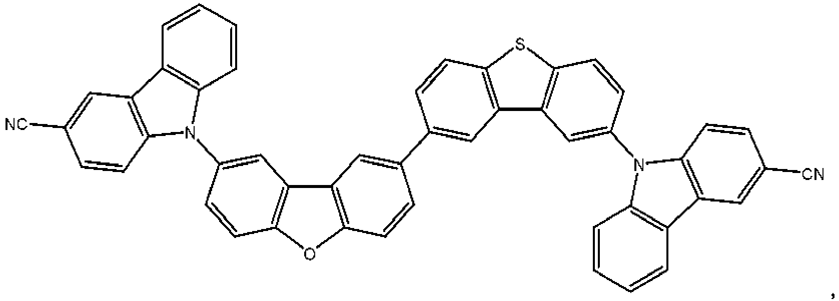

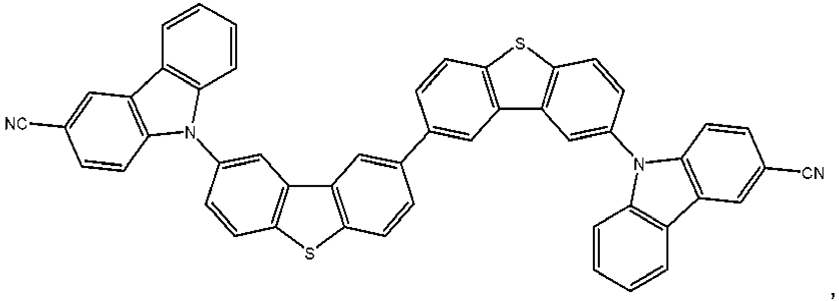

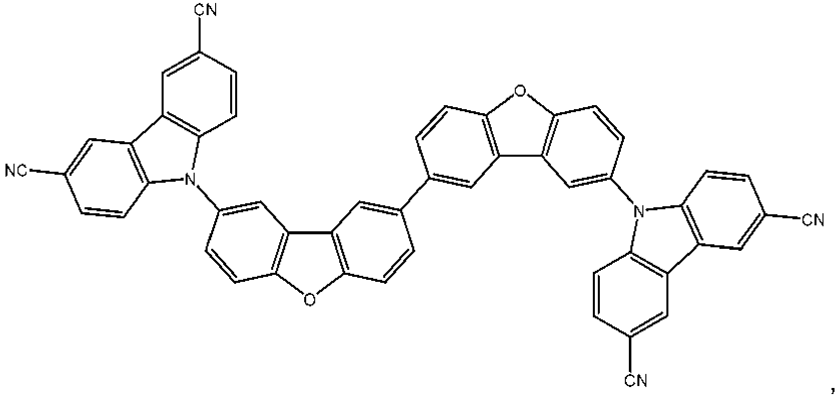

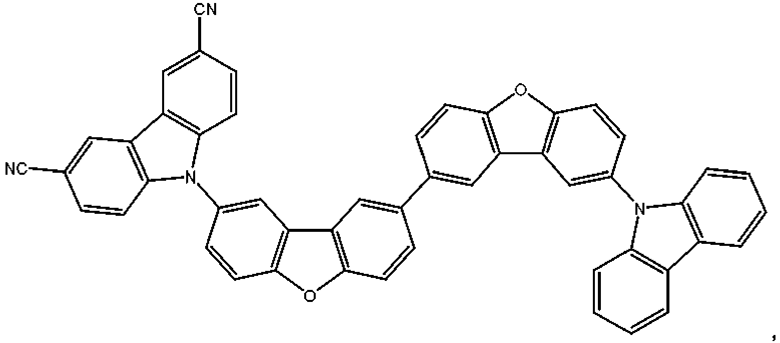

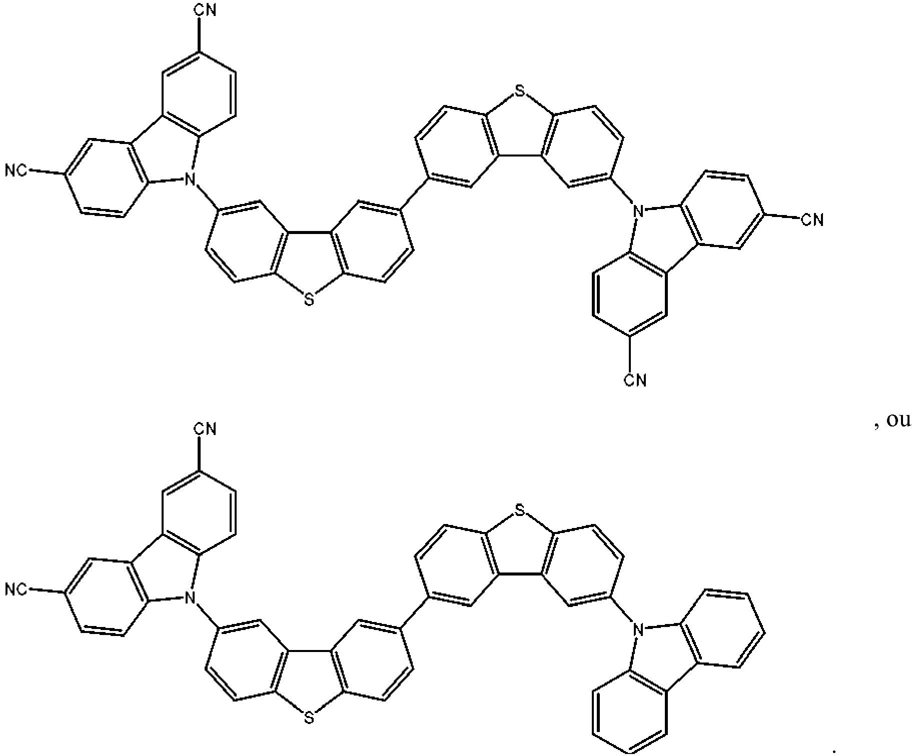

2. Composé cyclique condensé selon la revendication 1, dans lequel R₁ à R₈ et R₁₁ à R₁₈ sont chacun indépendamment sélectionnés parmi un hydrogène, un deutérium, -F, -Cl, -Br, -I, un groupe hydroxyle, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en C₁ à C₂₀, et un groupe alcoxy en C₁ à C₂₀ ;
un groupe alkyle en C₁ à C₂₀ et un groupe alcoxy en C₁ à C₂₀, chacun substitué par au moins un sélectionné parmi un deutérium, -F, -Cl, -Br, -I, un groupe hydroxyle, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe phényle, un groupe naphtyle, un groupe pyridinyle, un groupe pyrimidinyle, un groupe pyrazinyle, un groupe pyridazinyle, et un groupe triazinyle ;
un groupe cyclopentyle, un groupe cyclohexyle, un groupe cyclopentényle, un groupe cyclohexényle, un groupe cycloheptényle, un groupe phényle, un groupe pentalényle, un groupe indényle, un groupe naphtyle, un groupe azulényle, un groupe heptalényle, un groupe indacényle, un groupe acénaphtyle, un groupe fluorényle, un groupe spiro-fluorényle, un groupe phénalényle, un groupe phénanthrényle, un anthracényle, un groupe fluoranthrényle, un groupe triphénylényle, un groupe pyrényle, un groupe chrysényle, un groupe naphtacényle, un groupe picényle, un pérylényle, un groupe pentaphényle, un groupe hexacényle, un groupe pyrrolyle, un groupe imidazolyle, un groupe pyrazolyle, un groupe pyridinyle, un groupe pyrazinyle, un groupe pyrimidinyle, un groupe pyridazinyle, un groupe isoindolyle, un groupe indolyle, un groupe indazolyle, un groupe purinyle, un groupe quinolinyle, un groupe isoquinolinyle, un groupe benzoquinolinyle, un groupe phtalazinyle, un groupe naphtyridinyle, un groupe quinoxalinyle, un groupe quinazolinyle, un groupe cinnolinyle, un groupe phénanthridinyle, un groupe acridinyle, un groupe phénanthrolinyle, un groupe phénazinyle, un groupe benzoxazolyle, un groupe benzimidazolyle, un groupe furanyle, un groupe benzofuranyle, un groupe thiophényle, un groupe benzothiophényle, un groupe thiazolyle, un groupe isothiazolyle, un groupe benzothiazolyle, un groupe isoxazolyle, un groupe oxazolyle, un groupe triazolyle, un groupe tétrazolyle, un groupe oxadiazolyle, un groupe triazinyle, un groupe dibenzofuranyle, un groupe dibenzothiophényle, un groupe imidazopyridimidinyle, et un groupe imidazopyridinyle ;
un groupe cyclopentyle, un groupe cyclohexyle, un groupe cyclopentényle, un cyclohexényle, un groupe cycloheptényle, un groupe phényle, un groupe pentalényle, un groupe indényle, un groupe naphtyle, un groupe azulényle, un groupe heptalényle, un groupe indacényle, un groupe acénaphtyle, un groupe fluorényle, un groupe spiro-fluorényle, un groupe phénalényle, un groupe phénanthrényle, un groupe anthracényle, un groupe fluoranthrényle, un triphénylényle, un groupe pyrényle, un groupe chrysényle, un groupe naphtacényle, un groupe picényle, un groupe pérylényle, un groupe pentaphényle, un groupe hexacényle, un groupe pyrrolyle, un groupe imidazolyle, un groupe pyrazolyle, un groupe pyridinyle, un groupe pyrazinyle, un groupe pyrimidinyle, un groupe pyridazinyle, un groupe isoindolyle, un groupe indolyle, un groupe indazolyle, un groupe purinyle, un groupe quinolinyle, un groupe isoquinolinyle, un groupe benzoquinolinyle, un phtalazinyle groupe, un groupe naphtyridinyle, un groupe quinoxalinyle, un groupe quinazolinyle, un groupe cinnolinyle, un groupe phénanthridinyle, un groupe acridinyle, un groupe phénanthrolinyle, un groupe phénazinyle, un groupe benzoxazolyle, un groupe benzimidazolyle, un groupe furanyle, un groupe benzofuranyle, un groupe thiophényle, un groupe benzothiophényle, un groupe thiazolyle, un groupe isothiazolyle, un groupe benzothiazolyle, un groupe isoxazolyle, un groupe oxazolyle, un groupe triazolyle, un groupe tétrazolyle, un groupe oxadiazolyle, un groupe triazinyle, un groupe dibenzofuranyle, un groupe dibenzothiophényle, un groupe imidazopyridimidinyle, et un groupe imidazopyridinyle, chacun substitué par au moins un sélectionné parmi un deutérium, -F, -Cl, -Br, -I, un groupe hydroxyle, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en C₁ à C₂₀, un groupe alcényle en C₂ à C₂₀, un groupe alcycnyle en C₂ à C₂₀, un groupe alcoxy en C₁ à C₂₀, un groupe phényle, un groupe naphtyle, un groupe anthracényle, un groupe pyrényle, un groupe phénanthrényle, un groupe fluorényle, un groupe pyridinyle, un groupe pyrimidinyle, un groupe pyrazinyle, un groupe pyridazinyle, un groupe triazinyle, un groupe quinolinyle, un groupe isoquinolinyle, un groupe phtalazinyle, un groupe quinoxalinyle, un groupe cinnolinyle, un groupe quinazolinyle, et -Si(Q₃₁)(Q₃₂)(Q₃₃) ;
et -Si(Q₁)(Q₂)(Q₃),
dans lequel Q₁ à Q₃ et Q₃₁ à Q₃₃ sont chacun indépendamment sélectionnés parmi un hydrogène, un groupe alkyle en C₁ à C₂₀, un groupe alcoxy en C₁ à C₂₀, un groupe phényle, un groupe naphtyle, un groupe anthracényle, un groupe pyrényle, un groupe phénanthrényle, un groupe fluorényle, un groupe pyridinyle, un groupe pyrimidinyle, un groupe pyrazinyle, un groupe pyridazinyle, un groupe triazinyle, un groupe quinolinyle, un groupe isoquinolinyle un groupe phtalazinyle, un groupe quinoxalinyle, un groupe cinnolinyle, et un groupe quinazolinyle, préférablement dans lequel R₁ à R₈ et R₁₁ à R₁₈ sont chacun indépendamment sélectionnés parmi un hydrogène, un deutérium, un groupe cyano, un groupe méthyle, un groupe éthyle, un groupe n-propyle, un groupe isopropyle, un groupe n-butyle, un groupe isobutyle, un groupe sec-butyle, un groupe tert-butyle, un groupe n-pentyle, un groupe isopentyle, un groupe sec-pentyle, un groupe tert-pentyle, un groupe n-hexyle, un groupe iso-hexyle, un groupe sec-hexyle, un groupe tert-hexyle, et -Si(Q₁)(Q₂)(Q₃),
dans lequel Q₁ à Q₃ sont chacun indépendamment sélectionnés parmi un hydrogène, un groupe alkyle en C₁ à C₁₀, un groupe alcoxy en C₁ à C₁₀, et un groupe phényle.

3. Composé cyclique condensé selon la revendication 1, dans lequel R₁ à R₆ et R₁₁ à R₁₆ sont chacun indépendamment sélectionnés parmi un hydrogène, un deutérium, -F, -Cl, -Br, -I, un groupe hydroxyle, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en C₁ à C₁₀, et un groupe alcoxy en C₁ à C₁₀ ;
un groupe phényle, un groupe naphtyle, un groupe pyridinyle, un groupe pyrimidinyle, un groupe pyrazinyle, un groupe pyridazinyle, et un groupe triazinyle ;
un groupe phényle, un groupe naphtyle, un groupe pyridinyle, un groupe pyrimidinyle, un groupe pyrazinyle, un groupe pyridazinyle, et un groupe triazinyle, chacun substitué par au moins un sélectionné parmi un deutérium, -F, -Cl, -Br, -I, un groupe hydroxyle, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en C₁ à C₁₀, un groupe alcoxy en C₁ à C₁₀, un groupe phényle, un groupe naphtyle, un groupe pyridinyle, un groupe pyrimidinyle, un groupe pyrazinyle, un groupe pyridazinyle, un groupe triazinyle, et - Si(Q₃₁)(Q₃₂)(Q₃₃) ;
et -Si(Q₁)(Q₂)(Q₃),
R₇, R₈₉ R₁₇, et R₁₈ sont chacun indépendamment sélectionnés parmi un hydrogène, un deutérium, -F, -Cl, -Br, -I, un groupe hydroxyle, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en C₁ à C₁₀, et un groupe alcoxy en C₁ à C₁₀ ;
un groupe phényle, un groupe naphtyle, un groupe pyridinyle, un groupe pyrimidinyle, un groupe pyrazinyle, un groupe pyridazinyle, et un groupe triazinyle ;
un groupe phényle, un groupe naphtyle, un groupe pyridinyle, un groupe pyrimidinyle, un groupe pyrazinyle, un groupe pyridazinyle, et un groupe triazinyle, chacun substitué par au moins un sélectionné parmi un deutérium, -F, -Cl, -Br, -I, un groupe hydroxyle, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en C₁ à C₁₀, un groupe alcoxy en C₁ à C₁₀, un groupe phényle, un groupe naphtyle, un groupe pyridinyle, un groupe pyrimidinyle, un groupe pyrazinyle, un groupe pyridazinyle, un groupe triazinyle, et -Si(Q₃₁)(Q₃₂)(Q₃₃) ;
et -Si(Q₁)(Q₂)(Q₃),
dans lequel Q₁ à Q₃ et Q₃₁ à Q₃₃ sont chacun indépendamment sélectionnés parmi un hydrogène, un groupe alkyle en C₁ à C₁₀, un groupe alcoxy en C₁ à C₁₀, un groupe phényle, un groupe naphtyle, un groupe pyridinyle, un groupe pyrimidinyle, un groupe pyrazinyle, un groupe pyridazinyle, et un groupe triazinyle, et/ou dans lequel chacun de R₁ à R₆ et R₁₁ à R₁₆ n'est pas un groupe cyano.

4. Composé cyclique condensé selon la revendication 1, dans lequel Ar₁ est représenté par l'une des Formules 2A-1 à 2A-10, 2B-1 à 2B-8, et 2C-1 à 2C-9 : 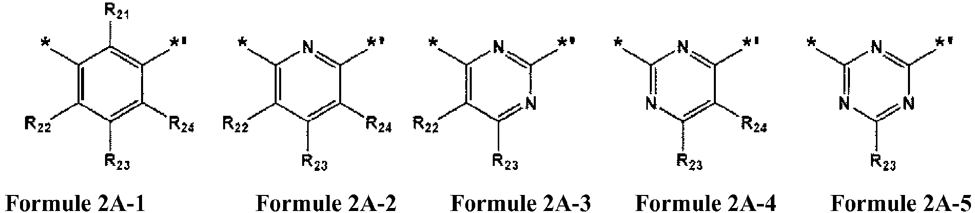 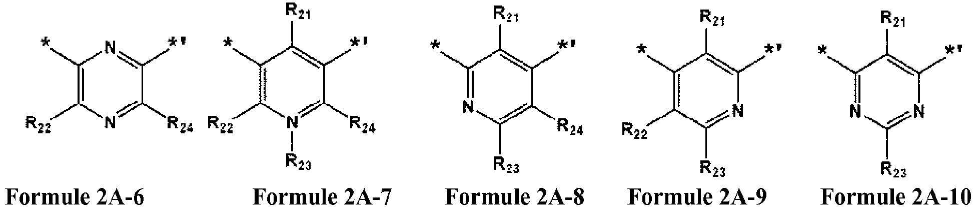 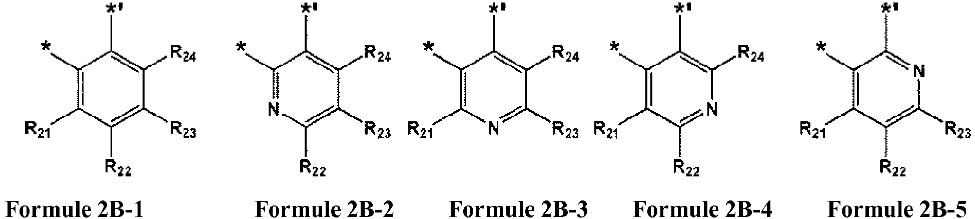 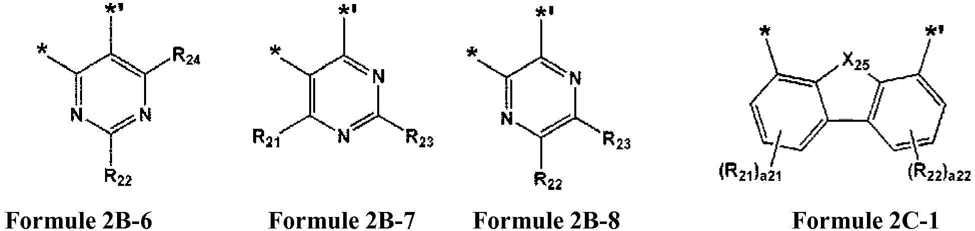 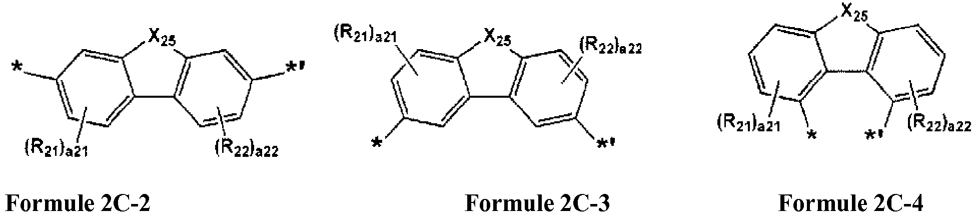 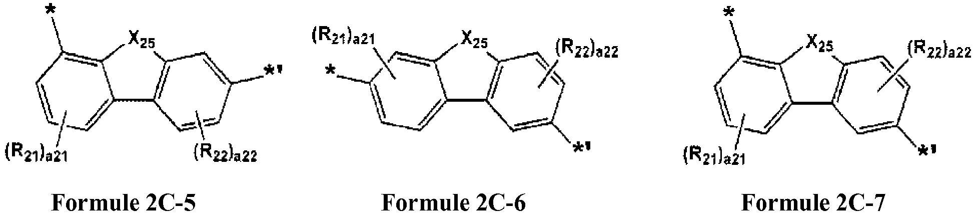 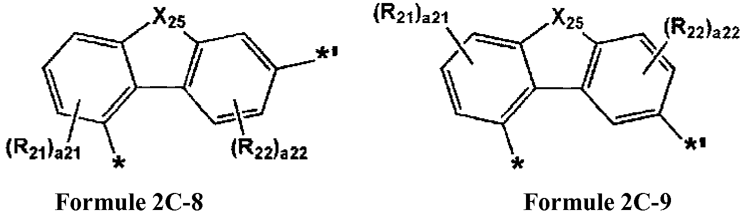 dans lequel dans les Formules 2A-1 à 2A-10, 2B-1 à 2B-8, et 2C-1 à 2C-9,
X₂₅ est O, S, P(=O)(R₂₅), Se ou Si(R₂₅)(R₂₆),
R₂₁ à R₂₄ sont chacun indépendamment sélectionnés parmi un hydrogène, un deutérium, un groupe méthyle, un groupe éthyle, un groupe n-propyle, un groupe isopropyle, un groupe n-butyle, un groupe isobutyle, un groupe sec-butyle, un groupe tert-butyle, un groupe phényle, un groupe pyridinyle, un groupe pyrimidinyle, un groupe pyrazinyle, un groupe pyridazinyle, un groupe triazinyle, et -Si(Q₁₁)(Q₁₂)(Q₁₃),
R₂₅ et R₂₆ sont chacun indépendamment sélectionnés parmi un hydrogène, un deutérium, un groupe méthyle, un groupe éthyle, un groupe n-propyle, un groupe isopropyle, un groupe n-butyle, un groupe isobutyle, un groupe sec-butyle, un groupe tert-butyle, un groupe phényle, un groupe pyridinyle, un groupe pyrimidinyle, un groupe pyrazinyle, un groupe pyridazinyle et un groupe triazinyle,
dans lequel Q₁₁ à Q₁₃ sont chacun indépendamment sélectionnés parmi un hydrogène, un groupe alkyle en C₁ à C₁₀, un groupe alcoxy en C₁ à C₁₀, et un groupe phényle,
a21 et a22 sont chacun indépendamment 0 ou 1,
et chacun de * et *' indique un site de liaison à un atome voisin.

5. Composé cyclique condensé selon l'une quelconque des revendications 1 à 3, dans lequel
L₁ et L₂ sont chacun indépendamment sélectionnés parmi
un groupe phénylène, un groupe pyridinylène, un groupe pyrimidinylène, et un groupe triazinylène ;
et un groupe phénylène, un groupe pyridinylène, un groupe pyrimidinylène, et un groupe triazinylène, chacun substitué par au moins un sélectionné parmi un deutérium, un groupe méthyle, un groupe éthyle, un groupe n-propyle, un groupe isopropyle, un groupe n-butyle, un groupe isobutyle, un groupe sec-butyle, un groupe tert-butyle, un groupe phényle, un groupe pyridinyle, un groupe pyrimidinyle, un groupe pyrazinyle, un groupe pyridazinyle, un groupe triazinyle, et -Si(Q₂₁)(Q₂₂)(Q₂₃), préférablement dans lequel
L₁ et L₂ sont chacun indépendamment sélectionnés parmi
un groupe phénylène ;
et un groupe phénylène, substitué avec au moins sélectionné parmi un deutérium, un groupe méthyle, un groupe éthyle, un groupe n-propyle, un groupe isopropyle, un groupe n-butyle, un groupe isobutyle, un groupe sec-butyle, un groupe tert-butyle, un groupe phényle, un groupe pyridinyle, un groupe pyrimidinyle, un groupe pyrazinyle, un groupe pyridazinyle, un groupe triazinyle, et - Si(Q₂₁)(Q₂₂)(Q₂₃), dans lequel Q₂₁ à Q₂₃ sont chacun indépendamment sélectionnés parmi un hydrogène, un groupe alkyle en C₁ à C₁₀, un groupe alcoxy en C₁ à C₁₀, et un groupe phényle,
et a1 et a2 sont chacun indépendamment 0 ou 1.

6. Composé cyclique condensé selon la revendication 4, dans lequel a1 et a2 dans les Formules 1B à 1D sont 0 et Ar₁ est représenté par l'une des Formules 2A-1, 2A-2, 2A-10, 2B-1, 2B-8 et 2C-3.

7. Composé cyclique condensé selon la revendication 1, dans lequel concernant les Formules 1B à 1D, la somme de a1 et de a2 est 1 ou plus, et *-(L₁)a₁-Ar₁-(L₂)a₂⁻*' est représenté par l'une des Formules 3-1 à 3-57 : 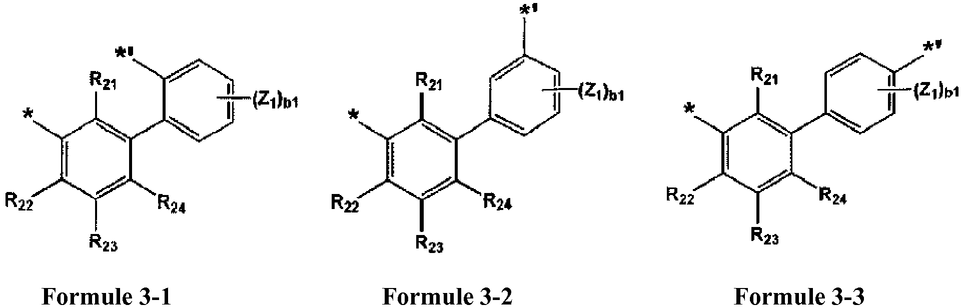 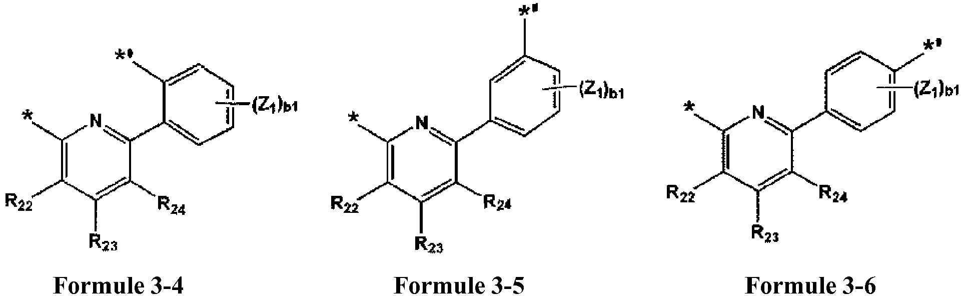 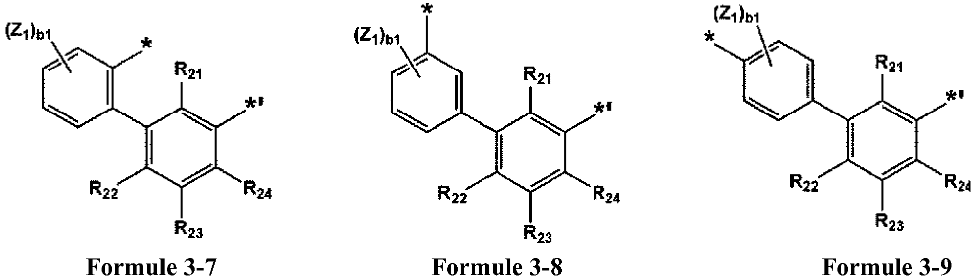 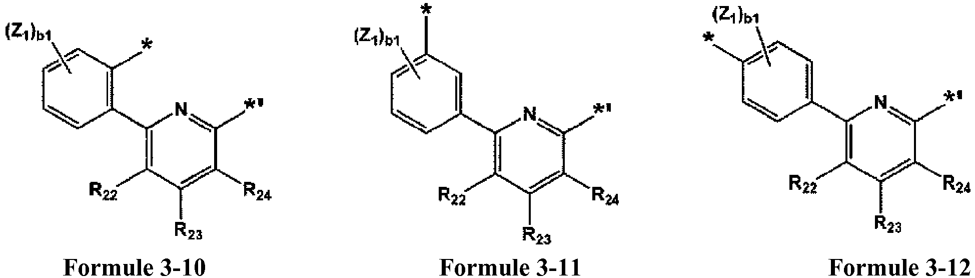 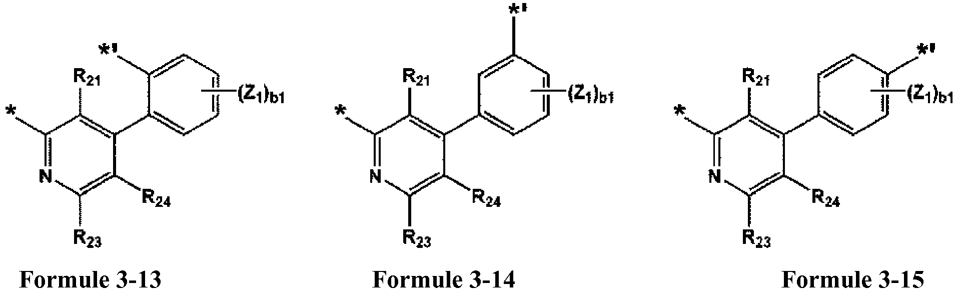 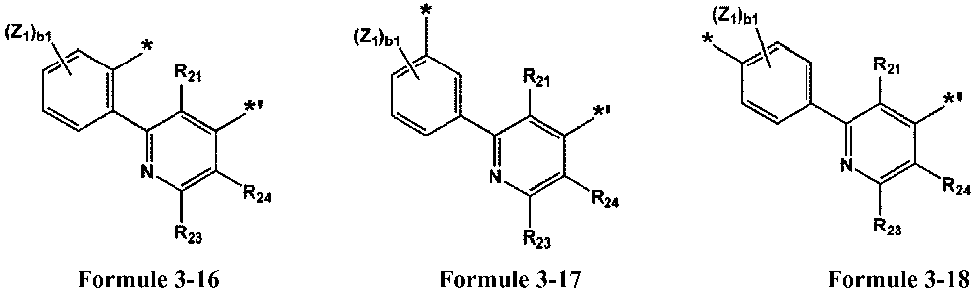 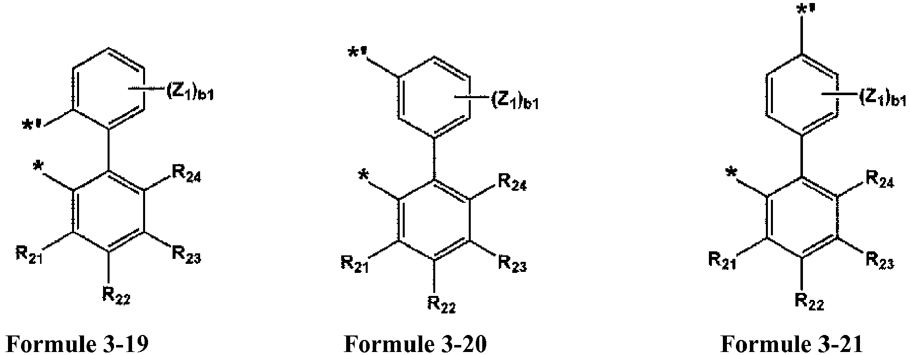 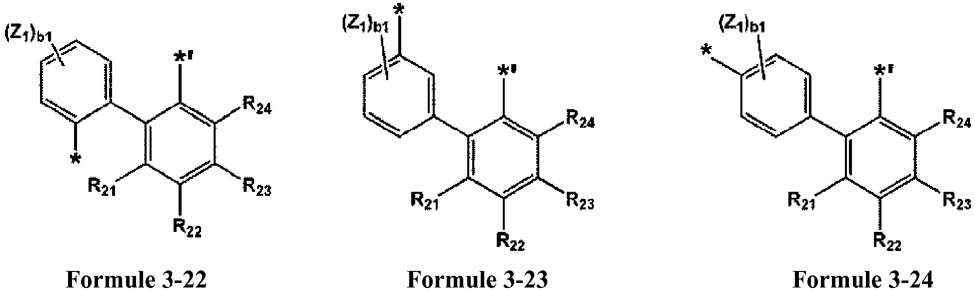 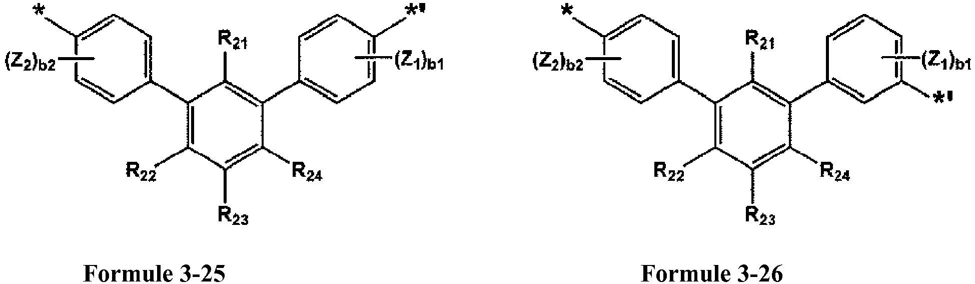 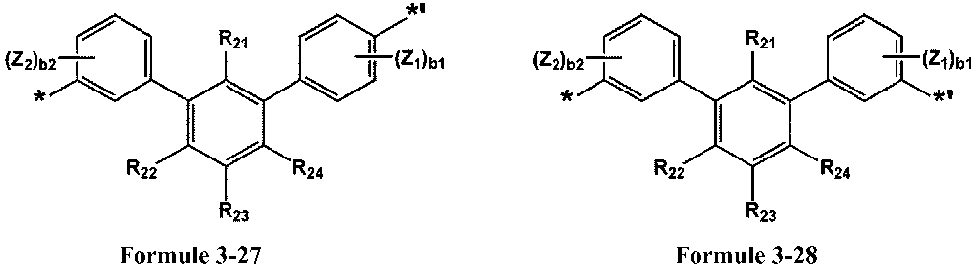 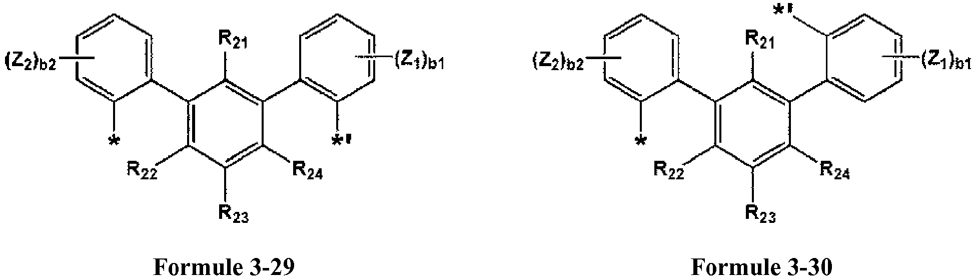 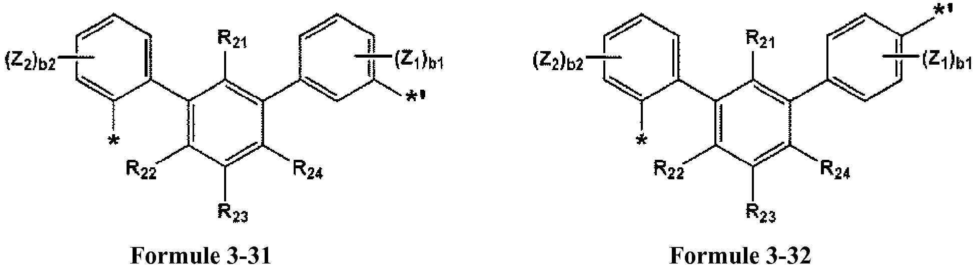 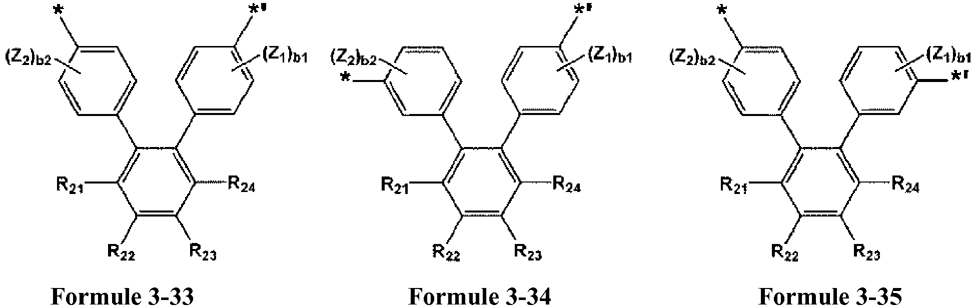 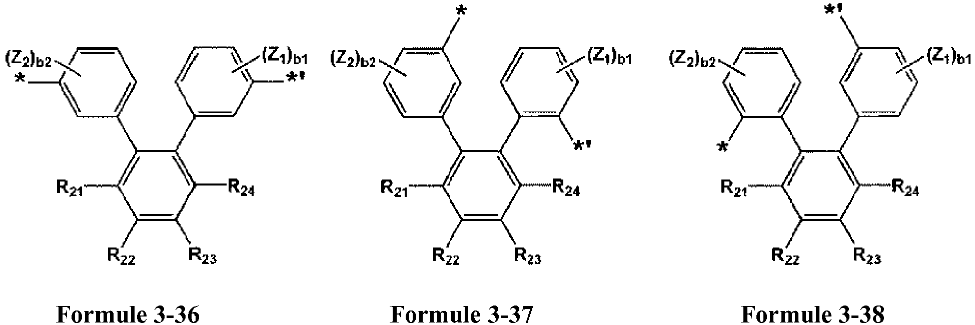 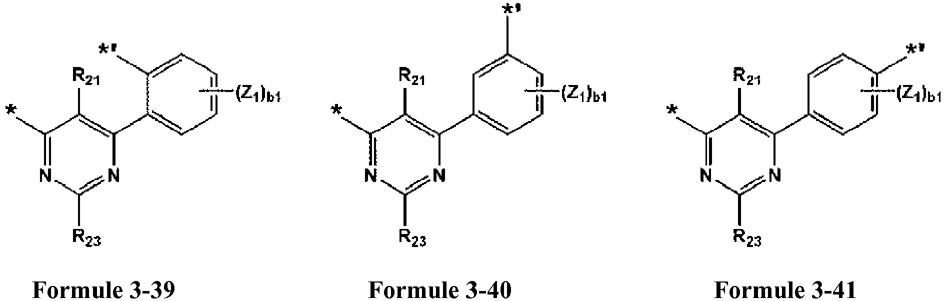 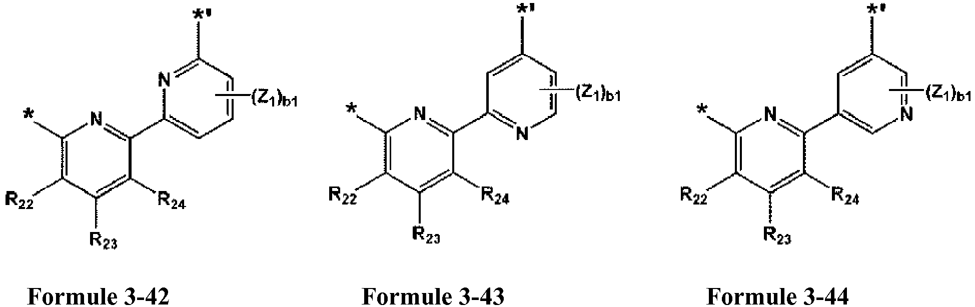 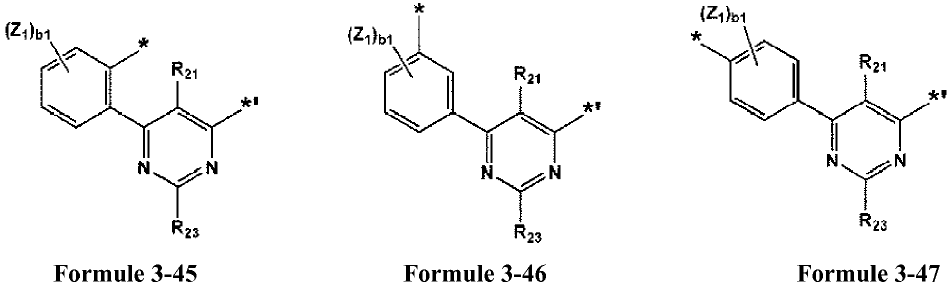 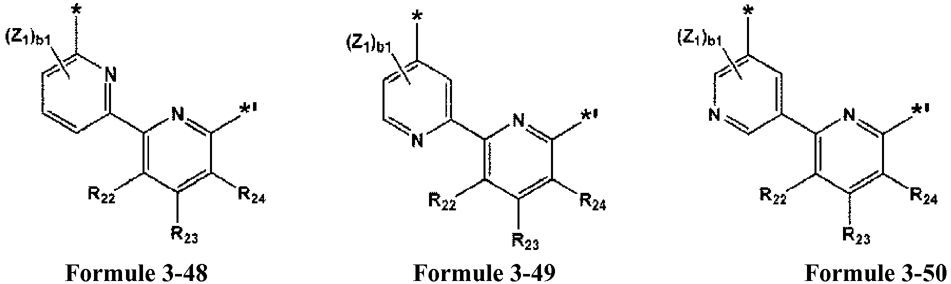 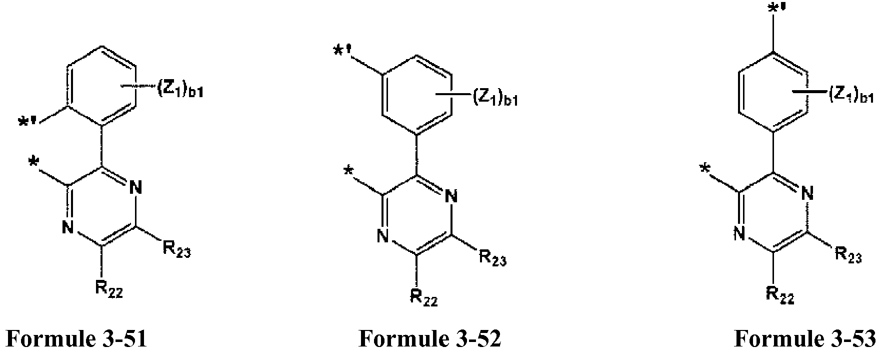 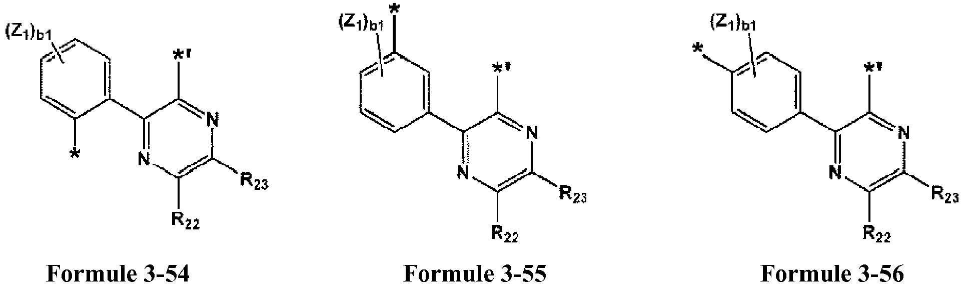 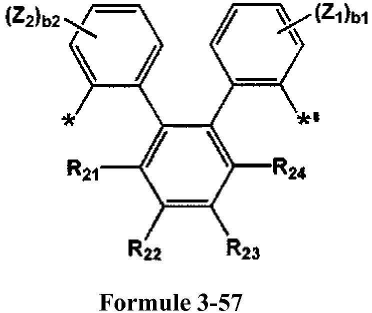 dans lequel dans les Formules 3-1 à 3-57,
R₂₁ à R₂₄, Z₁, et Z₂ sont chacun indépendamment sélectionnés parmi un hydrogène, un deutérium, un groupe méthyle, un groupe éthyle, un groupe n-propyle, un groupe isopropyle, un groupe n-butyle, un groupe isobutyle, un groupe sec-butyle, un groupe tert-butyle, un groupe phényle, un groupe pyridinyle, un groupe pyrimidinyle, un groupe pyrazinyle, un groupe pyridazinyle, un groupe triazinyle, et -Si(Q₁₁)(Q₁₂)(Q₁₃),
dans lequel Q₁₁ à Q₁₃ sont chacun indépendamment sélectionnés parmi un hydrogène, un groupe alkyle en C₁ à C₁₀, un groupe alcoxy en C₁ à C₁₀, et un groupe phényle,
b1 et b2 sont chacun indépendamment 0,1, ou 2,
et chacun de * et *' indique un site de liaison à un atome d'azote voisin.

8. Composé cyclique condensé selon la revendication 1, dans lequel le composé cyclique condensé est représenté par l'une des Formules 1B(1) à 1D(1), 1 B(2) à 1 D(2) et 1 B(3) à 1 D(3) : 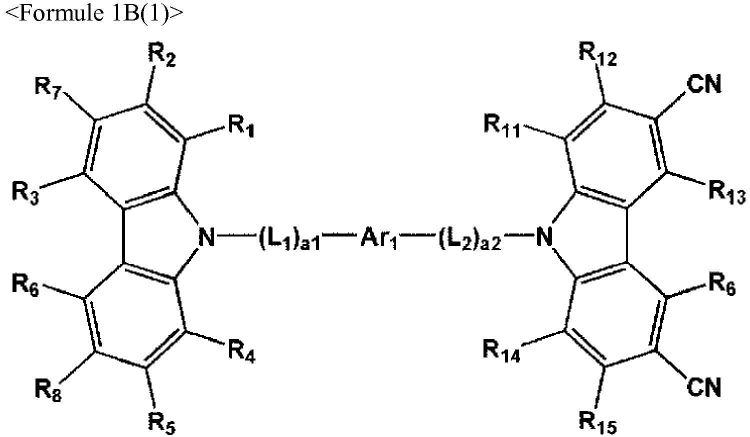 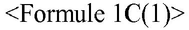 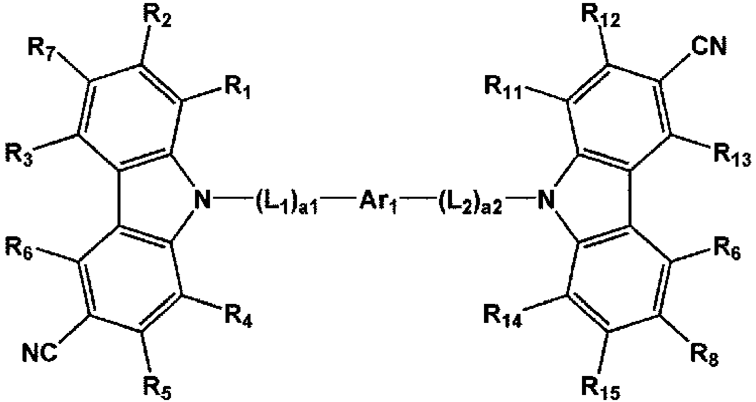 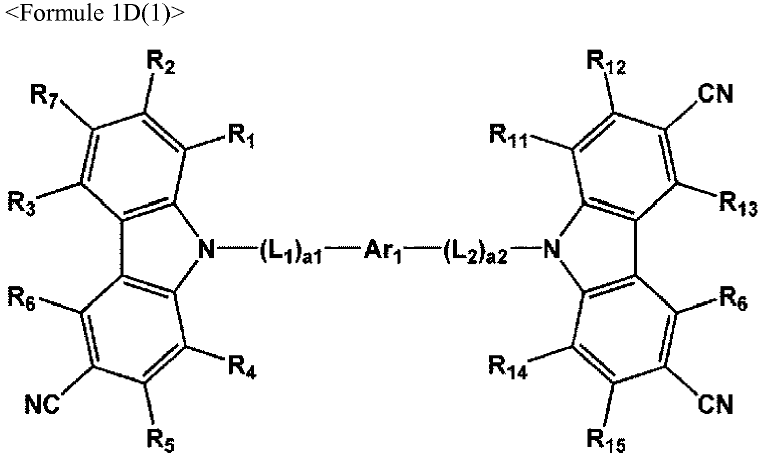 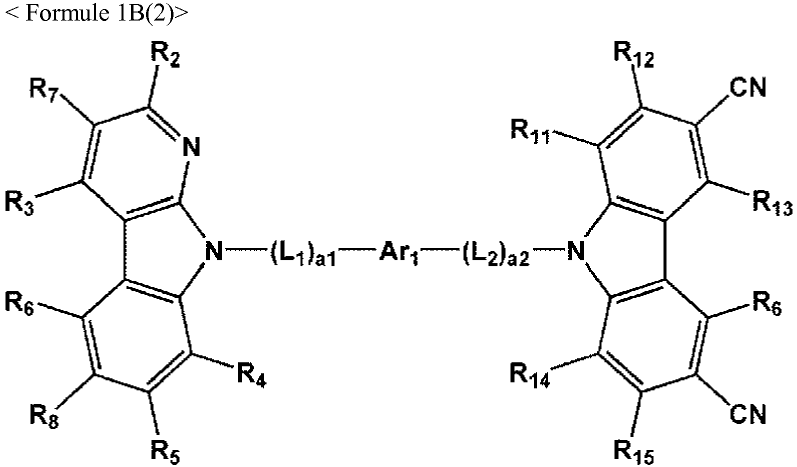 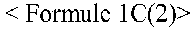 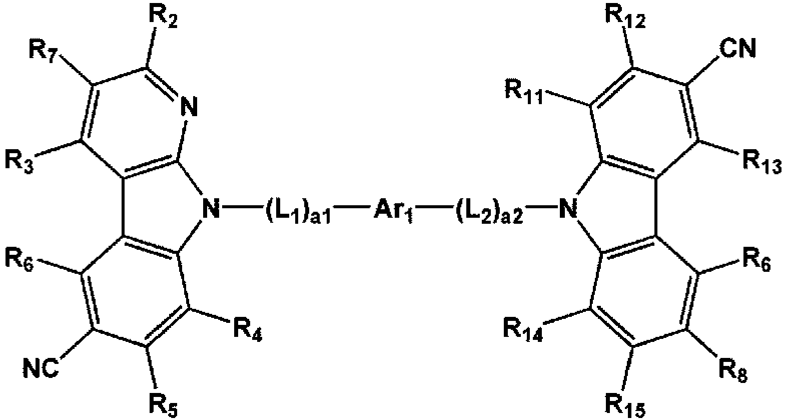 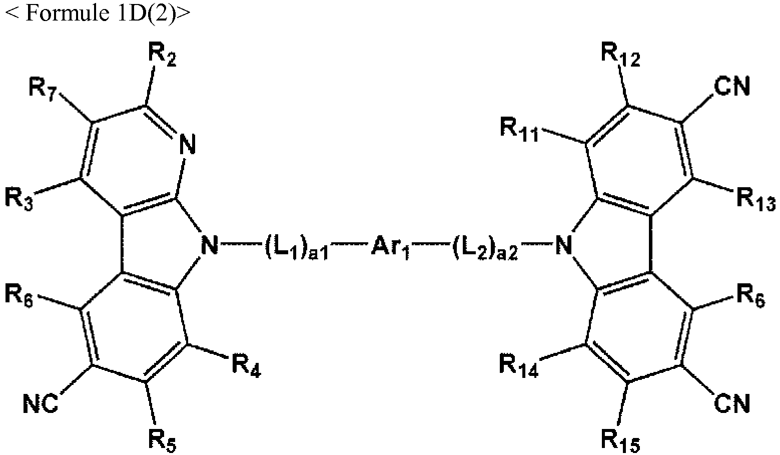 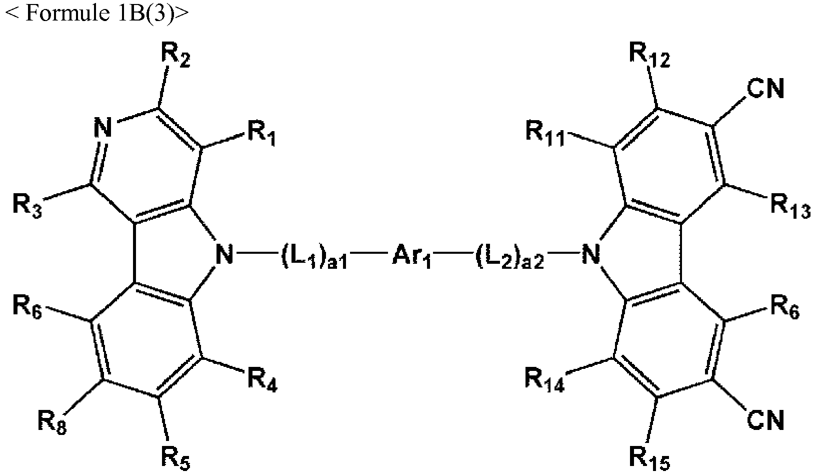 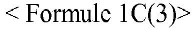 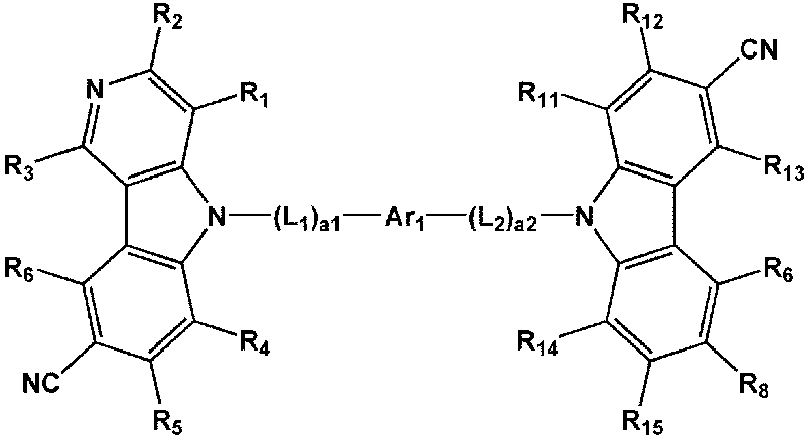 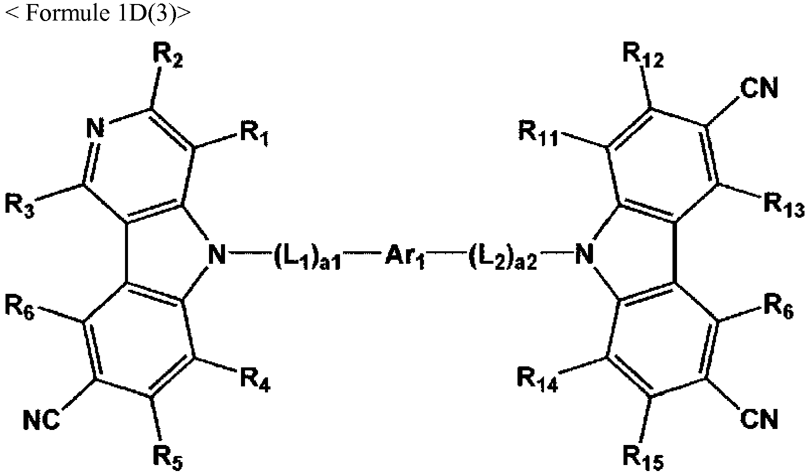 dans lequel dans les Formules 1 B(1) à 1 D(1), 1 B(2) à 1 D(2) et 1 B(3) à 1 D(3), R₁ à R₈, R₁₁ à R₁₆, et R₁₈ sont chacun indépendamment sélectionnés parmi un hydrogène, un deutérium, -F, -Cl, -Br, -I, un groupe hydroxyle, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe méthyle, un groupe éthyle, un groupe n-propyle, un groupe isopropyle, un groupe n-butyle, un groupe isobutyle, un groupe sec-butyle, un groupe tert-butyle, un groupe n-pentyle, un groupe isopentyle, un groupe sec-pentyle, un groupe tert-pentyle, un groupe n-hexyle, un groupe iso-hexyle, un groupe sec-hexyle, un groupe tert-hexyle, un groupe n-heptyle, un groupe iso-heptyle, un groupe sec-heptyle, un groupe tert-heptyle, un groupe n-octyle, un groupe iso-octyle, un groupe sec-octyle, un groupe tert-octyle, un groupe n-nonyle, un groupe iso-nonyle, un groupe sec-nonyle, un groupe tert-nonyle, un groupe n-décyle, un groupe isodécyle, un groupe sec-décyle, un groupe tert-décyle, un groupe méthoxy, un groupe éthoxy, un groupe propoxy, un groupe butoxy, un groupe pentoxy, un groupe phényle, un groupe naphtyle, un groupe pyridinyle, un groupe pyrimidinyle, un groupe triazinyle, et -Si(Q₁)(Q₂)(Q₃),
Ar₁ est représenté par les Formules 2A-1, 2A-2, 2A-10, 2B-1, 2B-8 ou 2C-3 :
L₁ et L₂ sont chacun indépendamment sélectionnés parmi :
un groupe phénylène, un groupe pyridinylène, un groupe pyrimidinylène, un groupe pyrazinylène, un groupe pyridazinylène, un groupe triazinylène, un groupe dibenzofuranylène, et un groupe dibenzothiophénylène ;
et un groupe phénylène, un groupe pyridinylène, un groupe pyrimidinylène, un groupe pyrazinylène, un groupe pyridazinylène, un groupe triazinylène, un groupe dibenzofuranylène, et un groupe dibenzothiophénylène, chacun substitué par au moins un sélectionné parmi un deutérium, -F, -Cl, -Br, -I, un groupe hydroxyle, un groupe cyano, un groupe nitro, un groupe amino, un groupe amidino, un groupe hydrazine, un groupe hydrazone, un groupe acide carboxylique ou un sel de celui-ci, un groupe acide sulfonique ou un sel de celui-ci, un groupe acide phosphorique ou un sel de celui-ci, un groupe alkyle en C₁ à C₁₀, un groupe alcoxy en C₁ à C₁₀, un groupe phényle, un groupe naphtyle, un groupe pyridinyle, un groupe pyrimidinyle, un groupe pyrazinyle, un groupe pyridazinyle, un groupe triazinyle et - Si(Q₂₁)(Q₂₂)(Q₂₃),
a1 et a2 sont chacun indépendamment 0 ou 1,
dans lequel Q₁ à Q₃, et Q₂₁ à Q₂₃ sont chacun indépendamment sélectionnés parmi un hydrogène, un groupe alkyle en C₁ à C₁₀, un groupe alcoxy en C₁ à C₁₀, et un groupe phényle : 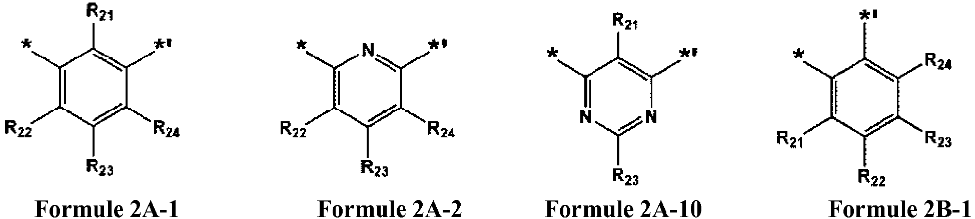 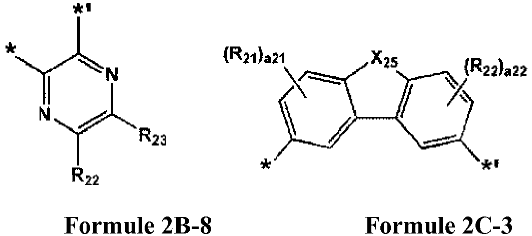 dans lequel dans les Formules 2A-1, 2A-2, 2A-10, 2B-1, 2B-8 et 2C-3,
X₂₅ est O, S, P(=O)(R₂₅), Se ou Si(R₂₅)(R₂₆),
R₂₁ à R₂₄ sont chacun indépendamment sélectionnés parmi un hydrogène, un deutérium, un groupe méthyle, un groupe éthyle, un groupe n-propyle, un groupe isopropyle, un groupe n-butyle, un groupe isobutyle, un groupe sec-butyle, un groupe tert-butyle, un groupe phényle, un groupe pyridinyle, un groupe pyrimidinyle, un groupe pyrazinyle, un groupe pyridazinyle, un groupe triazinyle, et -Si(Q₁₁)(Q₁₂)(Q₁₃),
R₂₅ et R₂₆ sont chacun indépendamment sélectionnés parmi un hydrogène, un deutérium, un groupe méthyle, un groupe éthyle, un groupe n-propyle, un groupe isopropyle, un groupe n-butyle, un groupe isobutyle, un groupe sec-butyle, un groupe tert-butyle, un groupe phényle, un groupe pyridinyle, un groupe pyrimidinyle, un groupe pyrazinyle, un groupe pyridazinyle et un groupe triazinyle,
a21 et a22 sont chacun indépendamment 0 ou 1,
dans lequel Q₁₁ à Q₁₃ sont chacun indépendamment sélectionnés parmi un hydrogène, un groupe alkyle en C₁ à C₁₀, un groupe alcoxy en C₁ à C₁₀, et un groupe phényle.

9. Composé cyclique condensé selon la revendication 1, dans lequel le composé cyclique condensé est un des Composés 1 à 7, 13 à 18, 26 à 27, 31 à 34, 36, 39, 42 à 44, 46, 47, 49, 50, 52, 53, 55 à 56 :

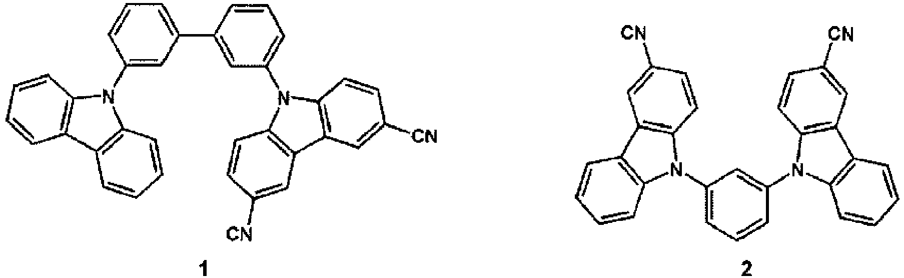

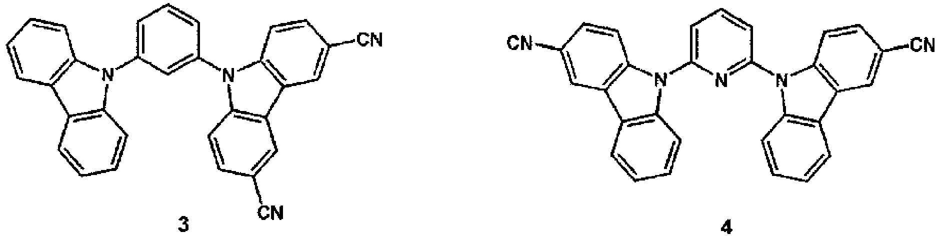

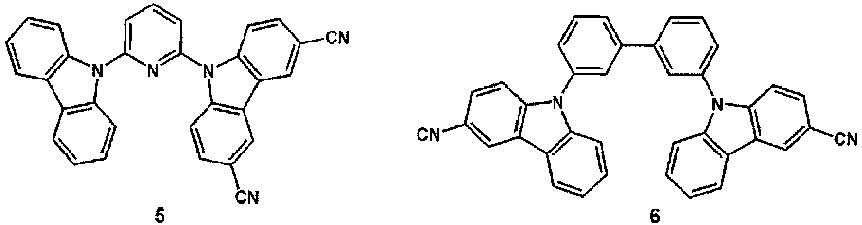

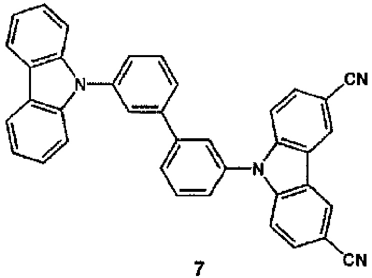

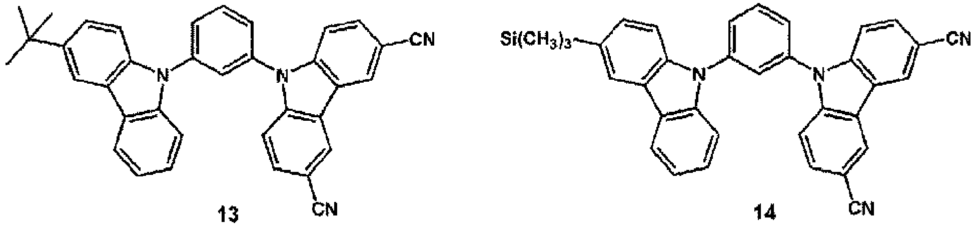

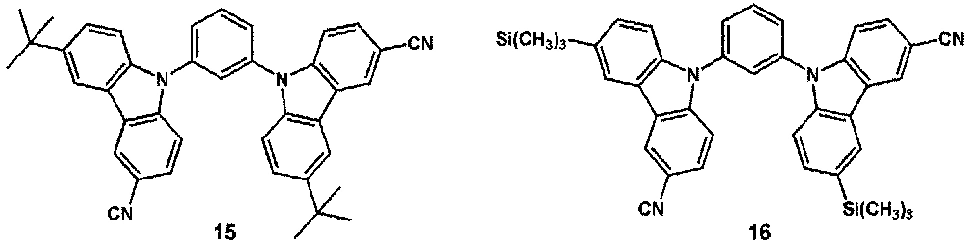

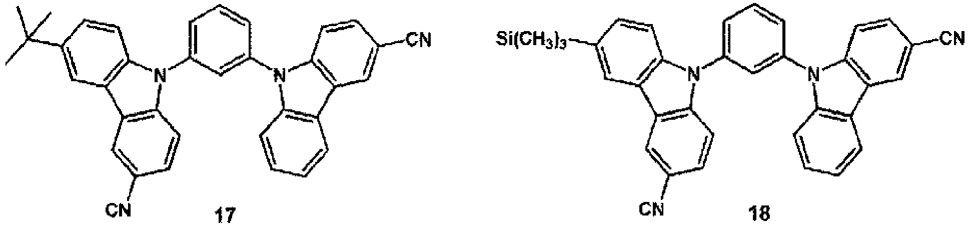

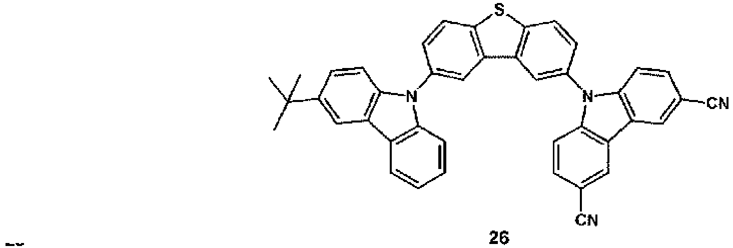

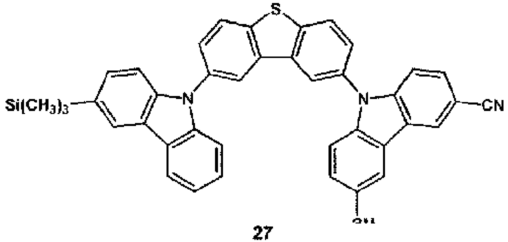

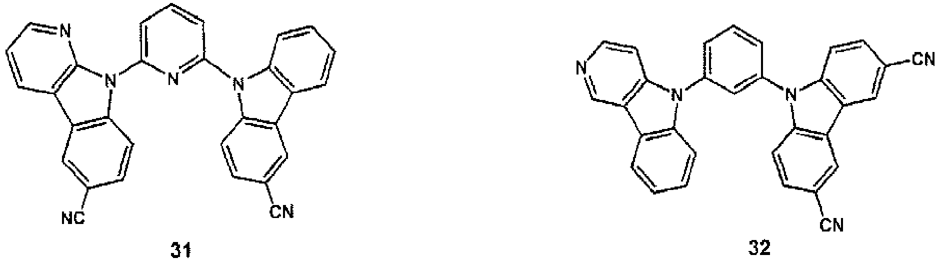

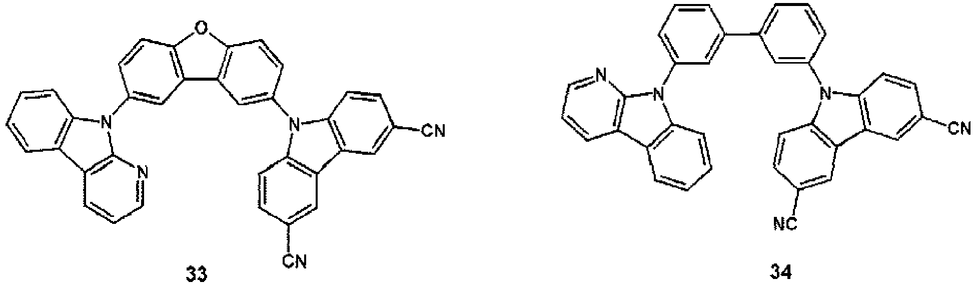

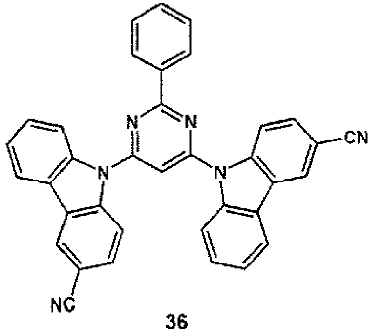

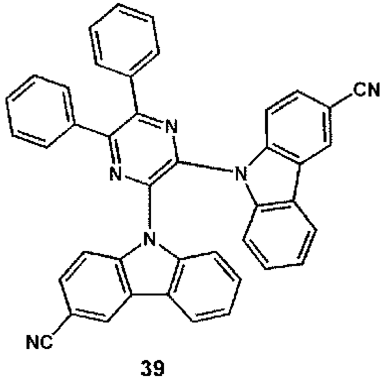

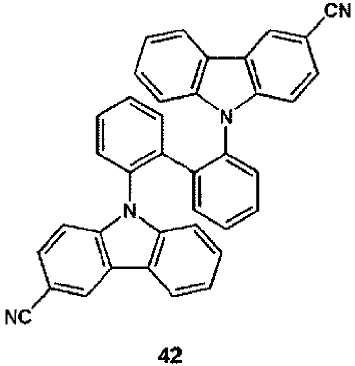

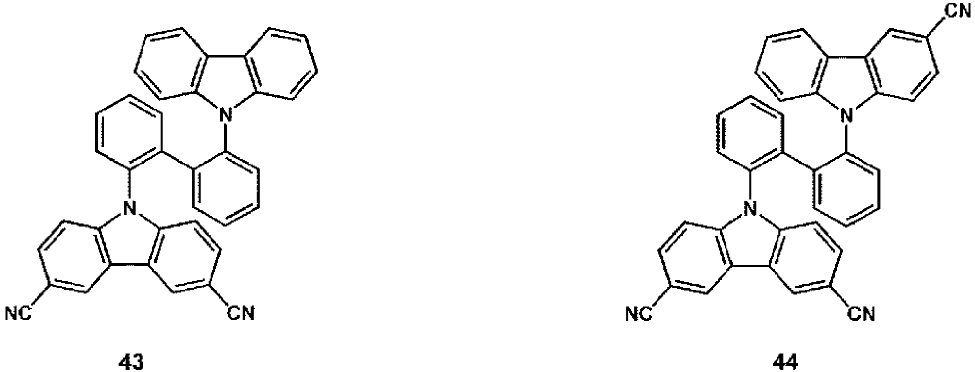

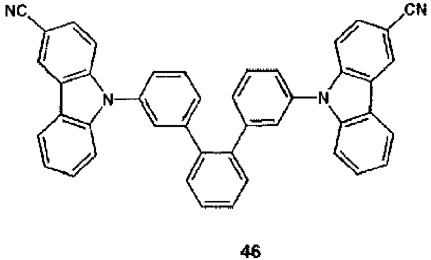

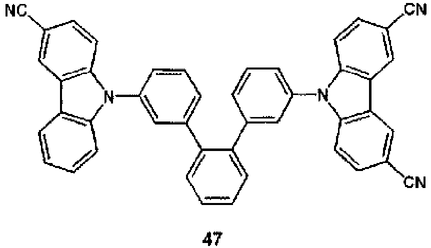

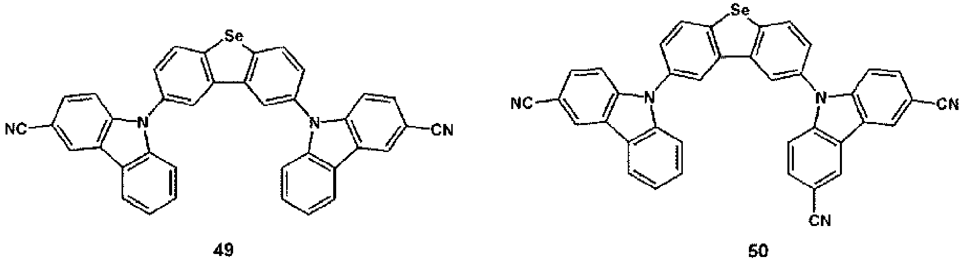

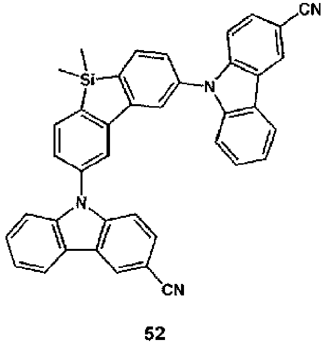

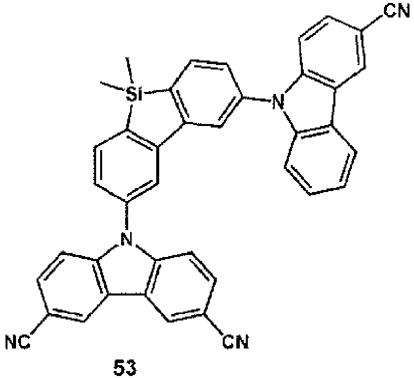

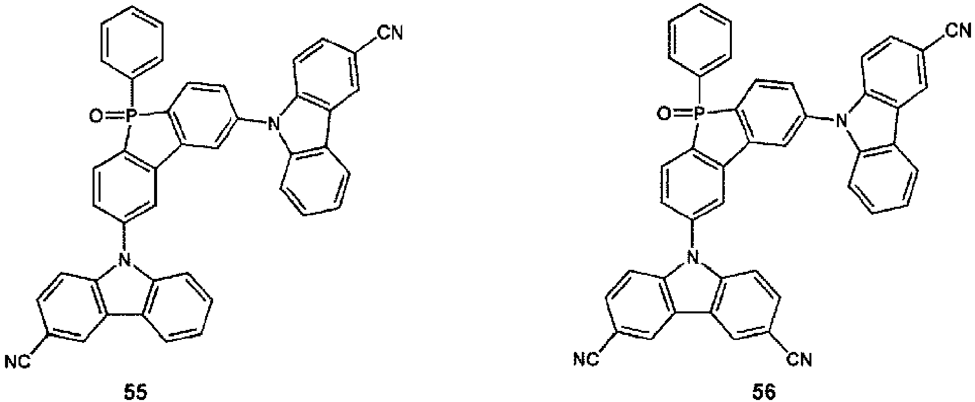

10. Dispositif émetteur de lumière organique comprenant une première électrode ; une deuxième électrode ; et une couche organique entre la première électrode et la deuxième électrode, la couche organique comprenant une couche d'émission, dans lequel la couche organique comprend au moins l'un des composés cycliques condensés représentés par les Formules 1B à 1D selon l'une quelconque des revendications 1 à 9.

11. Dispositif émetteur de lumière organique selon la revendication 10, dans lequel la première électrode est une anode, la deuxième électrode est une cathode, et la couche organique comprend :
i) une région de transport de trous entre la première électrode et la couche d'émission, la région de transport de trous comprenant au moins une couche sélectionnée parmi une couche d'injection de trous, une couche de transport de trous, et une couche de blocage des électrons ; et
ii) une région de transport d'électrons entre la couche d'émission et la deuxième électrode, la région de transport d'électrons comprenant au moins une couche sélectionnée parmi une couche de blocage de trous, une couche de transport d'électrons, et une couche d'injection d'électrons.

12. Dispositif émetteur de lumière organique selon la revendication 10 ou 11, dans lequel l'au moins un composé cyclique condensé représenté par les Formules 1B à 1D est inclus dans la couche d'émission, la couche d'émission émettant facultativement de la lumière bleue et/ou le composé cyclique condensé représenté par les Formules 1B à 1D inclus dans la couche d'émission facultativement est un émetteur de fluorescence retardée activé thermiquement.

13. Dispositif émetteur de lumière organique selon l'une quelconque des revendications 10 à 12, dans lequel l'au moins un composé cyclique condensé représenté par les Formules 1B à 1D est inclus dans la couche d'émission, la couche d'émission comprend en outre un dopant phosphorescent, et une quantité du composé cyclique condensé est supérieure à une quantité du dopant phosphorescent.
